# EUROPEAN PATENT APPLICATION

(11) **EP 4 467 535 A1**
(43) Date of publication of application: **27.11.2024**
(21) Application number: 23175523.2
(22) Date of filing: 25.05.2023
(51) Int. Cl.: C07D 401/04, C07D 403/04, C07D 417/14, A01N 43/56, A01N 43/653, A01N 43/78

(54) **LACTAM PESTICIDAL COMPOUNDS**

(71) Applicant: BASF SE, 67056 Ludwigshafen am Rhein (DE)
(72) Inventor: WAKEHAM, Matthew Charles Linford, 67056 Ludwigshafen am Rhein (DE); DEFIEBER, Christian, 67056 Ludwigshafen am Rhein (DE); MAITY, Pulakesh, 400705 Navi Mumbai (IN); KOERBER, Karsten, 67056 Ludwigshafen am Rhein (DE); CHAUDHURI, Rupsha, 400705 Navi Mumbai (IN); ADISECHAN, Ashokkumar, 400705 Navi Mumbai (IN); WINTER, Christian, 67056 Ludwigshafen am Rhein (DE); HANDORE, Kishor, 400705 Navi Mumbai (IN)
(74) Representative: Maiwald GmbH

(57) **Abstract**

The present invention relates to the compounds of formula (I), and the N-oxides, stereoisomers, tautomers and agriculturally or veterinarily acceptable salts thereof wherein the variables are defined according to the description,

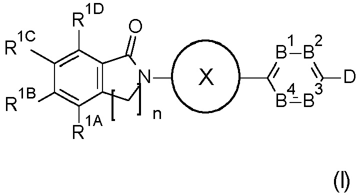

The compounds of formula (I), as well as the N-oxides, stereoisomers, tautomers and agriculturally or veterinarily acceptable salts thereof, are useful for combating or controlling invertebrate pests, in particular arthropod pests and nematodes. The invention also relates to a method for controlling invertebrate pests by using these compounds and to plant propagation material and to an agricultural and a veterinary composition comprising said compounds.

## Description

Invertebrate pests and in particular insects, arachnids and nematodes destroy growing and harvested crops and attack wooden dwelling and commercial structures, thereby causing large economic loss to the food supply and to property. Accordingly, there is an ongoing need for new agents for combating invertebrate pests.

Carbamoylated and thiocarbamoylated oxime derivatives are known for pesticidal use, for example, in patent publications WO 2016/156076, semi-carbazones and thiosemicarbazones derivatives are known for pesticidal use in patent publication WO 2016/116445, WO2021/013561.

Due to the ability of target pests to develop resistance to pesticidally-active agents, there is an ongoing need to identify further compounds, which are suitable for combating invertebrate pests such as insects, arachnids and nematodes. Furthermore, there is a need for new compounds having a high pesticidal activity and showing a broad activity spectrum against a large number of different invertebrate pests, especially against difficult to control insects, arachnids and nematodes.

Nevertheless, there remains a need for highly effective and versatile agents for combating invertebrate pests. It is therefore an object of the invention to provide compounds having a good pesticidal activity and showing a broad activity spectrum against a large number of different invertebrate pests, especially against difficult to control pests, such as insects.

It has been found that these objects can be achieved by compounds of formula I, as depicted and defined below, including their stereoisomers, their salts, in particular their agriculturally or veterinarily acceptable salts, their tautomers and their N-oxides.

In a first aspect, the present invention relates to the Compounds of the formula I 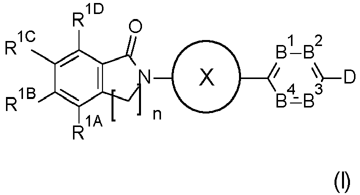 wherein
R^{1A}, R^{1B}, R^{1C} and R^{1D} are, identical or different, H, halogen, SF₅, OH, CN, C₁-C₆-alkyl, C₁-C₆-alkoxy, C₃-C₆-cycloalkyl, C₃-C₆-cycloalkoxy wherein the alkyl, alkoxy, cycloalkyl, and cycloalkoxy moieties are unsubstituted or substituted with halogen or CN;
NR⁸R⁹, C=O(NR⁸R⁹), S(=O)ₘ(NR⁸R⁹), or NHS(=O)ₘR⁸
m is 1 or 2
n is 1, 2 or 3;
X is defined by either one of the following two rings X1 or X2 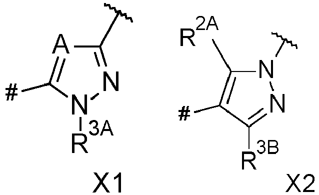
wherein
# denotes connection to the lactam moiety;
A is N or CR^{2A};
R^{2A} is H, C₁-C₆-alkyl, C₃-C₆-cycloalkyl, C₁-C₆-alkoxy, wherein the alkyl, alkoxy and cycloalkyl moieties are unsubstituted or substituted with halogen or CN;
halogen, CN, OR⁶, or NR⁶R⁷
R^{3A} is H, C₁-C₆-alkyl, C₃-C₆-cycloalkyl, C₁-C₆-alkoxy, halogen, or CN wherein the alkyl, alkoxy, and cycloalkyl moieties are unsubstituted or substituted with halogen, CN, or C₁-C₆-alkoxy;
R^{3B} is H, C₁-C₆-alkyl, C₃-C₆-cycloalkyl, C₁-C₆-alkoxy, wherein the alkyl, alkoxy, and cycloalkyl moieties are unsubstituted or substituted with halogen or CN;
halogen, CN, OR⁶, or NR⁶R⁷,
B¹ is N or CR^{B1};
B² is N or CR^{B2};
B³ is N or CR^{B3};
B⁴ is CR^{B4};
R^{B1}, R^{B2}, R^{B3}, and R^{B4} are, identical or different, H, halogen, OH, CN, C₁-C₆-alkyl, C₃-C₆-cycloalkyl, or C₁-C₆-alkoxy, wherein the alkyl, alkoxy, and cycloalkyl moieties are unsubstituted or substituted with halogen;
D is the moiety DA, DB, DC, DD, DE, or DF 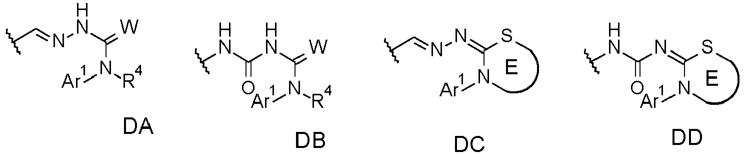 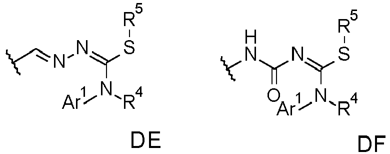
wherein
W is S or O;
R⁴ is H, C₁-C₆-alkyl, or C₃-C₆-cycloalkyl, wherein the alkyl and cycloalkyl moieties are unsubstituted or substituted with halogen or CN;
R⁵ is H, C₁-C₆-alkyl, or C₃-C₆-cycloalkyl, wherein the alkyl and cycloalkyl moieties are unsubstituted or substituted with -O-(C=O)-C₁-C₆-alkoxy, -O-(C=O)-C₁-C₆-alkyl or CN;
E is a 5- or 6-membered carbocyclic group, wherein 1 or 2 CH₂ moieties of the carbocyclic group may be replaced by a carbonyl group, O, or S, wherein the carbocyclic group is unsubstituted or substituted with R¹⁰;
R⁶and R⁷ are, identical or different, H, C₁-C₆-alkyl, C₃-C₆-cycloalkyl, phenyl, -CH₂-phenyl, 5- or 6- membered heteroaryl, -CH₂-5- or 6- membered heteroaryl, 1,3-dioxolan-2-ylmethyl, or 2-(methylamino)-2-oxo-ethyl, wherein the alkyl, cycloalkyl, phenyl and heteroaryl moieties are unsubstituted or substituted with halogen, CN, C₁-C₆-alkyl or C₁-C₆-alkoxy;
Ar¹ is phenyl or 5- or 6-membered heteroaryl, which are unsubstituted or substituted with R^{Ar1}, wherein
R^{Ar1} is halogen, SF₅, OH, CN, C₁-C₆-alkyl, C₁-C₆-alkoxy, C₃-C₆-cycloalkyl, or C₃-C₆-cycloalkoxy wherein the alkyl, alkoxy, cycloalkyl, and cycloalkoxy moieties are unsubstituted or substituted with halogen or CN;
NR⁸R⁹, C=O(NR⁸R⁹), S(=O)ₘ(NR⁸R⁹), NHS(=O)ₘR⁸;
R⁸ and R⁹ are, identical or different, H, C₁-C₆-alkyl, C₃-C₆-cycloalkyl wherein the alkyl, and cycloalkyl moieties are unsubstituted or substituted with halogen;
R¹⁰ is halogen, C₁-C₆-alkyl, or C₁-C₆-alkoxy;
and the N-oxides, stereoisomers, tautomers and agriculturally or veterinarily acceptable salts thereof.

Moreover, the present invention also relates to processes and intermediates for preparing compounds of formula I and to active compound combinations comprising them. Moreover, the present invention relates to agricultural or veterinary compositions comprising the compounds of formula I, and to the use of the compounds of formula I or compositions comprising them for combating or controlling invertebrate pests and/or for protecting crops, plants, plant propagation material and/or growing plants from attack and/or infestation by invertebrate pests. The present invention also relates to methods of applying the compounds of formula I. The present invention also relates to method for protecting crops, plants, plant propagation material and/or growing plants from attack or infestation by invertebrate pests comprising contacting or treating the crops, plants, plant propagation material and growing plants, or soil, material, surface, space, area or water in which the crops, plants, plant propagation material is stored or the plant is growing, with a pesticidally effective amount of at least one compound of formula (I) as defined above or a composition comprising at least one compound of formula (I);

Furthermore, the present invention relates to seed comprising compounds of formula I. Wherein the compounds of formula I includes N-oxides, stereoisomers, tautomers and agriculturally or veterinarily acceptable salts thereof.

With due modification of the starting compounds, the compounds of formula I can be prepared by procedures as given in below schemes.

The compounds of the formula (I) can be prepared by methods of organic chemistry, e.g, by the methods described herein after in schemes 1 to 16 in the synthesis description of the examples. In the schemes 1 to 16 the radicals Hal is halogen and R^{1A}, R^{1B}, R^{1C}, R^{1D}, m, n, X, Y, Z, X‴, A, L, D, DX', DX", R^{2A}, R^{3A}, R^{3B}, B¹, B², B³, B⁴, R⁴, R^{B1}, R^{B2}, R^{B3}, R^{B4}, R⁴, R⁵, E, R⁶, R⁷, W, Ar¹, R^{Ar1}, R⁸, R⁹ and R¹⁰ are as defined above for formula (I), unless otherwise specified. 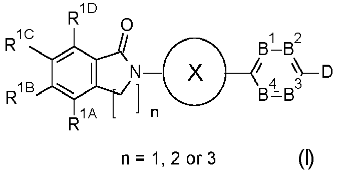 wherein X is 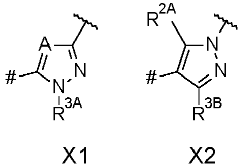 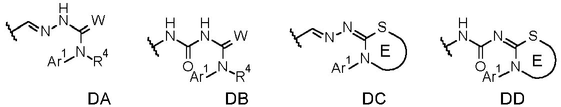 and D is

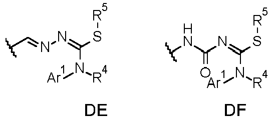

Compounds of formula (I), wherein D is DA, DC or DE are the compounds of formula (I-2) can be prepared analogously to the methods described in WO 2020/083733 or methods described in Scheme 4 and Scheme 6.

Compounds of formula (I), wherein D is DB, DD or DF are the compounds of formula (I-1) can be prepared analogously to the methods described in WO 2021/011722 or methods described in Scheme 1, Scheme 2, Scheme 4 and Scheme 5.

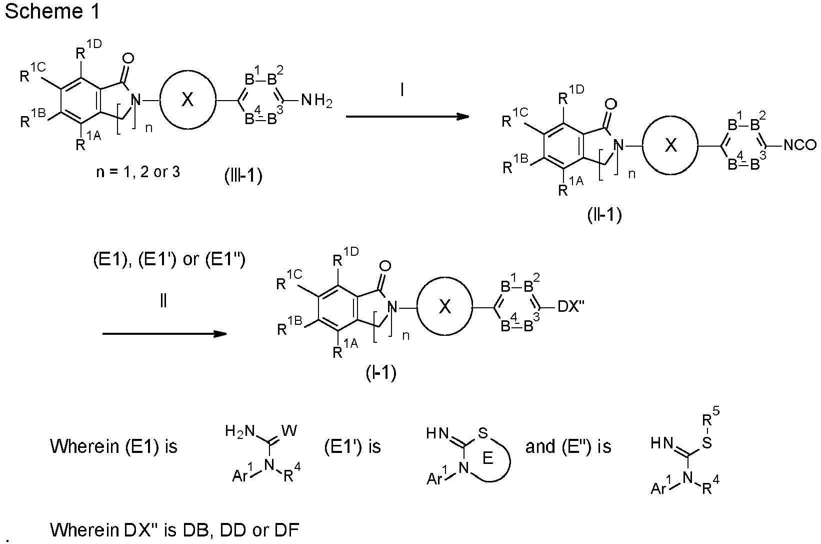

In one embodiment of Scheme 1, compounds of formula (II-1) are reacted directly with a compound of formula (E1), (E1') or (E1") in the presence of an inorganic base to form a compound of formula (I-1). An isocyanate compound of formula (II-1) can be generated *in situ* from either an amine of the formula (III-1) by using one of the common reagents such as phosgene, diphosgene, triphosgene or carbonyldiimidazole, as in step (I), in a mixed solvent system and in the presence of a base as described in March's Advanced Organic Chemistry 6th edition, Michael B. Smith and Jerry March or by the methods described in WO 2021/011722 or WO 2014/204622.

Compounds of formula (III-1) can also be prepared analogously to the methods described in WO 2021/011722, WO 2014/204622 or methods described in Scheme 8.

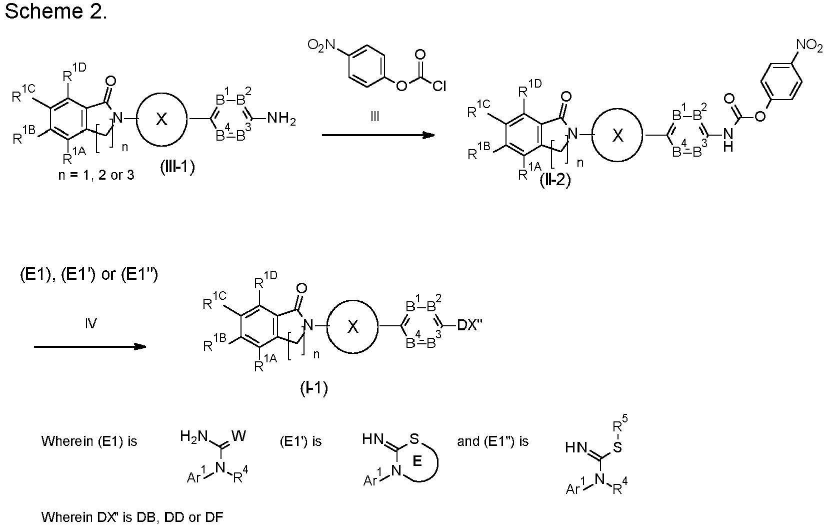

According to the method depicted in scheme 2, amine of formula (III-1) can be treated with an activating agent such as 4-nitrophenyl chloroformate in the presence of a polar aprotic solvent preferably tetrahydrofuran to generate an activated carbamate intermediate (II-2), which in turn is reacted with the compound of formula (E1), (E1') or (E1") in the presence of organic base such as DIPEA to form compounds of formula (I-1).

Compounds of formula (E1) (E1') or (E1") can be prepared analogously to the methods described in WO 2021/011722, J. of Med. Chem. 2010, 53(10), 4198-4211, Synthesis 1988, 1998(6), 460-466 or can be prepared analogously to the methods described in Scheme 3. Compounds of formula (III-1) can also be prepared analogously to the methods described in WO 2021/011722, WO 2014/204622 or methods described in Scheme 8.

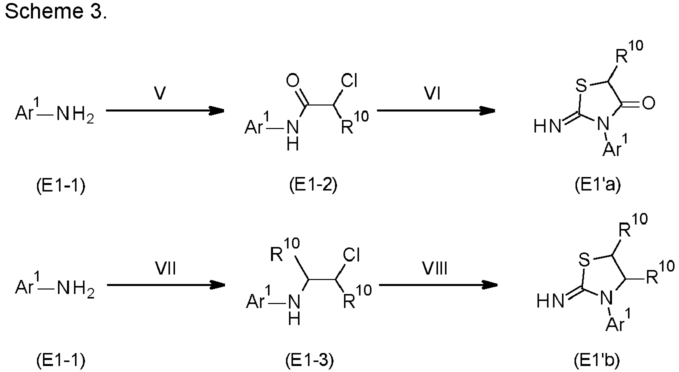

In the above reactions, compounds of formula (E1-1) can be converted into cyclized analogs of formulae (E1'a). Compounds of formula (E1-2) can be prepared by treatment of compounds of formula (E1-1) with unsubstituted or mono- or di- substituted 2-chloroacetylchloride in two steps as depicted in J. Med. Chem. 2010, 53(10), 4198-4211. Compounds of formula (E1-3) can be prepared by treatment of compounds of formula (E1-1) with unsubstituted or mono- or di- substituted 2-chloroactaldehyde in two steps as mentioned in J. Het. Chem. 2006, 43(6), 1523-1531. Compounds of formula (E1'b) can be prepared by treatment of compounds of formula (E1-3) with potassium thiocyanate in presence of inorganic bases such as cesium carbonate in an aprotic solvent such as acetone.

Compounds containing 6- membered rings substituted or unsubstituted with R¹⁰ can also be prepared analogously by the methods described in J. Med. Chem. 2010, 53(10), 4198-4211, and J. Het. Chem. 2006, 43(6), 1523-1531.

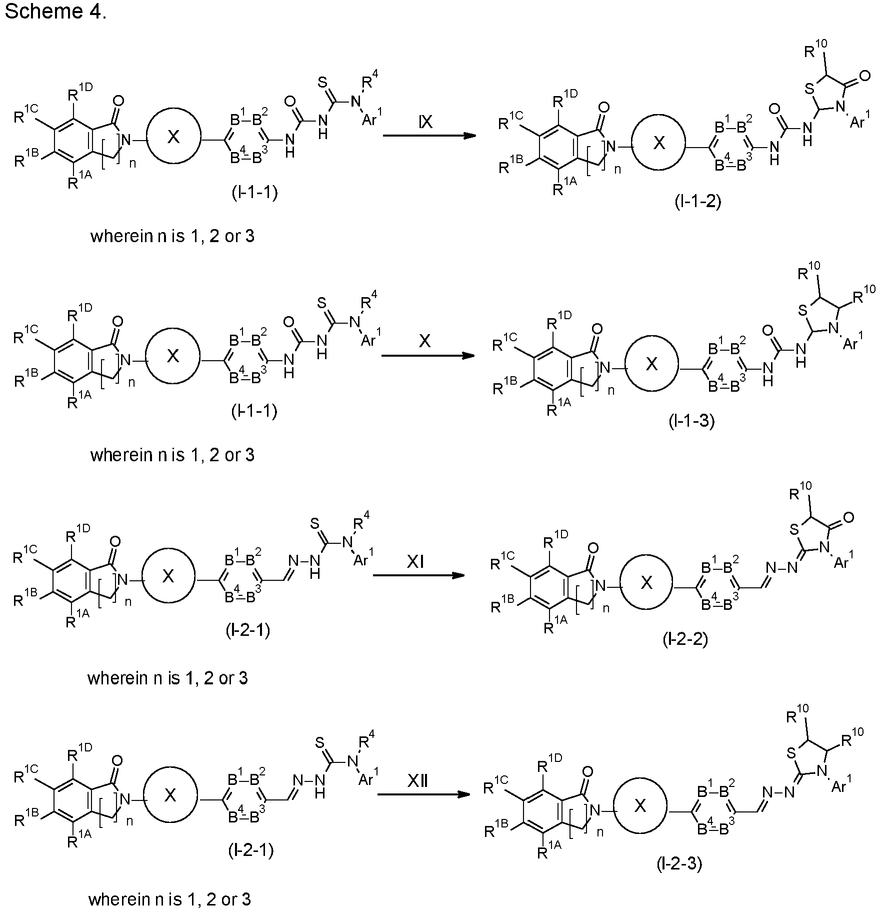

Compounds of formula (I-1-1) and (I-2-1) where R⁴ is H, can be converted into a variety of cyclized analogs of formulae (!-1-2) and (!-1-3) or (I-2-2) and (I-2-3), respectively. Cyclization can be achieved by treatment of compounds of formula (I-1-1) or (I-2-1) with α-halo esters such as methyl bromoacetate to form compounds of formula (I-1-2) or (I-2-2) unsubstituted or substituted with R¹⁰. Compounds of formula (I-1-3) or (I-2-3) unsubstituted or mono- or di-substituted with R¹⁰ can be prepared by treatment of compounds of formula (I-1-1) or (I-2-1) with vicinal dihalides. For steps (IX) and (XI), use of sodium acetate in aprotic solvent such as ACN, at temperatures ranging from about 20 °C to about 70 °C is preferred. For steps (X) and (XII), use of an inorganic base such as potassium carbonate in a solvent such as ACN or 2-butanone, at a temperature between about 0 °C and about 80 °C, is preferred. All the above reactions can be performed analogously to the methods described in WO 2021/011722.

Compounds of formula (I-1-1) can be prepared analogously to the methods described in Scheme 1, Scheme 2 or Scheme 5. Compounds of formula (I-2-1) can be prepared analogously to the methods described in Scheme 6.

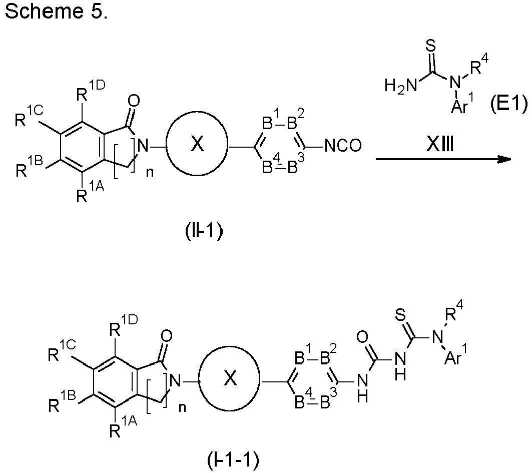

Compounds of the formula (I-1-1) can be prepared by treating aryl thiourea of formula (E1) with the isocyanate of formula (II-1) in the presence of inorganic bases such as cesium carbonate in an aprotic solvent. Compounds of formula (II-1) can be prepared analogously to the methods described in WO 2021/011722, WO 2014/204622 or by the methods described in Scheme 1. Compounds of formula (E1) can be prepared analogously to the methods described in J. Med. Chem. 2010, 53(10), 4198-4211 or in WO 2021/011722.

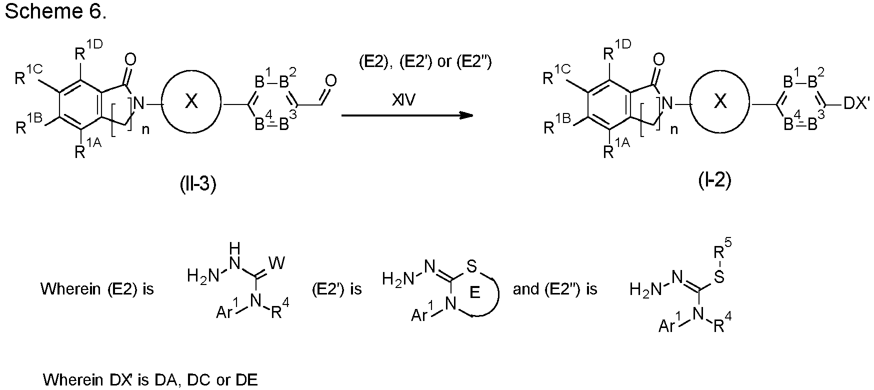

In one embodiment of Scheme 6, an aldehyde of the formula (II-3) is reacted with a compound of formula (E2), (E2') or (E2") in the presence or in the absence of a solvent. Suitable solvents are polar protic solvents. If the reaction is performed in the absence of a solvent, the compound of the formula (E2), (E2') or (E2") can also act as the solvent. Compounds of the formula (E2), (E2') or (E2") are commercially available or can be prepared using organic reactions analogy to method as described in March's Advanced Organic Chemistry 6th edition, Michael B. Smith and Jerry March, in Bioorg. Med. Chem., 2004, 12(17), 4633-4643 or in Bioorg. Med. Chem. Lett., 2005, 15(3), 539-543.

Compounds of formula (II-3) can be prepared analogously to the methods described in Scheme 7.

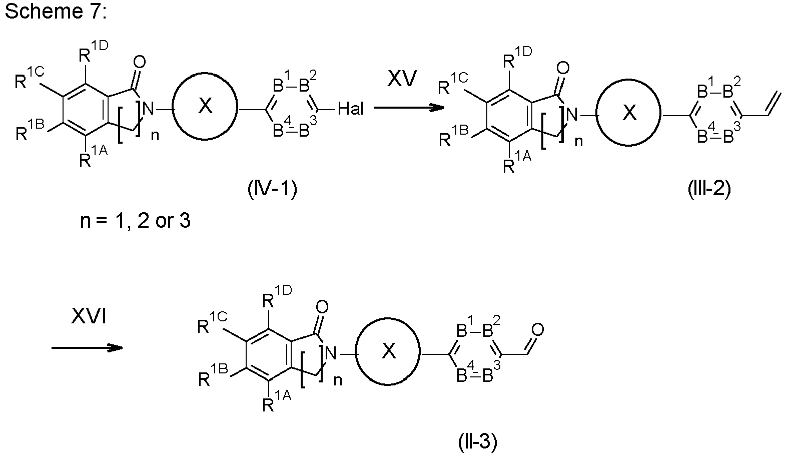

Reaction step (XV) can be performed by analogy to method described in WO 2015/051341. Reaction step (XVI) can be performed by analogy to method described in E. J. Med. Chem., 2012, 49, 310-323.

Compounds of the formula (IV-1) can be prepared by the methods described in Scheme 9.

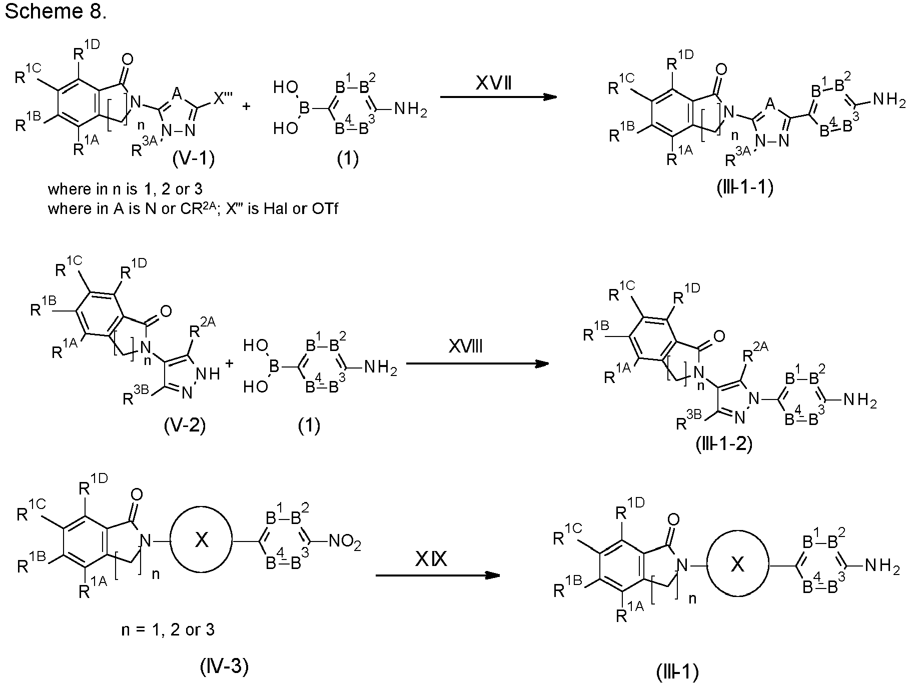

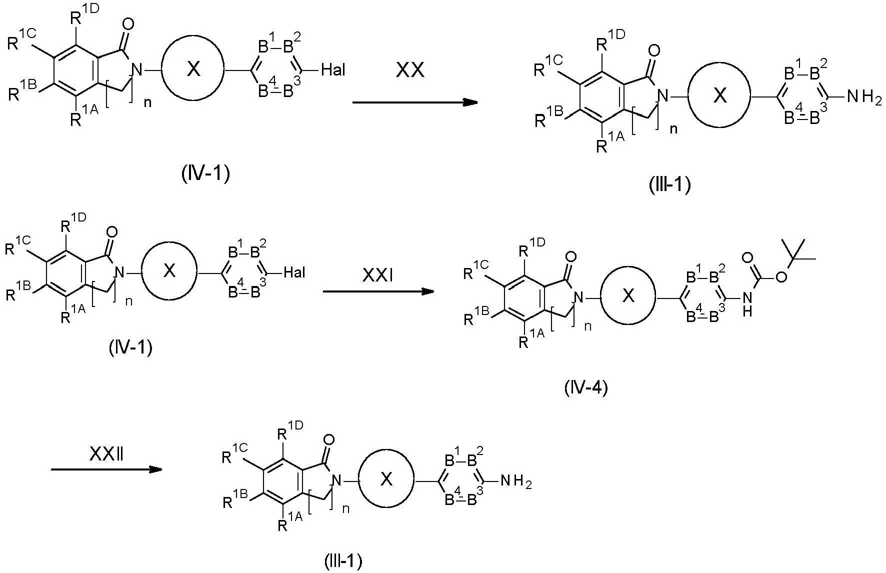

Compounds of formula (III-1-1) can be prepared with different synthetic routes. Step (XVII) can be performed via Suzuki cross coupling reaction starting from an appropriate aryl boronic acid/ ester, such as precursor (1), as described in either Tetrahedron, 2009, 65(37), 7817-7824 or WO2018/075937.

Compounds of formula (III-1-2) can be prepared by Chan-Lam coupling with an appropriate boronic acid, such as precursor (1), as depicted in step (XVIII) and as described in Bioorg. Med. Chem., 17(13), 2009, 4708-4717. Compounds of the formula (III-1-2) can also be prepared by aromatic nucleophilic substitution of the appropriate 4-fluoroaniline, analogously to the method described for preparation of compounds of the formula (IV-2) in Scheme 9.

Compounds of formula (III-1) can also be prepared by reduction of nitro compounds of formula (IV-3) using reducing agents such as SnCl₂ in acid medium as shown in step (XIX). Alternatively, compounds of formula (III-1) can also be prepared by reacting compounds of the formula (IV-1) with ammonia in the presence of a metal catalyst or its salts, preferably copper or its salts as depicted in step (XX) as described in Chem. Commun., 2009, 3035-3037. Additionally, compounds of formula (III-1) can also be prepared in two steps from compounds of the formula (IV-1). Treatment of compounds of formula (IV-3) with tert-butyl carbamate in the presence of metal catalyst or its salts, preferably palladium or its salts to form compounds of formula (IV-4) in step (XXI), followed by Boc-deprotection using trifluoroacetic acid or diluted hydrochloric acid to form the desired compound in step (XXII). All these reactions are performed as described in March's Advanced Organic Chemistry 6th edition, Michael B. Smith and Jerry March.

Compounds of formula (V-1) and (V-2) can be prepared by methods described in Scheme 10 and Scheme 11. Compounds of formula (IV-3) can be prepared analogously to the methods described in Scheme 9. Compounds of formula (IV-1) can be prepared analogously to the methods described in Scheme 9.

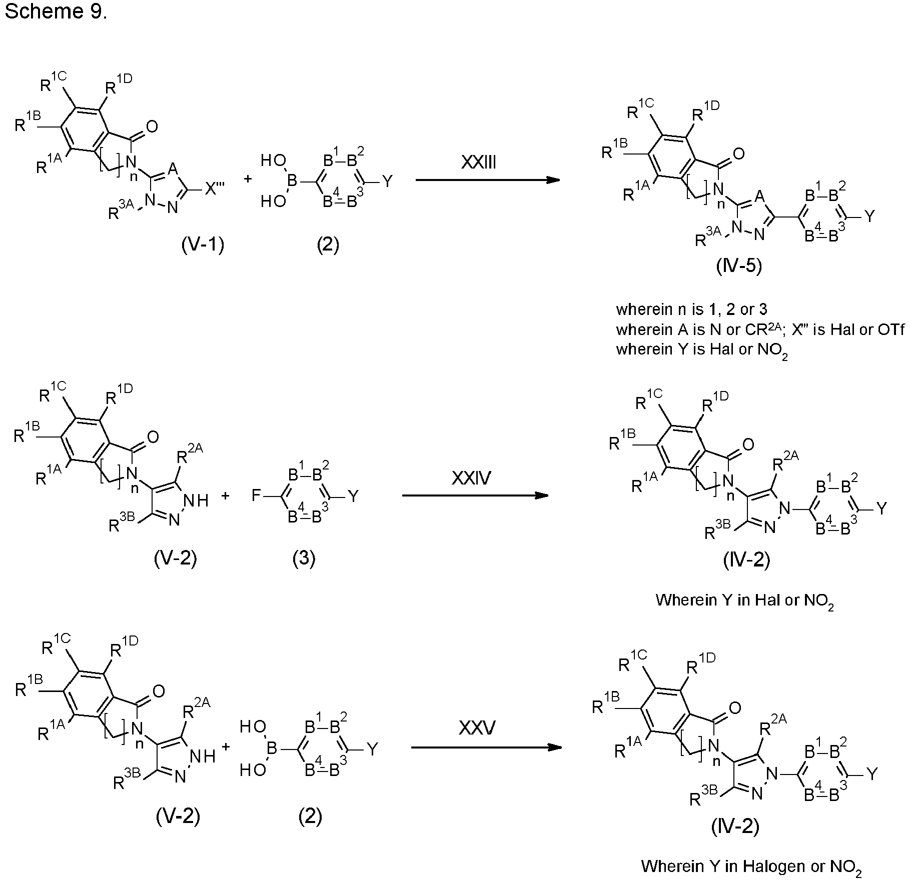

Step (XXIII) can be performed via Suzuki cross coupling reaction starting from an aryl boronic acid precursor (2) as described in Tetrahedron, 2009, 65(37), 7817-7824 or WO2018/075937. Similar reaction can also be carried with aryl boronate ester instead of aryl boronic acid precursor (2).

Step (XXIV) involves a nucleophilic aromatic substitution reaction between pyrazole of formula (IVa) and p-fluoro nitroarene or p-fluoro haloarene (3) as described in WO 2017/139274.

Step (XXV) can be performed via Chan-Lam coupling reaction starting from an aryl boronic acid such as precursor (2) as described in Chem. A Eur. J., 2017, 23(14), 3285-3290.

Compounds of formula (V-1) and (V-2) can be prepared by the methods described in Scheme 10 and Scheme 11.

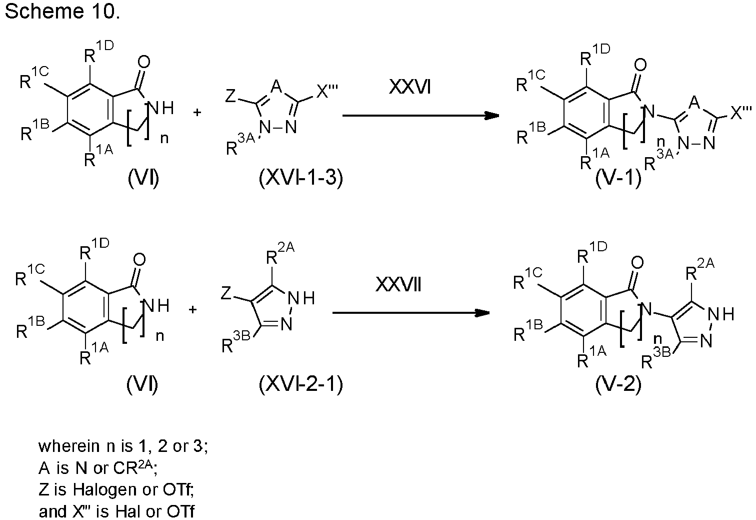

Compounds of the formula (V-1) can be prepared from the metal catalyzed Buchwald or Ullmann coupling of the lactam (VI) with the substituted heteroaryl ring (XVI-1-3) where Z is Hal or OTf, as in step (XXVI). Alternatively, compounds of the formula (V-1) can be prepared by nucleophilic aromatic substitution of the heteroaryl compounds (XVI-1-3), when Z is Hal, by (VI). Compounds of the formula (V-2) can be prepared from the metal catalyzed Buchwald or Ullmann coupling of the lactam (VI) with the substituted heteroaryl ring (XVI-2-1), as in step (XXVII). Alternatively, compounds of the formula (V-2) can be prepared by nucleophilic aromatic substitution of the heteroaryl compounds (XVI-2-1), when Z is Hal, by (VI).

Compounds of the formula (VI) can be purchased commercially or prepared according to the methods in Scheme 13.

Compounds of the formula (XVI-1-3) and (XVI-2-1) can be purchased commercially or prepared analogously according to the methods described in Scheme 14.

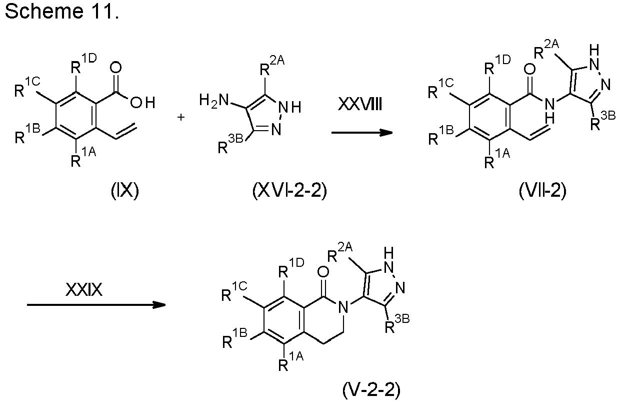

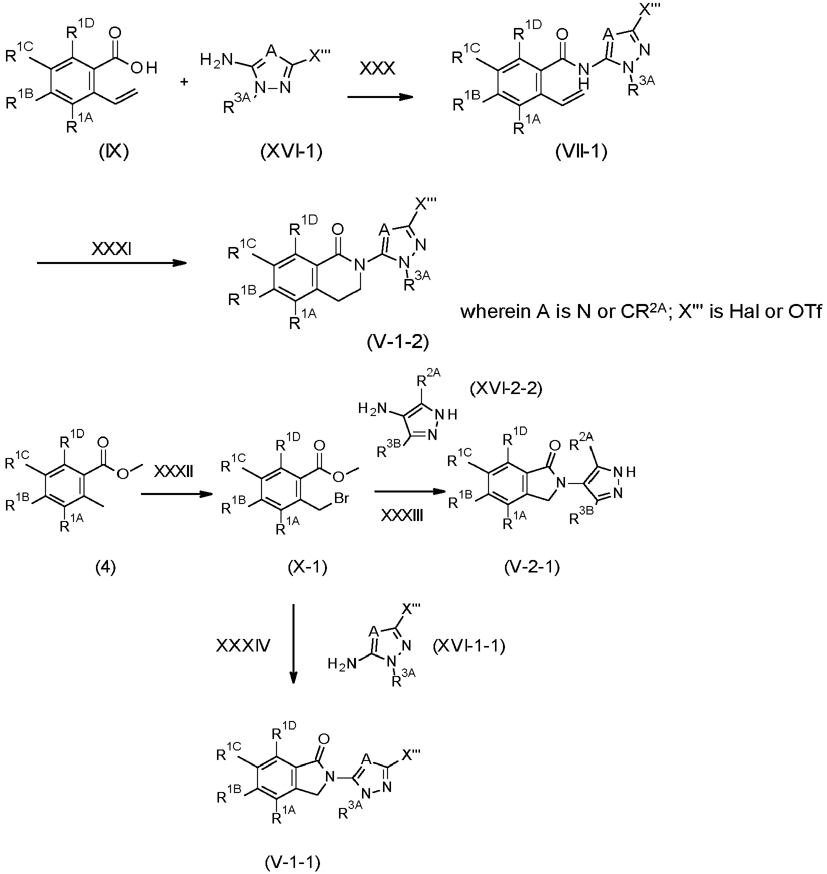

Compounds of the formula (V-2-2) can be prepared by the formation of the intermediates (VII-2) by amide coupling reagents, T3P, or by the *in-situ* generation of the acid chloride with thionyl chloride or oxalyl chloride and DMF, followed by the subsequent cyclization in the presence of an organic base such potassium tert-butoxide in DMF as the solvent.

Similarly, compounds of the formula (V-1-2) can be prepared by the formation of the intermediates (VII-1) by amide coupling reagents, T3P, or by the *in-situ* generation of the acid chloride with thionyl chloride or oxalyl chloride and DMF, followed by the subsequent cyclization in the presence of an organic base such potassium tert-butoxide in DMF as the solvent.

Compounds of the formula (V-2-1) and (V-1-1) can be prepared in 2 steps, first by bromination of the methyl group of starting materials of the formula (4) with *N*-bromosuccinimide in the presence of azobisisobutylnitrile as a catalyst to form the intermediates of the formula (X-1), as in step (XXXII), as described in the methods of Bioorganic & Medicinal Chemistry Letters, 2016, 26(10), 2526-2530. Compounds of the formula (V-1-1) and (V-2-1) can then be formed by heating with compounds of the formula (XVI-2-2) or (XVI-1-1), under basic conditions, as in steps (XXXIII) and (XXXIV), respectively, as described in WO2006/113140.

Compounds of the formula (IX) can be prepared by the methods described in Scheme 12.

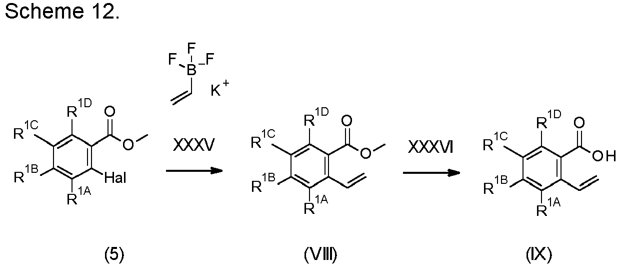

Compounds of the formula (VIII) can be prepared the corresponding building blocks (5) by palladium catalyzed Suzuki coupling, employing potassium vinyltrifluoroborate as in step (XXXV), or by the methods described in Org. Chem. Front., 2022, 9(4), 989-994. Compounds of the formula (IX) can then be prepared by hydrolysis of compounds (VIII) employing an inorganic base, for example NaOH or LiOH, in a mixture of water and a water-miscible organic solvent, for example THF or MeOH, or as described in March's Advanced Organic Chemistry 6th edition, Michael B. Smith and Jerry March.

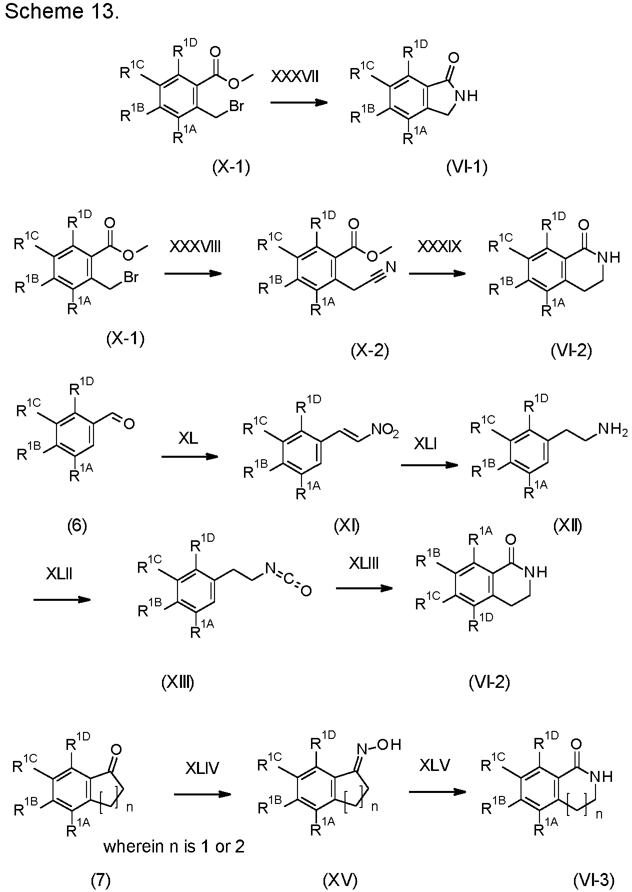

Compounds of the formula (VI-1) can be prepared from compounds (X-1) by heating with aqueous ammonia, as in step (XXXVII), or as described in Bioorg. Med. Chem. Lett., 2012, 22(2), 814-819.

Compounds of the formula (VI-2) can be prepared from compounds of the formula (X-1) in two steps, by first reacting with sodium cyanide as in step (XXXVIII) to give compounds of the formula (X-2), followed by cyclisation under reductive conditions, such as with hydrogen gas over Raney nickel, or with platinum oxide and a mild organic acid such as acetic acid, as in step (XXXIX).

Compounds of the formula (VI-2) can also be prepared from the corresponding aldehydes (6) in 5 steps. Firstly, Henry condensation with nitromethane as in step (XL) is performed, followed by reduction with LiAlH or DIBAL to the corresponding amines (XII). Isocyanate intermediates (XIII) can be prepared by treating compounds of the formula (XII) with phosgene, diphosgene, triphosgene or carbonyldiimidazole in a mixed solvent system and in the presence of a base as described in March's Advanced Organic Chemistry 6th edition, Michael B. Smith and Jerry March. Finally, compounds of the formula (VI-2) can then be prepared from the corresponding isocyanates (XIII) by treating with trifluoromethanesulfonic acid at 0 °C, as described in the methods of J. Org. Chem. 2012, 77(20), 9313-9328.

Compounds of the formula (VI-2) can be prepared in 2 steps from the building blocks (7) by first condensing with hydroxylamine to give compounds of the formula (XV), as in step (XLIV), as described in Tet. Lett. 2017, 58(23), 2240-2243, followed by Beckmann rearrangement, as in the methods described in Bioorg. Med. Chem. Lett. 2002, 12(3), 387-390.

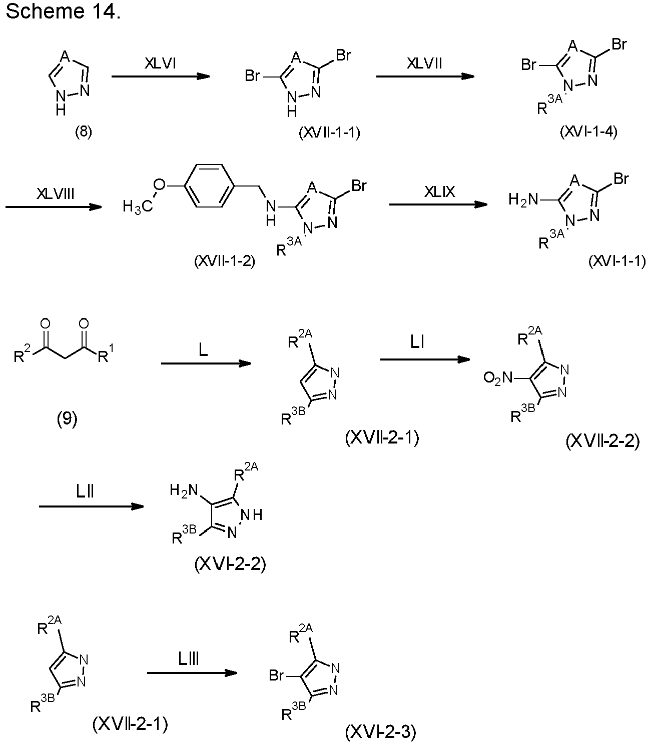

Compounds of formula (XVI-1-4) and (XVI-1-1) can be prepared from commercially available building blocks of the formula (8). Step (XLVI) includes bromination by reacting the compounds of formula (8) with bromine in presence of a weak base like sodium acetate, a protic solvent like ethanol and water. Step (XLVII) includes alkylation by reacting with the corresponding commercially available alkyl halides preferably iodides or bromides in presence of bases like cesium carbonate and polar aprotic solvent like DMF, analogous to as described in March's Advanced Organic Chemistry 6th edition, Michael B. Smith and Jerry March. Step (XLVIII) includes introduction of protecting group such as 2,4-dimethoxybenzylamine. Step (XLIX) involves deprotection to give of compounds of formula (XVII-1-1) by reacting compound of formula (XVII-1-2) with 5N Hydrochloric acid. All these steps can be performed as described in March's Advanced Organic Chemistry 6th edition, Michael B. Smith and Jerry March.

Compounds of formula (XVI-2-1) can be prepared from a commercially available 1,3 diketone derivative (9) by reacting it with hydrazine, analogously to the method described in WO 2016/044666. Then nitration of compounds of formula (XVII-2-1) using a mixture of nitric acid and sulfuric acid in an aprotic solvent such as dichloroethane can produce compounds of formula (XVII-2-2). Compounds of formula (XVII-2-2) can undergo reduction to compounds of formula (XVI-2-2) using reducing agents such as SnCl₂ in acid medium or Fe with NH₄Cl in a mixture of Ethanol and water as shown in step (LII), or as described in March's Advanced Organic Chemistry 6th edition, Michael B. Smith and Jerry March.

Compounds of the formula (XVI-2-3) can be prepared by treating compounds (XVII-2-1) with *N-*bromosuccinimide, as described in the methods of ACS Med. Chem. Lett., 2019, 10(4), 627-632

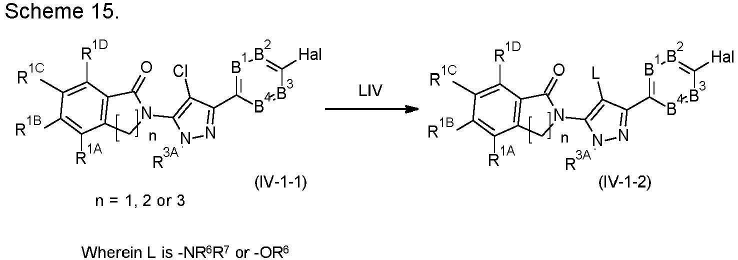

Compounds of formula (IV-1-2) can be prepared from compounds of formula (IV-1-1) in one step. Nucleophilic aromatic substitution of the chloro group of compounds of formula (IV-1-1) can be performed by reacting with the corresponding commercially available substituted amines, in presence of bases like TEA, and polar aprotic solvents like DMF to get compounds of formula (IV-1-2). Compounds of the formula (IV-1-2) wherein L is NR⁶R⁷ can be prepared by amination of compounds of the formula (IV-1-1) in presence of bases like TEA and polar aprotic solvents such as DMF. Compounds of the formula (IV-1-2) wherein L is -OR⁶ can be prepared analogously according to the methods described in WO2021/204626 or in US2016/0185785.

Compounds of the formula (IV-1-1) can be prepared according to the methods described in scheme 16.

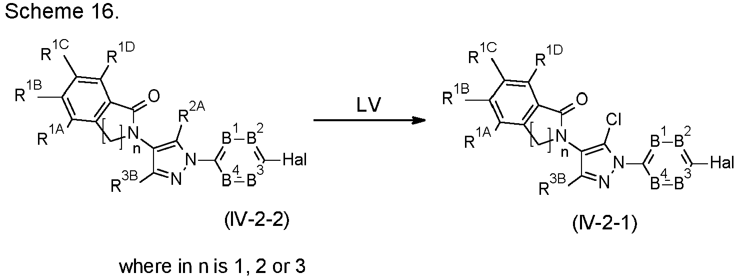

Compounds of formula (IV-2-1) can be prepared by treating compounds of formula (IV-2-2) with electrophilic halogenating agent such as Palau'Chlor in a non-polar aprotic solvent like chloroform analogous to as described in March's Advanced Organic Chemistry 6th edition, Michael B. Smith and Jerry March.

Individual compounds of formula I can also be prepared by derivatisation of other compounds of formula I or the intermediates thereof.

If the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the harmful fungus to be controlled.

A skilled person will readily understand that the preferences for the substituents, also in particular the ones given in the tables below for the respective substituents, given herein in connection with compounds I apply for the intermediates accordingly. Thereby, the substituents in each case have independently of each other or more preferably in combination the meanings as defined herein.

Unless otherwise indicated, the term "compound(s) according to the invention" or "compound(s) of the invention" or "compound(s) of formula (I)", refers to
the compounds of formula I.

The term "compound(s) according to the invention", or "compounds of formula I" comprises the compound(s) as defined herein as well as a stereoisomer, salt, tautomer or N-oxide thereof. The term "compound(s) of the present invention" is to be understood as equivalent to the term "compound(s) according to the invention", therefore also comprising a stereoisomer, salt, tautomer or N-oxide thereof.

The term "composition(s) according to the invention" or "composition(s) of the present invention" encompasses composition(s) comprising at least one compound of formula I according to the invention as defined above. The compositions of the invention are preferably agricultural or veterinary compositions.

Depending on the substitution pattern, the compounds according to the invention may have one or more centers of chirality, in which case they are present as mixtures of enantiomers or diastereomers. The invention provides both the single pure enantiomers or pure diastereomers of the compounds according to the invention, and their mixtures and the use according to the invention of the pure enantiomers or pure diastereomers of the compounds according to the invention or their mixtures. Suitable compounds according to the invention also include all possible geometrical stereoisomers (cis/trans isomers) and mixtures thereof. Cis/trans isomers may be present with respect to an alkene, carbon-nitrogen double-bond or amide group. The term "stereoisomer(s)" encompasses both optical isomers, such as enantiomers or diastereomers, the latter existing due to more than one center of chirality in the molecule, as well as geometrical isomers (cis/trans isomers). The present invention relates to every possible stereoisomer of the compounds of formula I, i.e. to single enantiomers or diastereomers, as well as to mixtures thereof.

The compounds according to the invention may be amorphous or may exist in one or more different crystalline states (polymorphs) which may have different macroscopic properties such as stability or show different biological properties such as activities. The present invention relates to amorphous and crystalline compounds according to the invention, mixtures of different crystalline states of the respective compounds according to the invention, as well as amorphous or crystalline salts thereof.

The term "tautomers" encompasses isomers, which are derived from the compounds of formula I by the shift of an H-atom involving at least one H-atom located at a nitrogen, oxygen or sulphur atom. Examples of tautomeric forms are keto-enol forms, imine-enamine forms, urea-isourea forms, thiourea-isothiourea forms, (thio)amide-(thio)imidate forms etc.

The term "stereoisomers" encompasses both optical isomers, such as enantiomers or diastereomers, the latter existing due to more than one center of chirality in the molecule, as well as geometrical isomers (cis/trans isomers).

Depending on the substitution pattern, the compounds of the formula I may have one or more centers of chirality, in which case they are present as mixtures of enantiomers or diastereomers. The invention provides both the pure enantiomers or diastereomers and their mixtures and the use according to the invention of the pure enantiomers or diastereomers of the compound I or its mixtures. Suitable compounds of the formula I also include all possible geometrical stereoisomers (cis/trans isomers) and mixtures thereof.

The term N-oxides relates to a form of compounds I in which at least one nitrogen atom is present in oxidized form (as NO). To be more precise, it relates to any compound of the present invention which has at least one tertiary nitrogen atom that is oxidized to an N-oxide moiety. N-oxides of compounds I can in particular be prepared by oxidizing e.g. the ring nitrogen atom of an N-heterocycle, e.g. a pyridine or pyrimidine ring present in Ar or R¹¹, or an imino-nitrogen present in central tricyclic core, with a suitable oxidizing agent, such as peroxo carboxylic acids or other peroxides. The person skilled in the art knows if and in which positions compounds of the present invention may form N-oxides.

Salts of the compounds of the formula I are preferably agriculturally and veterinarily acceptable salts. They can be formed in a customary method, e.g. by reacting the compound with an acid of the anion in question if the compound of formula I has a basic functionality or by reacting an acidic compound of formula I with a suitable base.

Suitable agriculturally or veterinarily acceptable salts are especially the salts of those cations or the acid addition salts of those acids whose cations and anions, which are known and accepted in the art for the formation of salts for agricultural or veterinary use respectively, and do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH⁴⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or -CH₂-phenyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethylammonium, bis(2-hydroxyethyl)ammonium, benzyltrimethylammonium and benzyl-triethylammonium, furthermore phosphonium ions, sulfonium ions, preferably tri(C₁-C₄-alkyl)sulfonium, and sulfoxonium ions, preferably tri(C₁-C₄-alkyl)sulfoxonium. Suitable acid addition veterinarily acceptable salts, e.g. formed by compounds of formula I containing a basic nitrogen atom, e.g. an amino group, include salts with inorganic acids, for example hydrochlorides, sulphates, phosphates, and nitrates and salts of organic acids for example acetic acid, maleic acid, dimaleic acid, fumaric acid, difumaric acid, methane sulfenic acid, methane sulfonic acid, and succinic acid.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting a compound of formulae I with an acid of the corresponding anion, preferably of hydrochloric acid, hydrobromic acid, sulfuric acid, phosphoric acid or nitric acid.

The term "invertebrate pest" as used herein encompasses animal populations, such as insects, arachnids and nematodes, which may attack plants, thereby causing substantial damage to the plants attacked, as well as ectoparasites which may infest animals, in particular warm blooded animals such as e.g. mammals or birds, or other higher animals such as reptiles, amphibians or fish, thereby causing substantial damage to the animals infested.

The term "plant propagation material" is to be understood to denote all the generative parts of the plant such as seeds and vegetative plant material such as cuttings and tubers (e. g. potatoes), which can be used for the multiplication of the plant. This includes seeds, roots, fruits, tubers, bulbs, rhizomes, shoots, sprouts and other parts of plants, including seedlings and young plants, which are to be transplanted after germination or after emergence from soil. The plant propagation materials may be treated prophylactically with a plant protection compound either at or before planting or transplanting. Said young plants may also be protected before transplantation by a total or partial treatment by immersion or pouring.

The term "plants" comprises any types of plants including "modified plants" and in particular "cultivated plants".

The term "modified plants" refers to any wild type species or related species or related genera of a cultivated plant.

The term "cultivated plants" is to be understood as including plants which have been modified by breeding, mutagenesis or genetic engineering including but not limiting to agricultural biotech products on the market or in development (cf. http://www.bio.org/speeches/pubs/er/agri_products.asp). Genetically modified plants are plants, which genetic material has been so modified by the use of recombinant DNA techniques that under natural circumstances cannot readily be obtained by cross breeding, mutations or natural recombination. Typically, one or more genes have been integrated into the genetic material of a genetically modified plant in order to improve certain properties of the plant. Such genetic modifications also include but are not limited to targeted post-translational modification of protein(s), oligo- or polypeptides e. g. by glycosylation or polymer additions such as prenylated, acetylated or farnesylated moieties or PEG moieties.

Plants that have been modified by breeding, mutagenesis or genetic engineering, e. g. have been rendered tolerant to applications of specific classes of herbicides, such as auxin herbicides such as dicamba or 2,4-D; bleacher herbicides such as hydroxylphenylpyruvate dioxygenase (HPPD) inhibitors or phytoene desaturase (PDS) inhibittors; acetolactate synthase (ALS) inhibitors such as sulfonyl ureas or imidazolinones; enolpyruvylshikimate-3-phosphate synthase (EPSPS) inhibitors, such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering. Furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are e. g. described in Pest Managem. Sci. 61, 2005, 246; 61, 2005, 258; 61, 2005, 277; 61, 2005, 269; 61, 2005, 286; 64, 2008, 326; 64, 2008, 332; Weed Sci. 57, 2009, 108; Austral. J. Agricult. Res. 58, 2007, 708; Science 316, 2007, 1185; and references quoted therein. Several cultivated plants have been rendered tolerant to herbicides by conventional methods of breeding (mutagenesis), e. g. Clearfield^{®} summer rape (Canola, BASF SE, Germany) being tolerant to imidazolinones, e. g. imazamox, or ExpressSun^{®} sunflowers (DuPont, USA) being tolerant to sulfonyl ureas, e. g. tribenuron. Genetic engineering methods have been used to render cultivated plants such as soybean, cotton, corn, beets and rape, tolerant to herbicides such as glyphosate and glufosinate, some of which are commercially available under the trade names RoundupReady^{®} (glyphosate-tolerant, Monsanto, U.S.A.), Cultivance^{®} (imidazolinone tolerant, BASF SE, Germany) and LibertyLink^{®} (glufosinate-tolerant, Bayer CropScience, Germany).

Furthermore, plants are also covered that are by the use of recombinant DNA techniques capable to synthesize one or more insecticidal proteins, especially those known from the bacterial genus *Bacillus,* particularly from *Bacillus thuringiensis,* such as δ-endotoxins, e. g. CrylA(b), CrylA(c), CrylF, CrylF(a2), CryllA(b), CrylllA, CryIlIB(b1) or Cry9c; vegetative insecticidal proteins (VIP), e. g. VIP1, VIP2, VIP3 or VIP3A; insecticidal proteins of bacteria colonizing nematodes, e. g. *Photorhabdus* spp. or *Xenorhabdus* spp.; toxins produced by animals, such as scorpion toxins, arachnid toxins, wasp toxins, or other insect-specific neurotoxins; toxins produced by fungi, such Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilben synthase, bibenzyl synthase, chitinases or glucanases. In the context of the present invention these insecticidal proteins or toxins are to be understood expressly also as pre-toxins, hybrid proteins, truncated or otherwise modified proteins. Hybrid proteins are characterized by a new combination of protein domains, (see, e. g. WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g. in the publications mentioned above. These insecticidal proteins contained in the genetically modified plants impart to the plants producing these proteins tolerance to harmful pests from all taxonomic groups of athropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard^{®} (corn cultivars producing the Cry1Ab toxin), YieldGard^{®} Plus (corn cultivars producing Cry1Ab and Cry3Bb1 toxins), Starlink^{®} (corn cultivars producing the Cry9c toxin), Herculex^{®} RW (corn cultivars producing Cry34Ab1, Cry35Ab1 and the enzyme Phosphinothricin-N-Acetyltransferase [PAT]); NuCOTN^{®} 33B (cotton cultivars producing the Cry1Ac toxin), Bollgard^{®} I (cotton cultivars producing the Cry1Ac toxin), Bollgard^{®} II (cotton cultivars producing Cry1Ac and Cry2Ab2 toxins); VIPCOT^{®} (cotton cultivars producing a VIP-toxin); NewLeaf^{®} (potato cultivars producing the Cry3A toxin); Bt-Xtra^{®}, NatureGard^{®}, KnockOut^{®}, BiteGard^{®}, Protecta^{®}, Bt11 (e. g. Agrisure^{®} CB) and Bt176 from Syngenta Seeds SAS, France, (corn cultivars producing the Cry1Ab toxin and PAT enyzme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e. g. EP-A 392 225), plant disease resistance genes (e. g. potato cultivars, which express resistance genes acting against *Phytophthora infestans* derived from the mexican wild potato *Solanum bulbocastanum*) or T4-lysozym (e. g. potato cultivars capable of synthesizing these proteins with increased resistance against bacteria such as *Erwinia amylvora*)*.* The methods for producing such genetically modified plants are generally known to the person skilled in the art and are described, e. g. in the publications mentioned above.

Furthermore, plants are also covered that are by the use of recombinant DNA techniques capable to synthesize one or more proteins to increase the productivity (e. g. bio mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of substances of content or new substances of content, specifically to improve human or animal nutrition, e. g. oil crops that produce health-promoting long-chain omega-3 fatty acids or unsaturated omega-9 fatty acids (e. g. Nexera^{®} rape, DOW Agro Sciences, Canada). Furthermore, plants are also covered that contain by the use of recombinant DNA techniques a modified amount of substances of content or new substances of content, specifically to improve raw material production, e. g. potatoes that produce increased amounts of amylopectin (e. g. Amflora^{®} potato, BASF SE, Germany).

The organic moieties mentioned in the above definitions of the variables are - like the term halogen - collective terms for individual listings of the individual members. The prefix Cₙ-Cₘ indicates in each case the possible number of carbon atoms in the group.

The term halogen denotes in each case F, Br, Cl or I, in particular F, Cl or Br.

The term "alkyl" as used herein and in the alkyl moieties of alkoxy, alkylthio, and the like refers to saturated straight-chain or branched hydrocarbon radicals having 1 to 2 ("C₁-C₂-alkyl"), 1 to 3 ("C₁-C₃-alkyl"),1 to 4 ("C₁-C₄-alkyl") or 1 to 6 ("C₁-C₆-alkyl") carbon atoms. C₁-C₂-Alkyl is CH₃ or C₂H₅. C₁-C₃-Alkyl is additionally propyl and isopropyl. C₁-C₄-Alkyl is additionally butyl, 1-methylpropyl (sec-butyl), 2-methylpropyl (isobutyl) or 1,1-dimethylethyl (tert-butyl). C₁-C₆-Alkyl is additionally also, for example, pentyl, 1-methylbutyl, 2-methylbutyl, 3-methylbutyl, 2,2-dimethylpropyl, 1-ethylpropyl, 1,1-dimethylpropyl, 1,2-dimethylpropyl, hexyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, or 1-ethyl-2-methylpropyl.

The term "haloalkyl" as used herein, which is also expressed as "alkyl which is partially or fully halogenated", refers to straight-chain or branched alkyl groups having 1 to 2 ("C₁-C₂-haloalkyl"), 1 to 3 ("C₁-C₃-haloalkyl"), 1 to 4 ("C₁-C₄-haloalkyl") or 1 to 6 ("C₁-C₆-haloalkyl") carbon atoms (as mentioned above), where some or all of the hydrogen atoms in these groups are replaced by halogen atoms as mentioned above: in particular C₁-C₂-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl or pentafluoroethyl. C₁-C₃-haloalkyl is additionally, for example, 1-fluoropropyl, 2-fluoropropyl, 3-fluoropropyl, 1,1-difluoropropyl, 2,2-difluoropropyl, 1,2-difluoropropyl, 3,3-difluoropropyl, 3,3,3-trifluoropropyl, heptafluoropropyl, 1,1,1-trifluoroprop-2-yl, 3-chloropropyl and the like. Examples for C₁-C₄-haloalkyl are, apart those mentioned for C₁-C₃-haloalkyl, 4-chlorobutyl and the like.

The term "alkylene" (or alkanediyl) as used herein in each case denotes an alkyl radical as defined above, wherein one hydrogen atom at any position of the carbon backbone is replaced by one further binding site, thus forming a bivalent moiety. Alkylene has preferably 1 to 6 carbon atoms (C₁-C₆-alkylene), 2 to 6 carbon atoms (C₂-C₆-alkylene), in particular 1 to 4 carbon atoms (C₁-C₄-alkylene) or 2 to 4 carbon atoms (C₂-C₄-alkylene). Examples of alkylene are methylene (CH2), 1,1-ethandiyl, 1,2-ethandiyl, 1,3-propandiyl, 1,2-propandiyl, 2,2-propandiyl, 1,4-butandiyl, 1,2-butandiyl, 1,3-butandiyl, 2,3-butandiyl, 2,2-butandiyl, 1,5-pentandiyl, 2,2-dimethylpropan-1,3-diyl, 1,3-dimethyl-1,3-propandiyl, 1,6-hexandiyl etc.

The term "alkenyl" as used herein refers to monounsaturated straight-chain or branched hydrocarbon radicals having 2 to 3 ("C₂-C₃-alkenyl"), 2 to 4 ("C₂-C₄-alkenyl") or 2 to 6 ("C₂-C₆-alkenyl) carbon atoms and a double bond in any position, for example C₂-C₃-alkenyl, such as ethenyl, 1-propenyl, 2-propenyl or 1-methylethenyl; C₂-C₄-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl or 2-methyl-2-propenyl; C₂-C₆-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl and the like.

The term "alkynyl" as used herein refers to straight-chain or branched hydrocarbon groups having 2 to 3 ("C₂-C₃-alkynyl"), 2 to 4 ("C₂-C₄-alkynyl") or 2 to 6 ("C₂-C₆-alkynyl") carbon atoms and one or two triple bonds in any position, for example C₂-C₃-alkynyl, such as ethynyl, 1-propynyl or 2-propynyl; C₂-C₄-alkynyl, such as ethynyl, 1-propynyl, 2-propynyl, 1-butynyl, 2-butynyl, 3-butynyl, 1-methyl-2-propynyl and the like, C₂-C₆-alkynyl, such as ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, 1-ethyl-1-methyl-2-propynyl and the like;

The term "cycloalkyl" as used herein refers to mono- or bi- or polycyclic saturated hydrocarbon radicals having in particular 3 to 6 ("C₃-C₆-cycloalkyl") or 3 to 5 ("C₃-C₅-cycloalkyl") or 3 to 4 ("C₃-C₄-cycloalkyl") carbon atoms. Examples of monocyclic radicals having 3 to 4 carbon atoms comprise cyclopropyl and cyclobutyl. Examples of monocyclic radicals having 3 to 5 carbon atoms comprise cyclopropyl, cyclobutyl and cyclopentyl. Examples of monocyclic radicals having 3 to 6 carbon atoms comprise cyclopropyl, cyclobutyl, cyclopentyl and cyclohexyl. Examples of monocyclic radicals having 3 to 8 carbon atoms comprise cyclopropyl, cyclobutyl, cyclopentyl, cyclohexyl, cycloheptyl and cyclooctyl. Examples of bicyclic radicals having 7 or 8 carbon atoms comprise bicyclo[2.2.1]heptyl, bicyclo[3.1.1]heptyl, bicyclo[2.2.2]octyl and bicyclo[3.2.1]octyl. Preferably, the term cycloalkyl denotes a monocyclic saturated hydrocarbon radical.

The term "cycloalkoxy" as used herein refers to a cycloalkyl radical, in particular a monocyclic cycloalkyl radical, as defined above having in particular 3 to 6 ("C₃-C₆-cycloalkoxy") or 3 to 5 ("C₃-C₅-cycloalkoxy") or 3 to 4 ("C₃-C₄-cycloalkoxy") carbon atoms, which is bound via an oxygen atom to the remainder of the molecule.

The term "cycloalkyl-C₁-C₄-alkyl" refers to a C₃-C₈-cycloalkyl ("C₃-C₈-cycloalkyl-C₁-C₄-alkyl"), preferably a C₃-C₈-cycloalkyl ("C₃-C₆-cycloalkyl-C₁-C₄-alkyl"), more preferably a C₃-C₄-cycloalkyl ("C₃-C₄-cycloalkyl-C₁-C₄-alkyl") as defined above (preferably a monocyclic cycloalkyl group) which is bound to the remainder of the molecule via a C₁-C₄-alkyl group, as defined above. Examples for C₃-C₄-cycloalkyl-C₁-C₄-alkyl are cyclopropylmethyl, cyclopropylethyl, cyclopropylpropyl, cyclobutylmethyl, cyclobutylethyl and cyclobutylpropyl, Examples for C₃-C₆-cycloalkyl-C₁-C₄-alkyl, apart those mentioned for C₃-C₄-cycloalkyl-C₁-C₄-alkyl, are cyclopentylmethyl, cyclopentylethyl, cyclopentylpropyl, cyclohexylmethyl, cyclohexylethyl and cyclohexylpropyl.

The term "C₁-C₂-alkoxy" is a C₁-C₂-alkyl group, as defined above, attached via an oxygen atom. The term "C₁-C₃-alkoxy" is a C₁-C₃-alkyl group, as defined above, attached via an oxygen atom. The term "C₁-C₄-alkoxy" is a C₁-C₄-alkyl group, as defined above, attached via an oxygen atom. The term "C₁-C₈-alkoxy" is a C₁-C₆-alkyl group, as defined above, attached via an oxygen atom. The term "C₁-C₁₀-alkoxy" is a C₁-C₁₀-alkyl group, as defined above, attached via an oxygen atom. C₁-C₂-Alkoxy is OCH₃ or OC₂H₅. C₁-C₃-Alkoxy is additionally, for example, n-propoxy and 1-methylethoxy (isopropoxy). C₁-C₄-Alkoxy is additionally, for example, butoxy, 1-methylpropoxy (sec-butoxy), 2-methylpropoxy (isobutoxy) or 1,1-dimethylethoxy (tert-butoxy). C₁-C₆-Alkoxy is additionally, for example, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy or 1-ethyl-2-methylpropoxy. C₁-C₈-Alkoxy is additionally, for example, heptyloxy, octyloxy, 2-ethylhexyloxy and positional isomers thereof. C₁-C₁₀-Alkoxy is additionally, for example, nonyloxy, decyloxy and positional isomers thereof.

The term "C₁-C₂-haloalkoxy" is a C₁-C₂-haloalkyl group, as defined above, attached via an oxygen atom. The term "C₁-C₃-haloalkoxy" is a C₁-C₃-haloalkyl group, as defined above, attached via an oxygen atom. The term "C₁-C₄-haloalkoxy" is a C₁-C₄-haloalkyl group, as defined above, attached via an oxygen atom. The term "C₁-C₈-haloalkoxy" is a C₁-C₆-haloalkyl group, as defined above, attached via an oxygen atom. C₁-C₂-Haloalkoxy is, for example, OCH₂F, OCHF₂, OCF₃, OCH₂Cl, OCHCl₂, OCCl₃, chlorofluoromethoxy, dichlorofluoromethoxy, chlorodifluoromethoxy, 2-fluoroethoxy, 2-chloroethoxy, 2-bromoethoxy, 2-iodoethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy or OC₂F₅. C₁-C₃-Haloalkoxy is additionally, for example, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 2-bromopropoxy, 3-bromopropoxy, 3,3,3-trifluoropropoxy, 3,3,3-trichloropropoxy, OCH₂-C₂F₅, OCF₂-C₂F₅, 1-(CH₂F)-2-fluoroethoxy, 1-(CH₂Cl)-2-chloroethoxy or 1-(CH₂Br)-2-bromoethoxy. C₁-C₄-Haloalkoxy is additionally, for example, 4-fluorobutoxy, 4-chlorobutoxy, 4-bromobutoxy or nonafluorobutoxy. C₁-C₆-Haloalkoxy is additionally, for example, 5-fluoropentoxy, 5-chloropentoxy, 5-brompentoxy, 5-iodopentoxy, undecafluoropentoxy, 6-fluorohexoxy, 6-chlorohexoxy, 6-bromohexoxy, 6-iodohexoxy or dodecafluorohexoxy.

The term "C₁-C₆-alkoxy-C₁-C₄-alkyl" as used herein, refers to a straight-chain or branched alkyl having 1 to 4 carbon atoms, as defined above, where one hydrogen atom is replaced by a C₁-C₆-alkoxy group, as defined above. Examples are methoxymethyl, ethoxymethyl, propoxymethyl, isopropoxymethyl, n-butoxymethyl, sec-butoxymethyl, isobutoxymethyl, tert-butoxymethyl, 1-methoxyethyl, 1-ethoxyethyl, 1-propoxyethyl, 1-isopropoxyethyl, 1-n-butoxyethyl, 1-sec-butoxyethyl, 1-isobutoxyethyl, 1-tert-butoxyethyl, 2-methoxyethyl, 2-ethoxyethyl, 2-propoxyethyl, 2-isopropoxyethyl, 2-n-butoxyethyl, 2-sec-butoxyethyl, 2-isobutoxyethyl, 2-tert-butoxyethyl, 1-methoxypropyl, 1-ethoxypropyl, 1-propoxypropyl, 1-isopropoxypropyl, 1-n-butoxypropyl, 1-sec-butoxypropyl, 1-isobutoxypropyl, 1-tert-butoxypropyl, 2-methoxypropyl, 2-ethoxypropyl, 2-propoxypropyl, 2-isopropoxypropyl, 2-n-butoxypropyl, 2-sec-butoxypropyl, 2-isobutoxypropyl, 2-tert-butoxypropyl, 3-methoxypropyl, 3-ethoxypropyl, 3-propoxypropyl, 3-isopropoxypropyl, 3-n-butoxypropyl, 3-sec-butoxypropyl, 3-isobutoxypropyl, 3-tert-butoxypropyl and the like.

The term "alkoxyalkoxy" as used herein refers to an alkoxyalkyl radical, in particular a C₁-C₆-alkoxy-C₁-C₄-alkyl radical, as defined above, which is bound via an oxygen atom to the remainder of the molecule. Examples thereof are OCH₂-OCH₃, OCH₂-OC₂H₅, n-propoxymethoxy, OCH₂-OCH(CH₃)₂, n-butoxymethoxy, (1-methylpropoxy)methoxy, (2-methylpropoxy)methoxy, OCH₂-OC(CH₃)₃, 2-(methoxy)ethoxy, 2-(ethoxy)ethoxy, 2-(n-propoxy)ethoxy, 2-(1-methylethoxy)ethoxy, 2-(n-butoxy)ethoxy, 2-(1-methylpropoxy)ethoxy, 2-(2-methylpropoxy)ethoxy, 2-(1,1-dimethylethoxy)ethoxy, etc.

The substituent "oxo" replaces a CH₂ by a C(=O) group.

The term "aryl" relates to phenyl and bi- or polycyclic carbocycles having at least one fused phenylene ring, which is bound to the remainder of the molecule. Examples of bi- or polycyclic carbocycles having at least one phenylene ring include naphthyl, tetrahydronaphthyl, indanyl, indenyl, anthracenyl, fluorenyl etc.

The term "5- to 6-membered carbocyclic group" as used herein refers to cyclopentane and cyclohexane rings which may contain heteroatoms selected from N, O, and S.

The term "aryl-C₁-C₄-alkyl" relates to C₁-C₄-alkyl, as defined above, wherein one hydrogen atom has been replaced by an aryl radical, in particular a phenyl radical. Particular examples of aryl-C₁-C₄-alkyl include -CH₂-phenyl, 1-phenethyl, 2-phenetyl, 1-phenylpropyl, 2-phenylpropyl, 3-phenyl-1-propyl and 2-phenyl-2-propyl.

The term "aryloxy-C₁-C₄-alkyl" relates to C₁-C₄-alkyl, as defined above, wherein one hydrogen atom has been replaced by an aryloxy radical, in particular a phenoxy radical. Particular examples of aryloxy-C₁-C₄-alkyl include phenoxymethyl, 1-phenoxyethyl, 2-phenoxyetyl, 1-phenoxypropyl, 2-phenoxypropyl, 3-phenoxy-1-propyl and 2-phenoxy-2-propyl.

The term "aryl-C₁-C₄-carbonyl" relates to aryl as defined above, in particular a phenyl radical, which is bound by a carbonyl to the remainder of the molecule. Particular examples of arylcarbonyl include benzoyl, 1-naphthoyl and 2-naphthoyl.

The term "hetaryl" relates to aromatic heterocyclyl or heterocycles having either 5 or 6 ring atoms (5- or 6-membered hetaryl) and being monocyclic or 8, 9 or 10 ring atoms and bing bicyclic. Hetaryl will generally have at least one ring atom selected from O, S and N, which in case of N may be an imino-nitrogen or an amino-nitrogen, which carries hydrogen or a radical different from hydrogen. Hetaryl may have 1, 2, 3 or 4 further nitrogen atoms as ring members, which are imino nitrogens. Examples of 5- or 6-membered hetaryl include 2-furyl, 3-furyl, 2-thienyl, 3-thienyl, 1-pyrrolyl, 2-pyrrolyl, 3-pyrrolyl, 1-pyrazolyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 1-imidazolyl, 2-imidazolyl, 4-imidazolyl, 1,3,4-triazol-1-yl, 1,3,4-triazol-2-yl, 1,3,4-oxadiazolyl-2-yl, 1,3,4-thiadiazol-2-yl, 2-pyridinyl, 3-pyridinyl, 4-pyridinyl, 3-pyridazinyl, 4-pyridazinyl, 2-pyrimidinyl, 4-pyrimidinyl, 5-pyrimidinyl, 2-pyrazinyl and 1,3,5-triazin-2-yl.. Examples of 8-, 9- or 10-membered hetaryl include, for example, quinolinyl, isoquinolinyl, cinnolinyl, indolyl, indolizynyl, isoindolyl, indazolyl, benzofuryl, benzothienyl, benzo[b]thiazolyl, benzoxazolyl, benzthiazolyl, benzimidazolyl, imidazo[1,2-a]pyridine-2-yl, thieno[3,2-b]pyridine-5-yl, imidazo-[2,1-b]-thiazol-6-yl and 1,2,4-triazolo[1,5-a]pyridine-2-yl. Examples of N-bound 5-, 6-, 7 or 8-membered saturated heterocyclyl or heterocycles include: pyrrolidin-1-yl, pyrazolidin-1-yl, imidazolidin-1-yl, oxazolidin-3-yl, isoxazolidin-2-yl, thiazolidin-3-yl, isothiazolidin-2-yl, piperidin-1-yl, piperazin-1-yl, morpholin-4-yl, thiomorpholin-4-yl, 1-oxothiomorpholin-4-yl, 1,1-dioxothiomorpholin-4-yl, azepan-1-yl and the like.

The term "hetaryl-C₁-C₄-alkyl" relates to C₁-C₄-alkyl, as defined above, wherein one hydrogen atom has been replaced by a hetaryl radical, in particular a pyridyl radical. Particular examples of hetaryl-C₁-C₄-alkyl include 2-pyridylmethyl, 3-pyridylmethyl, 4-pyridylmethyl, 1-(2-pyridyl)ethyl, 2-(2-pyridyl)ethyl, 1-(3-pyridyl)ethyl, 2-(3-pyridyl)ethyl, 1-(4-pyridyl)ethyl, 2-(4-pyridyl)ethyl etc..

The term "hetaryloxy-C₁-C₄-alkyl" relates to C₁-C₄-alkyl, as defined above, wherein one hydrogen atom has been replaced by an hetaryloxy radical, in particular a pyridyloxy radical. Particular examples of hetaryloxy-C₁-C₄-alkyl include 2-pyridyloxymethyl, 3-pyridyloxymethyl, 4-pyridyloxymethyl, 1-(2-pyridyloxy)ethyl, 2-(2-pyridyloxy)ethyl, 1-(3-pyridyloxy)ethyl, 2-(3-pyridyloxy)ethyl, 1-(4-pyridyloxy)ethyl, 2-(4-pyridyloxy)ethyl etc.

The term "hetaryl-C₁-C₄-carbonyl" relates to hetaryl as defined above, in particular a C-bound hetaryl radical, e.g. 2-, 3-or 4-pyridyl, 2- or 3-thienyl, 2- or 3-furyl, 1-, 2- or 3-pyrrolyl, 2- or 4-pyrimidinyl, pyridazinyl, 1-, 3- or 4-pyrazolyl, 1-, 2- or 4-imidazolyl radical, which is bound by a carbonyl to the remainder of the molecule.

The term "substituted" if not specified otherwise refers to substituted with 1, 2, or up to maximum possible number of substituents. If substituents as defined in compounds of formula I are more than one then they are independently from each other are same or different if not mentioned otherwise.

With respect to the variables, the embodiments of the compounds of the formula I are,
In one preferred embodiment, maximum two of B¹, B,² and B³ are N;
In another preferred embodiment, B¹ is CR^{B1}, B² is CR^{B2}, and B³ is CR^{B3};
In another preferred embodiment, B¹ is N, B² is CR^{B2}, and B³ is CR^{B3};
In another preferred embodiment, B¹ is CR^{B1}, B² is N, and B³ is CR^{B3};
In another preferred embodiment, B¹ is CR^{B1}, B² is N, and B³ is N;
In another preferred embodiment, B¹ is N, B² is N, and B³ is CR^{B3};
In another preferred embodiment, B¹ is CR^{B1}, and B² is N or CR^{B2}, B³ is N or CR^{B3};
In another preferred embodiment, B³ is CR^{B3}, and B¹ is N or CR^{B2}, B² is N or CR^{B3};
In one preferred embodiment, R^{B1}, R^{B2}, R^{B3}, and R^{B4} independently of each other are selected from H, halogen, CN, C₁-C₆-alkyl, and C₁-C₆-alkoxy, wherein the alkyl, alkoxy moieties are unsubstituted or substituted with halogen, or CN;
In another preferred embodiment, R^{B1}, R^{B2}, R^{B3}, and R^{B4} independently of each other are selected from H, halogen, CN, and C₁-C₆-alkyl, wherein the alkyl moieties are unsubstituted or substituted with halogen, or CN;
In another preferred embodiment, R^{B1}, R^{B2}, R^{B3}, and R^{B4} independently of each other are selected from H, halogen, CN, and C₁-C₆-alkyl, wherein the alkyl moieties are unsubstituted or substituted with halogen;
In another preferred embodiment, R^{B1}, R^{B2}, R^{B3}, and R^{B4} independently of each other are selected from H, halogen, CN, and C₁-C₆-alkyl, wherein the alkyl moieties are unsubstituted;
In another preferred embodiment, R^{B1}, R^{B2}, R^{B3}, and R^{B4} independently of each other are selected from H, halogen, and C₁-C₆-alkyl, wherein the alkyl moieties are unsubstituted;
In another preferred embodiment, R^{B1}, R^{B2}, R^{B3}, and R^{B4} independently of each other are selected from H and halogen;
In another preferred embodiment, R^{B1}, R^{B2}, R^{B3}, and R^{B4} independently of each other are selected from H, halogen, and CN;
In one preferred embodiment, R^{1A}, R^{1B}, R^{1C} and R^{1D} independently of each other are selected from H, halogen, CN, C₁-C₆-alkyl, and C₁-C₆-alkoxy, wherein the alkyl, alkoxy moieties are unsubstituted or substituted with halogen or CN;
In another preferred embodiment, R^{1A}, R^{1B}, R^{1C} and R^{1D} independently of each other are selected from H, halogen, CN, C₁-C₆-alkyl, and C₁-C₆-alkoxy, wherein the alkyl, alkoxy moieties are unsubstituted or substituted with halogen;
In another preferred embodiment, R^{1A}, R^{1B}, R^{1C} and R^{1D} independently of each other are selected from H, halogen, CN, and C₁-C₆-alkoxy, wherein the alkoxy moieties are unsubstituted or substituted with halogen or CN;
In another preferred embodiment, R^{1A}, R^{1B}, R^{1C} and R^{1D} independently of each other are selected from H, halogen, CN, C₁-C₆-haloalkyl, and C₁-C₆-haloalkoxy;
In another preferred embodiment, R^{1A}, R^{1B}, R^{1C} and R^{1D} independently of each other are selected from H and C₁-C₆-haloalkoxy;
In another preferred embodiment, R^{1A}, R^{1B}, R^{1C} and R^{1D} independently of each other are selected from H, CI, F, Br, CN, CF₃, and OCF₃;
In another preferred embodiment, R^{1A}, R^{1B}, R^{1C} and R^{1D} independently of each other are selected from H, CI, F, CN, CF₃, and OCF₃;
In another preferred embodiment, R^{1A}, R^{1B}, R^{1C} and R^{1D} independently of each other are selected from H and OCF₃;
In another preferred embodiment, R^{1B} is other than H;
In another preferred embodiment, R^{1C} is other than H;
In another preferred embodiment, R^{1D} is other than H;
In one preferred embodiment, n is 1 or 2;
In another preferred embodiment, n is 2;
In another preferred embodiment, n is 1;
In one preferred embodiment, X is X1;
In another preferred embodiment, X is X2;
In one preferred embodiment, A is N;
In another preferred embodiment, A is CR^{2A}.
In one preferred embodiment, R^{2A} is H, C₁-C₆-alkyl, C₃-C₆-cycloalkyl, or halogen, wherein the alkyl and cycloalkyl moieties are unsubstituted or substituted with halogen or CN;
In another preferred embodiment, R^{2A} is H, halogen, or C₁-C₆-alkyl;
In another preferred embodiment, R^{2A} is H or C₁-C₆-alkyl;
In another preferred embodiment, R^{2A} is H, or C₁-C₆-alkyl, wherein the alkyl moieties are unsubstituted or substituted with halogen or CN;
In another preferred embodiment, R^{2A} is C₁-C₆-alkyl, which is unsubstituted;
In another preferred embodiment, R^{2A} is C₁-C₆-alkyl, which is substituted with halogen or CN;
In another preferred embodiment, R^{2A} is C₃-C₆-cycloalkyl, which is unsubstituted or substituted with halogen or CN;
In another preferred embodiment, R^{2A} is C₃-C₆-cycloalkyl, which is unsubstituted;
In another preferred embodiment, R^{2A} is C₃-C₆-cycloalkyl, which is substituted with halogen or CN;
In another preferred embodiment, R^{2A} is H or halogen;
In another preferred embodiment, R^{2A} is H;
In another preferred embodiment, R^{2A} is halogen;
In another preferred embodiment, R^{2A} is H, halogen, or CH₃;
In another preferred embodiment, R^{2A} is H, Cl, or CH₃;
In another preferred embodiment, R^{2A} is H or CH₃;
In another preferred embodiment, A is N, C(C₁-C₆-alkyl), or CH;
In another preferred embodiment, A is N, C(CH₃), or CH;
In one preferred embodiment, R^{3A} is H, C₁-C₆-alkyl, or C₃-C₆-cycloalkyl, wherein the alkyl and cycloalkyl moieties are unsubstituted or substituted with halogen, CN or C₁-C₆-alkoxy;
In another preferred embodiment, R^{3A} is H or C₁-C₆-alkyl;
In another preferred embodiment, R^{3A} is H, or C₁-C₆-alkyl, wherein the alkyl moieties are unsubstituted or substituted with halogen or C₁-C₆-alkoxy;
In another preferred embodiment, R^{3A} is C₁-C₆-alkyl, which is unsubstituted;
In another preferred embodiment, R^{3A} is C₁-C₆-alkyl, which is substituted with halogen, CN or C₁-C₆-alkoxy;
In another preferred embodiment, R^{3A} is C₃-C₆-cycloalkyl, which is unsubstituted or substituted with halogen or C₁-C₆-alkoxy;
In another preferred embodiment, R^{3A} is C₃-C₆-cycloalkyl, which is unsubstituted;
In another preferred embodiment, R^{3A} is C₃-C₆-cycloalkyl, which is substituted with halogen or C₁-C₆-alkoxy;
In another preferred embodiment, R^{3A} is CH₃ or C₂H₅;
In another preferred embodiment, R^{3A} is H;
In another preferred embodiment, R^{3A} is H, CH₃ or C₂H₅;
In one preferred embodiment, R^{3B} is H or C₁-C₆-alkyl, wherein the alkyl and cycloalkyl moieties are unsubstituted or substituted with halogen or CN;
In another preferred embodiment, R^{3B} is H or C₁-C₆-alkyl;
In another preferred embodiment, R^{3B} is C₁-C₆-alkyl, which is unsubstituted;
In another preferred embodiment, R^{3B} is C₁-C₆-alkyl, which is substituted with halogen or CN;
In another preferred embodiment, R^{3B} is CH₃;
In another preferred embodiment, R^{3B} is H;
In another preferred embodiment, R^{3B} is H or CH₃;
In one preferred embodiment, D is DA, DB, DC, DD, DE, or DF;
In another preferred embodiment, D is DA, DB, DC, DD, or DE;
In another preferred embodiment, D is DA, DB, DC, or DD;
In another preferred embodiment, D is DA or DB;
In another preferred embodiment, D is DC or DD;
In another preferred embodiment, D is DE or DF;
In another preferred embodiment, D is DA;
In another preferred embodiment, D is DB;
In another preferred embodiment, D is DC;
In another preferred embodiment, D is DD;
In another preferred embodiment, D is DE;
In another preferred embodiment, D is DA, DC, or DE;
In another preferred embodiment, D is DB, DD, or DF;
In one preferred embodiment, W is S;
In another preferred embodiment, W is O;
In one preferred embodiment, R⁴ is H or C₁-C₆-alkyl, wherein the alkyl is unsubstituted or substituted with halogen or CN;
In another preferred embodiment, R⁴ is H;
In another preferred embodiment, R⁴ is C₁-C₆-alkyl, which is unsubstituted or substituted with halogen or CN;
In one preferred embodiment, R⁵ is H or C₁-C₆-alkyl, wherein the alkyl moieties are unsubstituted or substituted with -O-(C=O)-C₁-C₆-alkoxy, -O-(C=O)-C₁-C₆-alkyl or CN;
In another preferred embodiment, R⁵ is C₁-C₆-alkyl, which is unsubstituted or substituted with - O-(C=O)-C₁-C₆-alkoxy or -O-(C=O)-C₁-C₆-alkyl;
In another preferred embodiment, R⁵ is H;
In one preferred embodiment, E is a 5- or 6-membered carbocyclic group, wherein 1 or 2 CH₂ moieties of the carbocyclic group may be replaced by a carbonyl group, O, or S, wherein the carbocyclic group is unsubstituted or substituted with R¹⁰;
In another preferred embodiment, E is a 5- or 6-membered carbocyclic group, wherein 1 or 2 CH₂ moieties of the carbocyclic group may be replaced by a carbonyl group, or S, wherein the carbocyclic group is unsubstituted or substituted with R¹⁰;
In another preferred embodiment, E is a 5- or 6-membered carbocyclic group, wherein 1 or 2 CH₂ moieties of the carbocyclic group may be replaced by a carbonyl group, wherein the carbocyclic group is unsubstituted or substituted with R¹⁰;
In another preferred embodiment, E is a 5- or 6-membered carbocyclic group, wherein 1 or 2 CH₂ moieties of the carbocyclic group may be replaced by a carbonyl group, wherein the carbocyclic group is unsubstituted;
In another preferred embodiment, E is a 5-membered carbocyclic group, wherein 1 or 2 CH₂ moieties of the carbocyclic group may be replaced by a carbonyl group, wherein the carbocyclic group is unsubstituted or substituted with R¹⁰;
In another preferred embodiment, E is a 6-membered carbocyclic group, wherein 1 or 2 CH₂ moieties of the carbocyclic group may be replaced by a carbonyl group, wherein the carbocyclic group is unsubstituted or substituted with R¹⁰;
In another preferred embodiment, E is E1 or E2, which is unsubstituted or substituted with R¹⁰; 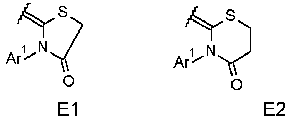
In one preferred embodiment, Ar¹ is phenyl which is unsubstituted or substituted with R^{Ar1};
In another preferred embodiment, Ar¹ is 5- or 6-membered hetaryl, which is unsubstituted or substituted with R^{Ar1};
In another preferred embodiment, Ar¹ is phenyl, pyrimidinyl, pyridazinyl, thiophenyl, thiazolyl, or pyridyl, which are unsubstituted or substituted with R^{Ar1};
In another preferred embodiment, Ar¹ is phenyl, which is substituted with R^{Ar1}.
In one preferred embodiment, R^{Ar1} is halogen, SF₅, OH, CN, NR⁸R⁹, C₁-C₆-alkyl, C₁-C₆-alkoxy, C₃-C₆-cycloalkyl, C₃-C₆-cycloalkoxy wherein the alkyl, alkoxy, cycloalkyl and cycloalkoxy moieties are unsubstituted or substituted with halogen or CN;
In another preferred embodiment, R^{Ar1} is halogen, CN, C₁-C₆-alkyl, C₁-C₆-alkoxy, C₁-C₆-alkoxy-C₁-C₄-alkyl, C₃-C₆-cycloalkyl, wherein the alkyl, alkoxy, and cycloalkyl moieties are unsubstituted or substituted with halogen,
In another preferred embodiment, R^{Ar1} is halogen, CN, C₁-C₆-alkyl, C₁-C₆-alkoxy, C₃-C₆-cycloalkyl, wherein the alkyl, alkoxy, and cycloalkyl moieties are unsubstituted or substituted with halogen,
In another preferred embodiment, R^{Ar1} is CN, C₁-C₆-alkyl, C₃-C₆-cycloalkyl, NR⁸R⁹ wherein alkyl and cycloalkyl moieties are unsubstituted or substituted with halogen;
In another preferred embodiment, R^{Ar1} is CN, C₁-C₆-alkyl, C₁-C₆-alkyl, C₃-C₆-cycloalkyl, or NR⁸R⁹;
In another preferred embodiment, R^{Ar1} is halogen, CN, C₁-C₆-alkyl, wherein the alkyl moieties are unsubstituted or substituted with halogen;
In another preferred embodiment, R^{Ar1} is halogen or C₁-C₆-alkyl, wherein the alkyl moieties are unsubstituted or substituted with halogen;
In another preferred embodiment, R^{Ar1} is halogen or C₁-C₆-alkyl;
In another preferred embodiment, R^{Ar1} is halogen;
In another preferred embodiment, R^{Ar1} is C₁-C₆-alkyl;
In another preferred embodiment, R^{Ar1} is halogen or CN,
In one preferred embodiment, R⁸ and R⁹ are, identical or different, H or C₁-C₆-alkyl;
In another preferred embodiment, Ar¹ is phenyl which is unsubstituted or substituted with R^{Ar1}, and wherein
R^{Ar1} is halogen, CN, C₁-C₆-alkyl, C₁-C₆-alkoxy, C₃-C₆-cycloalkyl, wherein the alkyl, alkoxy, and cycloalkyl moieties are unsubstituted or substituted with halogen.

In another preferred embodiment, Ar¹ is selected from Ar¹-1 to Ar¹-19 as shown in Table Ar1,

**Table Ar1:**

| Ar¹ | Structure |
|---|---|
| Ar¹-1 | 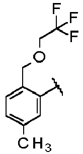 |
| Ar¹-2 | 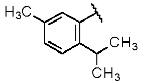 |
| Ar¹-3 | 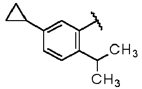 |
| Ar¹-4 | 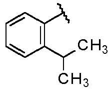 |
| Ar¹-5 | 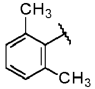 |
| Ar¹-6 | 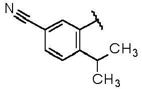 |
| Ar¹-7 | 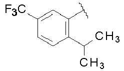 |
| Ar¹-8 | 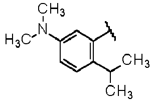 |
| Ar¹-9 | 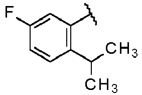 |
| Ar¹-10 | 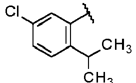 |
| Ar¹-11 | 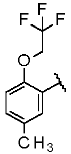 |
| Ar¹-12 | 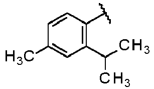 |
| Ar¹-13 | 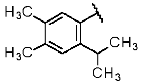 |
| Ar¹-14 | 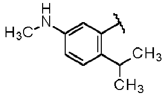 |
| Ar¹-15 | 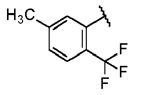 |
| Ar¹-16 | 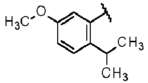 |
| Ar¹-17 | 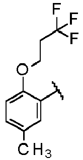 |
| Ar¹-18 | 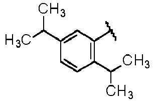 |
| Ar¹-19 | 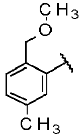 |

In another preferred embodiment, Ar¹ is selected from Ar¹-2 to Ar¹-8;
In another preferred embodiment, Ar¹ is selected from Ar¹-1 to Ar¹-7;
In another preferred embodiment, Ar¹ is Ar¹-2 to Ar¹-5.
In another preferred embodiment of compound of formula I, wherein n is 1 or 2;
R^{1A}, R^{1B}, R^{1C} and R^{1D} independently of each other are selected from H, halogen, CN, C₁-C₆-haloalkyl, and C₁-C₆-haloalkoxy;
X is X1 or X2;
A is N or CR^{2A};
R^{2A} is H, halogen, or C₁-C₆-alkyl;
R^{3A} is H or C₁-C₆-alkyl, wherein the alkyl is unsubstituted or substituted with halogen, CN or C₁-C₆-alkoxy;
R^{3B} is H or C₁-C₆-alkyl, preferably CH₃;
B¹ is CR^{B1}, B² is CR^{B2}, and B³ is CR^{B3};
R^{B1}, R^{B2}, R^{B3}, and R^{B4} independently of each other are H, halogen, C₁-C₆-alkyl, or CN;
D is DA, DB, DC, DD, or DE;
W is O or S;
R⁴ is H;
R⁵ is C₁-C₆-alkyl, which is unsubstituted or substituted with -O-(C=O)-C₁-C₆-alkoxy or -O-(C=O)-C₁-C₆-alkyl;
Ar¹ is phenyl which is unsubstituted or substituted with R^{Ar1};
R^{Ar1} is CN, C₁-C₆-alkyl, C₃-C₆-cycloalkyl, NR⁸R⁹ wherein alkyl and cycloalkyl moieties are unsubstituted or substituted with halogen;
R⁸ and R⁹ are, identical or different, H or C₁-C₆-alkyl.

In another preferred embodiment of compound of formula I, wherein
n is 1 or 2;
R^{1A}, R^{1B}, R^{1C} and R^{1D} independently of each other are selected from H, halogen, CN, C₁-C₆-haloalkyl, and C₁-C₆-haloalkoxy;
X is X1 or X2;
A is N or CR^{2A};
R^{2A} is H, halogen, or C₁-C₆-alkyl;
R^{3A} is H or C₁-C₆-alkyl, wherein the alkyl is unsubstituted or substituted with halogen or C₁-C₆-alkoxy;
R^{3B} is H or C₁-C₆-alkyl, preferably CH₃;
B¹ is CR^{B1}, B² is CR^{B2}, and B³ is CR^{B3};
R^{B1}, R^{B2}, R^{B3}, and R^{B4} independently of each other are H, halogen, C₁-C₆-alkyl, or CN;
D is DA, DB, DC or DD; preferably DC or DD;
W is O or S, preferably S;
R⁴ is H;
R⁵ is C₁-C₆-alkyl, which is unsubstituted or substituted with -O-(C=O)-C₁-C₆-alkoxy or -O-(C=O)-C₁-C₆-alkyl;
Ar¹ is phenyl which is unsubstituted or substituted with R^{Ar1};
R^{Ar1} is CN, C₁-C₆-alkyl, C₃-C₆-cycloalkyl, NR⁸R⁹ wherein alkyl and cycloalkyl moieties are unsubstituted or substituted with halogen;
R⁸ and R⁹ are, identical or different, H or C₁-C₆-alkyl.

In another preferred embodiment of compound of formula I, wherein
n is 1 or 2;
R^{1A}, R^{1B}, R^{1C} and R^{1D} independently of each other are selected from H, CI, F, Br, CN, CF₃, and OCF₃;
X is X1 or X2;
A is N, C(CH₃), or CH;
R^{2A} is H, Cl, or CH₃;
R^{3A} is C₁-C₆-alkyl;
R^{3B} is CH₃;
B¹ is CR^{B1}, B² is CR^{B2}, and B³ is CR^{B3};
R^{B1}, R^{B2}, R^{B3}, and R^{B4} independently of each other are H, halogen, or CN;
D is DA, DB, DC, DD, or DE;
W is O or S, preferably S;
R⁴ is H;
R⁵ is C₁-C₆-alkyl, which is unsubstituted or substituted with -O-(C=O)-C₁-C₆-alkoxy or -O-(C=O)-C₁-C₆-alkyl;
Ar¹ is selected from Ar¹-2 to Ar¹-8.

In another preferred embodiment of compound of formula I, wherein
n is 1 or 2;
R^{1A}, R^{1B}, R^{1C} and R^{1D} independently of each other are selected from H, halogen, CN, C₁-C₆-haloalkyl, and C₁-C₆-haloalkoxy;
X is X1;
A is N, C(C₁-C₆-alkyl), or CH;
R^{3A} is H or C₁-C₆-alkyl, wherein the alkyl is unsubstituted or substituted with halogen, CN or C₁-C₆-alkoxy;
B¹ is N or CR^{B1}, B² is CR^{B2}, and B³ is CR^{B3};
R^{B1}, R^{B2}, R^{B3}, and R^{B4} independently of each other are H, halogen, or CN;
D is DA or DB;
W is S or O, preferably S;
R⁴ is H or C₁-C₆-alkyl;
Ar¹ is phenyl which is unsubstituted or substituted with R^{Ar1};
R^{Ar1} is CN, C₁-C₆-alkyl, C₃-C₆-cycloalkyl, NR⁸R⁹ wherein alkyl and cycloalkyl moieties are unsubstituted or substituted with halogen;
R⁸ and R⁹ are, identical or different, H or C₁-C₆-alkyl.

In another preferred embodiment of compound of formula I, wherein
n is 1 or 2;
R^{1A}, R^{1B}, R^{1C} and R^{1D} independently of each other are selected from H, halogen, CN, C₁-C₆-haloalkyl, and C₁-C₆-haloalkoxy;
X is X1;
A is N, C(C₁-C₆-alkyl), or CH;
R^{3A} is H or C₁-C₆-alkyl, wherein the alkyl is unsubstituted or substituted with halogen, CN or C₁-C₆-alkoxy;
B¹ is N or CR^{B1}, B² is CR^{B2}, and B³ is CR^{B3};
R^{B1}, R^{B2}, R^{B3}, and R^{B4} independently of each other are H, halogen, or CN;
D is DC or DD;
Ar¹ is phenyl which is unsubstituted or substituted with R^{Ar1};
R^{Ar1} is CN, C₁-C₆-alkyl, C₃-C₆-cycloalkyl, NR⁸R⁹ wherein alkyl and cycloalkyl moieties are unsubstituted or substituted with halogen;
R⁸ and R⁹ are, identical or different, H or C₁-C₆-alkyl.

In another preferred embodiment of compound of formula I, wherein
n is 1 or 2;
R^{1A}, R^{1B}, R^{1C} and R^{1D} independently of each other are selected from H, halogen, CN, C₁-C₆-haloalkyl, and C₁-C₆-haloalkoxy;
X is X1;
A is N, C(C₁-C₆-alkyl), or CH;
R^{3A} is H or C₁-C₆-alkyl, wherein the alkyl is unsubstituted or substituted with halogen, CN or C₁-C₆-alkoxy;
B¹ is N or CR^{B1}, B² is CR^{B2}, and B³ is CR^{B3};
R^{B1}, R^{B2}, R^{B3}, and R^{B4} independently of each other are H, halogen, or CN;
D is DE or DF;
R⁴ is H or C₁-C₆-alkyl;
R⁵ is C₁-C₆-alkyl, which is unsubstituted or substituted with -O-(C=O)-C₁-C₆-alkoxy or -O-(C=O)-C₁-C₆-alkyl;
Ar¹ is phenyl which is unsubstituted or substituted with R^{Ar1};
R^{Ar1} is CN, C₁-C₆-alkyl, C₃-C₆-cycloalkyl, NR⁸R⁹ wherein alkyl and cycloalkyl moieties are unsubstituted or substituted with halogen;
R⁸ and R⁹ are, identical or different, H or C₁-C₆-alkyl.

In another preferred embodiment of compound of formula I, wherein
n is 1 or 2;
R^{1A}, R^{1B}, R^{1C} and R^{1D} independently of each other are selected from H, halogen, CN, C₁-C₆-haloalkyl, and C₁-C₆-haloalkoxy;
X is X2;
R^{2A} is H, halogen, or C₁-C₆-alkyl;
R^{3B} is H or C₁-C₆-alkyl, wherein the alkyl is unsubstituted or substituted with halogen or CN; B¹ is N or CR^{B1}, B² is CR^{B2}, and B³ is CR^{B3};
R^{B1}, R^{B2}, R^{B3}, and R^{B4} independently of each other are H, halogen, or CN;
D is DA or DB;
W is S or O, preferably S;
R⁴ is H or C₁-C₆-alkyl;
Ar¹ is phenyl which is unsubstituted or substituted with R^{Ar1};
R^{Ar1} is CN, C₁-C₆-alkyl, C₃-C₆-cycloalkyl, or NR⁸R⁹ wherein alkyl and cycloalkyl moieties are unsubstituted or substituted with halogen;
R⁸ and R⁹ are, identical or different, H or C₁-C₆-alkyl.

In another preferred embodiment of compound of formula I, wherein
n is 1 or 2;
R^{1A}, R^{1B}, R^{1C} and R^{1D} independently of each other are selected from H, halogen, CN, C₁-C₆-haloalkyll and C₁-C₆-haloalkoxy;
X is X2;
R^{2A} is H, halogen, or C₁-C₆-alkyl;
R^{3B} is H or C₁-C₆-alkyl, wherein the alkyl is unsubstituted or substituted with halogen or CN; B¹ is N or CR^{B1}, B² is CR^{B2}, and B³ is CR^{B3};
R^{B1}, R^{B2}, R^{B3}, and R^{B4} independently of each other are H, halogen, or CN;
D is DC orDD;
Ar¹ is phenyl which is unsubstituted or substituted with R^{Ar1};
R^{Ar1} is CN, C₁-C₆-alkyl, C₃-C₆-cycloalkyl, NR⁸R⁹ wherein alkyl and cycloalkyl moieties are unsubstituted or substituted with halogen;
R⁸ and R⁹ are, identical or different, H or C₁-C₆-alkyl.

In another preferred embodiment of compound of formula I, wherein
n is 1 or 2;
R^{1A}, R^{1B}, R^{1C} and R^{1D} independently of each other are selected from H, halogen, CN, C₁-C₆-haloalkyl, and C₁-C₆-haloalkoxy;
X is X2;
R^{2A} is H, halogen, or C₁-C₆-alkyl;
R^{3B} is H or C₁-C₆-alkyl, wherein the alkyl is unsubstituted or substituted with halogen or CN; B¹ is N or CR^{B1}, B² is CR^{B2}, and B³ is CR^{B3};
R^{B1}, R^{B2}, R^{B3}, and R^{B4} independently of each other are H, halogen, or CN;
D is DE or DF;
R⁴ is H or C₁-C₆-alkyl;
R⁵ is C₁-C₆-alkyl, which is unsubstituted or substituted with -O-(C=O)-C₁-C₆-alkoxy or -O-(C=O)-C₁-C₆-alkyl;
Ar¹ is phenyl which is unsubstituted or substituted with R^{Ar1};
R^{Ar1} is CN, C₁-C₆-alkyl, C₃-C₆-cycloalkyl, NR⁸R⁹ wherein alkyl and cycloalkyl moieties are unsubstituted or substituted with halogen;
R⁸ and R⁹ are, identical or different, H or C₁-C₆-alkyl;

In another preferred embodiment, compounds of formula I are selected from compounds of formulae A.1 to A.120, wherein the variables are as defined herein above, 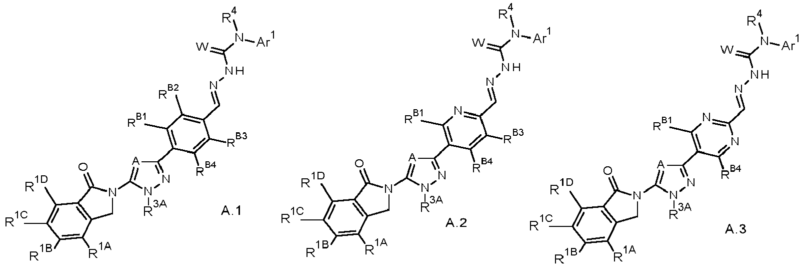 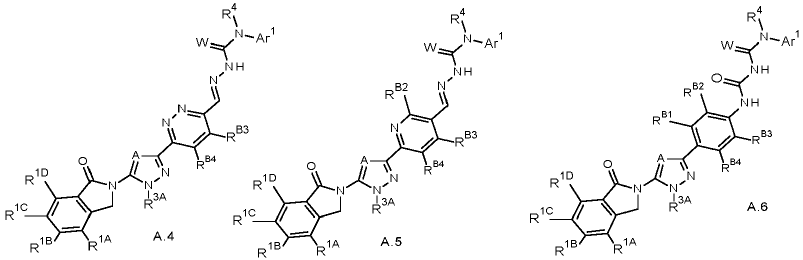 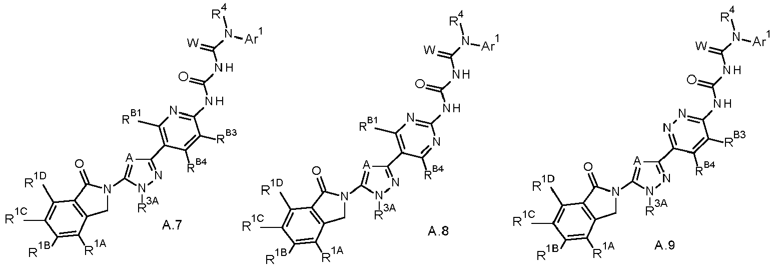 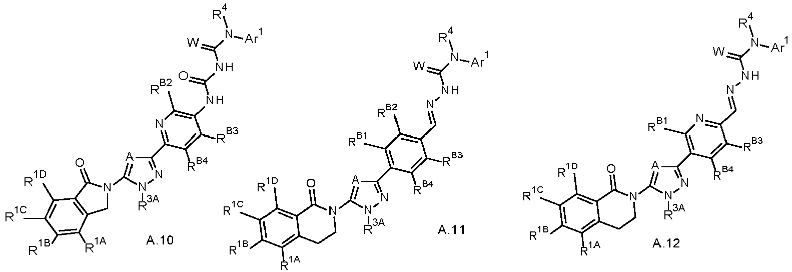 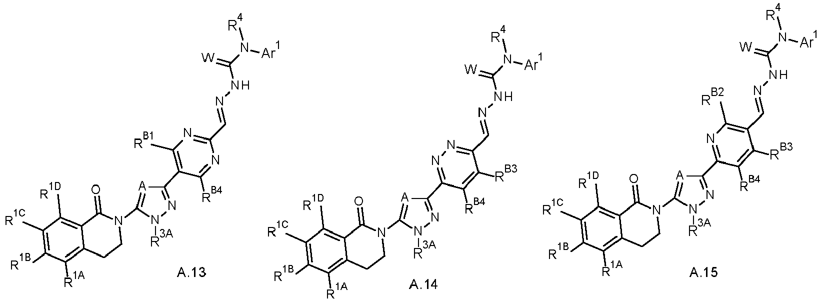 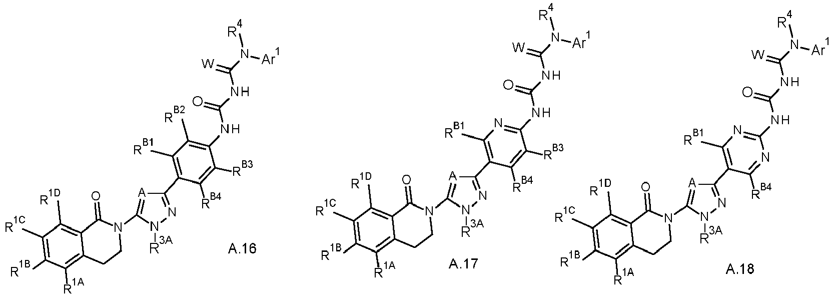 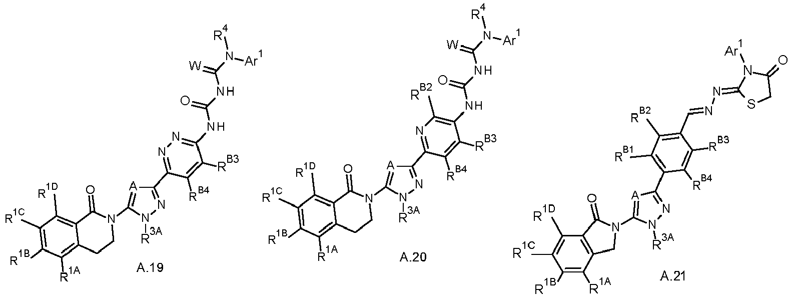 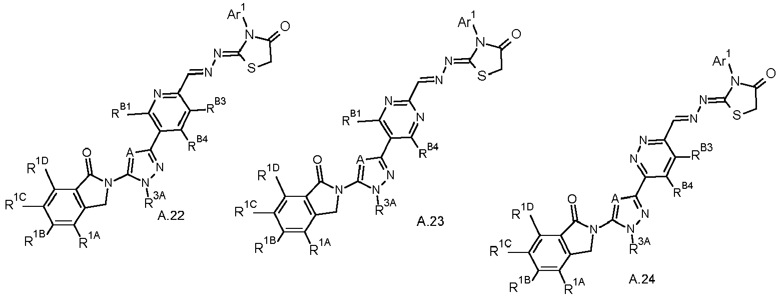 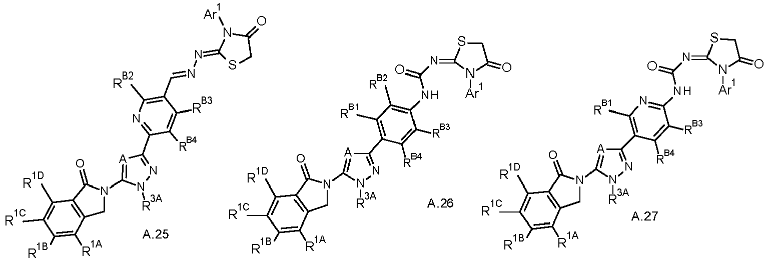 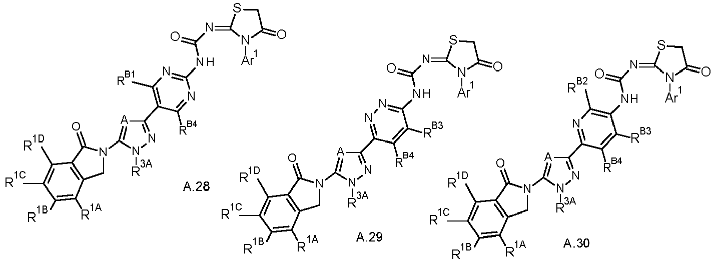 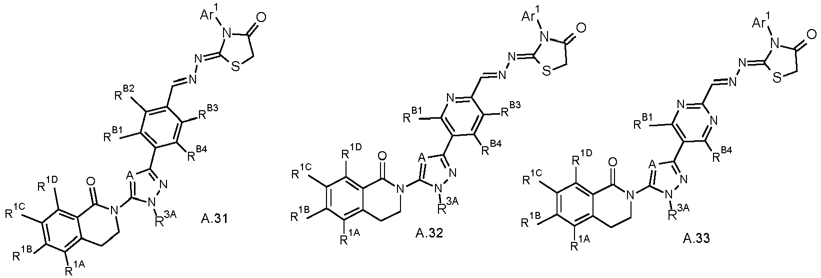 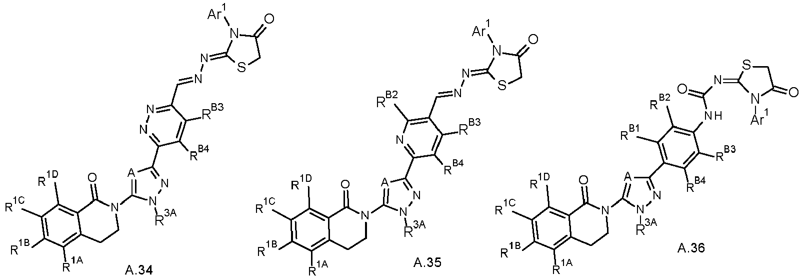 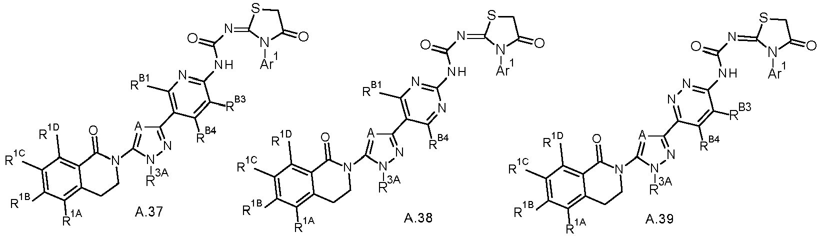 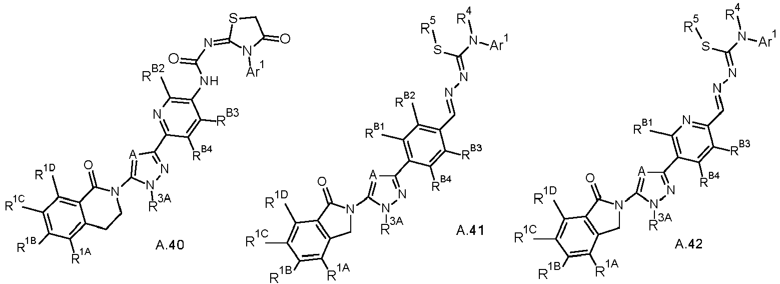 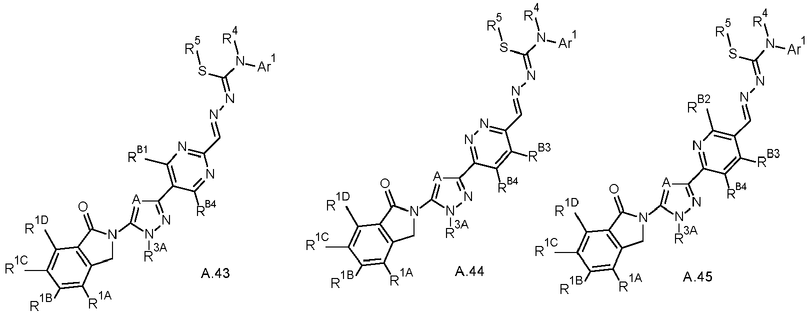 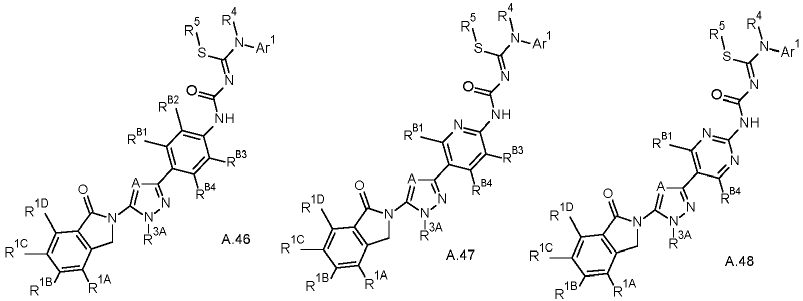 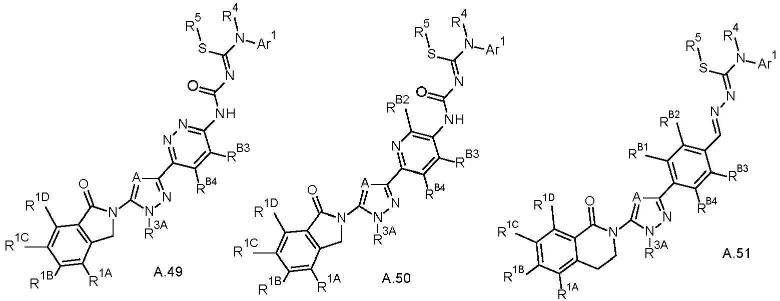 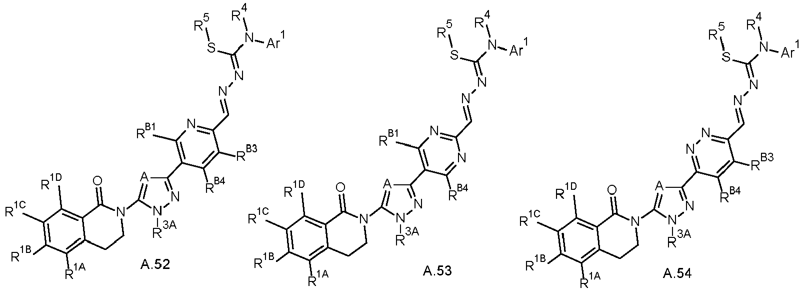 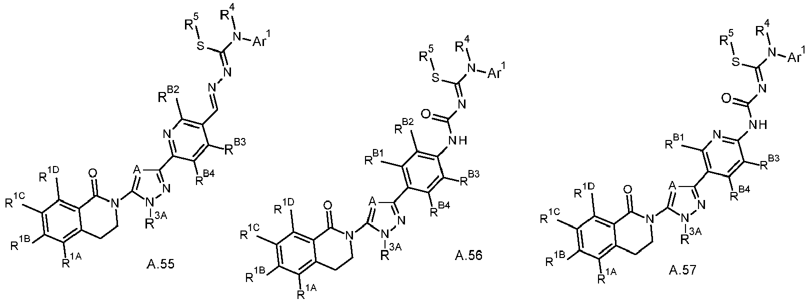 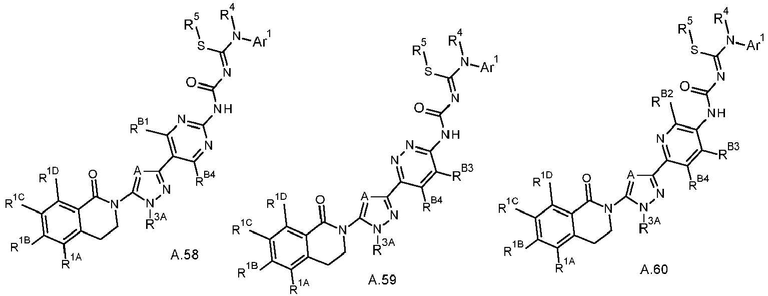 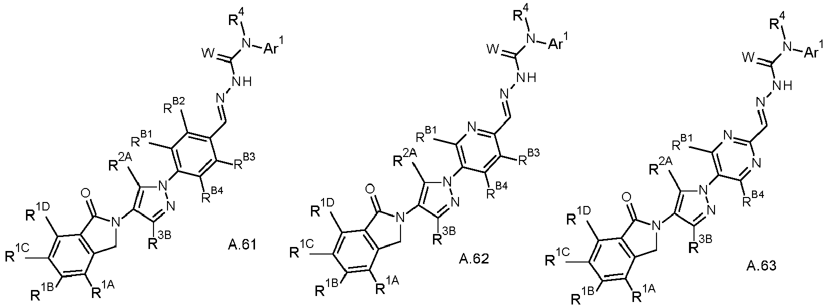 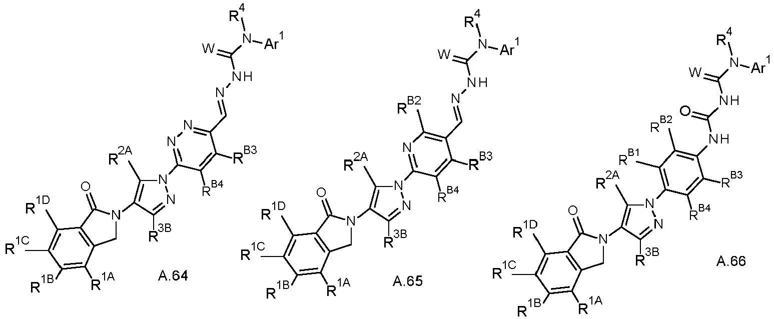 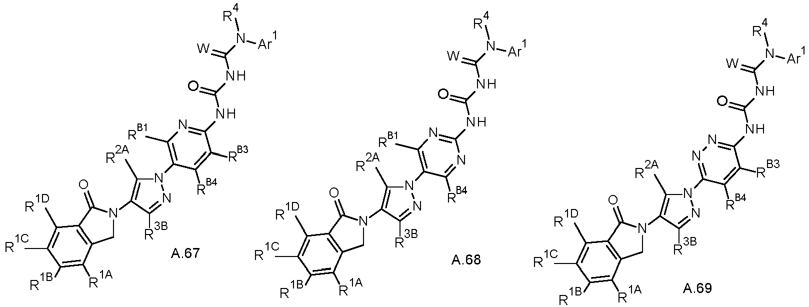 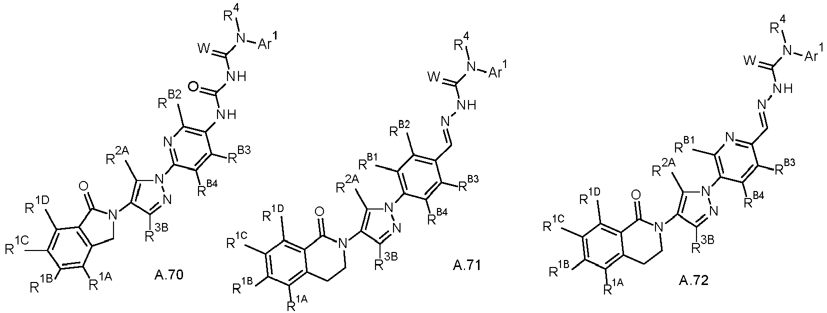 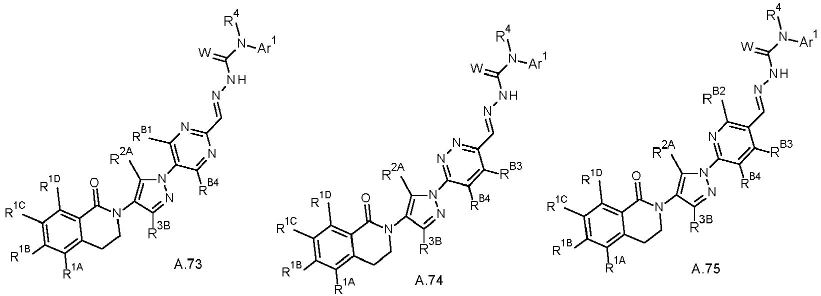 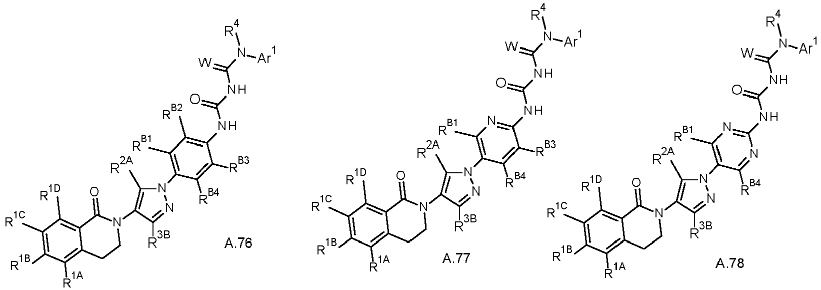 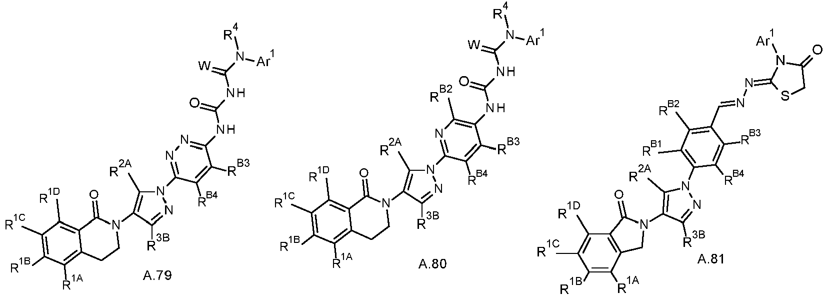 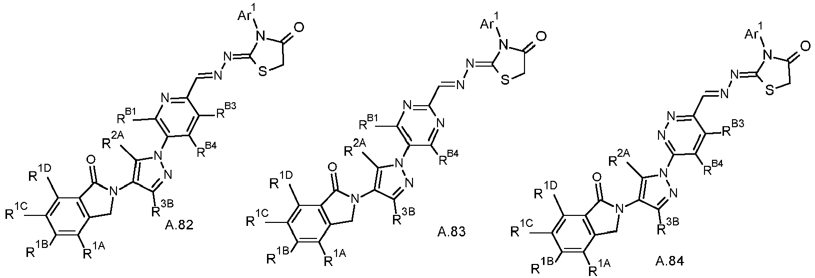 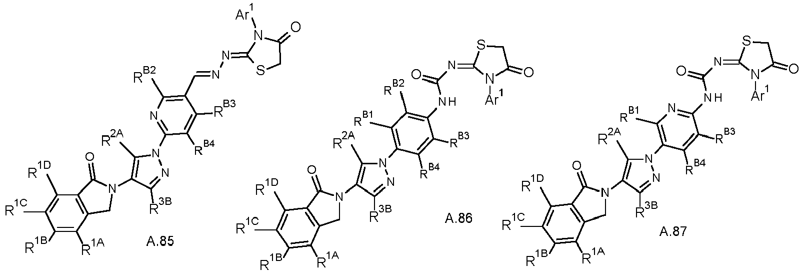 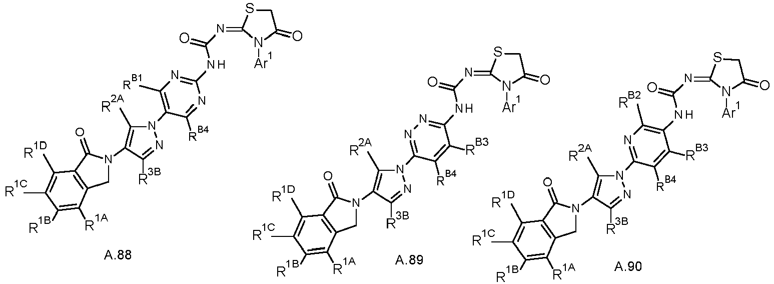 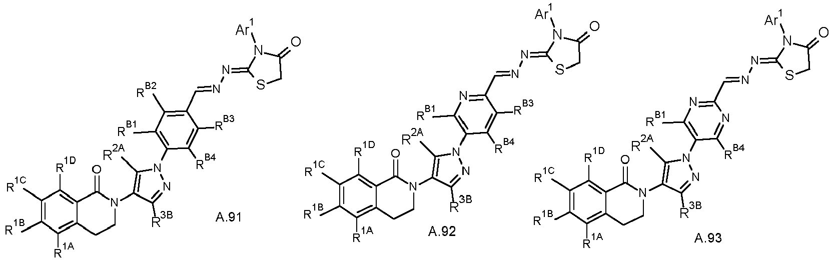 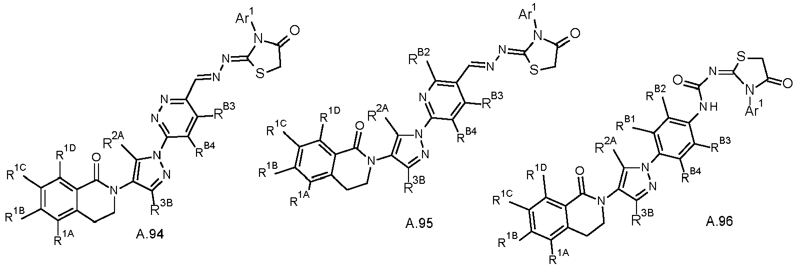 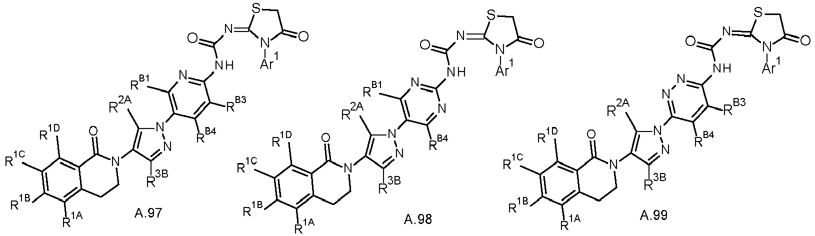 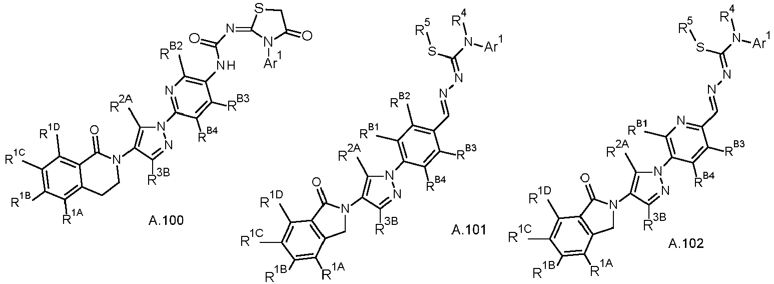 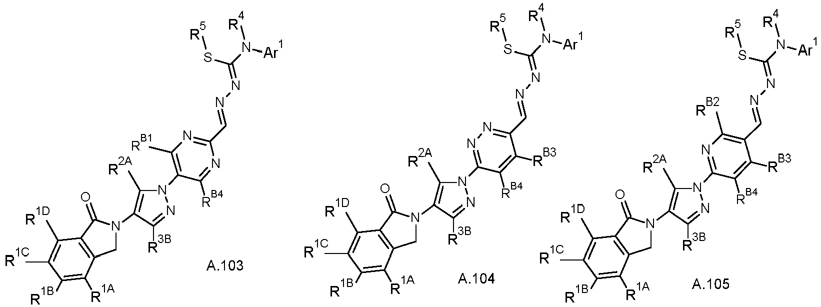 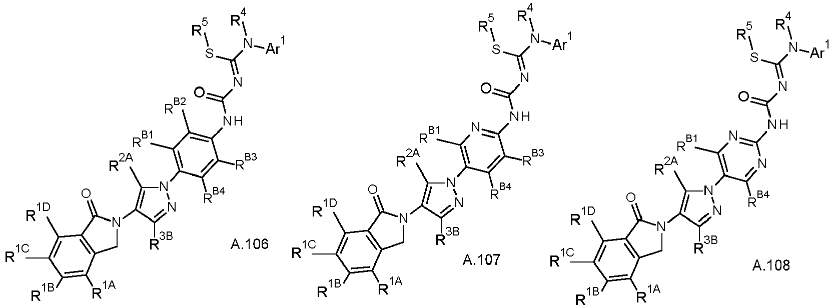 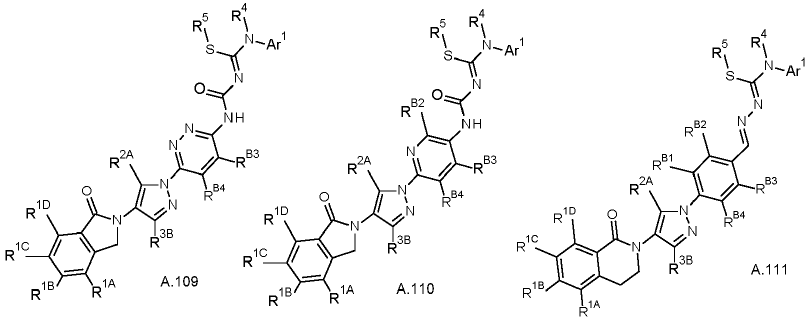 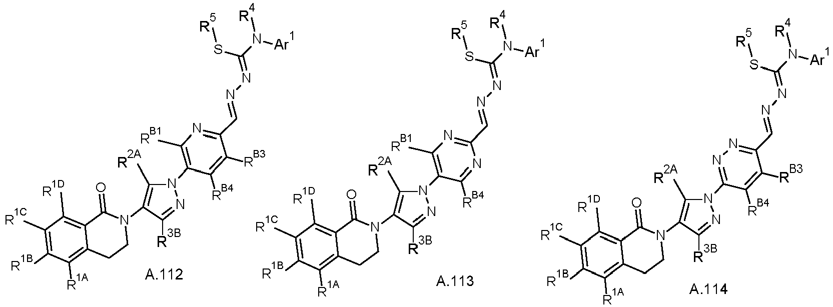 In another preferred embodiment, compounds of formula I are selected from compounds of formula A.1 to A.60;
In another preferred embodiment, compounds of formula I are selected from compounds of formula A.1 to A.20;
In another preferred embodiment, compounds of formula I are selected from compounds of formula A.1, A.6, A.11, and A.16;
In another preferred embodiment, compounds of formula I are selected from compounds of formula A.21 to A.40;
In another preferred embodiment, compounds of formula I are selected from compounds of formula A.21, A.26, A.31, and A.36;
In another preferred embodiment compounds of formula I are selected from compounds of formula A.41 to A.60
In another preferred embodiment, compounds of formula I are selected from compounds of formula A.41, A.46, A.51, and A.56;
In another preferred embodiment, compounds of formula I are selected from compounds of formula A.1, A.6, A.11, A.16, A.21, A.26, A.31, A.36, A.41, A.46, A.51, and A.56;
In another preferred embodiment, compounds of formula I are selected from compounds of formula A.21, A.26, A.31, and A.36;
In another preferred embodiment, compounds of formula I are selected from compounds of formula A.26, and A.36;
In another preferred embodiment, compounds of formula I are selected from compounds of formula A.61 to A.120;
In another preferred embodiment, compounds of formula I are selected from compounds of formula A.61 to A.80;
In another preferred embodiment, compounds of formula I are selected from compounds of formula A.61, A.66, A.71, and A.76;
In another preferred embodiment, compounds of formula I are selected from compounds of formula A.81 to A.100;
In another preferred embodiment, compounds of formula I are selected from compounds of formula A.81, A.86, A.91, and A.96;
In another preferred embodiment compounds of formula I are selected from compounds of formula A.101 to A.120
In another preferred embodiment, compounds of formula I are selected from compounds of formula A.101, A.106, A.111, and A.116;
In another preferred embodiment, compounds of formula I are selected from compounds of formula A.61, A.66, A.71, A.66, A.81, A.86, A.91, A.96, A.101, A.106, A.111, and A.116;
In another preferred embodiment, compounds of formula I are selected from compounds of formula A.81, A.86, A.91, and A.96;
In another preferred embodiment, compounds of formula I are selected from compounds of formula A.86, and A.96;
In another preferred embodiment, compounds of formula I are selected from compounds of formula A.1, A.6, A.11, A.16, A.21, A.26, A.31, A.36, A.41, A.46, A.51, A.56, A.61, A.66, A.71, A.66, A.81, A.86, A.91, A.96, A.101, A.106, A.111, and A.116;
In another preferred embodiment, compounds of formula I are selected from compounds of formula A.21, A.26, A.31, A.36, A.81, A.86, A.91, and A.96;
In another preferred embodiment, compounds of formula I are selected from compounds of formula A.26, A.36, A.86, and A.96;
In another preferred embodiment compounds of formula I are selected from compounds of formula A.21, A.26, A.31, and A.36, wherein
R^{1A}, R^{1B}, R^{1C} and R^{1D} independently of each other are selected from H, halogen, CN, C₁-C₆-haloalkyl, and C₁-C₆-haloalkoxy;
A is N or CR^{2A};
R^{2A} is H, halogen, or C₁-C₆-alkyl;
R^{3A} is H or C₁-C₆-alkyl, wherein the alkyl is unsubstituted or substituted with halogen or C₁-C₆-alkoxy;
R^{B1}, R^{B2}, R^{B3}, and R^{B4} independently of each other are H, halogen, or CN;
Ar¹ is phenyl which is unsubstituted or substituted with R^{Ar1};
R^{Ar1} is CN, C₁-C₆-alkyl, C₃-C₆-cycloalkyl, NR⁸R⁹ wherein alkyl and cycloalkyl moieties are unsubstituted or substituted with halogen;
R⁸ and R⁹ are, identical or different, H or C₁-C₆-alkyl.
In another preferred embodiment compounds of formula I are selected from compounds of formula A.21, A.26, A.31, and A.36, wherein
R^{1A}, R^{1B}, R^{1C} and R^{1D} independently of each other are selected from H, CI, F, Br, CN, CF₃, and OCF₃;
A is N, C(CH₃), or CH;
R^{3A} is C₁-C₆-alkyl;
R^{B1}, R^{B2}, R^{B3}, and R^{B4} independently of each other are H, halogen, or CN;
Ar¹ is Ar¹-2 to Ar¹-8;
In another preferred embodiment, compounds of formula I are selected from compounds of formula A.81, A.86, A.91, and A.96;
R^{1A}, R^{1B}, R^{1C} and R^{1D} independently of each other are selected from H, halogen, CN, C₁-C₆-haloalkyl, and C₁-C₆-haloalkoxy;
R^{2A} is H, halogen, or C₁-C₆-alkyl;
R^{3B} is H or C₁-C₆-alkyl, wherein the alkyl is unsubstituted or substituted with halogen or CN;
R^{B1}, R^{B2}, R^{B3}, and R^{B4} independently of each other are H, halogen, or CN;
Ar¹ is phenyl which is unsubstituted or substituted with R^{Ar1};
R^{Ar1} is CN, C₁-C₆-alkyl, C₃-C₆-cycloalkyl, NR⁸R⁹ wherein alkyl and cycloalkyl moieties are unsubstituted or substituted with halogen;
R⁸ and R⁹ are, identical or different, H or C₁-C₆-alkyl;
In another preferred embodiment, compounds of formula I are selected from compounds of formula A.81, A.86, A.91, and A.96;
R^{1A}, R^{1B}, R^{1C} and R^{1D} independently of each other are selected from H, CI, F, CN, CF₃, and OCF₃;
R^{2A} is H, halogen, or C₁-C₆-alkyl;
R^{3B} is C₁-C₆-alkyl;
R^{B1}, R^{B2}, R^{B3}, and R^{B4} independently of each other are H, halogen, or CN;
R^{Ar1} is Ar¹-2 to Ar¹-8;
In another preferred embodiment, the compound of formula I is selected from the compounds of formulae I-1 to I-20, wherein the variables are as defined herein 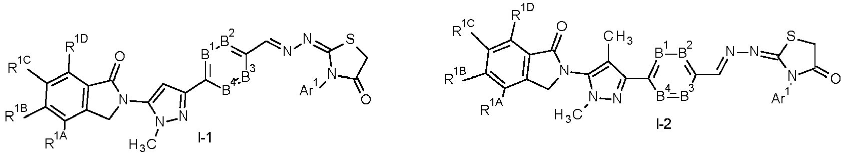 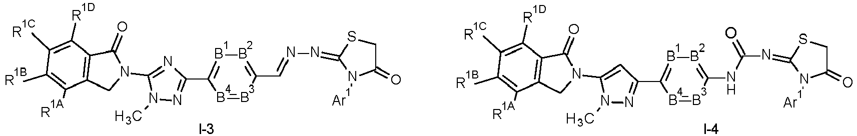 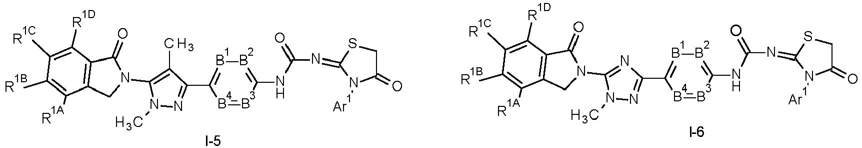 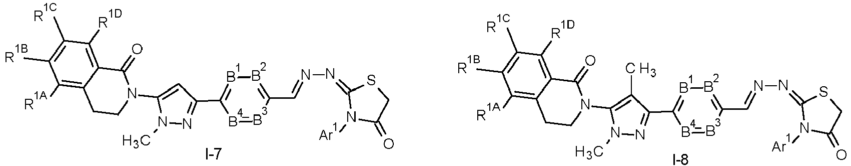 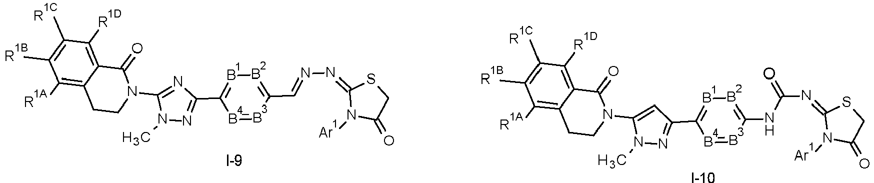 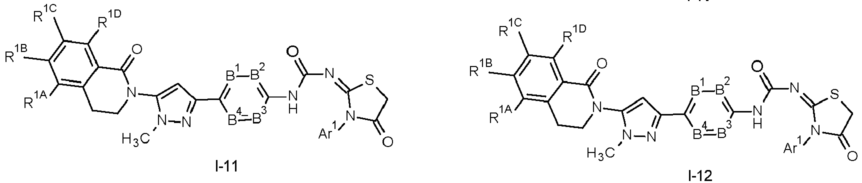 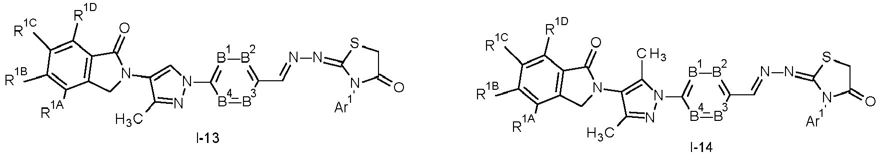 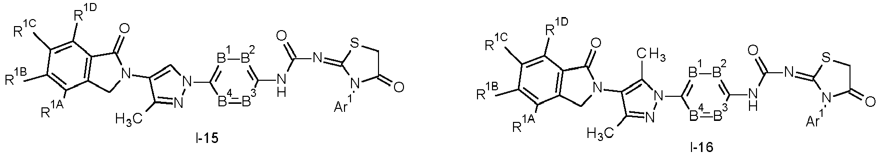 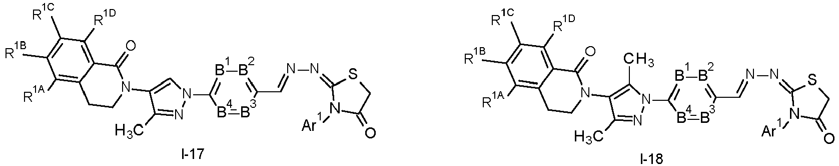 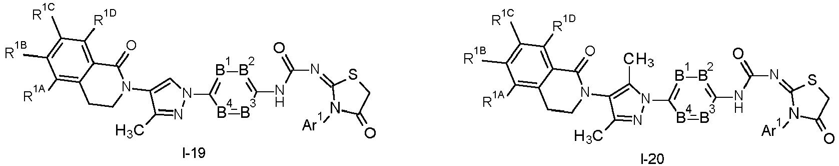 In another preferred embodiment, the compound of formula I is selected from the compounds of formulae I-1 to I-12;
In another preferred embodiment, the compound of formula I is selected from the compounds of formulae I-13 to I-20;
In another preferred embodiment, the compound of formula I is selected from the compounds of formulae I-1 to I-6;
In another preferred embodiment, the compound of formula I is selected from the compounds of formulae I-4 and I-5;
In another preferred embodiment, the compound of formula I is selected from the compounds of formulae I-4, I-5, I-6, I-10, I-11,and I-12;
In another preferred embodiment, the compound of formula I is selected from the compounds of formulae I-7 to I-12;
In another preferred embodiment, the compound of formula I is selected from the compounds of formulae I-13 to !-16;
In another preferred embodiment, the compound of formula I is selected from the compounds of formulae I-17 to I-20;
In another preferred embodiment, the compound of formula I is selected from the compounds of formulae I-15, I-16, I-19, and I-20;
In another preferred embodiment, the compound of formula I is compound of formula I-1 to I-20, wherein
R^{1A}, R^{1B}, R^{1C} and R^{1D} independently of each other are selected from H, halogen, CN, C₁-C₆-haloalkyl, and C₁-C₆-haloalkoxy;
B¹ is N or CR^{B1};
B² is N or CR^{B2};
B³ is N or CR^{B3};
B⁴ is CR^{B4};
R^{B1}, R^{B2}, R^{B3}, and R^{B4} independently of each other are selected from H, halogen, CN, and C₁-C₆-alkyl, preferably CH₃;
Ar¹ is selected from Ar¹-2 to Ar¹-8;
Particular compounds of formula I are the compounds that are compiled in the following tables 1 to 42 and the tables 43 to 840, wherein the combination of variables B¹, B², B³, and B⁴ for each compound corresponds to each line of Table B. Each of the groups mentioned for a substituent in the tables is furthermore per se, independently of the combination in which it is mentioned, a particularly preferred aspect of the substituent in question.
Table 1. Compounds of formula I-1 where R^{1A} is H, R^{1B} is H, R^{1C} is H, R^{1D} is H, Ar¹ is Ar¹-2,
Table 2. Compounds of formula I-1 where R^{1A} is H, R^{1B} is H, R^{1C} is H, R^{1D} is H, Ar¹ is Ar¹-3.
Table 3. Compounds of formula I-1 where R^{1A} is H, R^{1B} is H, R^{1C} is H, R^{1D} is H, Ar¹ is Ar¹-4.
Table 4. Compounds of formula I-1 where R^{1A} is H, R^{1B} is H, R^{1C} is H, R^{1D} is H, Ar¹ is Ar¹-5.
Table 5. Compounds of formula I-1 where R^{1A} is H, R^{1B} is H, R^{1C} is H, R^{1D} is H, Ar¹ is Ar¹-6.
Table 6. Compounds of formula I-1 where R^{1A} is H, R^{1B} is H, R^{1C} is H, R^{1D} is H, Ar¹ is Ar¹-7.
Table 7. Compounds of formula I-1 where R^{1A} is H, R^{1B} is H, R^{1C} is H, R^{1D} is H, Ar¹ is Ar¹-8.
Table 8. Compounds of formula I-1 where R^{1A} is H, R^{1B} is OCF₃, R^{1C} is H, R^{1D} is H, Ar¹ is Ar¹-2,
Table 9. Compounds of formula I-1 where R^{1A} is H, R^{1B} is OCF₃, R^{1C} is H, R^{1D} is H, Ar¹ is Ar¹-3.
Table 10. Compounds of formula I-1 where R^{1A} is H, R^{1B} is OCF₃, R^{1C} is H, R^{1D} is H, Ar¹ is Ar¹-4.
Table 11. Compounds of formula I-1 where R^{1A} is H, R^{1B} is OCF₃, R^{1C} is H, R^{1D} is H, Ar¹ is Ar¹-5.
Table 12. Compounds of formula I-1 where R^{1A} is H, R^{1B} is OCF₃, R^{1C} is H, R^{1D} is H, Ar¹ is Ar¹-6.
Table 13. Compounds of formula I-1 where R^{1A} is H, R^{1B} is OCF₃, R^{1C} is H, R^{1D} is H, Ar¹ is Ar¹-7.
Table 14. Compounds of formula I-1 where R^{1A} is H, R^{1B} is OCF₃, R^{1C} is H, R^{1D} is H, Ar¹ is Ar¹-8.
Table 15. Compounds of formula I-1 where R^{1A} is H, R^{1B} is CF₃, R^{1C} is H, R^{1D} is H, Ar¹ is Ar¹-2,
Table 16. Compounds of formula I-1 where R^{1A} is H, R^{1B} is CF₃, R^{1C} is H, R^{1D} is H, Ar¹ is Ar¹-3.
Table 17. Compounds of formula I-1 where R^{1A} is H, R^{1B} is CF₃, R^{1C} is H, R^{1D} is H, Ar¹ is Ar¹-4.
Table 18. Compounds of formula I-1 where R^{1A} is H, R^{1B} is CF₃, R^{1C} is H, R^{1D} is H, Ar¹ is Ar¹-5.
Table 19. Compounds of formula I-1 where R^{1A} is H, R^{1B} is CF₃, R^{1C} is H, R^{1D} is H, Ar¹ is Ar¹-6.
Table 20. Compounds of formula I-1 where R^{1A} is H, R^{1B} is CF₃, R^{1C} is H, R^{1D} is H, Ar¹ is Ar¹-7.
Table 21. Compounds of formula I-1 where R^{1A} is H, R^{1B} is CF₃, R^{1C} is H, R^{1D} is H, Ar¹ is Ar¹-8.
Table 22. Compounds of formula I-1 where R^{1A} is H, R^{1B} is CI, R^{1C} is H, R^{1D} is H, Ar¹ is Ar¹-2,
Table 23. Compounds of formula I-1 where R^{1A} is H, R^{1B} is CI, R^{1C} is H, R^{1D} is H, Ar¹ is Ar¹-3.
Table 24. Compounds of formula I-1 where R^{1A} is H, R^{1B} is Cl, R^{1C} is H, R^{1D} is H, Ar¹ is Ar¹-4.
Table 25. Compounds of formula I-1 where R^{1A} is H, R^{1B} is CI, R^{1C} is H, R^{1D} is H, Ar¹ is Ar¹-5.
Table 26. Compounds of formula I-1 where R^{1A} is H, R^{1B} is Cl, R^{1C} is H, R^{1D} is H, Ar¹ is Ar¹-6.
Table 27. Compounds of formula I-1 where R^{1A} is H, R^{1B} is CI, R^{1C} is H, R^{1D} is H, Ar¹ is Ar¹-7.
Table 28. Compounds of formula I-1 where R^{1A} is H, R^{1B} is CI, R^{1C} is H, R^{1D} is H, Ar¹ is Ar¹-8.
Table 29. Compounds of formula I-1 where R^{1A} is H, R^{1B} is F, R^{1C} is H, R^{1D} is H, Ar¹ is Ar¹-2,
Table 30. Compounds of formula I-1 where R^{1A} is H, R^{1B} is F, R^{1C} is H, R^{1D} is H, Ar¹ is Ar¹-3.
Table 31. Compounds of formula I-1 where R^{1A} is H, R^{1B} is F, R^{1C} is H, R^{1D} is H, Ar¹ is Ar¹-4.
Table 32. Compounds of formula I-1 where R^{1A} is H, R^{1B} is F, R^{1C} is H, R^{1D} is H, Ar¹ is Ar¹-5.
Table 33. Compounds of formula I-1 where R^{1A} is H, R^{1B} is F, R^{1C} is H, R^{1D} is H, Ar¹ is Ar¹-6.
Table 34. Compounds of formula I-1 where R^{1A} is H, R^{1B} is F, R^{1C} is H, R^{1D} is H, Ar¹ is Ar¹-7.
Table 35. Compounds of formula I-1 where R^{1A} is H, R^{1B} is F, R^{1C} is H, R^{1D} is H, Ar¹ is Ar¹-8.
Table 36. Compounds of formula I-1 where R^{1A} is H, R^{1B} is CN, R^{1C} is H, R^{1D} is H, Ar¹ is Ar¹-2,
Table 37. Compounds of formula I-1 where R^{1A} is H, R^{1B} is CN, R^{1C} is H, R^{1D} is H, Ar¹ is Ar¹-3.
Table 38. Compounds of formula I-1 where R^{1A} is H, R^{1B} is CN, R^{1C} is H, R^{1D} is H, Ar¹ is Ar¹-4.
Table 39. Compounds of formula I-1 where R^{1A} is H, R^{1B} is CN, R^{1C} is H, R^{1D} is H, Ar¹ is Ar¹-5.
Table 40. Compounds of formula I-1 where R^{1A} is H, R^{1B} is CN, R^{1C} is H, R^{1D} is H, Ar¹ is Ar¹-6.
Table 41. Compounds of formula I-1 where R^{1A} is H, R^{1B} is CN, R^{1C} is H, R^{1D} is H, Ar¹ is Ar¹-7.
Table 42. Compounds of formula I-1 where R^{1A} is H, R^{1B} is CN, R^{1C} is H, R^{1D} is H, Ar¹ is Ar¹-8.

**Table B:**

| **Line** | **B¹** | **B²** | **B³** | **B⁴** |
|---|---|---|---|---|
| 1. | N | N | CH | CH |
| 2. | N | N | CH | C-Cl |
| 3. | N | N | CH | C-F |
| 4. | N | N | CH | C-Br |
| 5. | N | N | CH | C-CH₃ |
| 6. | N | N | C-CI | CH |
| 7. | N | N | C-Cl | C-Cl |
| 8. | N | N | C-Cl | C-F |
| 9. | N | N | C-Cl | C-Br |
| 10. | N | N | C-CI | C-CH₃ |
| 11. | N | N | C-F | CH |
| 12. | N | N | C-F | C-CI |
| 13. | N | N | C-F | C-F |
| 14. | N | N | C-F | C-Br |
| 15. | N | N | C-F | C-CH₃ |
| 16. | N | N | C-Br | CH |
| 17. | N | N | C-Br | C-Cl |
| 18. | N | N | C-Br | C-F |
| 19. | N | N | C-Br | C-Br |
| 20. | N | N | C-Br | C-CH₃ |
| 21. | N | N | C-CH₃ | CH |
| 22. | N | N | C-CH₃ | C-Cl |
| 23. | N | N | C-CH₃ | C-F |
| 24. | N | N | C-CH₃ | C-Br |
| 25. | N | N | C-CH₃ | C-CH₃ |
| 26. | N | CH | N | CH |
| 27. | N | CH | N | C-CI |
| 28. | N | CH | N | C-F |
| 29. | N | CH | N | C-Br |
| 30. | N | CH | N | C-CH₃ |
| 31. | N | CH | CH | CH |
| 32. | N | CH | CH | C-Cl |
| 33. | N | CH | CH | C-F |
| 34. | N | CH | CH | C-Br |
| 35. | N | CH | CH | C-CH₃ |
| 36. | N | CH | C-Cl | CH |
| 37. | N | CH | C-Cl | C-CI |
| 38. | N | CH | C-Cl | C-F |
| 39. | N | CH | C-CI | C-Br |
| 40. | N | CH | C-Cl | C-CH₃ |
| 41. | N | CH | C-F | CH |
| 42. | N | CH | C-F | C-CI |
| 43. | N | CH | C-F | C-F |
| 44. | N | CH | C-F | C-Br |
| 45. | N | CH | C-F | C-CH₃ |
| 46. | N | CH | C-Br | CH |
| 47. | N | CH | C-Br | C-CI |
| 48. | N | CH | C-Br | C-F |
| 49. | N | CH | C-Br | C-Br |
| 50. | N | CH | C-Br | C-CH₃ |
| 51. | N | CH | C-CH₃ | CH |
| 52. | N | CH | C-CH₃ | C-Cl |
| 53. | N | CH | C-CH₃ | C-F |
| 54. | N | CH | C-CH₃ | C-Br |
| 55. | N | CH | C-CH₃ | C-CH₃ |
| 56. | N | C-Cl | N | CH |
| 57. | N | C-Cl | N | C-Cl |
| 58. | N | C-Cl | N | C-F |
| 59. | N | C-Cl | N | C-Br |
| 60. | N | C-Cl | N | C-CH₃ |
| 61. | N | C-CI | CH | CH |
| 62. | N | C-Cl | CH | C-Cl |
| 63. | N | C-Cl | CH | C-F |
| 64. | N | C-Cl | CH | C-Br |
| 65. | N | C-CI | CH | C-CH₃ |
| 66. | N | C-Cl | C-Cl | CH |
| 67. | N | C-Cl | C-CI | C-CI |
| 68. | N | C-Cl | C-Cl | C-F |
| 69. | N | C-Cl | C-Cl | C-Br |
| 70. | N | C-CI | C-CI | C-CH₃ |
| 71. | N | C-CI | C-F | CH |
| 72. | N | C-Cl | C-F | C-Cl |
| 73. | N | C-Cl | C-F | C-F |
| 74. | N | C-Cl | C-F | C-Br |
| 75. | N | C-CI | C-F | C-CH₃ |
| 76. | N | C-Cl | C-Br | CH |
| 77. | N | C-Cl | C-Br | C-CI |
| 78. | N | C-Cl | C-Br | C-F |
| 79. | N | C-Cl | C-Br | C-Br |
| 80. | N | C-CI | C-Br | C-CH₃ |
| 81. | N | C-Cl | C-CH₃ | CH |
| 82. | N | C-Cl | C-CH₃ | C-Cl |
| 83. | N | C-Cl | C-CH₃ | C-F |
| 84. | N | C-Cl | C-CH₃ | C-Br |
| 85. | N | C-CI | C-CH₃ | C-CH₃ |
| 86. | N | C-F | N | CH |
| 87. | N | C-F | N | C-Cl |
| 88. | N | C-F | N | C-F |
| 89. | N | C-F | N | C-Br |
| 90. | N | C-F | N | C-CH₃ |
| 91. | N | C-F | CH | CH |
| 92. | N | C-F | CH | C-Cl |
| 93. | N | C-F | CH | C-F |
| 94. | N | C-F | CH | C-Br |
| 95. | N | C-F | CH | C-CH₃ |
| 96. | N | C-F | C-Cl | CH |
| 97. | N | C-F | C-Cl | C-CI |
| 98. | N | C-F | C-Cl | C-F |
| 99. | N | C-F | C-Cl | C-Br |
| 100. | N | C-F | C-CI | C-CH₃ |
| 101. | N | C-F | C-F | CH |
| 102. | N | C-F | C-F | C-CI |
| 103. | N | C-F | C-F | C-F |
| 104. | N | C-F | C-F | C-Br |
| 105. | N | C-F | C-F | C-CH₃ |
| 106. | N | C-F | C-Br | CH |
| 107. | N | C-F | C-Br | C-Cl |
| 108. | N | C-F | C-Br | C-F |
| 109. | N | C-F | C-Br | C-Br |
| 110. | N | C-F | C-Br | C-CH₃ |
| 111. | N | C-F | C-CH₃ | CH |
| 112. | N | C-F | C-CH₃ | C-CI |
| 113. | N | C-F | C-CH₃ | C-F |
| 114. | N | C-F | C-CH₃ | C-Br |
| 115. | N | C-F | C-CH₃ | C-CH₃ |
| 116. | N | C-Br | N | CH |
| 117. | N | C-Br | N | C-CI |
| 118. | N | C-Br | N | C-F |
| 119. | N | C-Br | N | C-Br |
| 120. | N | C-Br | N | C-CH₃ |
| 121. | N | C-Br | CH | CH |
| 122. | N | C-Br | CH | C-Cl |
| 123. | N | C-Br | CH | C-F |
| 124. | N | C-Br | CH | C-Br |
| 125. | N | C-Br | CH | C-CH₃ |
| 126. | N | C-Br | C-Cl | CH |
| 127. | N | C-Br | C-CI | C-CI |
| 128. | N | C-Br | C-Cl | C-F |
| 129. | N | C-Br | C-CI | C-Br |
| 130. | N | C-Br | C-Cl | C-CH₃ |
| 131. | N | C-Br | C-F | CH |
| 132. | N | C-Br | C-F | C-CI |
| 133. | N | C-Br | C-F | C-F |
| 134. | N | C-Br | C-F | C-Br |
| 135. | N | C-Br | C-F | C-CH₃ |
| 136. | N | C-Br | C-Br | CH |
| 137. | N | C-Br | C-Br | C-CI |
| 138. | N | C-Br | C-Br | C-F |
| 139. | N | C-Br | C-Br | C-Br |
| 140. | N | C-Br | C-Br | C-CH₃ |
| 141. | N | C-Br | C-CH₃ | CH |
| 142. | N | C-Br | C-CH₃ | C-Cl |
| 143. | N | C-Br | C-CH₃ | C-F |
| 144. | N | C-Br | C-CH₃ | C-Br |
| 145. | N | C-Br | C-CH₃ | C-CH₃ |
| 146. | N | C-CH₃ | N | CH |
| 147. | N | C-CH₃ | N | C-Cl |
| 148. | N | C-CH₃ | N | C-F |
| 149. | N | C-CH₃ | N | C-Br |
| 150. | N | C-CH₃ | N | C-CH₃ |
| 151. | N | C-CH₃ | CH | CH |
| 152. | N | C-CH₃ | CH | C-CI |
| 153. | N | C-CH₃ | CH | C-F |
| 154. | N | C-CH₃ | CH | C-Br |
| 155. | N | C-CH₃ | CH | C-CH₃ |
| 156. | N | C-CH₃ | C-CI | CH |
| 157. | N | C-CH₃ | C-CI | C-Cl |
| 158. | N | C-CH₃ | C-CI | C-F |
| 159. | N | C-CH₃ | C-CI | C-Br |
| 160. | N | C-CH₃ | C-Cl | C-CH₃ |
| 161. | N | C-CH₃ | C-F | CH |
| 162. | N | C-CH₃ | C-F | C-Cl |
| 163. | N | C-CH₃ | C-F | C-F |
| 164. | N | C-CH₃ | C-F | C-Br |
| 165. | N | C-CH₃ | C-F | C-CH₃ |
| 166. | N | C-CH₃ | C-Br | CH |
| 167. | N | C-CH₃ | C-Br | C-Cl |
| 168. | N | C-CH₃ | C-Br | C-F |
| 169. | N | C-CH₃ | C-Br | C-Br |
| 170. | N | C-CH₃ | C-Br | C-CH₃ |
| 171. | N | C-CH₃ | C-CH₃ | CH |
| 172. | N | C-CH₃ | C-CH₃ | C-Cl |
| 173. | N | C-CH₃ | C-CH₃ | C-F |
| 174. | N | C-CH₃ | C-CH₃ | C-Br |
| 175. | N | C-CH₃ | C-CH₃ | C-CH₃ |
| 176. | CH | N | N | CH |
| 177. | CH | N | N | C-Cl |
| 178. | CH | N | N | C-F |
| 179. | CH | N | N | C-Br |
| 180. | CH | N | N | C-CH₃ |
| 181. | CH | N | CH | CH |
| 182. | CH | N | CH | C-Cl |
| 183. | CH | N | CH | C-F |
| 184. | CH | N | CH | C-Br |
| 185. | CH | N | CH | C-CH₃ |
| 186. | CH | N | C-Cl | CH |
| 187. | CH | N | C-Cl | C-CI |
| 188. | CH | N | C-Cl | C-F |
| 189. | CH | N | C-Cl | C-Br |
| 190. | CH | N | C-Cl | C-CH₃ |
| 191. | CH | N | C-F | CH |
| 192. | CH | N | C-F | C-Cl |
| 193. | CH | N | C-F | C-F |
| 194. | CH | N | C-F | C-Br |
| 195. | CH | N | C-F | C-CH₃ |
| 196. | CH | N | C-Br | CH |
| 197. | CH | N | C-Br | C-Cl |
| 198. | CH | N | C-Br | C-F |
| 199. | CH | N | C-Br | C-Br |
| 200. | CH | N | C-Br | C-CH₃ |
| 201. | CH | N | C-CH₃ | CH |
| 202. | CH | N | C-CH₃ | C-Cl |
| 203. | CH | N | C-CH₃ | C-F |
| 204. | CH | N | C-CH₃ | C-Br |
| 205. | CH | N | C-CH₃ | C-CH₃ |
| 206. | CH | CH | N | CH |
| 207. | CH | CH | N | C-CI |
| 208. | CH | CH | N | C-F |
| 209. | CH | CH | N | C-Br |
| 210. | CH | CH | N | C-CH₃ |
| 211. | CH | CH | CH | CH |
| 212. | CH | CH | CH | C-CI |
| 213. | CH | CH | CH | C-F |
| 214. | CH | CH | CH | C-Br |
| 215. | CH | CH | CH | C-CH₃ |
| 216. | CH | CH | C-Cl | CH |
| 217. | CH | CH | C-CI | C-CI |
| 218. | CH | CH | C-Cl | C-F |
| 219. | CH | CH | C-CI | C-Br |
| 220. | CH | CH | C-Cl | C-CH₃ |
| 221. | CH | CH | C-F | CH |
| 222. | CH | CH | C-F | C-CI |
| 223. | CH | CH | C-F | C-F |
| 224. | CH | CH | C-F | C-Br |
| 225. | CH | CH | C-F | C-CH₃ |
| 226. | CH | CH | C-Br | CH |
| 227. | CH | CH | C-Br | C-CI |
| 228. | CH | CH | C-Br | C-F |
| 229. | CH | CH | C-Br | C-Br |
| 230. | CH | CH | C-Br | C-CH₃ |
| 231. | CH | CH | C-CH₃ | CH |
| 232. | CH | CH | C-CH₃ | C-CI |
| 233. | CH | CH | C-CH₃ | C-F |
| 234. | CH | CH | C-CH₃ | C-Br |
| 235. | CH | CH | C-CH₃ | C-CH₃ |
| 236. | CH | C-Cl | N | CH |
| 237. | CH | C-Cl | N | C-CI |
| 238. | CH | C-Cl | N | C-F |
| 239. | CH | C-Cl | N | C-Br |
| 240. | CH | C-Cl | N | C-CH₃ |
| 241. | CH | C-Cl | CH | CH |
| 242. | CH | C-CI | CH | C-CI |
| 243. | CH | C-Cl | CH | C-F |
| 244. | CH | C-Cl | CH | C-Br |
| 245. | CH | C-Cl | CH | C-CH₃ |
| 246. | CH | C-Cl | C-Cl | CH |
| 247. | CH | C-Cl | C-Cl | C-CI |
| 248. | CH | C-Cl | C-Cl | C-F |
| 249. | CH | C-Cl | C-Cl | C-Br |
| 250. | CH | C-Cl | C-CI | C-CH₃ |
| 251. | CH | C-Cl | C-F | CH |
| 252. | CH | C-Cl | C-F | C-CI |
| 253. | CH | C-Cl | C-F | C-F |
| 254. | CH | C-Cl | C-F | C-Br |
| 255. | CH | C-Cl | C-F | C-CH₃ |
| 256. | CH | C-Cl | C-Br | CH |
| 257. | CH | C-Cl | C-Br | C-CI |
| 258. | CH | C-Cl | C-Br | C-F |
| 259. | CH | C-Cl | C-Br | C-Br |
| 260. | CH | C-Cl | C-Br | C-CH₃ |
| 261. | CH | C-Cl | C-CH₃ | CH |
| 262. | CH | C-Cl | C-CH₃ | C-Cl |
| 263. | CH | C-Cl | C-CH₃ | C-F |
| 264. | CH | C-Cl | C-CH₃ | C-Br |
| 265. | CH | C-Cl | C-CH₃ | C-CH₃ |
| 266. | CH | C-F | N | CH |
| 267. | CH | C-F | N | C-CI |
| 268. | CH | C-F | N | C-F |
| 269. | CH | C-F | N | C-Br |
| 270. | CH | C-F | N | C-CH₃ |
| 271. | CH | C-F | CH | CH |
| 272. | CH | C-F | CH | C-CI |
| 273. | CH | C-F | CH | C-F |
| 274. | CH | C-F | CH | C-Br |
| 275. | CH | C-F | CH | C-CH₃ |
| 276. | CH | C-F | C-Cl | CH |
| 277. | CH | C-F | C-Cl | C-CI |
| 278. | CH | C-F | C-Cl | C-F |
| 279. | CH | C-F | C-Cl | C-Br |
| 280. | CH | C-F | C-Cl | C-CH₃ |
| 281. | CH | C-F | C-F | CH |
| 282. | CH | C-F | C-F | C-CI |
| 283. | CH | C-F | C-F | C-F |
| 284. | CH | C-F | C-F | C-Br |
| 285. | CH | C-F | C-F | C-CH₃ |
| 286. | CH | C-F | C-Br | CH |
| 287. | CH | C-F | C-Br | C-Cl |
| 288. | CH | C-F | C-Br | C-F |
| 289. | CH | C-F | C-Br | C-Br |
| 290. | CH | C-F | C-Br | C-CH₃ |
| 291. | CH | C-F | C-CH₃ | CH |
| 292. | CH | C-F | C-CH₃ | C-Cl |
| 293. | CH | C-F | C-CH₃ | C-F |
| 294. | CH | C-F | C-CH₃ | C-Br |
| 295. | CH | C-F | C-CH₃ | C-CH₃ |
| 296. | CH | C-Br | N | CH |
| 297. | CH | C-Br | N | C-Cl |
| 298. | CH | C-Br | N | C-F |
| 299. | CH | C-Br | N | C-Br |
| 300. | CH | C-Br | N | C-CH₃ |
| 301. | CH | C-Br | CH | CH |
| 302. | CH | C-Br | CH | C-CI |
| 303. | CH | C-Br | CH | C-F |
| 304. | CH | C-Br | CH | C-Br |
| 305. | CH | C-Br | CH | C-CH₃ |
| 306. | CH | C-Br | C-Cl | CH |
| 307. | CH | C-Br | C-CI | C-CI |
| 308. | CH | C-Br | C-CI | C-F |
| 309. | CH | C-Br | C-CI | C-Br |
| 310. | CH | C-Br | C-Cl | C-CH₃ |
| 311. | CH | C-Br | C-F | CH |
| 312. | CH | C-Br | C-F | C-CI |
| 313. | CH | C-Br | C-F | C-F |
| 314. | CH | C-Br | C-F | C-Br |
| 315. | CH | C-Br | C-F | C-CH₃ |
| 316. | CH | C-Br | C-Br | CH |
| 317. | CH | C-Br | C-Br | C-CI |
| 318. | CH | C-Br | C-Br | C-F |
| 319. | CH | C-Br | C-Br | C-Br |
| 320. | CH | C-Br | C-Br | C-CH₃ |
| 321. | CH | C-Br | C-CH₃ | CH |
| 322. | CH | C-Br | C-CH₃ | C-Cl |
| 323. | CH | C-Br | C-CH₃ | C-F |
| 324. | CH | C-Br | C-CH₃ | C-Br |
| 325. | CH | C-Br | C-CH₃ | C-CH₃ |
| 326. | CH | C-CH₃ | N | CH |
| 327. | CH | C-CH₃ | N | C-CI |
| 328. | CH | C-CH₃ | N | C-F |
| 329. | CH | C-CH₃ | N | C-Br |
| 330. | CH | C-CH₃ | N | C-CH₃ |
| 331. | CH | C-CH₃ | CH | CH |
| 332. | CH | C-CH₃ | CH | C-Cl |
| 333. | CH | C-CH₃ | CH | C-F |
| 334. | CH | C-CH₃ | CH | C-Br |
| 335. | CH | C-CH₃ | CH | C-CH₃ |
| 336. | CH | C-CH₃ | C-CI | CH |
| 337. | CH | C-CH₃ | C-CI | C-CI |
| 338. | CH | C-CH₃ | C-CI | C-F |
| 339. | CH | C-CH₃ | C-CI | C-Br |
| 340. | CH | C-CH₃ | C-Cl | C-CH₃ |
| 341. | CH | C-CH₃ | C-F | CH |
| 342. | CH | C-CH₃ | C-F | C-CI |
| 343. | CH | C-CH₃ | C-F | C-F |
| 344. | CH | C-CH₃ | C-F | C-Br |
| 345. | CH | C-CH₃ | C-F | C-CH₃ |
| 346. | CH | C-CH₃ | C-Br | CH |
| 347. | CH | C-CH₃ | C-Br | C-CI |
| 348. | CH | C-CH₃ | C-Br | C-F |
| 349. | CH | C-CH₃ | C-Br | C-Br |
| 350. | CH | C-CH₃ | C-Br | C-CH₃ |
| 351. | CH | C-CH₃ | C-CH₃ | CH |
| 352. | CH | C-CH₃ | C-CH₃ | C-CI |
| 353. | CH | C-CH₃ | C-CH₃ | C-F |
| 354. | CH | C-CH₃ | C-CH₃ | C-Br |
| 355. | CH | C-CH₃ | C-CH₃ | C-CH₃ |
| 356. | C-Cl | N | N | CH |
| 357. | C-Cl | N | N | C-CI |
| 358. | C-Cl | N | N | C-F |
| 359. | C-Cl | N | N | C-Br |
| 360. | C-Cl | N | N | C-CH₃ |
| 361. | C-Cl | N | CH | CH |
| 362. | C-Cl | N | CH | C-Cl |
| 363. | C-Cl | N | CH | C-F |
| 364. | C-Cl | N | CH | C-Br |
| 365. | C-Cl | N | CH | C-CH₃ |
| 366. | C-Cl | N | C-Cl | CH |
| 367. | C-Cl | N | C-Cl | C-CI |
| 368. | C-Cl | N | C-Cl | C-F |
| 369. | C-Cl | N | C-Cl | C-Br |
| 370. | C-Cl | N | C-Cl | C-CH₃ |
| 371. | C-Cl | N | C-F | CH |
| 372. | C-Cl | N | C-F | C-CI |
| 373. | C-Cl | N | C-F | C-F |
| 374. | C-Cl | N | C-F | C-Br |
| 375. | C-Cl | N | C-F | C-CH₃ |
| 376. | C-Cl | N | C-Br | CH |
| 377. | C-Cl | N | C-Br | C-CI |
| 378. | C-Cl | N | C-Br | C-F |
| 379. | C-Cl | N | C-Br | C-Br |
| 380. | C-Cl | N | C-Br | C-CH₃ |
| 381. | C-Cl | N | C-CH₃ | CH |
| 382. | C-Cl | N | C-CH₃ | C-Cl |
| 383. | C-Cl | N | C-CH₃ | C-F |
| 384. | C-Cl | N | C-CH₃ | C-Br |
| 385. | C-Cl | N | C-CH₃ | C-CH₃ |
| 386. | C-Cl | CH | N | CH |
| 387. | C-Cl | CH | N | C-Cl |
| 388. | C-Cl | CH | N | C-F |
| 389. | C-Cl | CH | N | C-Br |
| 390. | C-Cl | CH | N | C-CH₃ |
| 391. | C-Cl | CH | CH | CH |
| 392. | C-Cl | CH | CH | C-CI |
| 393. | C-Cl | CH | CH | C-F |
| 394. | C-Cl | CH | CH | C-Br |
| 395. | C-Cl | CH | CH | C-CH₃ |
| 396. | C-Cl | CH | C-Cl | CH |
| 397. | C-Cl | CH | C-Cl | C-CI |
| 398. | C-Cl | CH | C-Cl | C-F |
| 399. | C-Cl | CH | C-CI | C-Br |
| 400. | C-Cl | CH | C-Cl | C-CH₃ |
| 401. | C-Cl | CH | C-F | CH |
| 402. | C-Cl | CH | C-F | C-CI |
| 403. | C-Cl | CH | C-F | C-F |
| 404. | C-Cl | CH | C-F | C-Br |
| 405. | C-Cl | CH | C-F | C-CH₃ |
| 406. | C-Cl | CH | C-Br | CH |
| 407. | C-Cl | CH | C-Br | C-CI |
| 408. | C-Cl | CH | C-Br | C-F |
| 409. | C-Cl | CH | C-Br | C-Br |
| 410. | C-Cl | CH | C-Br | C-CH₃ |
| 411. | C-Cl | CH | C-CH₃ | CH |
| 412. | C-Cl | CH | C-CH₃ | C-Cl |
| 413. | C-Cl | CH | C-CH₃ | C-F |
| 414. | C-Cl | CH | C-CH₃ | C-Br |
| 415. | C-Cl | CH | C-CH₃ | C-CH₃ |
| 416. | C-Cl | C-CI | N | CH |
| 417. | C-Cl | C-CI | N | C-CI |
| 418. | C-Cl | C-CI | N | C-F |
| 419. | C-Cl | C-CI | N | C-Br |
| 420. | C-Cl | C-CI | N | C-CH₃ |
| 421. | C-Cl | C-CI | CH | CH |
| 422. | C-Cl | C-CI | CH | C-CI |
| 423. | C-Cl | C-CI | CH | C-F |
| 424. | C-Cl | C-CI | CH | C-Br |
| 425. | C-Cl | C-CI | CH | C-CH₃ |
| 426. | C-Cl | C-CI | C-CI | CH |
| 427. | C-Cl | C-CI | C-Cl | C-CI |
| 428. | C-Cl | C-CI | C-Cl | C-F |
| 429. | C-Cl | C-CI | C-Cl | C-Br |
| 430. | C-Cl | C-CI | C-CI | C-CH₃ |
| 431. | C-Cl | C-CI | C-F | CH |
| 432. | C-Cl | C-CI | C-F | C-Cl |
| 433. | C-Cl | C-CI | C-F | C-F |
| 434. | C-Cl | C-CI | C-F | C-Br |
| 435. | C-Cl | C-CI | C-F | C-CH₃ |
| 436. | C-Cl | C-CI | C-Br | CH |
| 437. | C-Cl | C-CI | C-Br | C-CI |
| 438. | C-Cl | C-CI | C-Br | C-F |
| 439. | C-Cl | C-CI | C-Br | C-Br |
| 440. | C-Cl | C-CI | C-Br | C-CH₃ |
| 441. | C-Cl | C-CI | C-CH₃ | CH |
| 442. | C-Cl | C-CI | C-CH₃ | C-Cl |
| 443. | C-Cl | C-CI | C-CH₃ | C-F |
| 444. | C-Cl | C-CI | C-CH₃ | C-Br |
| 445. | C-Cl | C-CI | C-CH₃ | C-CH₃ |
| 446. | C-Cl | C-F | N | CH |
| 447. | C-Cl | C-F | N | C-CI |
| 448. | C-Cl | C-F | N | C-F |
| 449. | C-Cl | C-F | N | C-Br |
| 450. | C-Cl | C-F | N | C-CH₃ |
| 451. | C-Cl | C-F | CH | CH |
| 452. | C-Cl | C-F | CH | C-CI |
| 453. | C-Cl | C-F | CH | C-F |
| 454. | C-Cl | C-F | CH | C-Br |
| 455. | C-Cl | C-F | CH | C-CH₃ |
| 456. | C-Cl | C-F | C-Cl | CH |
| 457. | C-Cl | C-F | C-Cl | C-CI |
| 458. | C-Cl | C-F | C-Cl | C-F |
| 459. | C-Cl | C-F | C-Cl | C-Br |
| 460. | C-Cl | C-F | C-Cl | C-CH₃ |
| 461. | C-Cl | C-F | C-F | CH |
| 462. | C-Cl | C-F | C-F | C-Cl |
| 463. | C-Cl | C-F | C-F | C-F |
| 464. | C-Cl | C-F | C-F | C-Br |
| 465. | C-Cl | C-F | C-F | C-CH₃ |
| 466. | C-Cl | C-F | C-Br | CH |
| 467. | C-Cl | C-F | C-Br | C-Cl |
| 468. | C-Cl | C-F | C-Br | C-F |
| 469. | C-Cl | C-F | C-Br | C-Br |
| 470. | C-Cl | C-F | C-Br | C-CH₃ |
| 471. | C-Cl | C-F | C-CH₃ | CH |
| 472. | C-Cl | C-F | C-CH₃ | C-Cl |
| 473. | C-Cl | C-F | C-CH₃ | C-F |
| 474. | C-Cl | C-F | C-CH₃ | C-Br |
| 475. | C-Cl | C-F | C-CH₃ | C-CH₃ |
| 476. | C-Cl | C-Br | N | CH |
| 477. | C-Cl | C-Br | N | C-Cl |
| 478. | C-Cl | C-Br | N | C-F |
| 479. | C-Cl | C-Br | N | C-Br |
| 480. | C-Cl | C-Br | N | C-CH₃ |
| 481. | C-Cl | C-Br | CH | CH |
| 482. | C-Cl | C-Br | CH | C-Cl |
| 483. | C-Cl | C-Br | CH | C-F |
| 484. | C-Cl | C-Br | CH | C-Br |
| 485. | C-Cl | C-Br | CH | C-CH₃ |
| 486. | C-Cl | C-Br | C-Cl | CH |
| 487. | C-Cl | C-Br | C-Cl | C-Cl |
| 488. | C-Cl | C-Br | C-Cl | C-F |
| 489. | C-Cl | C-Br | C-Cl | C-Br |
| 490. | C-Cl | C-Br | C-Cl | C-CH₃ |
| 491. | C-Cl | C-Br | C-F | CH |
| 492. | C-Cl | C-Br | C-F | C-Cl |
| 493. | C-Cl | C-Br | C-F | C-F |
| 494. | C-Cl | C-Br | C-F | C-Br |
| 495. | C-Cl | C-Br | C-F | C-CH₃ |
| 496. | C-Cl | C-Br | C-Br | CH |
| 497. | C-Cl | C-Br | C-Br | C-Cl |
| 498. | C-Cl | C-Br | C-Br | C-F |
| 499. | C-Cl | C-Br | C-Br | C-Br |
| 500. | C-Cl | C-Br | C-Br | C-CH₃ |
| 501. | C-Cl | C-Br | C-CH₃ | CH |
| 502. | C-Cl | C-Br | C-CH₃ | C-Cl |
| 503. | C-Cl | C-Br | C-CH₃ | C-F |
| 504. | C-Cl | C-Br | C-CH₃ | C-Br |
| 505. | C-Cl | C-Br | C-CH₃ | C-CH₃ |
| 506. | C-Cl | C-CH₃ | N | CH |
| 507. | C-Cl | C-CH₃ | N | C-Cl |
| 508. | C-Cl | C-CH₃ | N | C-F |
| 509. | C-Cl | C-CH₃ | N | C-Br |
| 510. | C-Cl | C-CH₃ | N | C-CH₃ |
| 511. | C-Cl | C-CH₃ | CH | CH |
| 512. | C-Cl | C-CH₃ | CH | C-CI |
| 513. | C-Cl | C-CH₃ | CH | C-F |
| 514. | C-Cl | C-CH₃ | CH | C-Br |
| 515. | C-Cl | C-CH₃ | CH | C-CH₃ |
| 516. | C-Cl | C-CH₃ | C-Cl | CH |
| 517. | C-Cl | C-CH₃ | C-Cl | C-Cl |
| 518. | C-Cl | C-CH₃ | C-Cl | C-F |
| 519. | C-Cl | C-CH₃ | C-Cl | C-Br |
| 520. | C-Cl | C-CH₃ | C-Cl | C-CH₃ |
| 521. | C-Cl | C-CH₃ | C-F | CH |
| 522. | C-Cl | C-CH₃ | C-F | C-Cl |
| 523. | C-Cl | C-CH₃ | C-F | C-F |
| 524. | C-Cl | C-CH₃ | C-F | C-Br |
| 525. | C-Cl | C-CH₃ | C-F | C-CH₃ |
| 526. | C-Cl | C-CH₃ | C-Br | CH |
| 527. | C-Cl | C-CH₃ | C-Br | C-Cl |
| 528. | C-Cl | C-CH₃ | C-Br | C-F |
| 529. | C-Cl | C-CH₃ | C-Br | C-Br |
| 530. | C-Cl | C-CH₃ | C-Br | C-CH₃ |
| 531. | C-Cl | C-CH₃ | C-CH₃ | CH |
| 532. | C-Cl | C-CH₃ | C-CH₃ | C-Cl |
| 533. | C-Cl | C-CH₃ | C-CH₃ | C-F |
| 534. | C-Cl | C-CH₃ | C-CH₃ | C-Br |
| 535. | C-Cl | C-CH₃ | C-CH₃ | C-CH₃ |
| 536. | C-F | N | N | CH |
| 537. | C-F | N | N | C-Cl |
| 538. | C-F | N | N | C-F |
| 539. | C-F | N | N | C-Br |
| 540. | C-F | N | N | C-CH₃ |
| 541. | C-F | N | CH | CH |
| 542. | C-F | N | CH | C-Cl |
| 543. | C-F | N | CH | C-F |
| 544. | C-F | N | CH | C-Br |
| 545. | C-F | N | CH | C-CH₃ |
| 546. | C-F | N | C-Cl | CH |
| 547. | C-F | N | C-Cl | C-Cl |
| 548. | C-F | N | C-Cl | C-F |
| 549. | C-F | N | C-Cl | C-Br |
| 550. | C-F | N | C-Cl | C-CH₃ |
| 551. | C-F | N | C-F | CH |
| 552. | C-F | N | C-F | C-Cl |
| 553. | C-F | N | C-F | C-F |
| 554. | C-F | N | C-F | C-Br |
| 555. | C-F | N | C-F | C-CH₃ |
| 556. | C-F | N | C-Br | CH |
| 557. | C-F | N | C-Br | C-Cl |
| 558. | C-F | N | C-Br | C-F |
| 559. | C-F | N | C-Br | C-Br |
| 560. | C-F | N | C-Br | C-CH₃ |
| 561. | C-F | N | C-CH₃ | CH |
| 562. | C-F | N | C-CH₃ | C-Cl |
| 563. | C-F | N | C-CH₃ | C-F |
| 564. | C-F | N | C-CH₃ | C-Br |
| 565. | C-F | N | C-CH₃ | C-CH₃ |
| 566. | C-F | CH | N | CH |
| 567. | C-F | CH | N | C-Cl |
| 568. | C-F | CH | N | C-F |
| 569. | C-F | CH | N | C-Br |
| 570. | C-F | CH | N | C-CH₃ |
| 571. | C-F | CH | CH | CH |
| 572. | C-F | CH | CH | C-Cl |
| 573. | C-F | CH | CH | C-F |
| 574. | C-F | CH | CH | C-Br |
| 575. | C-F | CH | CH | C-CH₃ |
| 576. | C-F | CH | C-Cl | CH |
| 577. | C-F | CH | C-Cl | C-Cl |
| 578. | C-F | CH | C-Cl | C-F |
| 579. | C-F | CH | C-Cl | C-Br |
| 580. | C-F | CH | C-Cl | C-CH₃ |
| 581. | C-F | CH | C-F | CH |
| 582. | C-F | CH | C-F | C-Cl |
| 583. | C-F | CH | C-F | C-F |
| 584. | C-F | CH | C-F | C-Br |
| 585. | C-F | CH | C-F | C-CH₃ |
| 586. | C-F | CH | C-Br | CH |
| 587. | C-F | CH | C-Br | C-Cl |
| 588. | C-F | CH | C-Br | C-F |
| 589. | C-F | CH | C-Br | C-Br |
| 590. | C-F | CH | C-Br | C-CH₃ |
| 591. | C-F | CH | C-CH₃ | CH |
| 592. | C-F | CH | C-CH₃ | C-Cl |
| 593. | C-F | CH | C-CH₃ | C-F |
| 594. | C-F | CH | C-CH₃ | C-Br |
| 595. | C-F | CH | C-CH₃ | C-CH₃ |
| 596. | C-F | C-Cl | N | CH |
| 597. | C-F | C-Cl | N | C-Cl |
| 598. | C-F | C-Cl | N | C-F |
| 599. | C-F | C-Cl | N | C-Br |
| 600. | C-F | C-Cl | N | C-CH₃ |
| 601. | C-F | C-Cl | CH | CH |
| 602. | C-F | C-Cl | CH | C-Cl |
| 603. | C-F | C-Cl | CH | C-F |
| 604. | C-F | C-Cl | CH | C-Br |
| 605. | C-F | C-Cl | CH | C-CH₃ |
| 606. | C-F | C-Cl | C-Cl | CH |
| 607. | C-F | C-Cl | C-Cl | C-Cl |
| 608. | C-F | C-Cl | C-Cl | C-F |
| 609. | C-F | C-Cl | C-Cl | C-Br |
| 610. | C-F | C-Cl | C-Cl | C-CH₃ |
| 611. | C-F | C-Cl | C-F | CH |
| 612. | C-F | C-Cl | C-F | C-Cl |
| 613. | C-F | C-Cl | C-F | C-F |
| 614. | C-F | C-Cl | C-F | C-Br |
| 615. | C-F | C-Cl | C-F | C-CH₃ |
| 616. | C-F | C-Cl | C-Br | CH |
| 617. | C-F | C-Cl | C-Br | C-Cl |
| 618. | C-F | C-Cl | C-Br | C-F |
| 619. | C-F | C-Cl | C-Br | C-Br |
| 620. | C-F | C-Cl | C-Br | C-CH₃ |
| 621. | C-F | C-Cl | C-CH₃ | CH |
| 622. | C-F | C-Cl | C-CH₃ | C-Cl |
| 623. | C-F | C-Cl | C-CH₃ | C-F |
| 624. | C-F | C-Cl | C-CH₃ | C-Br |
| 625. | C-F | C-Cl | C-CH₃ | C-CH₃ |
| 626. | C-F | C-F | N | CH |
| 627. | C-F | C-F | N | C-Cl |
| 628. | C-F | C-F | N | C-F |
| 629. | C-F | C-F | N | C-Br |
| 630. | C-F | C-F | N | C-CH₃ |
| 631. | C-F | C-F | CH | CH |
| 632. | C-F | C-F | CH | C-Cl |
| 633. | C-F | C-F | CH | C-F |
| 634. | C-F | C-F | CH | C-Br |
| 635. | C-F | C-F | CH | C-CH₃ |
| 636. | C-F | C-F | C-Cl | CH |
| 637. | C-F | C-F | C-Cl | C-Cl |
| 638. | C-F | C-F | C-Cl | C-F |
| 639. | C-F | C-F | C-Cl | C-Br |
| 640. | C-F | C-F | C-Cl | C-CH₃ |
| 641. | C-F | C-F | C-F | CH |
| 642. | C-F | C-F | C-F | C-Cl |
| 643. | C-F | C-F | C-F | C-F |
| 644. | C-F | C-F | C-F | C-Br |
| 645. | C-F | C-F | C-F | C-CH₃ |
| 646. | C-F | C-F | C-Br | CH |
| 647. | C-F | C-F | C-Br | C-Cl |
| 648. | C-F | C-F | C-Br | C-F |
| 649. | C-F | C-F | C-Br | C-Br |
| 650. | C-F | C-F | C-Br | C-CH₃ |
| 651. | C-F | C-F | C-CH₃ | CH |
| 652. | C-F | C-F | C-CH₃ | C-Cl |
| 653. | C-F | C-F | C-CH₃ | C-F |
| 654. | C-F | C-F | C-CH₃ | C-Br |
| 655. | C-F | C-F | C-CH₃ | C-CH₃ |
| 656. | C-F | C-Br | N | CH |
| 657. | C-F | C-Br | N | C-Cl |
| 658. | C-F | C-Br | N | C-F |
| 659. | C-F | C-Br | N | C-Br |
| 660. | C-F | C-Br | N | C-CH₃ |
| 661. | C-F | C-Br | CH | CH |
| 662. | C-F | C-Br | CH | C-Cl |
| 663. | C-F | C-Br | CH | C-F |
| 664. | C-F | C-Br | CH | C-Br |
| 665. | C-F | C-Br | CH | C-CH₃ |
| 666. | C-F | C-Br | C-Cl | CH |
| 667. | C-F | C-Br | C-Cl | C-Cl |
| 668. | C-F | C-Br | C-Cl | C-F |
| 669. | C-F | C-Br | C-Cl | C-Br |
| 670. | C-F | C-Br | C-Cl | C-CH₃ |
| 671. | C-F | C-Br | C-F | CH |
| 672. | C-F | C-Br | C-F | C-Cl |
| 673. | C-F | C-Br | C-F | C-F |
| 674. | C-F | C-Br | C-F | C-Br |
| 675. | C-F | C-Br | C-F | C-CH₃ |
| 676. | C-F | C-Br | C-Br | CH |
| 677. | C-F | C-Br | C-Br | C-Cl |
| 678. | C-F | C-Br | C-Br | C-F |
| 679. | C-F | C-Br | C-Br | C-Br |
| 680. | C-F | C-Br | C-Br | C-CH₃ |
| 681. | C-F | C-Br | C-CH₃ | CH |
| 682. | C-F | C-Br | C-CH₃ | C-Cl |
| 683. | C-F | C-Br | C-CH₃ | C-F |
| 684. | C-F | C-Br | C-CH₃ | C-Br |
| 685. | C-F | C-Br | C-CH₃ | C-CH₃ |
| 686. | C-F | C-CH₃ | N | CH |
| 687. | C-F | C-CH₃ | N | C-Cl |
| 688. | C-F | C-CH₃ | N | C-F |
| 689. | C-F | C-CH₃ | N | C-Br |
| 690. | C-F | C-CH₃ | N | C-CH₃ |
| 691. | C-F | C-CH₃ | CH | CH |
| 692. | C-F | C-CH₃ | CH | C-Cl |
| 693. | C-F | C-CH₃ | CH | C-F |
| 694. | C-F | C-CH₃ | CH | C-Br |
| 695. | C-F | C-CH₃ | CH | C-CH₃ |
| 696. | C-F | C-CH₃ | C-Cl | CH |
| 697. | C-F | C-CH₃ | C-Cl | C-Cl |
| 698. | C-F | C-CH₃ | C-Cl | C-F |
| 699. | C-F | C-CH₃ | C-Cl | C-Br |
| 700. | C-F | C-CH₃ | C-Cl | C-CH₃ |
| 701. | C-F | C-CH₃ | C-F | CH |
| 702. | C-F | C-CH₃ | C-F | C-Cl |
| 703. | C-F | C-CH₃ | C-F | C-F |
| 704. | C-F | C-CH₃ | C-F | C-Br |
| 705. | C-F | C-CH₃ | C-F | C-CH₃ |
| 706. | C-F | C-CH₃ | C-Br | CH |
| 707. | C-F | C-CH₃ | C-Br | C-Cl |
| 708. | C-F | C-CH₃ | C-Br | C-F |
| 709. | C-F | C-CH₃ | C-Br | C-Br |
| 710. | C-F | C-CH₃ | C-Br | C-CH₃ |
| 711. | C-F | C-CH₃ | C-CH₃ | CH |
| 712. | C-F | C-CH₃ | C-CH₃ | C-Cl |
| 713. | C-F | C-CH₃ | C-CH₃ | C-F |
| 714. | C-F | C-CH₃ | C-CH₃ | C-Br |
| 715. | C-F | C-CH₃ | C-CH₃ | C-CH₃ |
| 716. | C-Br | N | N | CH |
| 717. | C-Br | N | N | C-Cl |
| 718. | C-Br | N | N | C-F |
| 719. | C-Br | N | N | C-Br |
| 720. | C-Br | N | N | C-CH₃ |
| 721. | C-Br | N | CH | CH |
| 722. | C-Br | N | CH | C-Cl |
| 723. | C-Br | N | CH | C-F |
| 724. | C-Br | N | CH | C-Br |
| 725. | C-Br | N | CH | C-CH₃ |
| 726. | C-Br | N | C-Cl | CH |
| 727. | C-Br | N | C-Cl | C-Cl |
| 728. | C-Br | N | C-Cl | C-F |
| 729. | C-Br | N | C-Cl | C-Br |
| 730. | C-Br | N | C-Cl | C-CH₃ |
| 731. | C-Br | N | C-F | CH |
| 732. | C-Br | N | C-F | C-Cl |
| 733. | C-Br | N | C-F | C-F |
| 734. | C-Br | N | C-F | C-Br |
| 735. | C-Br | N | C-F | C-CH₃ |
| 736. | C-Br | N | C-Br | CH |
| 737. | C-Br | N | C-Br | C-Cl |
| 738. | C-Br | N | C-Br | C-F |
| 739. | C-Br | N | C-Br | C-Br |
| 740. | C-Br | N | C-Br | C-CH₃ |
| 741. | C-Br | N | C-CH₃ | CH |
| 742. | C-Br | N | C-CH₃ | C-Cl |
| 743. | C-Br | N | C-CH₃ | C-F |
| 744. | C-Br | N | C-CH₃ | C-Br |
| 745. | C-Br | N | C-CH₃ | C-CH₃ |
| 746. | C-Br | CH | N | CH |
| 747. | C-Br | CH | N | C-Cl |
| 748. | C-Br | CH | N | C-F |
| 749. | C-Br | CH | N | C-Br |
| 750. | C-Br | CH | N | C-CH₃ |
| 751. | C-Br | CH | CH | CH |
| 752. | C-Br | CH | CH | C-Cl |
| 753. | C-Br | CH | CH | C-F |
| 754. | C-Br | CH | CH | C-Br |
| 755. | C-Br | CH | CH | C-CH₃ |
| 756. | C-Br | CH | C-Cl | CH |
| 757. | C-Br | CH | C-Cl | C-Cl |
| 758. | C-Br | CH | C-Cl | C-F |
| 759. | C-Br | CH | C-Cl | C-Br |
| 760. | C-Br | CH | C-Cl | C-CH₃ |
| 761. | C-Br | CH | C-F | CH |
| 762. | C-Br | CH | C-F | C-Cl |
| 763. | C-Br | CH | C-F | C-F |
| 764. | C-Br | CH | C-F | C-Br |
| 765. | C-Br | CH | C-F | C-CH₃ |
| 766. | C-Br | CH | C-Br | CH |
| 767. | C-Br | CH | C-Br | C-Cl |
| 768. | C-Br | CH | C-Br | C-F |
| 769. | C-Br | CH | C-Br | C-Br |
| 770. | C-Br | CH | C-Br | C-CH₃ |
| 771. | C-Br | CH | C-CH₃ | CH |
| 772. | C-Br | CH | C-CH₃ | C-Cl |
| 773. | C-Br | CH | C-CH₃ | C-F |
| 774. | C-Br | CH | C-CH₃ | C-Br |
| 775. | C-Br | CH | C-CH₃ | C-CH₃ |
| 776. | C-Br | C-Cl | N | CH |
| 777. | C-Br | C-Cl | N | C-Cl |
| 778. | C-Br | C-Cl | N | C-F |
| 779. | C-Br | C-Cl | N | C-Br |
| 780. | C-Br | C-Cl | N | C-CH₃ |
| 781. | C-Br | C-Cl | CH | CH |
| 782. | C-Br | C-Cl | CH | C-Cl |
| 783. | C-Br | C-Cl | CH | C-F |
| 784. | C-Br | C-Cl | CH | C-Br |
| 785. | C-Br | C-Cl | CH | C-CH₃ |
| 786. | C-Br | C-Cl | C-CI | CH |
| 787. | C-Br | C-Cl | C-Cl | C-Cl |
| 788. | C-Br | C-Cl | C-Cl | C-F |
| 789. | C-Br | C-Cl | C-Cl | C-Br |
| 790. | C-Br | C-Cl | C-CI | C-CH₃ |
| 791. | C-Br | C-Cl | C-F | CH |
| 792. | C-Br | C-Cl | C-F | C-Cl |
| 793. | C-Br | C-Cl | C-F | C-F |
| 794. | C-Br | C-Cl | C-F | C-Br |
| 795. | C-Br | C-Cl | C-F | C-CH₃ |
| 796. | C-Br | C-Cl | C-Br | CH |
| 797. | C-Br | C-Cl | C-Br | C-Cl |
| 798. | C-Br | C-Cl | C-Br | C-F |
| 799. | C-Br | C-Cl | C-Br | C-Br |
| 800. | C-Br | C-Cl | C-Br | C-CH₃ |
| 801. | C-Br | C-Cl | C-CH₃ | CH |
| 802. | C-Br | C-Cl | C-CH₃ | C-Cl |
| 803. | C-Br | C-Cl | C-CH₃ | C-F |
| 804. | C-Br | C-Cl | C-CH₃ | C-Br |
| 805. | C-Br | C-Cl | C-CH₃ | C-CH₃ |
| 806. | C-Br | C-F | N | CH |
| 807. | C-Br | C-F | N | C-Cl |
| 808. | C-Br | C-F | N | C-F |
| 809. | C-Br | C-F | N | C-Br |
| 810. | C-Br | C-F | N | C-CH₃ |
| 811. | C-Br | C-F | CH | CH |
| 812. | C-Br | C-F | CH | C-Cl |
| 813. | C-Br | C-F | CH | C-F |
| 814. | C-Br | C-F | CH | C-Br |
| 815. | C-Br | C-F | CH | C-CH₃ |
| 816. | C-Br | C-F | C-Cl | CH |
| 817. | C-Br | C-F | C-Cl | C-Cl |
| 818. | C-Br | C-F | C-Cl | C-F |
| 819. | C-Br | C-F | C-Cl | C-Br |
| 820. | C-Br | C-F | C-Cl | C-CH₃ |
| 821. | C-Br | C-F | C-F | CH |
| 822. | C-Br | C-F | C-F | C-Cl |
| 823. | C-Br | C-F | C-F | C-F |
| 824. | C-Br | C-F | C-F | C-Br |
| 825. | C-Br | C-F | C-F | C-CH₃ |
| 826. | C-Br | C-F | C-Br | CH |
| 827. | C-Br | C-F | C-Br | C-Cl |
| 828. | C-Br | C-F | C-Br | C-F |
| 829. | C-Br | C-F | C-Br | C-Br |
| 830. | C-Br | C-F | C-Br | C-CH₃ |
| 831. | C-Br | C-F | C-CH₃ | CH |
| 832. | C-Br | C-F | C-CH₃ | C-Cl |
| 833. | C-Br | C-F | C-CH₃ | C-F |
| 834. | C-Br | C-F | C-CH₃ | C-Br |
| 835. | C-Br | C-F | C-CH₃ | C-CH₃ |
| 836. | C-Br | C-Br | N | CH |
| 837. | C-Br | C-Br | N | C-Cl |
| 838. | C-Br | C-Br | N | C-F |
| 839. | C-Br | C-Br | N | C-Br |
| 840. | C-Br | C-Br | N | C-CH₃ |
| 841. | C-Br | C-Br | CH | CH |
| 842. | C-Br | C-Br | CH | C-Cl |
| 843. | C-Br | C-Br | CH | C-F |
| 844. | C-Br | C-Br | CH | C-Br |
| 845. | C-Br | C-Br | CH | C-CH₃ |
| 846. | C-Br | C-Br | C-Cl | CH |
| 847. | C-Br | C-Br | C-Cl | C-Cl |
| 848. | C-Br | C-Br | C-Cl | C-F |
| 849. | C-Br | C-Br | C-Cl | C-Br |
| 850. | C-Br | C-Br | C-Cl | C-CH₃ |
| 851. | C-Br | C-Br | C-F | CH |
| 852. | C-Br | C-Br | C-F | C-Cl |
| 853. | C-Br | C-Br | C-F | C-F |
| 854. | C-Br | C-Br | C-F | C-Br |
| 855. | C-Br | C-Br | C-F | C-CH₃ |
| 856. | C-Br | C-Br | C-Br | CH |
| 857. | C-Br | C-Br | C-Br | C-Cl |
| 858. | C-Br | C-Br | C-Br | C-F |
| 859. | C-Br | C-Br | C-Br | C-Br |
| 860. | C-Br | C-Br | C-Br | C-CH₃ |
| 861. | C-Br | C-Br | C-CH₃ | CH |
| 862. | C-Br | C-Br | C-CH₃ | C-Cl |
| 863. | C-Br | C-Br | C-CH₃ | C-F |
| 864. | C-Br | C-Br | C-CH₃ | C-Br |
| 865. | C-Br | C-Br | C-CH₃ | C-CH₃ |
| 866. | C-Br | C-CH₃ | N | CH |
| 867. | C-Br | C-CH₃ | N | C-Cl |
| 868. | C-Br | C-CH₃ | N | C-F |
| 869. | C-Br | C-CH₃ | N | C-Br |
| 870. | C-Br | C-CH₃ | N | C-CH₃ |
| 871. | C-Br | C-CH₃ | CH | CH |
| 872. | C-Br | C-CH₃ | CH | C-Cl |
| 873. | C-Br | C-CH₃ | CH | C-F |
| 874. | C-Br | C-CH₃ | CH | C-Br |
| 875. | C-Br | C-CH₃ | CH | C-CH₃ |
| 876. | C-Br | C-CH₃ | C-Cl | CH |
| 877. | C-Br | C-CH₃ | C-Cl | C-Cl |
| 878. | C-Br | C-CH₃ | C-Cl | C-F |
| 879. | C-Br | C-CH₃ | C-Cl | C-Br |
| 880. | C-Br | C-CH₃ | C-Cl | C-CH₃ |
| 881. | C-Br | C-CH₃ | C-F | CH |
| 882. | C-Br | C-CH₃ | C-F | C-Cl |
| 883. | C-Br | C-CH₃ | C-F | C-F |
| 884. | C-Br | C-CH₃ | C-F | C-Br |
| 885. | C-Br | C-CH₃ | C-F | C-CH₃ |
| 886. | C-Br | C-CH₃ | C-Br | CH |
| 887. | C-Br | C-CH₃ | C-Br | C-Cl |
| 888. | C-Br | C-CH₃ | C-Br | C-F |
| 889. | C-Br | C-CH₃ | C-Br | C-Br |
| 890. | C-Br | C-CH₃ | C-Br | C-CH₃ |
| 891. | C-Br | C-CH₃ | C-CH₃ | CH |
| 892. | C-Br | C-CH₃ | C-CH₃ | C-Cl |
| 893. | C-Br | C-CH₃ | C-CH₃ | C-F |
| 894. | C-Br | C-CH₃ | C-CH₃ | C-Br |
| 895. | C-Br | C-CH₃ | C-CH₃ | C-CH₃ |
| 896. | C-CH₃ | N | N | CH |
| 897. | C-CH₃ | N | N | C-Cl |
| 898. | C-CH₃ | N | N | C-F |
| 899. | C-CH₃ | N | N | C-Br |
| 900. | C-CH₃ | N | N | C-CH₃ |
| 901. | C-CH₃ | N | CH | CH |
| 902. | C-CH₃ | N | CH | C-Cl |
| 903. | C-CH₃ | N | CH | C-F |
| 904. | C-CH₃ | N | CH | C-Br |
| 905. | C-CH₃ | N | CH | C-CH₃ |
| 906. | C-CH₃ | N | C-Cl | CH |
| 907. | C-CH₃ | N | C-Cl | C-Cl |
| 908. | C-CH₃ | N | C-Cl | C-F |
| 909. | C-CH₃ | N | C-Cl | C-Br |
| 910. | C-CH₃ | N | C-Cl | C-CH₃ |
| 911. | C-CH₃ | N | C-F | CH |
| 912. | C-CH₃ | N | C-F | C-Cl |
| 913. | C-CH₃ | N | C-F | C-F |
| 914. | C-CH₃ | N | C-F | C-Br |
| 915. | C-CH₃ | N | C-F | C-CH₃ |
| 916. | C-CH₃ | N | C-Br | CH |
| 917. | C-CH₃ | N | C-Br | C-Cl |
| 918. | C-CH₃ | N | C-Br | C-F |
| 919. | C-CH₃ | N | C-Br | C-Br |
| 920. | C-CH₃ | N | C-Br | C-CH₃ |
| 921. | C-CH₃ | N | C-CH₃ | CH |
| 922. | C-CH₃ | N | C-CH₃ | C-Cl |
| 923. | C-CH₃ | N | C-CH₃ | C-F |
| 924. | C-CH₃ | N | C-CH₃ | C-Br |
| 925. | C-CH₃ | N | C-CH₃ | C-CH₃ |
| 926. | C-CH₃ | CH | N | CH |
| 927. | C-CH₃ | CH | N | C-Cl |
| 928. | C-CH₃ | CH | N | C-F |
| 929. | C-CH₃ | CH | N | C-Br |
| 930. | C-CH₃ | CH | N | C-CH₃ |
| 931. | C-CH₃ | CH | CH | CH |
| 932. | C-CH₃ | CH | CH | C-Cl |
| 933. | C-CH₃ | CH | CH | C-F |
| 934. | C-CH₃ | CH | CH | C-Br |
| 935. | C-CH₃ | CH | CH | C-CH₃ |
| 936. | C-CH₃ | CH | C-Cl | CH |
| 937. | C-CH₃ | CH | C-Cl | C-Cl |
| 938. | C-CH₃ | CH | C-Cl | C-F |
| 939. | C-CH₃ | CH | C-Cl | C-Br |
| 940. | C-CH₃ | CH | C-Cl | C-CH₃ |
| 941. | C-CH₃ | CH | C-F | CH |
| 942. | C-CH₃ | CH | C-F | C-Cl |
| 943. | C-CH₃ | CH | C-F | C-F |
| 944. | C-CH₃ | CH | C-F | C-Br |
| 945. | C-CH₃ | CH | C-F | C-CH₃ |
| 946. | C-CH₃ | CH | C-Br | CH |
| 947. | C-CH₃ | CH | C-Br | C-Cl |
| 948. | C-CH₃ | CH | C-Br | C-F |
| 949. | C-CH₃ | CH | C-Br | C-Br |
| 950. | C-CH₃ | CH | C-Br | C-CH₃ |
| 951. | C-CH₃ | CH | C-CH₃ | CH |
| 952. | C-CH₃ | CH | C-CH₃ | C-Cl |
| 953. | C-CH₃ | CH | C-CH₃ | C-F |
| 954. | C-CH₃ | CH | C-CH₃ | C-Br |
| 955. | C-CH₃ | CH | C-CH₃ | C-CH₃ |
| 956. | C-CH₃ | C-Cl | N | CH |
| 957. | C-CH₃ | C-Cl | N | C-CI |
| 958. | C-CH₃ | C-Cl | N | C-F |
| 959. | C-CH₃ | C-Cl | N | C-Br |
| 960. | C-CH₃ | C-Cl | N | C-CH₃ |
| 961. | C-CH₃ | C-Cl | CH | CH |
| 962. | C-CH₃ | C-Cl | CH | C-Cl |
| 963. | C-CH₃ | C-Cl | CH | C-F |
| 964. | C-CH₃ | C-Cl | CH | C-Br |
| 965. | C-CH₃ | C-Cl | CH | C-CH₃ |
| 966. | C-CH₃ | C-Cl | C-Cl | CH |
| 967. | C-CH₃ | C-Cl | C-Cl | C-CI |
| 968. | C-CH₃ | C-Cl | C-Cl | C-F |
| 969. | C-CH₃ | C-Cl | C-Cl | C-Br |
| 970. | C-CH₃ | C-Cl | C-Cl | C-CH₃ |
| 971. | C-CH₃ | C-Cl | C-F | CH |
| 972. | C-CH₃ | C-Cl | C-F | C-CI |
| 973. | C-CH₃ | C-Cl | C-F | C-F |
| 974. | C-CH₃ | C-Cl | C-F | C-Br |
| 975. | C-CH₃ | C-Cl | C-F | C-CH₃ |
| 976. | C-CH₃ | C-Cl | C-Br | CH |
| 977. | C-CH₃ | C-Cl | C-Br | C-CI |
| 978. | C-CH₃ | C-Cl | C-Br | C-F |
| 979. | C-CH₃ | C-Cl | C-Br | C-Br |
| 980. | C-CH₃ | C-Cl | C-Br | C-CH₃ |
| 981. | C-CH₃ | C-Cl | C-CH₃ | CH |
| 982. | C-CH₃ | C-Cl | C-CH₃ | C-Cl |
| 983. | C-CH₃ | C-Cl | C-CH₃ | C-F |
| 984. | C-CH₃ | C-Cl | C-CH₃ | C-Br |
| 985. | C-CH₃ | C-Cl | C-CH₃ | C-CH₃ |
| 986. | C-CH₃ | C-F | N | CH |
| 987. | C-CH₃ | C-F | N | C-Cl |
| 988. | C-CH₃ | C-F | N | C-F |
| 989. | C-CH₃ | C-F | N | C-Br |
| 990. | C-CH₃ | C-F | N | C-CH₃ |
| 991. | C-CH₃ | C-F | CH | CH |
| 992. | C-CH₃ | C-F | CH | C-CI |
| 993. | C-CH₃ | C-F | CH | C-F |
| 994. | C-CH₃ | C-F | CH | C-Br |
| 995. | C-CH₃ | C-F | CH | C-CH₃ |
| 996. | C-CH₃ | C-F | C-Cl | CH |
| 997. | C-CH₃ | C-F | C-Cl | C-CI |
| 998. | C-CH₃ | C-F | C-Cl | C-F |
| 999. | C-CH₃ | C-F | C-Cl | C-Br |
| 1000. | C-CH₃ | C-F | C-Cl | C-CH₃ |
| 1001. | C-CH₃ | C-F | C-F | CH |
| 1002. | C-CH₃ | C-F | C-F | C-Cl |
| 1003. | C-CH₃ | C-F | C-F | C-F |
| 1004. | C-CH₃ | C-F | C-F | C-Br |
| 1005. | C-CH₃ | C-F | C-F | C-CH₃ |
| 1006. | C-CH₃ | C-F | C-Br | CH |
| 1007. | C-CH₃ | C-F | C-Br | C-Cl |
| 1008. | C-CH₃ | C-F | C-Br | C-F |
| 1009. | C-CH₃ | C-F | C-Br | C-Br |
| 1010. | C-CH₃ | C-F | C-Br | C-CH₃ |
| 1011. | C-CH₃ | C-F | C-CH₃ | CH |
| 1012. | C-CH₃ | C-F | C-CH₃ | C-CI |
| 1013. | C-CH₃ | C-F | C-CH₃ | C-F |
| 1014. | C-CH₃ | C-F | C-CH₃ | C-Br |
| 1015. | C-CH₃ | C-F | C-CH₃ | C-CH₃ |
| 1016. | C-CH₃ | C-Br | N | CH |
| 1017. | C-CH₃ | C-Br | N | C-CI |
| 1018. | C-CH₃ | C-Br | N | C-F |
| 1019. | C-CH₃ | C-Br | N | C-Br |
| 1020. | C-CH₃ | C-Br | N | C-CH₃ |
| 1021. | C-CH₃ | C-Br | CH | CH |
| 1022. | C-CH₃ | C-Br | CH | C-Cl |
| 1023. | C-CH₃ | C-Br | CH | C-F |
| 1024. | C-CH₃ | C-Br | CH | C-Br |
| 1025. | C-CH₃ | C-Br | CH | C-CH₃ |
| 1026. | C-CH₃ | C-Br | C-Cl | CH |
| 1027. | C-CH₃ | C-Br | C-CI | C-Cl |
| 1028. | C-CH₃ | C-Br | C-CI | C-F |
| 1029. | C-CH₃ | C-Br | C-CI | C-Br |
| 1030. | C-CH₃ | C-Br | C-Cl | C-CH₃ |
| 1031. | C-CH₃ | C-Br | C-F | CH |
| 1032. | C-CH₃ | C-Br | C-F | C-Cl |
| 1033. | C-CH₃ | C-Br | C-F | C-F |
| 1034. | C-CH₃ | C-Br | C-F | C-Br |
| 1035. | C-CH₃ | C-Br | C-F | C-CH₃ |
| 1036. | C-CH₃ | C-Br | C-Br | CH |
| 1037. | C-CH₃ | C-Br | C-Br | C-Cl |
| 1038. | C-CH₃ | C-Br | C-Br | C-F |
| 1039. | C-CH₃ | C-Br | C-Br | C-Br |
| 1040. | C-CH₃ | C-Br | C-Br | C-CH₃ |
| 1041. | C-CH₃ | C-Br | C-CH₃ | CH |
| 1042. | C-CH₃ | C-Br | C-CH₃ | C-Cl |
| 1043. | C-CH₃ | C-Br | C-CH₃ | C-F |
| 1044. | C-CH₃ | C-Br | C-CH₃ | C-Br |
| 1045. | C-CH₃ | C-Br | C-CH₃ | C-CH₃ |
| 1046. | C-CH₃ | C-CH₃ | N | CH |
| 1047. | C-CH₃ | C-CH₃ | N | C-Cl |
| 1048. | C-CH₃ | C-CH₃ | N | C-F |
| 1049. | C-CH₃ | C-CH₃ | N | C-Br |
| 1050. | C-CH₃ | C-CH₃ | N | C-CH₃ |
| 1051. | C-CH₃ | C-CH₃ | CH | CH |
| 1052. | C-CH₃ | C-CH₃ | CH | C-Cl |
| 1053. | C-CH₃ | C-CH₃ | CH | C-F |
| 1054. | C-CH₃ | C-CH₃ | CH | C-Br |
| 1055. | C-CH₃ | C-CH₃ | CH | C-CH₃ |
| 1056. | C-CH₃ | C-CH₃ | C-CI | CH |
| 1057. | C-CH₃ | C-CH₃ | C-CI | C-Cl |
| 1058. | C-CH₃ | C-CH₃ | C-CI | C-F |
| 1059. | C-CH₃ | C-CH₃ | C-CI | C-Br |
| 1060. | C-CH₃ | C-CH₃ | C-Cl | C-CH₃ |
| 1061. | C-CH₃ | C-CH₃ | C-F | CH |
| 1062. | C-CH₃ | C-CH₃ | C-F | C-Cl |
| 1063. | C-CH₃ | C-CH₃ | C-F | C-F |
| 1064. | C-CH₃ | C-CH₃ | C-F | C-Br |
| 1065. | C-CH₃ | C-CH₃ | C-F | C-CH₃ |
| 1066. | C-CH₃ | C-CH₃ | C-Br | CH |
| 1067. | C-CH₃ | C-CH₃ | C-Br | C-Cl |
| 1068. | C-CH₃ | C-CH₃ | C-Br | C-F |
| 1069. | C-CH₃ | C-CH₃ | C-Br | C-Br |
| 1070. | C-CH₃ | C-CH₃ | C-Br | C-CH₃ |
| 1071. | C-CH₃ | C-CH₃ | C-CH₃ | CH |
| 1072. | C-CH₃ | C-CH₃ | C-CH₃ | C-Cl |
| 1073. | C-CH₃ | C-CH₃ | C-CH₃ | C-F |
| 1074. | C-CH₃ | C-CH₃ | C-CH₃ | C-Br |
| 1075. | C-CH₃ | C-CH₃ | C-CH₃ | C-CH₃ |

Table 43 to Table 84: Includes all the compounds as disclosed in Table 1 to Table 42 respectively wherein compound of formula I-1 is replaced by compound of formula I-2;
Table 85 to Table 126: Includes all the compounds as disclosed in Table 1 to Table 42 respectively wherein compound of formula I-1 is replaced by compound of formula I-3;
Table 127 to Table 168: Includes all the compounds as disclosed in Table 1 to Table 42 respectively wherein compound of formula I-1 is replaced by compound of formula I-4;
Table 169 to Table 210: Includes all the compounds as disclosed in Table 1 to Table 42 respectively wherein compound of formula I-1 is replaced by compound of formula I-5;
Table 211 to Table 252: Includes all the compounds as disclosed in Table 1 to Table 42 respectively wherein compound of formula I-1 is replaced by compound of formula I-6;
Table 253 to Table 294: Includes all the compounds as disclosed in Table 1 to Table 42 respectively wherein compound of formula I-1 is replaced by compound of formula I-7;
Table 295 to Table 336: Includes all the compounds as disclosed in Table 1 to Table 42 respectively wherein compound of formula I-1 is replaced by compound of formula I-8;
Table 337 to Table 378: Includes all the compounds as disclosed in Table 1 to Table 42 respectively wherein compound of formula I-1 is replaced by compound of formula I-9;
Table 379 to Table 420: Includes all the compounds as disclosed in Table 1 to Table 42 respectively wherein compound of formula I-1 is replaced by compound of formula I-10;
Table 421 to Table 462: Includes all the compounds as disclosed in Table 1 to Table 42 respectively wherein compound of formula I-1 is replaced by compound of formula I-11;
Table 463 to Table 504: Includes all the compounds as disclosed in Table 1 to Table 42 respectively wherein compound of formula I-1 is replaced by compound of formula I-12;
Table 505 to Table 546: Includes all the compounds as disclosed in Table 1 to Table 42 respectively wherein compound of formula I-1 is replaced by compound of formula I-13;
Table 547 to Table 588: Includes all the compounds as disclosed in Table 1 to Table 42 respectively wherein compound of formula I-1 is replaced by compound of formula I-14;
Table 589 to Table 630: Includes all the compounds as disclosed in Table 1 to Table 42 respectively wherein compound of formula I-1 is replaced by compound of formula I-15;
Table 631 to Table 672: Includes all the compounds as disclosed in Table 1 to Table 42 respectively wherein compound of formula I-1 is replaced by compound of formula I-16;
Table 673 to Table 714: Includes all the compounds as disclosed in Table 1 to Table 42 respectively wherein compound of formula I-1 is replaced by compound of formula I-17;
Table 715 to Table 756: Includes all the compounds as disclosed in Table 1 to Table 42 respectively wherein compound of formula I-1 is replaced by compound of formula I-18;
Table 757 to Table 798: Includes all the compounds as disclosed in Table 1 to Table 42 respectively wherein compound of formula I-1 is replaced by compound of formula I-19;
Table 799 to Table 840: Includes all the compounds as disclosed in Table 1 to Table 42 respectively wherein compound of formula I-1 is replaced by compound of formula I-20;

As used herein, the term "compound(s) of the present invention" or "compound(s) according to the invention" refers to the compound(s) of formula (I) as defined above, which are also referred to as "compound(s) of formula I" or "compound(s) I" or "formula I compound(s)", and includes their salts, tautomers, stereoisomers, and N-oxides.

As used herein, the term "compound(s) of the invention" or "compound(s) according to the invention" refers to the compound(s) of formula (I) as defined above, which are also referred to as "compound(s) of formula I" or "compound(s) I" or "formula I compound(s)", and includes their salts, tautomers, stereoisomers, and N-oxides.

The invention also relates to a mixture of at least one compound of the invention with at least one mixing partner. Preferred are binary mixtures of one compound of the invention as component I with one mixing partner herein as component II. Preferred weight ratios for such binary mixtures are from 5000:1 to 1:5000, preferably from 1000:1 to 1:1000, more preferably from 100:1 to 1:100, particularly from 10:1 to 1:10. In such binary mixtures, components I and II may be used in equal amounts, or an excess of component I, or an excess of component II may be used.

Mixing partners can be selected from pesticides, in particular insecticides, nematicides, and acaricides, fungicides, herbicides, plant growth regulators, fertilizers. Preferred mixing partners are insecticides, nematicides, and fungicides.

The following list M of pesticides, grouped according the Mode of Action Classification of the Insecticide Resistance Action Committee (IRAC), together with which the compounds of the invention can be used and with which potential synergistic effects might be produced, illustrates the possible combinations:
M.1 AChE inhibitors: aldicarb, alanycarb, bendiocarb, benfuracarb, butocarboxim, butoxycar-boxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thi-ofanox, trimethacarb, XMC, xylylcarb, triazamate; acephate, azamethiphos, azinphos-ethyl, azinphosmethyl, cadusafos, chlorethoxyfos, chlorfenvinphos, chlormephos, chlorpyrifos, chlorpyrifosmethyl, coumaphos, cyanophos, demeton-S-methyl, diazinon, dichlorvos/ DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothio-phosphoryl) salicylate, isoxathion, malathion, mecarbam, methamidophos, methidathion, mevinphos, monocrotophos, naled, omethoate, oxydemeton-methyl, parathion, parathion-methyl, phenthoate, phorate, phosalone, phosmet, phos-phamidon, phoxim, pirimiphos-methyl, profenofos, propetamphos, prothiofos, pyraclofos, pyri-daphenthion, quinalphos, sulfotep, tebupirimfos, temephos, terbufos, tetrachlorvinphos, thiometon, triazophos, trichlorfon, vamidothion;
M.2. GABA-gated chloride channel antagonists: cyclodiene organochlorine compounds: endosulfan, chlordane; phenylpyrazoles: ethiprole, fipronil, flufiprole, pyrafluprole, pyriprole;
M.3 Sodium channel modulators: pyrethroids: acrinathrin, allethrin, d-cis-trans allethrin, d-trans allethrin, bifenthrin, kappa-bifenthrin, bioallethrin, bioallethrin S-cylclopentenyl, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin, deltamethrin, empenthrin, esfenvalerate, etofenprox, fenpropathrin, fenvalerate, flucythrinate, flumethrin, tau-fluvalinate, halfenprox, heptafluthrin, imiprothrin, meperfluthrin,metofluthrin, momfluorothrin, epsilon-momfluorothrin, permethrin, phenothrin, prallethrin, profluthrin, pyrethrin (pyrethrum), resmethrin, silafluofen, tefluthrin, kappa-tefluthrin, tetramethylfluthrin, tetramethrin, tralomethrin, transfluthrin; sodium channel modulators, e.g.: DDT, methoxychlor;
M.4 nAChR agonists: neonicotinoids: acetamiprid, clothianidin, cycloxaprid, dinotefuran, im-idacloprid, nitenpyram, thiacloprid, thiamethoxam; 4,5-dihydro-N-nitro-1-(2-oxiranylmethyl)-1H-imidazol-2-amine, (2E-)-1-[(6-Chloropyridin-3-yl)methyl]-N'-nitro-2-pentylidenehydrazine-carboximidamide; 1-[(6-Chloropyridin-3-yl)methyl]-7-methyl-8-nitro-5-propoxy-1,2,3,5,6,7-hex-ahydroimidazo[1,2-a]pyridine; nicotine; sulfoxaflor; flupyradifurone; triflumezopyrim, fenmezo-ditiaz, flupyrimin;
M.5 Nicotinic acetylcholine receptor allosteric activators:spinosyns, e.g. spinosad or spineto-ram;
M.6 Chloride channel activators from the class of avermectins and milbemycins, e.g. abamectin, emamectin benzoate, ivermectin, lepimectin, or milbemectin;
M.7 Juvenile hormone mimics, such as hydroprene, kino-prene, methoprene; fenoxycarb, or pyriproxyfen;
M.8 miscellaneous multi-site inhibitors: CH₃Br, other alkyl halides, chloropicrin, sulfuryl fluoride, borax, tartar emetic;
M.9 Chordotonal organ TRPV channel modulators: afidopyropen, pymetrozine; pyrifluquina-zon;
M.10 Mite growth inhibitors: clofentezine, hexythiazox, diflovidazin, etoxazole;
M.11 Microbial disruptors of insect midgut membranes: *bacillus thuringiensis, bacillus sphaericus,* and insecticdal proteins they produce e.g.: *bacillus thuringiensis* subsp. *israelensis, bacillus sphaericus, bacillus thuringiensis* subsp. *aizawai, bacillus thuringiensis* subsp. *kurstaki, bacillus thuringiensis* subsp. *tenebrionis,* Bt crop proteins: Cry1Ab, Cry1Ac, Cry1Fa, Cry2Ab, mCry3A, Cry3Ab, Cry3Bb, Cry34/35Ab1;
M.12 Inhibitors of mitochondrial ATP synthase: diafenthiuron, organotin miticides, e.g.: azo-cyclotin, cyhexatin, fenbutatin oxide, propargite, tetradifon;
M.13 Uncouplers of oxidative phosphorylation via disruption of the proton gradient: chlorfenapyr, DNOC, sulfluramid;
M.14 nAChR channel blockers: nereistoxin analogues bensultap, cartap hydrochloride, thiocyclam, thiosultap-sodium;
M.15 Inhibitors of the chitin biosynthesis type 0, e.g.: bistrifluron, chlorfluazuron, difluben-zuron, flucycloxuron, flufenoxuron, hexaflumuron, lufenuron, novaluron, noviflumuron, teflubenzuron, triflumuron;
M.16 Inhibitors of the chitin biosynthesis type 1: buprofezin;
M.17 Moulting disruptors: Dipteran, cyromazine;
M.18 Ecdyson receptor agonists, e.g.: methoxyfenozide, tebufenozide, halofenozide, fufeno-zide, chromafenozide;
M.19 Octopamin receptor agonists: amitraz;
M.20 Mitochondrial complex III electron transport inhibitors: hydramethylnon, acequinocyl, fluacrypyrim; bifenazate;
M.21 METI acaricides and insecticides, e.g.: fenazaquin, fenpyroximate, pyrimidifen, pyridaben, tebufenpyrad, tolfenpyrad, rotenone;
M.22 Voltage-dependent sodium channel blockers: indoxacarb, metaflumizone, 2-[2-(4-cyanophenyl)-1-[3-(trifluoromethyl)phenyl]-,ethylidene]-N-[4-(difluoromethoxy)phenyl]-hydra-zinecarboxamide, N-(3-chloro-2-methyl-phenyl)-2-[(4-chlorophenyl)[4-[methy1(methylsulfonyl)amino]phenyl]¬methylene]-hydrazinecarboxamide, N-[4-chloro-2-[[(1,1-dimethylethyl)amino]carbonyl]-6-methylphenyl]-1-(3-chloro-2-pyridinyl)-3-(fluoromethoxy)-1H-pyrazole-5-carboxamide, 2-[2-(4-cyanophenyl)-1-[3-(trifluoromethyl)phenyl]ethylidene]-N-[4-(difluoromethoxy)phenyl]-hydrazinecarboxamide;
M.23 Inhibitors of the of acetyl CoA carboxylase, e.g.: spirodiclofen, spiromesifen, spirotetramat; spiropidion; spirobudifen, 11-(4-chloro-2,6-dimethylphenyl)-12-hydroxy-1,4-dioxa-9-azadispiro[4.2.4.2]tetradec-11-en-10-one, spidoxamat;
M.24 Mitochondrial complex IV electron transport inhibitors: e.g. aluminium phosphide, calcium phosphide, zinc phosphide, cyanide;
M.25 Mitochondrial complex II electron transport inhibitors, e.g.: cyenopyrafen, cyflumetofen, cyetpyrafen, pyflubumide;
M.28 Ryanodine receptor-modulators: chlor¬antraniliprole, cyantraniliprole, cyclaniliprole, flubendiamide, fluchlordiniliprole, (R)-3-chloro-N1-{2-methyl-4-[1,2,2,2-tetrafluoro-1-(trifluoromethyl)ethyl]phenyl}-N2-(1-methyl-2-methylsulfonylethyl)phthalamid, (S)-3-chloro-N1-{2-methyl-4-[1,2,2,2-tetrafluoro-1-(trifluoromethyl)ethyl]phenyl}-N2-(1-methyl-2-methylsulfonylethyl)phthalamide, methyl-2-[3,5-dibromo-2-({[3-bromo-1-(3-chlorpyridin-2-yl)-1H-pyrazol-5-yl]carbonyl}¬amino)benzoyl]-1,2-dimethylhydrazine-carboxylate; N-[2-(5-amino-1,3,4-thiadiazol-2-yl)-4-chloro-6-methyl¬phenyl]-3-bromo-1-(3-chloro-2-pyridinyl)-1H-pyrazole-5-carboxamide; 3-chloro-1-(3-chloro-2-pyridinyl)-N-[2,4-dichloro-6-[[(1-cyano-1-methylethyl)amino]carbonyl]phenyl]-1H-pyrazole-5-carboxamide; tetrachlorantraniliprole; tetraniliprole; tiorantraniliprole; N-[4-chloro-2-[[(1,1-dimethylethyl)amino]carbonyl]-6-methylphenyl]-1-(3-chloro-2-pyridinyl)-3-(fluoromethoxy)-1H-pyrazole-5-carboxamide; cyhalodi-amide; N-[2-(5-amino-1,3,4-thiadiazol-2-yl)-4-chloro-6-methylphenyl]-3-bromo-1-(3-chloro-2-pyridinyl)-1H-pyrazole-5-carboxamide;
M.29: Chordotonal organ Modulators: flonicamid;
M.30: broflanilide; fluxametamide, isocycloseram;
M.33 acynonapyr;
M.UN. Unknown mode of action: afoxolaner, azadirachtin, amidoflumet, ben-zoximate, bromopropylate, chino¬methionat, cryolite, cyproflanilid, dicloromezotiaz, dicofol, dimpropyridaz, flufenerim, flometoquin, fluensulfone, fluhexafon, fluopyram, fluralaner, metaldehyde, metoxadiazone, mivorilaner, modoflaner, piperonyl butoxide, pyridalyl, tioxazafen, trifluen-furonate, umifoxolaner, 11-(4-chloro-2,6-dimethylphenyl)-12-hydroxy-1,4-dioxa-9-aza-dispiro[4.2.4.2]-tetradec-11-en-10-one, 3-(4'-fluoro-2,4-dimethylbiphenyl-3-yl)-4-hydroxy-8-oxa-1-azaspiro[4.5]dec-3-en-2-one, 4-cyano-N-[2-cyano-5-[[[2,6-dibromo-4-[1,2,2,3,3,3-hexafluoro-1-(trifluoromethyl)propyl]phenyl]amino]carbonyl]phenyl]-2-methyl-benzamide, 4-cyano-3-[(4-cyano-2-methyl-benzoyl)amino]-N-[2,6-dichloro-4-[1,2,2,3,3,3-hexafluoro-1-(trifluoromethyl)propyl]phenyl]-2-fluoro-benzamide, N-[5-[[[2-chloro-6-cyano-4-[1,2,2,3,3,3-hexafluoro-1-(trifluoromethyl)propyl]phenyl]amino]carbonyl]-2-cyano-phenyl]-4-cyano-2-methyl-benzamide, N-[5-[[[2-bromo-6-chloro-4-[2,2,2-trifluoro-1-hydroxy-1-(trifluoromethyl)ethyl]phenyl]amino]carbonyl]-2-cyano-phenyl]-4-cyano-2-methyl-benzamide, N-[5-[[[2-bromo-6-chloro-4-[1,2,2,3,3,3-hexafluoro-1-(trifluoromethyl)propyl]phenyl]amino]carbonyl]-2-cyano-phenyl]-4-cyano-2-methyl-benzamide, 4-cyano-N-[2-cyano-5-[[[2,6-dichloro-4-[1,2,2,3,3,3-hexafluoro-1-(trifluoromethyl)propyl]phenyl]amino]carbonyl]phenyl]-2-methyl-benzamide, 1-[2-fluoro-4-methyl-5-[(2,2,2-trifluoroethyl)sulfinyl]phenyl]-3-(trifluoromethyl)-1H-1,2,4-triazole-5-amine, N-[5-[[[2-bromo-6-chloro-4-[1,2,2,2-tetrafluoro-1-(trifluoromethyl)ethyl]phenyl]amino]carbonyl]-2-cyano-phenyl]-4-cyano-2-methyl-benzamide, 4-cyano-N-[2-cyano-5-[[[2,6-dichloro-4-[1,2,2,2-tetrafluoro-1-(trifluoromethyl)ethyl]phenyl]amino]carbonyl]phenyl]-2-methyl-benzamide, actives on basis of *bacillus firmus* (Votivo, I-1582); fluazaindolizine; 5-[3-[2,6-dichloro-4-(3,3-dichloroallyloxy)phenoxy]propoxy]-1H-pyrazole; N-[5-[[2-bromo-6-chloro-4-[1,2,2,3,3,3-hexafluoro-1-(trifluoromethyl)-propyl]phenyl]carbamoyl]-2-cyano-phenyl]-4-cyano-2-methyl-benzamide; 4-cyano-N-[2-cyano-5-[[2,6-dichloro-4-[1,2,2,3,3,3-hexafluoro-1-(trifluoromethyl)-propyl]phenyl]carbamoyl]phenyl]-2-methyl-benzamide; 4-cyano-N-[2-cyano-5-[[2,6-dichloro-4-[1,2,2,2-tetrafluoro-1-(trifluoromethyl)ethyl]phenyl]carbamoyl]¬phenyl]-2-methyl-benzamide; N-[5-[[2-bromo-6-chloro-4-[1,2,2,2-tetrafluoro-1-(trifluoromethyl)ethyl]phenyl]carba¬moyl]-2-cyano-phenyl]-4-cyano-2-methyl-benzamide;
2-(1,3-dioxan-2-yl)-6-[2-(3-pyridinyl)-5-thiazolyl]-pyridine; 2-[6-[2-(5-fluoro-3-pyridinyl)-5-thiazo-lyl]-2-pyridinyl]-pyrimidine; 2-[6-[2-(3-pyridinyl)-5-thiazolyl]-2-pyridinyl]-pyrimidine; N-methylsuhfonyl-6-[2-(3-pyridyl)thiazol-5-yl]pyridine-2-carboxamide; N-methylsulfonyl-6-[2-(3-pyridyl)thiazol-5-yl]pyridine-2-carboxamide; 1-[(6-chloro-3-pyridinyl)methyl]-1,2,3,5,6,7-hexahydro-5-methoxy-7-methyl-8-nitro-imidazo[1,2-a]pyridine; 1-[(6-chloropyridin-3-yl)methyl]-7-methyl-8-nitro-1,2,3,5,6,7-hexahydroimidazo[1,2-a]pyridin-5-ol; N-(3-chloro-2-methylphenyl)-2-[(4-chlorophenyl)[4-[methyl(methylsulfonyl)amino]phenyl]methylene]-hydrazinecarboxamide; 1-[(6-chloro-3-pyridinyl)methyl]-1,2,3,5,6,7-hexahydro-5-methoxy-7-methyl-8-nitro-imidazo[1,2-a]pyridine; 2-(3-pyridinyl)-N-(2-pyrimidinylmethyl )-2H-indazole-5-carboxamide; tyclopyrazoflor; sarolaner, lotilaner; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; N-[4-chloro-3-[[(phenylmethyl)amino]carbonyl]phenyl]-1-methyl-3-(1,1,2,2,2-pentafluoroethyl)-4-(trifluoromethyl)-1H-pyrazole-5-carboxamide; 2-(3-ethylsulfonyl-2-pyridyl)-3-methyl-6-(tri-fluoromethyl)imidazo[4,5-b]pyridine, 2-[3-ethylsulfonyl-5-(trifluoromethyl)-2-pyridyl]-3-methyl-6-(trifluoromethyl)imidazo[4,5-b]pyridine; N-[4-chloro-3-(cyclopropylcarbamoyl)phenyl]-2-methyl-5-(1,1,2,2,2-pentafluoroethyl)-4-(trifluoromethyl)pyrazole-3-carboxamide, N-[4-chloro-3-[(1-cyanocyclopropyl)carbamoyl]phenyl]-2-methyl-5-(1,1,2,2,2-pentafluoroethyl)-4-(trifluoromethyl)pyrazole-3-carboxamide; benzpyrimoxan; tigolaner; oxazosulfyl; [(2S,3R,4R,5S,6S)-3,5-dimethoxy-6-methyl-4-propoxy-tetrahydropyran-2-yl] N-[4-[1-[4-(trifluoromethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(trifluoromethoxy)phenyl]-1,2,4-triazol-3-yl]phenyl]carbamate; [(2S,3R,4R,5S,6S)-3,5-dimethoxy-6-methyl-4-propoxy-tetrahydropyran-2-yl] N-[4-[1-[4-(1,1,2,2,2-pentafluoroethoxy)phenyl]-1,2,4-triazol-3-yl]phenyl]carbamate; [(2S,3R,4R,5S,6S)-3,4,5-trimethoxy-6-methyl-tetrahydropyran-2-yl] N-[4-[1-[4-(1,1,2,2,2-pentafluoroethoxy)phenyl]-1,2,4-triazol-3-yl]phenyl]carbamate; (2Z)-3-(2-isopropylphenyl)-2-[(E)-[4-[1-[4-(trifluoromethoxy)phenyl]-1,2,4-triazol-3-yl]phenyl]methylenehydrazono]thiazolidin-4-one, (2Z)-3-(2-isopropylphenyl)-2-[(E)-[4-[1-[4-(1,1,2,2,2-pentafluoroethoxy)phenyl]-1,2,4-triazol-3-yl]phenyl]methylenehydrazono]thiazolidin-4-one, (2Z)-3-(2-isopro¬pyl¬phenyl)-2-[(E)-[4-[1-[4-(1,1,2,2,2-pentafluoroethoxy)phenyl]-1,2,4-triazol-3-yl]phenyl]methylenehydrazono]thiazolidin-4-one; 2-(6-chloro-3-ethylsulfonyl-imidazo[1,2-a]pyridin-2-yl)-3-methyl-6-(trifluoromethyl)imidazo[4,5-b]pyridine, 2-(6-bromo-3-ethylsulfonyl-imidazo[1,2-a]pyridin-2-yl)-3-methyl-6-(trifluoromethyl)imidazo[4,5-b]pyridine, 2-(3-ethylsulfonyl-6-iodo-imidazo[1,2-a]pyridin-2-yl)-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(7-chloro-3-ethylsulfonyl-imidazo[1,2-a]pyridin-2-yl)-3-methyl-6-(trifluoromethyl)imidazo[4,5-b]pyridine, 2-(3-ethylsulfonyl-7-iodo-imidazo[1,2-a]pyridin-2-yl)-3-methyl-6-(trifluoromethyl)imidazo[4,5-b]pyridine, 3-ethylsulfonyl-6-iodo-2-[3-methyl-6-(trifluoromethyl)imidazo[4,5-b]pyridin-2-yl]imidazo[1,2-a]pyridine-8-carbonitrile, 2-[3-ethylsulfonyl-8-fluoro-6-(trifluoromethyl)imidazo[1,2-a]pyridin-2-yl]-3-methyl-6-(trifluoromethyl)imidazo[4,5-b]pyridine, 2-[3-ethylsulfonyl-7-(trifluoromethyl)imidazo[1,2-a]pyridin-2-yl]-3-methyl-6-(trifluoromethylsulfinyl)imidazo[4,5-b]pyridine, 2-[3-ethylsulfonyl-7-(trifluoromethyl)imidazo[1,2-a]pyridin-2-yl]-3-methyl-6-(trifluoromethyl)imidazo[4,5-c]pyridine, 2-(6-bromo-3-ethylsulfonyl-imidazo[1,2-a]pyridin-2-yl)-6-(trifluoromethyl)pyrazolo[4,3-c]pyridine; N-[[2-fluoro-4-[(2S,3S)-2-hydroxy-3-(3,4,5-trichlorophenyl)-3-(trifluoromethyl)pyrrolidin-1-yl]phenyl]methyl]cyclopropanecarboxamide; 2-[2-fluoro-4-methyl-5-(2,2,2-trifluoroethylsulfinyl)phenyl]imino-3-(2,2,2-trifluoroethyl)thiazolidin-4-one; flupentiofenox, N-[3-chloro-1-(3-pyridyl)pyrazol-4-yl]-2-methylsulfonyl-propanamide, cyclobutrifluram; N-[4-chloro-3-[(1-cyanocyclopropyl)carbamoyl]phenyl]-2-methyl-4-methylsulfonyl-5-(1,1,2,2,2-pentafluoroethyl)pyrazole-3-carboxamide, cyproflanilide, nicofluprole; 1,4-dimethyl-2-[2-(pyridin-3-yl)-2h-indazol-5-yl]-1,2,4-triazolidine-3,5-dione, 2-[2-fluoro-4-methyl-5-(2,2,2-trifluoroethylsulfanyl)phenyl]imino-3-(2,2,2-trifluoroethyl)thiazolidin-4-one, indazapyroxamet, N-[4-chloro-2-(3-pyridyl)thiazol-5-yl]-N-ethyl-3-methylsulfonyl-propanamide, N-cyclopropyl-5-[(5S)-5-(3,5-dichloro-4-fluoro-phenyl)-5-(trifluoromethyl)-4H-isoxazol-3-yl]isoquinoline-8-carboxamide, 5-[(5S)-5-(3,5-dichloro-4-fluoro-phenyl)-5-(trifluoromethyl)-4H-isoxazol-3-yl]-N-(pyrimidin-2-ylmethyl)isoquinoline-8-carboxamide, N-[1-(2,6-difluorophenyl)pyrazol-3-yl]-2-(trifluoromethyl)benzamide, 5-((1R,3R)-3-(3,5-Bis(trifluoromethyl)phenyl)-2,2-dichlorocyclopropane-1-carboxamido)-2-chloro-N-(3-(2,2-difluoroacetamido)-2,4-difluorophenyl)benzamide, 1-[6-(2,2-difluoro-7-methyl-[1,3]dioxolo[4,5-f]benzimidazol-6-yl)-5-ethylsulfonyl-3-pyridyl]cyclopropanecarbonitrile, 6-(5-cyclopropyl-3-ethylsulfonyl-2-pyridyl)-2,2-difluoro-7-methyl-[1,3]dioxolo[4,5-f]benzimidazole.

The commercially available compounds M listed above may be found in The Pesticide Manual, 18th Edition, C. MacBean, British Crop Protection Council (2018), or http://bcpcdata.com/pesticide-manual.html, http://www.alanwood.net/pesticides.

The active compounds described by IUPAC nomenclature are known from CN103814937; WO2013/003977, WO2007/101369, WO2018/177970, CN10171577, CN102126994, WO2007/101540, WO2007/043677, WO2011/085575, WO2008/134969, WO2012/034403, WO2006/089633, WO2008/067911, WO2006/043635, WO2009/124707, WO2013/050317, WO2010/060379, WO2010/127926, WO2010/006713, WO2012/000896, WO2007/101369, WO2012/143317, WO2015/038503, EP2910126, WO2015/059039, WO2015/190316, WO2012/126766, WO2009/102736, WO2013/116053, WO2018/052136, WO2015150252, WO2020055955, WO2021158455, WO2013092350, WO201811111, EP3608311, WO2019236274, WO2013092350, WO 2018052136, WO2009102736, WO2016174049, WO2012126766, CN106554335, WO2017054524, CN105153113, WO2022072650; WO2018071327, WO2022101502, WO2012158396, WO2007079162, WO2020013147.

The following list of fungicides, in conjunction with which the compounds of the invention can be used, illustrates the possible combinations:
A) Respiration inhibitors
   - Inhibitors of complex III at Qₒ site: azoxystrobin (A.1.1), coumethoxystrobin (A.1.2), coumoxystrobin (A.1.3), dimoxystrobin (A.1.4), enestroburin (A.1.5), fenaminstrobin (A.1.6), fenoxystrobin/flufenoxystrobin (A.1.7), fluoxastrobin (A.1.8), kresoxim-methyl (A.1.9), mande-strobin (A.1.10), metominostrobin (A.1.11), orysastrobin (A.1.12), picoxystrobin (A.1.13), pyraclostrobin (A.1.14), pyrametostrobin (A.1.15), pyraoxystrobin (A.1.16), trifloxystrobin (A.1.17), 2-(2-(3-(2,6-dichlorophenyl)-1-methyl-allylideneaminooxymethyl)-phenyl)-2-methoxyimino-*N*-methyl-acetamide (A.1.18), pyribencarb (A.1.19), triclopyricarb/chlorodincarb (A.1.20), famoxadone (A.1.21), fenamidone (A.1.21), methyl-*N*-[2-[(1,4-dimethyl-5-phenyl-pyrazol-3-yl)oxylmethyl]phenyl]-*N*-methoxy-carbamate (A.1.22), metyltetraprole (A.1.25), (*Z*,2*E*)-5-[1-(2,4-dichlorophenyl)pyrazol-3-yl]-oxy-2-methoxyimino-*N*,3-dimethyl-pent-3-enamide (A.1.34), (*Z*,2*E*)-5-[1-(4-chlorophenyl)pyrazol-3-yl]oxy-2-methoxyimino-*N*,3-dimethyl-pent-3-enamide (A.1.35), pyriminostrobin (A.1.36), bifujunzhi (A.1.37), 2-(ortho-((2,5-dimethylphenyl-oxymethylen)phenyl)-3-methoxy-acrylic acid methylester (A.1.38);
   - inhibitors of complex III at Qᵢ site: cyazofamid (A.2.1), amisulbrom (A.2.2), [(6S,7R,8R)-8-benzyl-3-[(3-hydroxy-4-methoxy-pyridine-2-carbonyl)amino]-6-methyl-4,9-dioxo-1,5-dioxonan-7-yl] 2-methylpropanoate (A.2.3), fenpicoxamid (A.2.4), florylpicoxamid (A.2.5), metarylpicoxamid (A.2.6);
   - inhibitors of complex II: benodanil (A.3.1), benzovindiflupyr (A.3.2), bixafen (A.3.3), boscalid (A.3.4), carboxin (A.3.5), fenfuram (A.3.6), fluopyram (A.3.7), flutolanil (A.3.8), fluxapyroxad (A.3.9), furametpyr (A.3.10), isofetamid (A.3.11), isopyrazam (A.3.12), mepronil (A.3.13), oxycarboxin (A.3.14), penflufen (A.3.15), penthiopyrad (A.3.16), pydiflumetofen (A.3.17), pyraziflumid (A.3.18), sedaxane (A.3.19), tecloftalam (A.3.20), thifluzamide (A.3.21), inpyrfluxam (A.3.22), pyrapropoyne (A.3.23), fluindapyr (A.3.28), N-[2-[2-chloro-4-(trifluoromethyl)phenoxy]phenyl]-3-(difluoromethyl)-5-fluoro-1-methyl-pyrazole-4-carboxamide (A.3.29), methyl (*E*)-2-[2-[(5-cyano-2-methyl-phenoxy)methyl]phenyl]-3-methoxy-prop-2-enoate (A.3.30), isoflucypram (A.3.31), 2-(difluoromethyl)-*N*-(1,1,3-trimethyl-indan-4-yl)pyridine-3-carboxamide (A.3.32), 2-(difluoromethyl)-*N*-[(3*R*)-1,1,3-trimethylindan-4-yl]pyridine-3-carboxamide (A.3.33), 2-(difluoromethyl)-*N*-(3-ethyl-1,1-dimethyl-indan-4-yl)pyridine-3-carboxamide (A.3.34), 2-(difluoromethyl)-*N*-[(3*R*)-3-ethyl-1,1-dimethyl-indan-4-yl]pyridine-3-carboxamide (A.3.35), 2-(difluoromethyl)-*N*-(1,1-dimethyl-3-propyl-indan-4-yl)pyridine-3-carboxamide (A.3.36), 2-(difluoromethyl)-*N*-[(3*R*)-1,1-dimethyl-3-propyl-indan-4-yl]pyridine-3-carboxamide (A.3.37), 2-(difluoromethyl)-*N*-(3-isobutyl-1,1-dimethyl-indan-4-yl)pyridine-3-carboxamide (A.3.38), 2-(difluoromethyl)-*N*-[(3*R*)-3-isobutyl-1,1-dimethyl-indan-4-yl]pyridine-3-carboxamide (A.3.39) cyclobutrifluram (A.3.24);
   - other respiration inhibitors: diflumetorim (A.4.1); nitrophenyl derivates: binapacryl (A.4.2), dinobuton (A.4.3), dinocap (A.4.4), fluazinam (A.4.5), meptyldinocap (A.4.6), ferimzone (A.4.7); organometal compounds: fentin salts, e.g. fentin-acetate (A.4.8), fentin chloride (A.4.9) or fentin hydroxide (A.4.10); ametoctradin (A.4.11); silthiofam (A.4.12);
B) Sterol biosynthesis inhibitors (SBI fungicides)
   - C14 demethylase inhibitors: triazoles: azaconazole (B.1.1), bitertanol (B.1.2), bromuconazole (B.1.3), cyproconazole (B.1.4), difenoconazole (B.1.5), diniconazole (B.1.6), diniconazole-M (B.1.7), epoxiconazole (B.1.8), fenbuconazole (B.1.9), fluquinconazole (B.1.10), flusilazole (B.1.11), flutriafol (B.1.12), hexaconazole (B.1.13), imibenconazole (B.1.14), ipconazole (B.1.15), metconazole (B.1.17), myclobutanil (B.1.18), oxpoconazole (B.1.19), paclobutrazole (B.1.20), penconazole (B.1.21), propiconazole (B.1.22), prothioconazole (B.1.23), simeconazole (B.1.24), tebuconazole (B.1.25), tetraconazole (B.1.26), triadimefon (B.1.27), triadimenol (B.1.28), triticonazole (B.1.29), uniconazole (B.1.30), 2-(2,4-difluorophenyl)-1,1-difluoro-3-(tetrazol-1-yl)-1-[5-[4-(2,2,2-trifluoroethoxy)phenyl]-2-pyridyl]propan-2-ol (B.1.31), 2-(2,4-difluorophenyl)-1,1-difluoro-3-(tetrazol-1-yl)-1-[5-[4-(trifluoromethoxy)phenyl]-2-pyridyl]propan-2-ol (B.1.32), fluoxytioconazole (B.1.33), ipfentrifluconazole (B.1.37), mefentrifluconazole (B.1.38), (2*R*)-2-[4-(4-chlorophenoxy)-2-(trifluoromethyl)phenyl]-1-(1,2,4-triazol-1-yl)propan-2-ol, (2S)-2-[4-(4-chlorophenoxy)-2-(trifluoromethyl)phenyl]-1-(1,2,4-triazol-1-yl)propan-2-ol, 2-(chloromethyl)-2-methyl-5-(*p*-tolylmethyl)-1-(1,2,4-triazol-1-ylmethyl)cyclopentanol (B.1.43); imidazoles: imazalil (B.1.44), pefurazoate (B.1.45), prochloraz (B.1.46), triflumizol (B.1.47); pyrimidines, pyridines, piperazines: fenarimol (B.1.49), pyrifenox (B.1.50), triforine (B.1.51), [3-(4-chloro-2-fluoro-phenyl)-5-(2,4-difluorophenyl)isoxazol-4-yl]-(3-pyridyl)methanol (B.1.52), 4-[[6-[2-(2,4-difluorophenyl)-1,1-difluoro-2-hydroxy-3-(1,2,4-triazol-1-yl)propyl]-3-pyridyl]oxy]benzonitrile (B.1.53), 2-[6-(4-bromophenoxy)-2-(trifluoromethyl)-3-pyridyl]-1-(1,2,4-triazol-1-yl)propan-2-ol (B.1.54), 2-[6-(4-chlorophenoxy)-2-(trifluoromethyl)-3-pyridyl]-1-(1,2,4-triazol-1-yl)propan-2-ol (B.1.55), methyl 2-[2-chloro-4-(4-chlorophenoxy)phenyl]-2-hydroxy-3-(1,2,4-triazol-1-yl)propanoate (B.1.56), methyl 2-[2-chloro-4-(4-chlorophenoxy)phenyl]-2-hydroxy-3-(1,2,4-triazol-1-yl)propanoic acid (B.1.57);
   - Delta14-reductase inhibitors: aldimorph (B.2.1), dodemorph (B.2.2), dodemorph-acetate (B.2.3), fenpropimorph (B.2.4), tridemorph (B.2.5), fenpropidin (B.2.6), piperalin (B.2.7), spiroxamine (B.2.8);
   - Inhibitors of 3-keto reductase: fenhexamid (B.3.1);
   - Other Sterol biosynthesis inhibitors: chlorphenomizole (B.4.1);
C) Nucleic acid synthesis inhibitors
   - phenylamides or acyl amino acid fungicides: benalaxyl (C.1.1), benalaxyl-M (C.1.2), kiralaxyl (C.1.3), metalaxyl (C.1.4), metalaxyl-M (C.1.5), ofurace (C.1.6), oxadixyl (C.1.7);
   - other nucleic acid synthesis inhibitors: hymexazole (C.2.1), octhilinone (C.2.2), oxolinic acid (C.2.3), bupirimate (C.2.4), 5-fluorocytosine (C.2.5), 5-fluoro-2-(p-tolylmethoxy)pyrimidin-4-amine (C.2.6), 5-fluoro-2-(4-fluorophenylmethoxy)pyrimidin-4-amine (C.2.7), 5-fluoro-2-(4-chlorophenylmethoxy)pyrimidin-4 amine (C.2.8);
D) Inhibitors of cell division and cytoskeleton
   - tubulin inhibitors: benomyl (D.1.1), carbendazim (D.1.2), fuberidazole (D1.3), thiabendazole (D.1.4), thiophanate-methyl (D.1.5), pyridachlometyl (D.1.6), *N*-ethyl-2-[(3-ethynyl-8-methyl-6-quinolyl)oxy]butanamide (D.1.8), *N*-ethyl-2-[(3-ethynyl-8-methyl-6-quinolyl)oxy]-2-methylsulfanyl-acetamide (D.1.9), 2-[(3-ethynyl-8-methyl-6-quinolyl)oxy]-*N*-(2-fluoroethyl)butanamide (D.1.10), 2-[(3-ethynyl-8-methyl-6-quinolyl)oxy]-*N*-(2-fluoroethyl)-2-methoxy-acetamide (D.1.11), 2-[(3-ethynyl-8-methyl-6-quinolyl)oxy]-*N*-propyl-butanamide (D.1.12), 2-[(3-ethynyl-8-methyl-6-quinolyl)oxy]-2-methoxy-*N*-propyl-acetamide (D.1.13), 2-[(3-ethynyl-8-methyl-6-quinolyl)oxy]-2-methylsulfanyl-*N*-propyl-acetamide (D.1.14), 2-[(3-ethynyl-8-methyl-6-quinolyl)oxy]-*N*-(2-fluoroethyl)-2-methylsulfanyl-acetamide (D.1.15), 4-(2-bromo-4-fluorophenyl)-*N*-(2-chloro-6-fluoro-phenyl)-2,5-dimethyl-pyrazol-3-amine (D.1.16);
   - other cell division inhibitors: diethofencarb (D.2.1), ethaboxam (D.2.2), pencycuron (D.2.3), fluopicolide (D.2.4), zoxamide (D.2.5), metrafenone (D.2.6), pyriofenone (D.2.7), phenamacril (D.2.8);
E) Inhibitors of amino acid and protein synthesis
   - methionine synthesis inhibitors: cyprodinil (E.1.1), mepanipyrim (E.1.2), pyrimethanil (E.1.3);
   - protein synthesis inhibitors: blasticidin-S (E.2.1), kasugamycin (E.2.2), kasugamycin hydrochloride-hydrate (E.2.3), mildiomycin (E.2.4), streptomycin (E.2.5), oxytetracyclin (E.2.6);
F) Signal transduction inhibitors
   - MAP / histidine kinase inhibitors: fluoroimid (F.1.1), iprodione (F.1.2), procymidone (F.1.3), vinclozolin (F.1.4), fludioxonil (F.1.5);
   - G protein inhibitors: quinoxyfen (F.2.1);
G) Lipid and membrane synthesis inhibitors
   - Phospholipid biosynthesis inhibitors: edifenphos (G.1.1), iprobenfos (G.1.2), pyrazophos (G.1.3), isoprothiolane (G.1.4);
   - lipid peroxidation: dicloran (G.2.1), quintozene (G.2.2), tecnazene (G.2.3), tolclofos-methyl (G.2.4), biphenyl (G.2.5), chloroneb (G.2.6), etridiazole (G.2.7), zinc thiazole (G.2.8);
   - phospholipid biosynthesis and cell wall deposition: dimethomorph (G.3.1), flumorph (G.3.2), mandipropamid (G.3.3), pyrimorph (G.3.4), benthiavalicarb (G.3.5), iprovalicarb (G.3.6), valifenalate (G.3.7);
   - compounds affecting cell membrane permeability and fatty acides: propamocarb (G.4.1);
   - inhibitors of oxysterol binding protein: oxathiapiprolin (G.5.1), fluoxapiprolin (G.5.3), 4-[1-[2-[3-(difluoromethyl)-5-methyl-pyrazol-1-yl]acetyl]-4-piperidyl]-*N*-tetralin-1-yl-pyridine-2-carboxamide (G.5.4), 4-[1-[2-[3,5-bis(difluoromethyl)pyrazol-1-yl]acetyl]-4-piperidyl]-*N*-tetralin-1-yl-pyridine-2-carboxamide (G.5.5), 4-[1-[2-[3-(difluoromethyl)-5-(trifluoromethyl)pyrazol-1-yl]acetyl]-4-piperidyl]-*N*-tetralin-1-yl-pyridine-2-carboxamide (G.5.6), 4-[1-[2-[5-cyclopropyl-3-(difluoromethyl)pyrazol-1-yl]acetyl]-4-piperidyl]-*N*-tetralin-1-yl-pyridine-2-carboxamide (G.5.7), 4-[1-[2-[5-methyl-3-(trifluoromethyl)pyrazol-1-yl]acetyl]-4-piperidyl]-*N*-tetralin-1-yl-pyridine-2-carboxamide (G.5.8), 4-[1-[2-[5-(difluoromethyl)-3-(trifluoromethyl)pyrazol-1-yl]acetyl]-4-piperidyl]-*N*-tetralin-1-yl-pyridine-2-carboxamide (G.5.9), 4-[1-[2-[3,5-bis(trifluoromethyl)pyrazol-1-yl]acetyl]-4-piperidyl]-*N*-tetralin-1-yl-pyridine-2-carboxamide (G.5.10), (4-[1-[2-[5-cyclopropyl-3-(trifluoromethyl)pyrazol-1-yl]acetyl]-4-piperidyl]-*N*-tetralin-1-yl-pyridine-2-carboxamide (G.5.11);
H) Inhibitors with Multi Site Action
   - inorganic active substances: Bordeaux mixture (H.1.1), copper (H.1.2), copper acetate (H.1.3), copper hydroxide (H.1.4), copper oxychloride (H.1.5), basic copper sulfate (H.1.6), sulfur (H.1.7);
   - thio- and dithiocarbamates: ferbam (H.2.1), mancozeb (H.2.2), maneb (H.2.3), metam (H.2.4), metiram (H.2.5), propineb (H.2.6), thiram (H.2.7), zineb (H.2.8), ziram (H.2.9);
   - organochlorine compounds: anilazine (H.3.1), chlorothalonil (H.3.2), captafol (H.3.3), captan (H.3.4), folpet (H.3.5), dichlofluanid (H.3.6), dichlorophen (H.3.7), hexachlorobenzene (H.3.8), pentachlorphenole (H.3.9) and its salts, phthalide (H.3.10), tolylfluanid (H.3.11);
   - guanidines and others: guanidine (H.4.1), dodine (H.4.2), dodine free base (H.4.3), guazatine (H.4.4), guazatine-acetate (H.4.5), iminoctadine (H.4.6), iminoctadine-triacetate (H.4.7), iminoctadine-tris(albesilate) (H.4.8), dithianon (H.4.9), 2,6-dimethyl-1*H*,5*H*-[1,4]dithiino[2,3-c:5,6-c']dipyrrole-1,3,5,7(2*H*,6*H*)-tetraone (H.4.10);
I) Cell wall synthesis inhibitors
   - inhibitors of glucan synthesis: validamycin (I.1.1), polyoxin B (I.1.2);
   - melanin synthesis inhibitors: pyroquilon (I.2.1), tricyclazole (I.2.2), carpropamid (I.2.3), dicyclomet (I.2.4), fenoxanil (I.2.5);
J) Plant defence inducers
   - acibenzolar-S-methyl (J.1.1), probenazole (J.1.2), isotianil (J.1.3), tiadinil (J.1.4), prohexadione-calcium (J.1.5); phosphonates: fosetyl (J.1.6), fosetyl-aluminum (J.1.7), phosphorous acid and its salts (J.1.8), calcium phosphonate (J.1.11), potassium phosphonate (J.1.12), potassium or sodium bicarbonate (J.1.9), 4-cyclopropyl-*N*-(2,4-dimethoxyphenyl)thiadiazole-5-carboxamide (J.1.10);
K) Unknown mode of action
   - bronopol (K.1.1), chinomethionat (K.1.2), cyflufenamid (K.1.3), cymoxanil (K.1.4), dazomet (K.1.5), debacarb (K.1.6), diclocymet (K.1.7), diclomezine (K.1.8), difenzoquat (K.1.9), difenzoquat-methylsulfate (K.1.10), diphenylamin (K.1.11), fenitropan (K.1.12), fenpyrazamine (K.1.13), flumetover (K.1.14), flumetylsulforim (K.1.60), flusulfamide (K.1.15), flutianil (K.1.16), harpin (K.1.17), methasulfocarb (K.1.18), nitrapyrin (K.1.19), nitrothal-isopropyl (K.1.20), tolprocarb (K.1.21), oxin-copper (K.1.22), proquinazid (K.1.23), seboctylamine (K.1.61), tebufloquin (K.1.24), tecloftalam (K.1.25), triazoxide (K.1.26), *N'*-(4-(4-chloro-3-trifluoromethyl-phenoxy)-2,5-dimethyl-phenyl)-*N*-ethyl-*N*-methyl formamidine (K.1.27), *N'*-(4-(4-fluoro-3-trifluoromethyl-phenoxy)-2,5-dimethyl-phenyl)-*N*-ethyl-*N*-methyl formamidine (K.1.28), *N'*-[4-[[3-[(4-chlorophenyl)methyl]-1,2,4-thiadiazol-5-yl]oxy]-2,5-dimethyl-phenyl]-*N*-ethyl-*N*-methyl-formamidine (K.1.29), *N'*-(5-bromo-6-indan-2-yloxy-2-methyl-3-pyridyl)-*N*-ethyl-*N*-methyl-formamidine (K.1.30), *N'*-[5-bromo-6-[1-(3,5-difluorophenyl)ethoxy]-2-methyl-3-pyridyl]-*N-*ethyl-*N*-methyl-formamidine (K.1.31), *N*'-[5-bromo-6-(4-isopropylcyclohexoxy)-2-methyl-3-pyridyl]-*N*-ethyl-*N*-methyl-formamidine (K.1.32), *N'*-[5-bromo-2-methyl-6-(1-phenylethoxy)-3-pyridyl]-*N*-ethyl-*N*-methyl-formamidine (K.1.33), *N'*-(2-methyl-5-trifluoromethyl-4-(3-trimethyl-silanyl-propoxy)-phenyl)-*N*-ethyl-*N*-methyl formamidine (K.1.34), *N*'-(5-difluoromethyl-2-methyl-4-(3-trimethylsilanyl-propoxy)-phenyl)-*N*-ethyl-*N*-methyl formamidine (K.1.35), 2-(4-chloro-phenyl)-*N*-[4-(3,4-dimethoxy-phenyl)-isoxazol-5-yl]-2-prop-2-ynyloxy-acetamide (K.1.36), 3-[5-(4-chloro-phenyl)-2,3-dimethyl-isoxazolidin-3-yl]-pyridine (pyrisoxazole) (K.1.37), 3-[5-(4-methylphenyl)-2,3-dimethyl-isoxazolidin-3-yl]-pyridine (K.1.38), 5-chloro-1-(4,6-dimethoxy-pyrimidin-2-yl)-2-methyl-1*H*-benzoimidazole (K.1.39), ethyl (Z)-3-amino-2-cyano-3-phenyl-prop-2-enoate (K.1.40), picarbutrazox (K.1.41), pentyl *N*-[6-[[(Z)-[(1-methyltetrazol-5-yl)-phenyl-methylene]amino]oxymethyl]-2-pyridyl]carbamate (K.1.42), but-3-ynyl *N*-[6-[[(Z)-[(1-methyltetrazol-5-yl)-phenyl-methylene]amino]oxymethyl]-2-pyridyl]carbamate (K.1.43), ipflufenoquin (K.1.44), quinofumelin (K.1.47), benziothiazolinone (K.1.48), bromotha-lonil (K.1.49), 2-(6-benzyl-2-pyridyl)quinazoline (K.1.50), 2-[6-(3-fluoro-4-methoxy-phenyl)-5-methyl-2-pyridyl]quinazoline (K.1.51), dichlobentiazox (K.1.52), *N'*-(2,5-dimethyl-4-phenoxyphenyl)-*N*-ethyl-*N*-methyl-formamidine (K.1.53), aminopyrifen (K.1.54), fluopimomide (K.1.55), *N*-[5-bromo-2-methyl-6-(1-methyl-2-propoxy-ethoxy)-3-pyridyl]-*N*-ethyl-*N*-methyl-formamidine (K.1.56), *N'*-[4-(4,5-dichlorothiazol-2-yl)oxy-2,5-dimethyl-phenyl]-*N*-ethyl-*N*-methyl-formamidine (K.1.57), flufenoxadiazam (K.1.58), *N*-methyl-4-[5-(trifluoromethyl)-1,2,4-oxadia-zol-3-yl]benzenecarbothioamide (K.1.59), *N*-methoxy-*N*-[[4-[5-(trifluoromethyl)-1,2,4-oxadia-zol-3-yl]phenyl]methyl]cyclopropanecarboxamide (K.1.60; WO2018/177894, WO 2020/212513), *N*-((4-[5-(trifluoromethyl)-1,2,4-oxadiazol-3-yl]phenyl)methyl)propanamide (K.1.62), 3,3,3-trifluoro-*N*-[[3-fluoro-4-[5-(trifluoromethyl)-1,2,4-oxadiazol-3-yl]phenyl]methyl]propanamide (K.1.63), 3,3,3-trifluoro-*N*-[[2-fluoro-4-[5-(trifluoromethyl)-1,2,4-oxadiazol-3-yl]phenyl]methyl]propanamide (K.1.64), *N*-[2,3-difluoro-4-[5-(trifluoromethyl)-1,2,4-oxadiazol-3-yl]benzyl]butanamide (K.1.65), *N*-[[2,3-difluoro-4-[5-(trifluoromethyl)-1,2,4-oxadiazol-3-yl]phenyl]methyl]-3,3,3-trifluoro-propanamide (K.1.66), 1-methoxy-1-methyl-3-[[4-[5-(trifluoromethyl)-1,2,4-oxadiazol-3-yl]phenyl]methyl]urea (K.1.67), 1,1-diethyl-3-[[4-[5-[trifluoromethyl]-1,2,4-oxadiazol-3-yl]phenyl]methyl]urea (K.1.68), *N*,2-dimethoxy-*N*-[[4-[5-(trifluoromethyl)-1,2,4-oxadiazol-3-yl]phenyl]methyl]propanamide (K.1.69), *N*-ethyl-2-methyl-*N*-[[4-[5-(trifluoromethyl)-1,2,4-oxadiazol-3-yl]phenyl]methyl]propanamide (K.1.70), 1-methoxy-3-methyl-1-[[4-[5-(trifluoromethyl)-1,2,4-oxadiazol-3-yl]phenyl]methyl]urea (K.1.71), 1-[[4-[5-(trifluoromethyl)-1,2,4-oxadiazol-3-yl]phenyl]methyl]pyrrolidin-2-one (K.1.72), 1-[[4-[5-(trifluoromethyl)-1,2,4-oxadiazol-3-yl]phenyl]methyl]piperidin-2-one (K.1.73), 4-[[4-[5-(trifluoromethyl)-1,2,4-oxadia-zol-3-yl]phenyl]methyl]morpholin-3-one (K.1.74), 4,4-dimethyl-2-[[4-[5-(trifluoromethyl)-1,2,4-oxadiazol-3-yl]phenyl]methyl]isoxazolidin-3-one (K.1.75), 2-[[4-[5-(trifluoromethyl)-1,2,4-oxadi-azol-3-yl]phenyl]methyl]isoxazolidin-3-one (K.1.76), 5,5-dimethyl-2-[[4-[5-(trifluoromethyl)-1,2,4-oxadiazol-3-yl]phenyl]methyl]isoxazolidin-3-one (K.1.77), 3,3-dimethyl-1-[[4-[5-(trifluoromethyl)-1,2,4-oxadiazol-3-yl]phenyl]methyl]piperidin-2-one (K.1.78), 2-[[4-[5-(trifluoromethyl)-1,2,4-oxadiazol-3-yl]phenyl]methyl]oxazinan-3-one (K.1.79), 1-[[3-fluoro-4-[5-(trifluoromethyl)-1,2,4-oxadiazol-3-yl]phenyl]methyl]azepan-2-one (K.1.80), 4,4-dimethyl-1-[[4-[5-(trifluoromethyl)-1,2,4-oxadiazol-3-yl]phenyl]methyl]pyrrolidin-2-one (K.1.81), 5-methyl-1-[[4-[5-(trifluoromethyl)-1,2,4-oxadiazol-3-yl]phenyl]methyl]pyrrolidin-2-one (K.1.82), ethyl 1-[[4-[5-(trifluoromethyl)-1,2,4-oxadiazol-3-yl]phenyl]methyl]pyrazole-4-carboxylate (K.1.83), *N*-methyl-1-[[4-[5-(trifluoromethyl)-1,2,4-oxadiazol-3-yl]phenyl]methyl]pyrazole-4-carboxamide (K.1.84), N,N-dimethyl-1-[4-[5-(trifluoromethyl)-1,2,4-oxadiazol-3-yl]benzyl]-1H-1,2,4-triazol-3-amine (K.1.85), *N*-methoxy-*N*-methyl-1-[[4-[5-(trifluoromethyl)-1,2,4-oxadiazol-3-yl]phenyl]methyl]pyrazole-4-carboxamide (K.1.86), propyl-1-[[4-[5-(trifluoromethyl)-1,2,4-oxadiazol-3-yl]phenyl]methyl]-pyrazole-4-carboxamide (K.1.87), *N*-methoxy-1-[[4-[5-(trifluoromethyl)-1,2,4-oxadiazol-3-yl]phenyl]methyl]pyrazole-4-carboxamide (K.1.88), *N*-allyl-*N*-[[4-[5-(trifluoromethyl)-1,2,4-oxadiazol-3-yl]phenyl]methyl]propanamide (K.1.89), 3-ethyl-1-methoxy-1-[[4-[5-(trifluoromethyl)-1,2,4-oxadiazol-3-yl]phenyl]methyl]urea (K.1.90), 1,3-dimethoxy-1-[[4-[5-(trifluoromethyl)-1,2,4-oxadiazol-3-yl]phenyl]methyl]urea (K.1.91), *N*-allyl-*N*-[[4-[5-(trifluoromethyl)-1,2,4-oxadiazol-3-yl]phenyl]methyl]acetamide (K.1.92), *N*-[4-[5-(trifluoromethyl)-1,2,4-oxadia-zol-3-yl]benzyl]cyclopropanecarboxamide (K.1.93), 1-methyl-3-[[4-[5-(trifluoromethyl)-1,2,4-oxadiazol-3-yl]phenyl]methyl]urea (K.1.94), *N'*-[2-chloro-4-(2-fluorophenoxy)-5-methyl-phenyl]-*N*-ethyl-*N*-methyl-formamidine (K.1.95), *N*-[2-chloro-4-[(4-methoxy-phenyl)methyl]-5-methyl-phenyl]-*N*-ethyl-*N*-methyl-formamidine (K.1.96), *N'*-[2-chloro-4-[(4-cyano-phenyl)methyl]-5-methyl-phenyl]-*N*-ethyl-*N*-methyl-formamidine (K.1.97), *N'*-[2,5-dimethyl-4-(*o*-tolylmethyl)phenyl]-*N*-ethyl-*N*-methyl-formamidine (K.1.98), 6-chloro-3-(3-cyclopropyl-2-fluoro-phenoxy)-*N*-[2-(2,4-dimethylphenyl)-2,2-difluoro-ethyl]-5-methyl-pyridazine-4-carboxamide (K.1.99), 3-(3-bromo-2-fluoro-phenoxy)-6-chloro-*N*-[2-(2-chloro-4-methyl-phenyl)-2,2-difluoroethyl]-5-methyl-pyridazine-4-carboxamide (K.1.100), 6-chloro-*N*-[2-(2-chloro-4-methyl-phenyl)-2,2-difluoro-ethyl]-3-(3-cyclopropyl-2-fluoro-phenoxy)-5-methyl-pyridazine-4-carboxamide (K.1.101), 6-chloro-3-(3-cyclopropyl-2-fluoro-phenoxy)-*N*-[2-(3,4-dimethylphenyl)-2,2-difluoro-ethyl]-5-methyl-pyridazine-4-carboxamide (K.1.102), 6-chloro-3-(3-chloro-2-fluoro-phenoxy)-*N*-[2-(2,4-dimethylphenyl)-2,2-difluoro-ethyl]-5-methyl-pyridazine-4-carboxamide (K.1.103), *N*-[2-(2-bromo-4-methyl-phenyl)-2,2-difluoro-ethyl]-6-chloro-3-(3-cyclopropyl-2-fluoro-phenoxy)-5-methyl-pyridazine-4-carboxamide (K.1.104);

The fungicides described by common names, their preparation and their activity e.g. against harmful fungi is known (cf.: http://www.alanwood.net/pesticides/); these substances are commercially available.

The active substances referred to as component 2, their preparation and their activity e.g. against harmful fungi is known (cf.: https://pesticidecompendium.bcpc.org/); these substances are commercially available. The compounds described by IUPAC nomenclature, their preparation and their pesticidal activity are also known (cf. Can. J. Plant Sci. 48(6), 587-94, 1968; EP-A 141 317; EP-A 152031; EP-A 226 917; EP-A 243 970; EP-A 256 503; EP-A 428 941; EP-A 532 022; EP-A 1 028 125; EP-A 1 035 122; EP-A 1 201 648; EP-A 1 122 244, JP 2002316902; DE 19650197; DE 10021412; DE 102005009458; US 3,296,272; US 3,325,503; WO 98/46608; WO 99/14187; WO 99/24413; WO 99/27783; WO 00/29404; WO 00/46148; WO 00/65913; WO 01/54501; WO 01/56358; WO 02/22583; WO 02/40431; WO 03/10149; WO 03/11853; WO 03/14103; WO 03/16286; WO 03/53145; WO 03/61388; WO 03/66609; WO 03/74491; WO 04/49804; WO 04/83193; WO 05/120234; WO 05/123689; WO 05/123690; WO 05/63721; WO 05/87772; WO 05/87773; WO 06/15866; WO 06/87325; WO 06/87343; WO 07/82098; WO 07/90624, WO 10/139271, WO 11/028657, WO 12/168188, WO 07/006670, WO 11/77514; WO 13/047749, WO 10/069882, WO 13/047441, WO 03/16303, WO 09/90181, WO 13/007767, WO 13/010862, WO 13/127704, WO 13/024009, WO 13/24010, WO 13/047441, WO 13/162072, WO 13/092224, WO 11/135833, CN 1907024, CN 1456054, CN 103387541, CN 1309897, WO 12/84812, CN 1907024, WO 09094442, WO 14/60177, WO 13/116251, WO 08/013622, WO 15/65922, WO 94/01546, EP 2865265, WO 07/129454, WO 12/165511, WO 11/081174, WO 13/47441, WO 16/156241, WO 16/162265). Some compounds are identified by their CAS Registry Number.

### Biopesticides

Suitable mixing partners for the compounds of the invention also include biopesticides. Biopesticides have been defined as a form of pesticides based on micro-organisms (bacteria, fungi, viruses, nematodes, etc.) or natural products (compounds, e.g. metabolites, proteins, or extracts from biological or other natural sources) (U.S. Environmental Protection Agency: http://www.epa.gov/pesticides/biopesticides/). Biopesticides fall into two major classes, microbial and biochemical pesticides:
(1) Microbial pesticides consist of bacteria, fungi or viruses (and often include the metabolites that bacteria and fungi produce). Entomopathogenic nematodes are also classified as microbial pesticides, even though they are multi-cellular.
(2) Biochemical pesticides are naturally occurring substances or or structurally-similar and functionally identical to a naturally-occurring substance and extracts from biological sources that control pests or provide other crop protection uses as defined below, but have non-toxic mode of actions (e.g. growth or developmental regulation, attractants, repellents or defence activators (e.g. induced resistance) and are relatively non-toxic to mammals.

The following list of biopesticides, in conjunction with which the compounds of the invention can be used, illustrates the possible combinations:
L) Biopesticides
L1) Microbial pesticides with fungicidal, bactericidal, viricidal and/or plant defense activator activity: *Ampelomyces quisqualis, Aspergillus flavus, Aureobasidium pullulans, Bacillus altitu-dinis, B. amyloliquefaciens, B. amyloliquefaciens* ssp. *plantarum* (also referred to as B. *vele-zensis), B. megaterium, B. mojavensis, B. mycoides, B. pumilus, B. simplex,* B. *solisalsi,* B. *subtilis, B. subtilis* var. *amyloliquefaciens, B. velezensis, Candida oleophila,* C. *saitoana, Clavi-bacter michiganensis* (bacteriophages), *Coniothyrium minitans, Cryphonectria parasitica, Cryptococcus albidus, Dilophosphora alopecuri, Fusarium oxysporum, Clonostachys rosea* f. *catenulate* (also named *Gliocladium catenulatum), Gliocladium roseum, Lysobacter antibioticus, L. enzymogenes, Metschnikowia fructicola, Microdochium dimerum, Microsphaeropsis ochracea, Muscodor albus, Paenibacillus alvei, Paenibacillus epiphyticus*, *P. polymyxa, Pantoea vagans, Penicillium bilaiae, Phlebiopsis gigantea*, *Pseudomonas* sp., *Pseudomonas chloraphis, Pseudozyma flocculosa, Pichia anomala, Pythium oligandrum, Sphaerodes mycoparasitica, Streptomyces griseoviridis, S. lydicus, S. violaceusniger, Talaromyces flavus, Trichoderma asperelloides, T. asperellum, T. atroviride, T. fertile, T. gamsii, T. harmatum, T. harzianum, T. polysporum, T. stromaticum, T. virens, T. viride, Typhula phacorrhiza, Ulocladium oudemansii, Verticillium dahlia,* zucchini yellow mosaic virus (avirulent strain);
L2) Biochemical pesticides with fungicidal, bactericidal, viricidal and/or plant defense activator activity: harpin protein, *Reynoutria sachalinensis* extract;
L3) Microbial pesticides with insecticidal, acaricidal, molluscidal and/or nematicidal activity: *Agrobacterium radiobacter, Bacillus cereus, B*. *firmus, B. thuringiensis, B. thuringiensis* ssp. *aizawai, B. t.* ssp. *israelensis, B. t.* ssp. *galleriae, B. t.* ssp. *kurstaki, B. t.* ssp. *tenebrionis, Beauveria bassiana, B. brongniartii, Burkholderia* spp., *Chromobacterium subtsugae, Cydia pomonella* granulovirus (CpGV), *Cryptophlebia leucotreta* granulovirus (CrleGV), *Flavobacterium* spp., *Helicoverpa armigera* nucleopolyhedrovirus (HearNPV), *Helicoverpa zea* nucleopolyhedrovirus (HzNPV), *Helicoverpa zea* single capsid nucleopolyhedrovirus (HzSNPV), *Heterorhabditis bacteriophora, Isaria fumosorosea, Lecanicillium longisporum, L. muscarium, Metarhizium anisopliae, M. anisopliae* var. *anisopliae, M. anisopliae* var. *acridum, Nomuraea rileyi, Paecilomyces fumosoroseus, P. lilacinus, Paenibacillus popilliae, Pasteuria* spp., *P. nishizawae, P. penetrans, P*. *ramosa, P. thornea, P. usgae, Pseudomonas fluorescens, Spodoptera littoralis* nucleopolyhedrovirus (SpliNPV), *Steinernema carpocapsae, S. feltiae, S*. *kraussei, Streptomyces galbus, S. microflavus;*
L4) Biochemical pesticides with insecticidal, acaricidal, molluscidal, pheromone and/or nematicidal activity: L-carvone, citral, (*E*,*Z*)-7,9-dodecadien-1-yl acetate, ethyl formate, (*E*,*Z*)-2,4-ethyl decadienoate (pear ester), (*Z*,*Z*,*E*)-7,11,13-hexadecatrienal, heptyl butyrate, isopropyl myristate, lavanulyl senecioate, cis-jasmone, 2-methyl 1-butanol, methyl eugenol, methyl jasmonate, (*E*,*Z*)-2,13-octadecadien-1-ol, (*E,Z*)-2,13-octadecadien-1-ol acetate, (*E*,*Z*)-3,13-octadecadien-1-ol, (*R*)-1-octen-3-ol, pentatermanone, (*E*,*Z*,*Z*)-3,8,11-tetradecatrienyl acetate, (*Z*,*E*)-9,12-tetradecadien-1-yl acetate, (*Z*)-7-tetradecen-2-one, (*Z*)-9-tetradecen-1-yl acetate, (*Z*)-11-tetradecenal, (*Z*)-11-tetradecen-1-ol, extract of *Chenopodium ambrosiodes,* Neem oil, Quillay extract;
L5) Microbial pesticides with plant stress reducing, plant growth regulator, plant growth promoting and/or yield enhancing activity: *Azospirillum amazonense, A. brasilense, A. lipoferum, A. irakense, A. halopraeferens, Bradyrhizobium* spp., *B. elkanii, B. japonicum, B. liaoningense, B. lupini, Delftia acidovorans, Glomus intraradices, Mesorhizobium* spp., *Rhizobium legumi-nosarum* bv. *phaseoli, R. I.* bv. *trifolii, R. I.* bv. *viciae, R. tropici, Sinorhizobium meliloti.*

The biopesticides from group L1) and/or L2) may also have insecticidal, acaricidal, molluscidal, pheromone, nematicidal, plant stress reducing, plant growth regulator, plant growth promoting and/or yield enhancing activity. The biopesticides from group L3) and/or L4) may also have fungicidal, bactericidal, viricidal, plant defense activator, plant stress reducing, plant growth regulator, plant growth promoting and/or yield enhancing activity. The biopesticides from group L5) may also have fungicidal, bactericidal, viricidal, plant defense activator, insecticidal, acaricidal, molluscidal, pheromone and/or nematicidal activity.

Many of these biopesticides have been deposited under deposition numbers mentioned herein (the prefices e.g. ATCC or DSM refer to the acronym of the respective culture collection, for details see e.g. here: http://www. wfcc.info/ccinfo/collection/by_acronym/), are referred to in literature, registered and/or are commercially available: mixtures of Aureobasidium pullulans DSM 14940 and DSM 14941 isolated in 1989 in Konstanz, Germany (e.g. blastospores in BlossomProtect^{®} from bio-ferm GmbH, Austria), Azospirillum brasilense Sp245 originally isolated in wheat reagion of South Brazil (Passo Fundo) at least prior to 1980 (BR 11005; e.g. GELFIX^{®} Gramíneas from BASF Agricultural Specialties Ltd., Brazil), A. brasilense strains Ab-V5 and Ab-V6 (e.g. in AzoMax from Novozymes BioAg Produtos papra Agricultura Ltda., Quattro Barras, Brazil or Simbiose-Malz® from Simbiose-Agro, Brazil; Plant Soil 331, 413-425, 2010), Bacillus amyloliquefaciens strain AP-188 (NRRL B-50615 and B-50331; US8,445,255); B. amylo-liquefaciens ssp. plantarum strains formerly also sometimes referred to as B. subtilis, recently together with B. methylotrophicus, and B. velezensis classified as B. velezensis (Int. J. Syst. Evol. Microbiol. 66, 1212-1217, 2016): B. a. ssp. plantarum or B. velezensis D747 isolated from air in Kikugawashi, Japan (US 20130236522 A1; FERM BP 8234; e.g. Double Nickel^{™} 55 WDG from Certis LLC, USA), B. a. ssp. plantarum or B. velezensis FZB24 isolated from soil in Brandenburg, Germany (also called SB3615; DSM 96-2; J. Plant Dis. Prot. 105, 181-197, 1998; e.g. Taegro^{®} from Novozyme Biologicals, Inc., USA), B. a. ssp. plantarum or B. velezensis FZB42 isolated from soil in Brandenburg, Germany (DSM 23117; J. Plant Dis. Prot. 105, 181-197, 1998; e.g. RhizoVital^{®} 42 from AbiTEP GmbH, Germany), B. a. ssp. plantarum or B. vele-zensis MBI600 isolated from faba bean in Sutton Bonington, Nottingham-shire, U.K. at least before 1988 (also called 1430; NRRL B 50595; US 2012/0149571 A1; e.g. Integral® from BASF Corp., USA), B. a. ssp. plantarum or B. velezensis QST-713 isolated from peach orchard in 1995 in California, U.S.A. (NRRL B 21661; e.g. Serenade^{®} MAX from Bayer Crop Science LP, USA), B. a. ssp. plantarum or B. velezensis TJ1000 isolated in 1992 in South Dakoda, U.S.A. (also called 1BE; ATCC BAA-390; CA 2471555 A1; e.g. QuickRoots™ from TJ Technologies, Watertown, SD, USA); B. firmus CNCM I-1582, a variant of parental strain EIP-N1 (CNCM I-1556) isolated from soil of central plain area of Israel (WO 2009/126473, US6,406,690; e.g. Votivo® from Bayer CropScience LP, USA), B. pumilus GHA 180 isolated from apple tree rhizo-sphere in Mexico (IDAC 260707-01; e.g. PRO-MIX^{®} BX from Premier Horticulture, Quebec, Canada), B. pumilus INR-7 otherwise referred to as BU F22 and BU-F33 isolated at least be-fore 1993 from cucumber infested by Erwinia tracheiphila (NRRL B-50185, NRRL B-50153; US 8,445,255), B. pumilus KFP9F isolated from the rhizosphere of grasses in South Africa at least before 2008 (NRRL B-50754; WO 2014/029697; e.g. BAC-UP or FUSION-P from BASF Agricultural Specialities (Pty) Ltd., South Africa), B. pumilus QST 2808 was isolated from soil collected in Pohnpei, Federated States of Micronesia, in 1998 (NRRL B 30087; e.g. Sonata^{®} or Ballad^{®} Plus from Bayer Crop Science LP, USA), B. simplex ABU 288 (NRRL B-50304; US8,445,255), B. subtilis FB17 also called UD 1022 or UD10-22 isolated from red beet roots in North America (ATCC PTA-11857; System. Appl. Microbiol. 27, 372-379, 2004; US2010/0260735; WO 2011/109395); B. thurin¬giensis ssp. aizawai ABTS-1857 isolated from soil taken from a lawn in Ephraim, Wisconsin, U.S.A., in 1987 (also called ABG 6346; ATCC SD-1372; e.g. XenTari^{®} from BioFa AG, Münsingen, Germany), B. t. ssp. kurstaki ABTS-351 identical to HD-1 isolated in 1967 from diseased Pink Bollworm black larvae in Brownsville, Texas, U.S.A. (ATCC SD-1275; e.g. Dipel^{®} DF from Valent BioSciences, IL, USA), B. t. ssp. kurstaki SB4 isolated from E. saccharina larval cadavers (NRRL B-50753; e.g. Beta Pro^{®} from BASF Agricultural Specialities (Pty) Ltd., South Africa), B. t. ssp. tenebrionis NB-176-1, a mutant of strain NB-125, a wild type strain isolated in 1982 from a dead pupa of the beetle Tenebrio molitor (DSM 5480; EP 585 215 B1; e.g. Novodor^{®} from Valent BioSciences, Switzerland), Beauveria bassiana GHA (ATCC 74250; e.g. BotaniGard^{®} 22WGP from Laverlam Int. Corp., USA), B. bassiana JW-1 (ATCC 74040; e.g. Naturalis^{®} from CBC (Europe) S.r.l., Italy), B. bassiana PPRI 5339 isolated from the larva of the tortoise beetle Conchyloctenia punctata (NRRL 50757; e.g. BroadBand^{®} from BASF Agricultural Specialities (Pty) Ltd., South Africa), Brady¬rhizobium elkanii strains SEMIA 5019 (also called 29W) isolated in Rio de Janeiro, Brazil and SEMIA 587 isolated in 1967 in the State of Rio Grande do Sul, from an area previously inoculated with a North American isolate, and used in commercial inoculants since 1968 (Appl. Environ. Microbiol. 73(8), 2635, 2007; e.g. GELFIX 5 from BASF Agricultural Specialties Ltd., Brazil), B. japonicum 532c isolated from Wisconsin field in U.S.A. (Nitragin 61A152; Can. J. Plant. Sci. 70, 661-666, 1990; e.g. in Rhizoflo^{®}, Histick^{®}, Hicoat^{®} Super from BASF Agricultural Specialties Ltd., Canada), B. japonicum E-109 variant of strain USDA 138 (INTA E109, SEMIA 5085; Eur. J. Soil Biol. 45, 28-35, 2009; Biol. Fertil. Soils 47, 81-89, 2011); B. japonicum strains deposited at SEMIA known from Appl. Environ. Microbiol. 73(8), 2635, 2007: SEMIA 5079 isolated from soil in Cerrados region, Brazil by Embrapa-Cerrados used in commercial inoculants since 1992 (CPAC 15; e.g. GELFIX 5 or ADHERE 60 from BASF Agricultural Specialties Ltd., Brazil), B. japonicum SEMIA 5080 obtained under lab condtions by Embrapa-Cerrados in Brazil and used in commercial inoculants since 1992, being a natural variant of SEMIA 586 (CB1809) originally isolated in U.S.A. (CPAC 7; e.g. GELFIX 5 or ADHERE 60 from BASF Agricultural Specialties Ltd., Brazil); Burkholderia sp. A396 isolated from soil in Nikko, Japan, in 2008 (NRRL B-50319; WO 2013/032693; Marrone Bio Innovations, Inc., USA), Coniothyrium minitans CON/M/91-08 isolated from oilseed rape (WO 1996/021358; DSM 9660; e.g. Contans^{®} WG, Intercept^{®} WG from Bayer CropScience AG, Germany), harpin (alpha-beta) protein (Science 257, 85-88, 1992; e.g. Messenger^{™} or HARP-N Tek from Plant Health Care plc, U.K.), Helicoverpa armigera nucleopolyhedrovirus (HearNPV) (J. Invertebrate Pathol. 107, 112-126, 2011; e.g. Helicovex^{®} from Adermatt Biocontrol, Switzerland; Diplomata^{®} from Koppert, Brazil; Vivus^{®} Max from AgBiTech Pty Ltd., Queensland, Australia), Helicoverpa zea single capsid nucleopolyhedrovirus (HzSNPV) (e.g. Gemstar^{®} from Certis LLC, USA), Helicoverpa zea nucleopolyhedrovirus ABA-NPV-U (e.g. Heligen^{®} from AgBiTech Pty Ltd., Queensland, Australia), Heterorhabditis bacteriophora (e.g. Nemasys^{®} G from BASF Agricultural Specialities Limited, UK), Isaria fumosorosea Apopka-97 isolated from mealy bug on gynura in Apopka, Florida, U.S.A. (ATCC 20874; Biocontrol Science Technol. 22(7), 747-761, 2012; e.g. PFR-97^{™} or PreFeRal^{®} from Certis LLC, USA), Metarhizium anisopliae var. anisopliae F52 also called 275 or V275 isolated from codling moth in Austria (DSM 3884, ATCC 90448; e.g. Met52^{®} Novozymes Biologicals BioAg Group, Canada), Metschnikowia fructicola 277 isolated from grapes in the central part of Israel (US 6,994,849; NRRL Y-30752; e.g. formerly Shemer^{®} from Agrogreen, Israel), Paecilomyces ilacinus 251 isolated from infected nematode eggs in the Philippines (AGAL 89/030550; WO1991/02051; Crop Protection 27, 352-361, 2008; e.g. BioAct^{®}from Bayer CropScience AG, Germany and MeloCon^{®} from Certis, USA), Paenibacillus alvei NAS6G6 isolated from the rhizosphere of grasses in South Africa at least before 2008 (WO 2014/029697; NRRL B-50755; e.g. BAC-UP from BASF Agricultural Specialities (Pty) Ltd., South Africa), Paenibacillus strains isolated from soil samples from a variety of European locations including Germany: P. epiphyticus Lu17015 (WO 2016/020371; DSM 26971), P. polymyxa ssp. plantarum Lu16774 (WO 2016/020371; DSM 26969), P. p. ssp. plantarum strain Lu17007 (WO 2016/020371; DSM 26970); Pasteuria nishizawae Pn1 isolated from a soybean field in the mid-2000s in Illinois, U.S.A. (ATCC SD 5833; Federal Register 76(22), 5808, February 2, 2011; e.g. Clariva^{™} PN from Syngenta Crop Protection, LLC, USA), Penicillium bilaiae (also called P. bilaii) strains ATCC 18309 (= ATCC 74319), ATCC 20851 and/or ATCC 22348 (=ATCC 74318) originally isolated from soil in Alberta, Canada (Fertilizer Res. 39, 97-103, 1994; Can. J. Plant Sci. 78(1), 91-102, 1998; US 5,026,417, WO 1995/017806; e.g. Jump Start^{®}, Provided from Novozymes Biologicals BioAg Group, Canada), Reynoutria sachalinensis extract (EP 0307510 B1; e.g. Regalia^{®} SC from Marrone Bio-Innovations, Davis, CA, USA or Milsana^{®} from BioFa AG, Germany), Steinernema carpocapsae (e.g. Millenium^{®} from BASF Agricultural Specialities Limited, UK), S. feltiae (e.g. Nemashield^{®} from BioWorks, Inc., USA; Nemasys^{®} from BASF Agricultural Specialities Limited, UK), Streptomyces microflavus NRRL B-50550 (WO 2014/124369; Bayer CropScience, Germany), Trichoderma asperelloides JM41R isolated in South Africa (NRRL 50759; also referred to as T. fertile; e.g. Trichoplus^{®} from BASF Agricultural Specialities (Pty) Ltd., South Africa), T. harzianum T-22 also called KRL-AG2 (ATCC 20847; BioControl 57, 687-696, 2012; e.g. Plantshield^{®} from BioWorks Inc., USA or SabrEx^{™} from Advanced Biological Marketing Inc., Van Wert, OH, USA).

According to the invention, the solid material (dry matter) of the biopesticides (with the exception of oils e.g. Neem oil) are considered as active components (e.g. to be obtained after drying or evaporation of the extraction or suspension medium in case of liquid formulations of the microbial pesticides).

In accordance with the invention, the weight ratios and percentages used herein for a biological extract e.g. Quillay extract are based on the total weight of the dry content (solid material) of the respective extract(s).

The total weight ratios of compositions comprising at least one microbial pesticide in the form of viable microbial cells including dormant forms, can be determined using the amount of CFU of the respective microorganism to calculate the total weight of the respective active component with the following equation that 1×10¹⁰ CFU equals one gram of total weight of the respective active component. Colony forming unit is measure of viable microbial cells, in particular fungal and bacterial cells. In addition, here "CFU" may also be understood as the number of (juvenile) individual nematodes in case of (entomo¬pathogenic) nematode biopesticides, e.g. Steinernema feltiae.

When mixtures comprising microbial pesticides are employed in crop protection, the application rates range from 1×10⁶ to 5×10¹⁶ (or more) CFU/ha, preferably from 1×10⁸ to 1×10¹³ CFU/ha, and even more preferably from 1×10⁹ to 5×10¹⁵ CFU/ha and in particular from 1×10¹² to 5×10¹⁴ CFU/ha. In the case of nematodes as microbial pesticides (e.g. Steinernema feltiae), the application rates regularly range from 1×10⁵ to 1×10¹² (or more), preferably from 1×10⁸ to 1×10¹¹, more preferably from 5×10⁸ to 1×10¹⁰ individuals (e.g. in the form of eggs, juvenile or any other live stages, preferably in an infetive juvenile stage) per ha.

When mixtures comprising microbial pesticides are employed in seed treatment, the application rates generally range from 1×10⁶ to 1×10¹² (or more) CFU/seed, preferably from 1×10⁶ to 1×10⁹ CFU/seed. Furthermore, the application rates with respect to seed treatment generally range from 1×10⁷ to 1×10¹⁴ (or more) CFU per 100 kg of seed, preferably from 1×10⁹ to 1×10¹² CFU per 100 kg of seed.

The invention also relates to agrochemical compositions comprising an auxiliary and at least one compound of the invention or a mixture thereof.

An agrochemical composition comprises a pesticidally effective amount of a compound of the invention or a mixture thereof.

The compounds of the invention or the mixtures thereof can be converted into customary types of agro-chemical compositions, e.g. solutions, emulsions, suspensions, dusts, powders, pastes, granules, pressings, capsules, and mixtures thereof. Examples for composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials e.g. seeds (e.g. GF). These and further compositions types are defined in the "Catalogue of pesticide formulation types and international coding system", Technical Monograph No. 2, 6th Ed. May 2008, CropLife International.

The compositions are prepared in a known manner, e.g. described by Mollet and Grubemann, Formulation technology, Wiley VCH, Weinheim, 2001; or Knowles, New developments in crop protection product formulation, Agrow Reports DS243, T&F Informa, London, 2005.

Examples for suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, e.g. mineral oil fractions of medium to high boiling point, e.g. kerosene, diesel oil; oils of vegetable or animal origin; hydrocarbons, e.g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, CaSO₄, MgSO₄, MgO; polysaccharide powders, e.g. cellulose, starch; fertilizers, e.g. (NH₄)₂SO₄, (NH₄)₃PO₄, NH₄NO₃, ureas; products of vegetable origin, e.g. cereal meal, tree bark meal, wood meal, nutshell meal, and mixtures thereof.

Suitable surfactants are surface-active compounds, e.g. anionic, cationic, nonionic and amphoteric surfactants, block polymers, polyelectrolytes, and mixtures thereof. Such surfactants can be used as emusifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-subsituted fatty acid amides, amine oxides, esters, sugar-based surfactants, polymeric surfactants, and mixtures thereof. Examples of alkoxylates are compounds e.g. alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-subsititued fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are homo- or copolymers of vinylpyrrolidone, vinylalcohols, or vinylacetate.

Suitable cationic surfactants are quaternary surfactants, e.g. quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines, or polyethyleneamines.

Suitable adjuvants are compounds, which have a neglectable or even no pesticidal activity themselves, and which improve the biological performance of the compounds of the invention on the target. Examples are surfactants, mineral or vegetable oils, and other auxilaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), anor-ganic clays (organically modified or unmodified), polycarboxylates, and silicates.

Suitable bactericides are bronopol and isothiazolinone derivatives e.g. alkylisothiazolinones and benzisothiazolinones.

Suitable anti-freezing agents are ethylene glycol, propylene glycol, urea, and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo-, and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for composition types and their preparation are:

### i) Water-soluble concentrates (SL, LS)

10-60 wt% of a compound I according to the invention and 5-15 wt% wetting agent (e.g. alcohol alkoxylates) are dissolved in water and/or in a water-soluble solvent (e.g. alcohols) up to 100 wt%. The active substance dissolves upon dilution with water.

### ii) Dispersible concentrates (DC)

5-25 wt% of a compound I according to the invention and 1-10 wt% dispersant (e.g. polyvinylpyrrolidone) are dissolved in up to 100 wt% organic solvent (e.g. cyclohexanone). Dilution with water gives a dispersion.

### iii) Emulsifiable concentrates (EC)

15-70 wt% of a compound I according to the invention and 5-10 wt% emulsifiers (e.g. calcium dodecylbenzenesulfonate and castor oil ethoxylate) are dissolved in up to 100 wt% water-insoluble organic solvent (e.g. aromatic hydrocarbon). Dilution with water gives an emulsion.

### iv) Emulsions (EW, EO, ES)

5-40 wt% of a compound I according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into up to 100 wt% water by means of an emulsifying machine and made into a homogeneous emulsion. Dilution with water gives an emulsion.

### v) Suspensions (SC, OD, FS)

In an agitated ball mill, 20-60 wt% of a compound I according to the invention are comminuted with addition of 2-10 wt% dispersants and wetting agents (e.g. sodium lignosulfonate and alcohol ethoxylate), 0,1-2 wt% thickener (e.g. xanthan gum) and up to 100 wt% water to give a fine active substance suspension. Dilution with water gives a stable suspension of the active sub-stance. For FS type composition up to 40 wt% binder (e.g. polyvinylalcohol) is added.

### vi) Water-dispersible granules and water-soluble granules (WG, SG)

50-80 wt% of a compound I according to the invention are ground finely with addition of up to 100 wt% dispersants and wetting agents (e.g. sodium lignosulfonate and alcohol ethoxylate) and prepared as water-dispersible or water-soluble granules by means of technical appliances (e.g. extrusion, spray tower, fluidized bed). Dilution with water gives a stable dispersion or solution of the active substance.

### vii) Water-dispersible powders and water-soluble powders (WP, SP, WS)

50-80 wt% of a compound I according to the invention are ground in a rotor-stator mill with addition of 1-5 wt% dispersants (e.g. sodium lignosulfonate), 1-3 wt% wetting agents (e.g. alcohol ethoxylate) and up to 100 wt% solid carrier, e.g. silica gel. Dilution with water gives a stable dispersion or solution of the active substance.

### viii) Gel (GW, GF)

In an agitated ball mill, 5-25 wt% of a compound I according to the invention are comminuted with addition of 3-10 wt% dispersants (e.g. sodium lignosulfonate), 1-5 wt% thickener (e.g. carboxymethylcellulose) and up to 100 wt% water to give a fine suspension of the active sub-stance. Dilution with water gives a stable suspension of the active substance.

### ix) Microemulsion (ME)

5-20 wt% of a compound I according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water up to 100 %. This mixture is stirred for 1 h to produce spontaneously a thermodynamically stable microemulsion.

### x) Microcapsules (CS)

An oil phase comprising 5-50 wt% of a compound I according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a compound I according to the invention, 0-40 wt% water insoluble organic solvent (e.g. aromatic hydrocarbon), and an isocyanate monomer (e.g. di-phenylme-thene-4,4'-diisocyanatae) are dispersed into an aqueous solution of a protective colloid (e.g. polyvinyl alcohol). The addition of a polyamine (e.g. hexamethylenediamine) results in the for-ation of a polyurea microcapsule. The monomers amount to 1-10 wt%. The wt% relate to the total CS composition.

### xi) Dustable powders (DP, DS)

1-10 wt% of a compound I according to the invention are ground finely and mixed intimately with up to 100 wt% solid carrier, e.g. finely divided kaolin.

### xii) Granules (GR, FG)

0.5-30 wt% of a compound I according to the invention is ground finely and associated with up to 100 wt% solid carrier (e.g. silicate). Granulation is achieved by extrusion, spray-drying or the fluidized bed.

### xiii) Ultra-low volume liquids (UL)

1-50 wt% of a compound I according to the invention are dissolved in up to 100 wt% organic solvent, e.g. aromatic hydrocarbon.

The compositions types i) to xi) may optionally comprise further auxiliaries, e.g. 0.1-1 wt% bactericides, 5-15 wt% anti-freezing agents, 0.1-1 wt% anti-foaming agents, and 0.1-1 wt% colorants.

The agrochemical compositions generally comprise between 0.01 and 95%, preferably between 0.1 and 90%, and most preferably between 0.5 and 75%, by weight of active substance. The active substances are employed in a purity of from 90% to 100%, preferably from 95% to 100% (according to NMR spectrum).

Various types of oils, wetters, adjuvants, fertilizer, or micronutrients, and other pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the composition according to the invention usually from a predosage device, a knapsack sprayer, a spray tank, a spray plane, or an irrigation system. Usually, the agro-chemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, individual components of the composition of the invention e.g. parts of a kit or parts of a binary or ternary mixture may be mixed by the user himself in a spray tank and further auxiliaries may be added, if appropriate.

In a further embodiment, either individual components of the composition according to the invention or partially premixed components, e.g. components comprising compounds of the invention and/or mixing partners as defined above, may be mixed by the user in a spray tank and further auxiliaries and additives may be added.

In a further embodiment, either individual components of the composition according to the invention or partially premixed components, e.g. components comprising compounds of the invention and/or mixing partners as defined above, can be applied jointly (e.g. after tank mix) or consecutively.

The compounds of the invention are suitable for use in protecting crops, plants, plant propagation materials, e.g. seeds, or soil or water, in which the plants are growing, from attack or infestation by animal pests. Therefore, the invention also relates to a plant protection method, which comprises contacting crops, plants, plant propagation materials, e.g. seeds, or soil or water, in which the plants are growing, to be protected from attack or infestation by animal pests, with a pesticidally effective amount of a compound of the invention.

The compounds of the invention are also suitable for use in combating or controlling animal pests. Therefore, the invention also relates to a method of combating or controlling animal pests, which comprises contacting the animal pests, their habitat, breeding ground, or food supply, or the crops, plants, plant propagation materials, e.g. seeds, or soil, or the area, material or environment in which the animal pests are growing or may grow, with a pesticidally effective amount of a compound of the invention.

The compounds of the invention are effective through both contact and ingestion. Furthermore, the compounds of the invention can be applied to any and all developmental stages, e.g. egg, larva, pupa, and adult.

The compounds of the invention can be applied as such or in form of compositions comprising them as defined above. Furthermore, the compounds of the invention can be applied together with a mixing partner or in form of compositions comprising said mixtures. The components of said mixture can be applied simultaneously, jointly or separately, or in succession, that is immediately one after another and thereby creating the mixture "in situ" on the desired location, e.g. the plant, the sequence, in the case of separate application, generally not having any effect on the result of the control measures.

The application can be carried out both before and after the infestation of the crops, plants, plant propagation materials, e.g. seeds, soil, or the area, material or environment by the pests. Suitable application methods include i.a. soil treatment, seed treatment, in furrow application, and foliar application. Soil treatment methods include drenching the soil, drip irrigation (drip application onto the soil), dipping roots, tubers or bulbs, or soil injection. Seed treatment techniques include seed dressing, seed coating, seed dusting, seed soaking, and seed pelleting. In furrow applications typically include the steps of making a furrow in cultivated land, seeding the furrow with seeds, applying the pesticidally active compound to the furrow, and closing the furrow. Foliar application refers to the application of the pesticidally active compound to plant foliage, e.g. through spray equipment. For foliar applications, it can be advantageous to modify the behavior of the pests by use of pheromones in combination with the compounds of the invention. Suitable pheromones for specific crops and pests are known and publicly available from databases of pheromones and semiochemicals, e.g. http://www.pherobase.com.

As used herein, the term "contacting" includes both direct contact (applying the compounds/compositions directly on the animal pest or plant - typically to the foliage, stem or roots of the plant) and indirect contact (applying the compounds/compositions to the locus, i.e. habitat, breeding ground, plant, seed, soil, area, material, or environment in which a pest is growing or may grow, of the animal pest or plant).

The term "animal pest" includes arthropods, gastropods, and nematodes. Preferred animal pests according to the invention are arthropods, preferably insects and arachnids, in particular insects. Insects, which are of particular relevance for crops, are typically referred to as crop insect pests.

The term "crop" refers to both, growing and harvested crops.

The term "plant" includes cereals, e.g. durum and other wheat, rye, barley, triticale, oats, rice, or maize (fodder maize and sugar maize / sweet and field corn); beet, e.g. sugar beet, or fodder beet; fruits, e.g. pomes, stone fruits, or soft fruits, e.g. apples, pears, plums, peaches, nectarines, almonds, cherries, papayas, strawberries, raspberries, blackberries or gooseberries; leguminous plants, e.g. beans, lentils, peas, alfalfa, or soybeans; oil plants, e.g. rapeseed (oilseed rape), turnip rape, mustard, olives, sunflowers, coconut, cocoa beans, castor oil plants, oil palms, ground nuts, or soybeans; cucurbits, e.g. squashes, pumpkins, cucumber or melons; fiber plants, e.g. cotton, flax, hemp, or jute; citrus fruit, e.g. oranges, lemons, grape-fruits or mandarins; vegetables, e.g. eggplant, spinach, lettuce (e.g. iceberg lettuce), chicory, cabbage, asparagus, cabbages, carrots, onions, garlic, leeks, tomatoes, potatoes, cucurbits or sweet peppers; lauraceous plants, e.g. avocados, cinnamon, or camphor; energy and raw material plants, e.g. corn, soybean, rapeseed, sugar cane or oil palm; tobacco; nuts, e.g. walnuts; pistachios; coffee; tea; bananas; vines; hop; sweet leaf (Stevia); natural rubber plants or ornamental and forestry plants, shrubs, broad-leaved trees or evergreens, eucalyptus; turf; lawn; grass. Preferred plants include potatoes sugar beets, tobacco, wheat, rye, barley, oats, rice, corn, cotton, soybeans, rapeseed, legumes, sunflowers, coffee, or sugar cane; fruits; vines; ornamentals; or vegetables, e.g. cucumbers, tomatoes, beans or squashes.

The term "cultivated plants" is to be understood as including plants which have been modified by mutagenesis or genetic engineering in order to provide a new trait to a plant or to modify an already present trait.

Mutagenesis includes techniques of random mutagenesis using X-rays or mutagenic chemicals, but also techniques of targeted mutagenesis, in order to create mutations at a specific locus of a plant genome. Targeted mutagenesis techniques frequently use oligonucleotides or proteins like CRISPR/Cas, zinc-finger nucleases, TALENs or meganucleases to achieve the targeting effect.

Genetic engineering usually uses recombinant DNA techniques to create modifications in a plant genome which under natural circumstances cannot readily be obtained by cross breeding, mutagenesis or natural recombination. Typically, one or more genes are integrated into the genome of a plant in order to add a trait or improve a trait. These integrated genes are also referred to as transgenes in the art, while plant comprising such transgenes are referred to as transgenic plants. The process of plant transformation usually produces several transformation events, which differ in the genomic locus in which a transgene has been integrated. Plants comprising a specific transgene on a specific genomic locus are usually described as comprising a specific "event", which is referred to by a specific event name. Traits which have been introduced in plants or have been modified include in particular herbicide tolerance, insect resistance, increased yield and tolerance to abiotic conditions, like drought.

Herbicide tolerance has been created by using mutagenesis as well as using genetic engineering. Plants which have been rendered tolerant to ALS inhibitor herbicides by conventional methods of mutagenesis and breeding comprise plant varieties commercially available under the name Clearfield^{®}.

Herbicide tolerance has been created to glyphosate, glufosinate, 2,4-D, dicamba, oxynil herbicides, like bromoxynil and ioxynil, sulfonylurea herbicides, ALS inhibitor herbicides and HPPD inhibitors, like isoxaflutole and mesotrione.

Transgenes which have been used to provide herbicide tolerance traits comprise: for tolerance to glyphosate: cp4 epsps, epsps grg23ace5, mepsps, 2mepsps, gat4601, gat4621 and goxv247, for tolerance to glufosinate: pat and bar, for tolerance to 2,4-D: aad-1 and aad-12, for tolerance to dicamba: dmo, for tolerance to oxynil herbicies: bxn, for tolerance to sulfonylurea herbicides: zm-hra, csr1-2, gm-hra, S4-HrA, for tolerance to ALS inhibitor herbicides: csr1-2, for tolerance to HPPD inhibitor herbicides: hppdPF, W336 and avhppd-03.

Transgenic corn events comprising herbicide tolerance genes are e.g., but not excluding others, DAS40278, MON801, MON802, MON809, MON810, MON832, MON87411, MON87419, MON87427, MON88017, MON89034, NK603, GA21, MZHG0JG, HCEM485, VCO-01981-5, 676, 678, 680, 33121, 4114, 59122, 98140, Bt10, Bt176, CBH-351, DBT418, DLL25, MS3, MS6, MZIR098, T25, TC1507 and TC6275.

Transgenic soybean events comprising herbicide tolerance genes are e.g., but not excluding others, GTS 40-3-2, MON87705, MON87708, MON87712, MON87769, MON89788, A2704-12, A2704-21, A5547-127, A5547-35, DP356043, DAS44406-6, DAS68416-4, DAS-81419-2, GU262, SYHTØH2, W62, W98, FG72 and CV127.

Transgenic cotton events comprising herbicide tolerance genes are e.g., but not excluding others, 19-51a, 31707, 42317, 81910, 281-24-236, 3006-210-23, BXN10211, BXN10215, BXN10222, BXN10224, MON1445, MON1698, MON88701, MON88913, GHB119, GHB614, LLCotton25, T303-3 and T304-40.

Transgenic canola events comprising herbicide tolerance genes are e.g., but not excluding others, MON88302, HCR-1, HCN10, HCN28, HCN92, MS1, MS8, PHY14, PHY23, PHY35, PHY36, RF1, RF2 and RF3.

Insect resistance has mainly been created by transferring bacterial genes for insecticidal proteins to plants. Transgenes which have most frequently been used are toxin genes of Bacillus spec. and synthetic variants thereof, like cry1A, cry1Ab, cry1Ab-Ac, cry1Ac, cry1A.105, cry1F, cry1Fa2, cry2Ab2, cry2Ae, mcry3A, ecry3.1Ab, cry3Bb1, cry34Ab1, cry35Ab1, cry9C, vip3A(a), vip3Aa20. However, also genes of plant origin have been transferred to other plants. In particular genes coding for protease inhibitors, like CpTI and pinll. A further approach uses transgenes in order to produce double stranded RNA in plants to target and downregulate insect genes. An example for such a transgene is dvsnf7.

Transgenic corn events comprising genes for insecticidal proteins or double stranded RNA are e.g., but not excluding others, Bt10, Bt11, Bt176, MON801, MON802, MON809, MON810, MON863, MON87411, MON88017, MON89034, 33121, 4114, 5307, 59122, TC1507, TC6275, CBH-351, MIR162, DBT418 and MZIR098.

Transgenic soybean events comprising genes for insecticidal proteins are e.g., but not excluding others, MON87701, MON87751 and DAS-81419.

Transgenic cotton events comprising genes for insecticidal proteins are e.g., but not excluding others, SGK321, MON531, MON757, MON1076, MON15985, 31707, 31803, 31807, 31808, 42317, BNLA-601, Event1, COT67B, COT102, T303-3, T304-40, GFM Cry1A, GK12, MLS 9124, 281-24-236, 3006-210-23, GHB119 and SGK321.

Increased yield has been created by increasing ear biomass using the transgene athb17, being present in corn event MON87403, or by enhancing photosynthesis using the transgene bbx32, being present in the soybean event MON87712.

Cultivated plants comprising a modified oil content have been created by using the transgenes: gm-fad2-1, Pj.D6D, Nc.Fad3, fad2-1A and fatb1-A. Soybean events comprising at least one of these genes are: 260-05, MON87705 and MON87769.

Tolerance to abiotic conditions, in particular to tolerance to drought, has been created by using the transgene cspB, comprised by the corn event MON87460 and by using the transgene Hahb-4, comprised by soybean event IND-00410-5.

Traits are frequently combined by combining genes in a transformation event or by combining different events during the breeding process. Preferred combination of traits are herbicide tolerance to different groups of herbicides, insect tolerance to different kind of insects, in particular tolerance to lepidopteran and coleopteran insects, herbicide tolerance with one or several types of insect resistance, herbicide tolerance with increased yield as well as a combination of herbicide tolerance and tolerance to abiotic conditions.

Plants comprising singular or stacked traits as well as the genes and events providing these traits are known (http://www.isaaa.org/gmapprovaldatabase) and (http://cera-gmc.org/GMCropDatabase).

Further information on specific events and methods to detect them can be found for canola events MS1, MS8, RF3, GT73, MON88302, KK179 in WO01/031042, WO01/041558, WO01/041558, WO02/036831, WO11/153186, WO13/003558, for cotton events MON1445, MON15985, MON531(MON15985), LLCotton25, MON88913, COT102, 281-24-236, 3006-210-23, COT67B, GHB614, T304-40, GHB119, MON88701, 81910 in WO02/034946, WO02/100163, WO02/100163, WO03/013224, WO04/072235, WO04/039986, WO05/103266, WO05/103266, WO06/128573, WO07/017186, WO08/122406, WO08/151780, WO12/134808, WO13/112527, for corn events GA21, MON810, DLL25, TC1507, MON863, MIR604, LY038, MON88017, 3272, 59122, NK603, MIR162, MON89034, 98140, 32138, MON87460, 5307, 4114, MON87427, DAS40278, MON87411, 33121, MON87403, MON87419 in WO98/044140, US02/102582, US03/126634, WO04/099447, WO04/011601, WO05/103301, WO05/061720, WO05/059103, WO06/098952, WO06/039376, US2007/292854, WO07/142840, WO07/140256, WO08/112019, WO09/103049, WO09/111263, WO10/077816, WO11/084621, WO11/062904, WO11/022469, WO13/169923, WO14/116854, WO15/053998, WO15/142571, for potato events E12, F10, J3, J55, V11, X17, Y9 in WO14/178910, WO14/178913, WO14/178941, WO14/179276, WO16/183445, WO17/062831, WO17/062825, for rice events LLRICE06, LLRICE601, LLRICE62 in WO00/026345, WO00/026356, WO00/026345 for soybean events H7-1, MON89788, A2704-12, A5547-127, DP305423, DP356043, MON87701, MON87769, CV127, MON87705, DAS68416-4, MON87708, MON87712, SYHT0H2, DAS81419, DAS81419 x DAS44406-6, MON87751 in WO04/074492, WO06/130436, WO06/108674, WO06/108675, WO08/054747, WO08/002872, WO09/064652, WO09/102873, WO10/080829, WO10/037016, WO11/066384, WO11/034704, WO12/051199, WO12/082548, WO13/016527, WO13/016516, WO14/201235.

The use of compositions according to the invention on cultivated plants may result in effects which are specific to a cultivated plant comprising a certain gene or event. These effects may comprise enhanced yield, enhanced resistance or tolerance to insects, nematodes, fungal, bacterial, mycoplasma, viral or viroid pathogens as well as early vigor, early or delayed ripening, cold or heat tolerance as well as changed amino acid or fatty acid spectrum or content.

It has been found that the pesticidal activity of the compounds of the invention may be enhanced by the insecticidal trait of a modified plant. Furthermore, it has been found that the compounds of the invention are suitable for preventing insects to become resistant to the insecticidal trait or for combating pests, which already have become resistant to the insecticidal trait of a modified plant. Moreover, the compounds of the invention are suitable for combating pests, against which the insecticidal trait is not effective, so that a complementary insecticidal activity can advantageously be used.

The term "plant propagation material" refers to all the generative parts of the plant e.g. seeds and vegetative plant material e.g. cuttings and tubers (e.g. potatoes), which can be used for the multiplication of the plant. This includes seeds, roots, fruits, tubers, bulbs, rhizomes, shoots, sprouts and other parts of plants. Seedlings and young plants, which are to be trans-planted after germination or after emergence from soil, may also be included. These plant propagation materials may be treated prophylactically with a plant protection compound either at or before planting or transplanting.

The term "seed" embraces seeds and plant propagules of all kinds including but not limited to true seeds, seed pieces, suckers, corms, bulbs, fruit, tubers, grains, cuttings, cut shoots and the like, and means in a preferred embodiment true seeds.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions e.g. desired pesticidal effect and duration, weather, target species, locus, mode of application.

In the case of soil treatment, in furrow application or of application to the pests dwelling place or nest, the quantity of active ingredient ranges from 0.0001 to 500 g per 100 m², preferably from 0.001 to 20 g per 100 m².

For use in treating crop plants, e.g. by foliar application, the rate of application of the active ingredients of this invention may be in the range of 0.0001 g to 4000 g per hectare, e.g. from 1 g to 2 kg per hectare or from 1 g to 750 g per hectare, desirably from 1 g to 100 g per hectare, more desirably from 10 g to 50 g per hectare, e.g., 10 to 20 g per hectare, 20 to 30 g per hectare, 30 to 40 g per hectare, or 40 to 50 g per hectare.

The compounds of the invention are particularly suitable for use in the treatment of seeds in order to protect the seeds from insect pests, in particular from soil-living insect pests, and the resulting seedling's roots and shoots against soil pests and foliar insects. The invention therefore also relates to a method for the protection of seeds from insects, in particular from soil insects, and of the seedling's roots and shoots from insects, in particular from soil and foliar insects, said method comprising treating the seeds before sowing and/or after pregermination with a compound of the invention. The protection of the seedling's roots and shoots is preferred. More preferred is the protection of seedling's shoots from piercing and sucking insects, chewing insects and nematodes.

The term "seed treatment" comprises e.g. seed dressing, seed coating, seed dusting, seed soaking, seed pelleting, and in-furrow application methods. Preferably, the seed treatment application of the active compound is carried out by spraying or by dusting the seeds before sowing of the plants and before emergence of the plants.

The invention also comprises seeds coated with or containing the active compound. The term "coated with and/or containing" generally signifies that the active ingredient is for the most part on the surface of the propagation product at the time of application, although a greater or lesser part of the ingredient may penetrate into the propagation product, depending on the method of application. When the said propagation product is (re)planted, it may absorb the active ingredient.

Suitable seed is e.g. seed of cereals, root crops, oil crops, vegetables, spices, ornamentals, e.g. seed of durum and other wheat, barley, oats, rye, maize (fodder maize and sugar maize / sweet and field corn), soybeans, oil crops, crucifers, cotton, sunflowers, bananas, rice, oilseed rape, turnip rape, sugarbeet, fodder beet, eggplants, potatoes, grass, lawn, turf, fodder grass, tomatoes, leeks, pumpkin/squash, cabbage, iceberg lettuce, pepper, cucumbers, melons, Brassica species, melons, beans, peas, garlic, onions, carrots, tuberous plants e.g. potatoes, sugar cane, tobacco, grapes, petunias, geranium/pelargoniums, pansies and impatiens.

In addition, the active compound may also be used for the treatment of seeds from plants, which have been modified by mutagenisis or genetic engineering, and which e.g. tolerate the action of herbicides or fungicides or insecticides.

Conventional seed treatment formulations include e.g. flowable concentrates FS, solutions LS, suspoemulsions (SE), powders for dry treatment DS, water dispersible powders for slurry treatment WS, water-soluble powders SS and emulsion ES and EC and gel formulation GF. These formulations can be applied to the seed diluted or undiluted. Application to the seeds is carried out before sowing, either directly on the seeds or after having pregerminated the latter. Preferably, the formulations are applied such that germination is not included.

The active substance concentrations in ready-to-use formulations, which may be obtained after two-to-tenfold dilution, are preferably from 0.01 to 60% by weight, more preferably from 0.1 to 40% by weight.

In a preferred embodiment a FS formulation is used for seed treatment. Typically, a FS formulation may comprise 1-800 g/l of active ingredient, 1-200 g/l Surfactant, 0 to 200 g/l anti-freezing agent, 0 to 400 g/l of binder, 0 to 200 g/l of a pigment and up to 1 liter of a solvent, preferably water.

Especially preferred FS formulations of the compounds of the invention for seed treatment usually comprise from 0.1 to 80% by weight (1 to 800 g/l) of the active ingredient, from 0.1 to 20% by weight (1 to 200 g/l) of at least one surfactant, e.g. 0.05 to 5% by weight of a wetter and from 0.5 to 15% by weight of a dispersing agent, up to 20% by weight, e.g. from 5 to 20% of an anti-freeze agent, from 0 to 15% by weight, e.g. 1 to 15% by weight of a pigment and/or a dye, from 0 to 40% by weight, e.g. 1 to 40% by weight of a binder (sticker/adhesion agent), optionally up to 5% by weight, e.g. from 0.1 to 5% by weight of a thickener, optionally from 0.1 to 2% of an anti-foam agent, and optionally a preservative e.g. a biocide, antioxidant or the like, e.g. in an amount from 0.01 to 1% by weight and a filler/vehicle up to 100% by weight.

In the treatment of seed, the application rates of the compounds of the invention are generally from 0.1 g to 10 kg per 100 kg of seed, preferably from 1 g to 5 kg per 100 kg of seed, more preferably from 1 g to 1000 g per 100 kg of seed and in particular from 1 g to 200 g per 100 kg of seed, e.g. from 1 g to 100 g or from 5 g to 100 g per 100 kg of seed.

The invention therefore also relates to seed comprising a compound of the invention, or an agriculturally useful salt thereof, as defined herein. The amount of the compound of the invention or the agriculturally useful salt thereof will in general vary from 0.1 g to 10 kg per 100 kg of seed, preferably from 1 g to 5 kg per 100 kg of seed, in particular from 1 g to 1000 g per 100 kg of seed. For specific crops e.g. lettuce the rate can be higher.

The compounds of the invention may also be used for improving the health of a plant. Therefore, the invention also relates to a method for improving plant health by treating a plant, plant propagation material and/or the locus where the plant is growing or is to grow with an effective and non-phytotoxic amount of a compound of the invention.

As used herein "an effective and non-phytotoxic amount" means that the compound is used in a quantity which allows to obtain the desired effect but which does not give rise to any phytotoxic symptom on the treated plant or on the plant grown from the treated propagule or treated soil.

"Plant health" is defined as a condition of the plant and/or its products which is determined by several aspects alone or in combination with each other e.g. yield (e.g. increased biomass and/or increased content of valuable ingredients), quality (e.g. improved content or composition of certain ingredients or shelf life), plant vigour (e.g. improved plant growth and/or greener leaves ("greening effect"), tolerance to abiotic (e.g. drought) and/or biotic stress (e.g. disease) and production efficiency (e.g., harvesting efficiency, processability).

The above identified indicators for the health condition of a plant may be interdependent and may result from each other. Each indicator is defined in the art and can be determined by methods known to a skilled person.

The compounds of the invention are also suitable for use against non-crop insect pests. For use against said non-crop pests, compounds of the invention can be used as bait composition, gel, general insect spray, aerosol, as ultra-low volume application and bed net (impregnated or surface applied). Furthermore, drenching and rodding methods can be used.

As used herein, the term "non-crop insect pest" refers to pests, which are particularly relevant for non-crop targets, e.g. ants, termites, wasps, flies, ticks, mosquitoes, bed bugs, crickets, or cockroaches.

The bait can be a liquid, a solid or a semisolid preparation (e.g. a gel). The bait employed in the composition is a product, which is sufficiently attractive to incite insects e.g. ants, termites, wasps, flies, mosquitoes, crickets etc. or cockroaches to eat it. The attractiveness can be manipulated by using feeding stimulants or sex pheromones. Food stimulants are preferably chosen from animal and/or plant proteins (meat-, fish- or blood meal, insect parts, egg yolk), from fats and oils of animal and/or plant origin, or mono-, oligo- or polyorgano¬saccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are known (http://www.pherobase.com).

For use in bait compositions, the typical content of active ingredient is from 0.001 wt% to 15 wt%, desirably from 0.001 wt% to 5 wt% of active compound.

Formulations of the compounds of the invention as aerosols (e.g in spray cans), oil sprays or pump sprays are highly suitable for professional or non-professional users for controlling pests e.g. flies, fleas, ticks, bed bugs, mosquitoes or cockroaches. Aerosol recipes are preferably composed of the active compound, solvents, furthermore auxiliaries e.g. emulsifiers, perfume oils, if appropriate stabilizers, and, if required, propellants.

The oil spray formulations differ from the aerosol recipes in that no propellants are used. For use in spray compositions, the content of active ingredient is from 0.001 to 80 wt%, preferably from 0.01 to 50 wt% and most preferably from 0.01 to 15 wt%.

The compounds of the invention and its respective compositions can also be used in mosquito and fumigating coils, smoke cartridges, vaporizer plates or long-term vaporizers and also in moth papers, moth pads or other heat-independent vaporizer systems.

Methods to control infectious diseases transmitted by insects (e.g. malaria, dengue and yellow fever, lymphatic filariasis, and leishmaniasis) with compounds of the invention and its respective compositions also comprise treating surfaces of huts and houses, air spraying and impregnation of curtains, tents, clothing items, bed nets, tsetse-fly trap. Insecticidal compositions for application to fibers, fabric, knitgoods, nonwovens, netting material or foils and tarpaulins preferably comprise a mixture including the insecticide, optionally a repellent and at least one binder.

The compounds of the invention and its compositions can be used for protecting wooden materials e.g. trees, board fences, sleepers, frames, artistic artifacts, etc. and buildings, but also construction materials, furniture, leathers, fibers, vinyl articles, electric wires and cables etc. from ants, termites and/or wood or textile destroying beetles, and for controlling ants and termites from doing harm to crops or human beings (e.g. when the pests invade into houses and public facilities or nest in yards, orchards or parks).

Customary application rates in the protection of materials are, e.g., from 0.001 g to 2000 g or from 0.01 g to 1000 g of active compound per m² treated material, desirably from 0.1 g to 50 g per m².

Insecticidal compositions for use in the impregnation of materials typically contain from 0.001 to 95 wt%, preferably from 0.1 to 45 wt%, and more preferably from 1 to 25 wt% of at least one repellent and/or insecticide.

The compounds of the invention and the compositions containing them may be applied in combination with, or by utilizing smart agricultural technologies, such as precision agriculture, remote and proximate imaging and image recognition, or smart agricultural site management programs. These smart agricultural technologies typically include models, e.g. computer programs, that support the user by considering information from a wide variety of sources to increase the quality and yield of harvested material, reduce damage by pests including the prediction of pest pressure and smart application of crop protection products, secure environmental protection, support quick and reliable agronomic decision making, reduce usage of fertilizers and crop protection products, reduce product residues in consumables increase spatial and temporal precision of agronomical measures, automate processes, and enable traceability of measures.

Commercially available systems which include agronomic models are e.g. FieldScripts^{™} from The Climate Corporation, Xarvio^{™} from BASF, AGLogic^{™} from John Deere, etc.

Information input for these models include but is not limited to soil data, information on the plants that are currently growing or that may grow at the area of interest including crop plants and/or unwanted vegetation, weather information, information on the location of the area and directly derivable information thereof, information on pest pressure, information on beneficial organisms, and / or historic information of any of the aforementioned.

The information usable for precision agriculture may be based on input by at least one user, be accessible from external data sources and databases, or be based on sensor data. Data sources typically includes proximate-detection systems like soil-borne sensors and remote sensing as may be achieved by imaging with unmanned airborne vehicles like drones, or satellites. Sensors may be included in an Internet-of-Things system and may be directly or indirectly connected to the processing unit, e.g. via a wireless network and/or cloud applications. The information is typically taken into account by at least one processing unit and used to provide recommendations and generate control signals.

Typical technologies that are used in smart agricultural technologies include self-steering robots (such as tractors, harvesters, drones), artificial intelligence (e.g. machine learning), imaging technologies (e.g. image segmentation technologies), big data analysis, and model generation, cloud computing, and machine-to-machine communication.

Precision agriculture such as precision farming is characterized by spatially and/or temporally resolved, targeted application of active ingredients like pesticides, plant-growth-regulators, ferti¬lizers, and/or water including the variation of application rates over the agronomic site, zone or spot application, and of the spatially and/or temporally resolved, targeted planting or seeding of desired plant propagation material to a agronomic site. Precision farming typically includes the use of geo-positioning technologies like GPS for gaining information on the location and bounda¬ries of the area of interest, the utilized application equipment, sensing equipment and recorded data, and to control the actions of farm vehicles such as spraying. By combining geo-positioning data with (digital) maps, it is possible to (semi)-automate agricultural measures at the site of interest, e.g. by using (semi)-autonomous spraying or seeding equipment.

Precision farming may typically include the application of smart spraying equipment, e.g. spot spraying, and precision spraying at a farm, e.g. by irrigation systems, tractors, robots, helicopters, airplanes, unmanned aerial vehicles, such as drones. Such equipment usually includes input sensors (such as e.g. a camera) and a processing unit configured to analyze the input data and configured to provide a recommendation or decision based on the analysis of the input data to apply the compounds of the invention or compositions comprising them to the agronomic site, e.g. the soil, the crop plants, or to control pests in a specific and precise manner. For example, pests may be detected, identified, and/or classified from imagery acquired by a camera. Such identification and/ classification can make use of image processing algorithms, which may utilize artificial intelligence (e.g. machine learning algorithms), or decision trees. In this manner, the compounds or com¬po¬sitions described herein can be applied only at the required location, point in time and dose rate.

The compounds of the invention are especially suitable for efficiently combating animal pests e.g. arthropods, gastropods and nematodes including:
insects from the order of Lepidoptera, e.g. Achroia grisella, Acleris spp. e.g. A. fimbriana, A. gloverana, A. variana; Acrolepiopsis assectella, Acronicta major, Adoxophyes spp. e.g. A. cyr-tosema, A. orana; Aedia leucomelas, Agrotis spp. e.g. A. exclamationis, A. fucosa, A. ipsilon, A. orthogoma, A. segetum, A. subterranea; Alabama argillacea, Aleurodicus dispersus, Alsophila pometaria, Ampelophaga rubiginosa, Amyelois transitella, Anacampsis sarcitella, Anagasta kuehniella, Anarsia lineatella, Anisota senatoria, Antheraea pernyi, Anticarsia (=Ther¬me¬sia) spp. e.g. A. gemmatalis; Apamea spp., Aproaerema modicella, Archips spp. e.g. A. argyrospila, A. fuscocupreanus, A. rosana, A. xyloseanus; Argyresthia conjugella, Argyroplo¬ce spp., Argyrotaenia spp. e.g. A. velutinana; Athetis mindara, Austroasca viridi¬gri¬sea, Autographa gamma, Autographa nigrisigna, Barathra brassicae, Bedellia spp., Bonagota salubricola, Borbo cinnara, Bucculatrix thurberiella, Bupalus piniarius, Busseola spp., Cacoecia spp. e.g. C. murinana, C. podana; Cactoblastis cactorum, Cadra cautella, Calingo brazilien¬sis, Caloptilis theivora, Capua reticulana, Carposina spp. e.g. C. niponensis, C. sasakii; Cephus spp., Chaetocnema aridula, Cheimatobia brumata, Chilo spp. e.g. C. Indicus, C. suppressalis, C. partellus; Choreutis pariana, Choristoneura spp. e.g. C. conflictana, C. fumife¬rana, C. longicellana, C. murinana, C. occidentalis, C. rosaceana; Chrysodeixis (=Pseudoplusia) spp., e.g. C. eriosoma, C. includens; Cirphis unipuncta, Clysia ambiguella, Cnaphalocerus spp., Cna¬phalocrocis medinalis, Cnephasia spp., Cochylis hospes, Coleophora spp., Colias eurytheme, Conopomorpha spp., Conotrachelus spp., Copitarsia spp., Corcyra cephalonica, Crambus caliginosellus, Crambus teterrellus, Crocidosema (=Epinotia) aporema, Cydalima (=Diaphania) perspectalis, Cydia (=Carpocapsa) spp., e.g. C. pomonella, C. latiferreana; Dalaca noctuides, Datana integerrima, Dasychira pinicola, Dendrolimus spp., e.g. D. pini, D. spectabilis, D. sibiricus; Desmia funeralis, Diaphania spp., e.g. D. nitidalis, D. hyalinata; Diatraea grandiosella, Diatraea saccharalis, Diphthera festiva, Earias spp. e.g. E. insulana, E. vittella; Ecdytolopha aurantianu, Egira (=Xylomyges) curialis, Elasmopalpus lignosellus, Eldana saccharina, Endo¬pi¬za viteana, Enno-mos subsignaria, Eoreuma loftini, Ephestia spp., e.g. E. cautella, E. elutella, E. kuehniella; Epinotia aporema, Epi¬phy¬as postvittana, Erannis tiliaria, Erio¬nota thrax, Etiella spp., Eulia spp., Eupoecilia ambiguella, Euproctis chrysorrhoea, Euxoa spp., Eve¬tria bouliana, Faronta albilinea, Feltia spp. e.g. F. subterranean; Galleria mellonella, Gra¬cillaria spp., Gra¬pholita spp. e.g. G. funebrana, G. molesta, G. inopinata; Halysidota spp., Harrisina americana, Hedylepta spp., Helicoverpa spp. e.g. H. armigera (=Heliothis armigera), H. zea (=Heliothis zea); Heliothis spp. e.g. H. assulta, H. subflexa, H. virescens; Hellula spp. e.g. H. undalis, H. rogatalis; Helocoverpa gelotopoeon, Hemileuca oliviae, Herpetogramma licarsisalis, Hibernia defoliaria, Hofmannophila pseudospretella, Homo¬eosoma electellum, Homona magna¬nima, Hypena scabra, Hyphantria cunea, Hyponomeuta padella, Hypono¬meuta malinellus, Kakivoria flavofasciata, Keiferia lycoper-sicella, Lambdina fiscellaria fiscellaria, Lambdina fiscellaria lugubrosa, Lamprosema indicata, Laspeyresia molesta, Leguminivora gly¬cinivorella, Lerodea eufala, Leucinodes orbonalis, Leucoma salicis, Leucoptera spp. e.g. L. coffeella, L. scitella; Leuminivora lycinivorella, Lithocolletis blancardella, Lithophane antennata, Llattia octo (=Amyna axis), Lobesia botrana, Lophocampa spp., Loxagrotis albicosta, Loxostege spp. e.g. L. sticticalis, L. cereralis; Lymantria spp., e.g. L. dispar, L. monacha; Lyone¬tia clerkella, Lyonetia prunifoliella, Malacosoma spp., e.g. M. americanum, M. californicum, M. constrictum, M. neu-stria; Mamestra spp., e.g. M. brassicae, M. configurata; Mamstra brassicae, Manduca spp. e.g. M. quinquemaculata, M. sexta; Marasmia spp, Marmara spp., Maruca testulalis, Megalopyge lanata, Melanchra picta, Melanitis leda, Mocis spp., e.g. M. lapites, M. repan¬da; Mocis latipes, Monochroa fragariae, Mythimna separata, Nemapogon cloacella, Neoleucino¬des elegantalis, Nepytia spp., Nymphula spp., Oiketicus spp., Omiodes indicata, Omphisa anastomosalis, Operophtera brumata, Orgyia pseudotsugata, Oria spp., Ortha¬ga thyrisalis, Ostrinia spp. e.g. O. nubilalis; Oulema oryzae, Paleacrita vernata, Panolis flammea, Parnara spp., Papa¬ipema nebris, Papilio cresphontes, Paramyelois transitella, Paranthrene regalis, Paysan¬disia archon, Pectinophora spp. e.g. P. gossypiella; Peridroma saucia, Perileucoptera spp., e.g. P. coffeella; Phalera bucephala, Phryganidia californica, Phtho¬rimaea spp. e.g. P. operculella; Phyllocnistis citrella, Phyllonorycter spp. e.g. P. blancardella, P. crataegella, P. issikii, P. ringoniella; Pieris spp. e.g. P. brassicae, P. rapae, P. napi; Pilocrocis tripunctata, Plathypena scabra, Pla¬tynota spp. e.g. P. flavedana, P. idaeusalis, P. stultana; Platyptilia carduidactyla, Plebejus argus, Plo-dia interpunctella, Plusia spp, Plutella maculipennis, Plutella xylostella, Pontia proto¬di¬ca, Prays spp., Prodenia spp., Proxenus lepigone, Pseudaletia spp. e.g. P. sequax, P. uni¬punc¬ta; Pyrausta nubilalis, Rachiplusia nu, Richia albicosta, Rhizobius ventralis, Rhyacionia frustra¬na, Sabulodes aegrotata, Schizura concinna, Schoenobius spp., Schreckensteinia festaliella, Scirpophaga spp. e.g. S. incertulas, S. innotata; Scotia sege¬tum, Sesamia spp. e.g. S. inferens, Seudyra subflava, Sitotroga cerealella, Sparganothis pilleriana, Spilonota lechriaspis, S. ocelli-na, Spodoptera (=Lamphygma) spp. e.g. S. cosmoides, S. eridania, S. exigua, S. frugiperda, S. latisfascia, S. littoralis, S. litura, S. omithogalli; Stigmella spp., Stomopteryx subsecivella, Strymon bazochii, Sylepta dero¬gata, Synanthedon spp. e.g. S. exitiosa, Tecia solanivora, Telehin licus, Thaumatopoea pityocampa, Thaumatotibia (=Cryptophlebia) leucotreta, Thaumetopoea pityocampa, Thecla spp., Theresimima ampelophaga, Thyrinteina spp, Tildenia inconspi-cuella, Tinea spp. e.g. T. cloacella, T. pellionella; Tineola bisselliella, Tortrix spp. e.g. T. virida¬na; Trichophaga tapetzella, Trichoplusia spp. e.g. T. ni; Tuta (=Scrobipalpula) absoluta, Udea spp. e.g. U. rubigalis, U. rubigalis; Virachola spp., Yponomeuta padella, and Zeiraphera canadensis;
insects from the order of Coleoptera, e.g. Acalymma vittatum, Acanthoscehdes obtectus, Ado¬retus spp., Agelastica alni, Agrilus spp. e.g. A. anxius, A. planipennis, A. sinuatus; Agriotes spp. e.g. A. fuscicollis, A. lineatus, A. obscurus; Alphitobius diaperinus, Amphimallus solstitialis, Anisandrus dispar, Anisoplia austriaca, Anobium punctatum, Anomala corpulen¬ta, Anoma¬la rufocuprea, Anoplophora spp. e.g. A. glabripennis; Anthonomus spp. e.g. A. eugenii, A. grandis, A. pomorum; Anthrenus spp., Aphthona euphoridae, Apion spp., Apogonia spp., Atho¬us haemorrhoidalis, Atomaria spp. e.g. A. linearis; Attagenus spp., Aulaco¬phora fe¬rnoralis, Blastophagus piniperda, Blitophaga undata, Bruchidius obtectus, Bruchus spp. e.g. B. lentis, B. pisorum, B. rufimanus; Byctiscus betulae, Callidiellum rufipenne, Callopistria floriden¬sis, Callosobruchus chinensis, Cameraria ohridella, Cassida nebulosa, Cerotoma trifurcata, Cetonia aurata, Ceuthorhynchus spp. e.g. C. assimilis, C. napi; Chaetocnema tibialis, Cleo¬nus mendicus, Conoderus spp. e.g. C. vespertinus; Conotrachelus nenuphar, Cosmopolites spp., Costelytra zealandica, Crioceris asparagi, Cryptolestes ferrugineus, Cryptorhynchus lapathi, Ctenicera spp. e.g. C. destructor; Curculio spp., Cylindrocopturus spp., Cyclo¬cephala spp., Dac-tylispa balyi, Dectes texanus, Dermestes spp., Diabrotica spp. e.g. D. undecim¬punctata, D. speciosa, D. longicornis, D. semipunctata, D. virgifera; Diaprepes abbreviates, Dichocrocis spp., Dicladispa armigera, Diloboderus abderus, Diocalandra frumenti (Diocalan¬dra stigmaticollis), Enaphalodes rufulus, Epilachna spp. e.g. E. varivestis, E. viginti-octomaculata; Epitrix spp. e.g. E. hirtipennis, E. similaris; Eutheola humilis, Eutinobothrus bra¬silien¬sis, Faustinus cubae, Gibbium psylloides, Gnathocerus cornutus, Hellula undalis, Heteronychus ara¬tor, Hylamorpha elegans, Hylobius abietis, Hylo¬trupes bajulus, Hypera spp., e.g. H. brunneipennis, H. postica; Hypomeces squamosus, Hypothenemus spp., Ips typographus, Lachno¬sterna consanguinea, Lasioderma serricorne, Latheticus oryzae, Lathridius spp., Lema spp. e.g. L. bilineata, L. melanopus; Leptinotarsa spp. e.g. L. decemlineata; Leptispa pygma¬ea, Limonius californi-cus, Lissorhoptrus oryzophilus, Lixus spp., Luperodes spp., Lyctus spp. e.g. L. bruneus; Liogenys fuscus, Macrodactylus spp. e.g. M. subspinosus; Mala¬de¬ra matrida, Megaplatypus mutates, Megascelis spp., Melanotus communis, Meligethes spp. e.g. M. aeneus; Melolontha spp. e.g. M. hippocastani, M. melolontha; Metamasius hemipterus, Microtheca spp., Migdolus spp. e.g. M. fryanus, Monochamus spp. e.g. M. alternatus; Naupactus xanthographus, Niptus hololeucus, Oberia brevis, Oemona hirta, Oryctes rhinoceros, Oryzaephilus surinamensis, Oryzaphagus oryzae, Otiorrhynchus sulcatus, Otiorrhynchus ovatus, Otiorrhynchus sulcatus, Oulema melanopus, Oulema oryzae, Oxycetonia jucunda, Phaedon spp. e.g. P. brassicae, P. cochleariae; Phoracantha recurva, Phyllobius pyri, Phyllopertha horticola, Phyllophaga spp. e.g. P. helleri; Phyllotreta spp. e.g. P. chry¬socephala, P. nemorum, P. striolata, P. vittula; Phyllopertha horticola, Popillia japonica, Premnotrypes spp., Psacothea hilaris, Psylliodes chrysocephala, Prostephanus truncates, Psylliodes spp., Ptinus spp., Pulga saltona, Rhizopertha dominica, Rhynchophorus spp. e.g. R. billineatus, R. ferrugineus, R. palmarum, R. phoenicis, R. vulneratus; Saperda candida, Scolytus schevyrewi, Scyphophorus acupunctatus, Sitona lineatus, Sitophilus spp. e.g. S. granaria, S. oryzae, S. zea¬mais; Sphenophorus spp. e.g. S. levis; Stegobium paniceum, Sternechus spp. e.g. S. subsignatus; Strophomorphus ctenotus, Symphyletes spp., Tanymecus spp., Tenebrio moli¬tor, Tenebrioides mauretanicus, Tribolium spp. e.g. T. castaneum; Trogoderma spp., Tychius spp., Xylo¬trechus spp. e.g. X. pyrrhoderus; and, Zabrus spp. e.g. Z. tenebrioides;
insects from the order of Diptera e.g. Aedes spp. e.g. A. aegypti, A. albopictus, A. vexans; Anastrepha ludens, Anopheles spp. e.g. A. albimanus, A. crucians, A. freeborni, A. gambiae, A. leucosphyrus, A. maculipennis, A. minimus, A. quadrimaculatus, A. sinensis; Bactro¬ce¬ra invadens, Bibio hortulanus, Calliphora erythrocephala, Calliphora vicina, Ceratitis capitata, Chrysomyia spp. e.g. C. bezziana, C. hominivorax, C. macellaria; Chrysops atlanticus, Chrysops discalis, Chrysops silacea, Cochliomyia spp. e.g. C. hominivorax; Contarinia spp. e.g. C. sorghicola; Cordylobia anthropophaga, Culex spp. e.g. C. nigripalpus, C. pipiens, C. quinquefasciatus, C. tarsalis, C. tritaeniorhynchus; Culicoides furens, Culiseta inornata, Culiseta melanura, Cuterebra spp., Dacus cucurbitae, Dacus oleae, Dasineura brassicae, Dasineura oxycoccana, Delia spp. e.g. D. antique, D. coarctata, D. platura, D. radicum; Dermatobia hominis, Drosophila spp. e.g. D. suzukii, Fannia spp. e.g. F. canicularis; Gastraphilus spp. e.g. G. intestinalis; Geomyza tipunctata, Glossina spp. e.g. G. fuscipes, G. morsitans, G. palpalis, G. tachinoides; Haematobia irritans, Haplodiplosis equestris, Hippelates spp., Hylemyia spp. e.g. H. platura; Hypoderma spp. e.g. H. lineata; Hyppobosca spp., Hydrellia philippina, Leptoconops torrens, Liriomyza spp. e.g. L. sativae, L. trifolii; Lucilia spp. e.g. L. caprina, L. cuprina, L. sericata; Lycoria pectoralis, Mansonia titillanus, Mayetiola spp. e.g. M. destructor; Musca spp. e.g. M. autumnalis, M. domestica; Muscina stabulans, Oestrus spp. e.g. O. ovis; Opomyza florum, Oscinella spp. e.g. O. frit; Orseolia oryzae, Pegomya hysocyami, Phlebotomus argentipes, Phorbia spp. e.g. P. antiqua, P. brassicae, P. coarctata; Phytomyza gymnostoma, Prosimulium mixtum, Psila rosae, Psorophora columbiae, Psorophora discolor, Rhagoletis spp. e.g. R. cerasi, R. cingulate, R. indifferens, R. mendax, R. pomonella; Rivellia quadrifasciata, Sarcophaga spp. e.g. S. haemorrhoidalis; Simulium vittatum, Sitodiplosis mosellana, Stomoxys spp. e.g. S. calcitrans; Tabanus spp. e.g. T. atratus, T. bovinus, T. lineola, T. similis; Tannia spp., Thecodiplo-sis japonensis, Tipula oleracea, Tipula paludosa, and Wohlfahrtia spp;
insects from the order of Thysanoptera e.g., Baliothrips biformis, Dichromothrips corbetti, Dichromothrips ssp., Echinothrips americanus, Enneothrips flavens, Frankliniella spp. e.g. F. fusca, F. occidentalis, F. tritici; Heliothrips spp., Hercinothrips femoralis, Kakothrips spp., Microcephalothrips abdominalis, Neohydatothrips samayunkur, Pezothrips kellyanus, Rhipiphorothrips cruentatus, Scirtothrips spp. e.g. S. citri, S. dorsalis, S. perseae; Stenchaetothrips spp, Taeniothrips cardamoni, Taeniothrips inconsequens, Thrips spp. e.g. T. imagines, T. hawaiiensis, T. oryzae, T. palmi, T. parvispinus, T. tabaci;
insects from the order of Hemiptera e.g., Acizzia jamatonica, Acrosternum spp., e.g. A. hilare; Acyrthosipon spp., e.g. A. onobrychis, A. pisum; Adelges laricis, Adelges tsugae, Adel¬phocoris spp., e.g. A. rapidus, A. superbus; Aeneolamia spp., Agonoscena spp., Aula¬cor¬thum solani, Aleurocanthus woglumi, Aleurodes spp., Aleurodicus disperses, Aleurolobus ba¬ro¬densis, Aleurothrixus spp., Amrasca spp., Anasa tristis, Antestiopsis spp., Anuraphis cardui, Aoni¬diella spp., Aphanostigma piri, Aphidula nasturtii, Aphis spp. e.g. A. craccivora, A. fabae, A. forbesi, A. gossypii, A. grossulariae, A. maidiradicis, A. pomi, A. sambuci, A. schnei¬deri, A. spiraecola; Arboridia apicalis, Arilus critatus, Aspidiella spp., Aspidiotus spp., Atanus spp., Aulacaspis yasumatsui, Aulacorthum solani, Bactericera cockerelli (Paratrioza cockerelli), Bemisia spp. e.g. B. argentifolii, B. tabaci (Aleurodes tabaci); Blissus spp. e.g. B. leucop¬terus; Brachycaudus spp. e.g. B. cardui, B. helichrysi, B. persicae, B. prunicola; Brachyco¬lus spp., Brachycorynella as-paragi, Brevicoryne brassicae, Cacopsylla spp. e.g. C. fulgura¬lis, C. pyricola (Psylla piri); Calligy¬pona marginata, Calocoris spp., Campylomma livida, Capito¬phorus horni, Carneocephala fulgida, Cavelerius spp., Ceraplastes spp., Ceratovacuna lanigera, Ceroplastes ceriferus, Cerosi¬pha gossypii, Chaetosiphon fragaefolii, Chionaspis tega¬lensis, Chlorita onukii, Chromaphis juglandicola, Chrysomphalus ficus, Cicadulina mbila, Cimex spp. e.g. C. hemipterus, C. lectularius; Coccomytilus halli, Coccus spp. e.g. C. hesperi¬dum, C. pseudomagnoliarum; Corythucha arcuata, Creontiades dilutus, Cryptomyzus ribis, Chrysomphalus aonidum, Cryptomyzus ribis, Ctenarytaina spatulata, Cyrtopeltis notatus, Dalbulus spp., Dasynus piperis, Dialeurodes spp. e.g. D. citrifolii; Dalbulus maidis, Diaphorina spp. e.g. D. citri; Diaspis spp. e.g. D. bromeliae; Dichelops furcatus, Diconocoris hewetti, Doralis spp., Dreyfusia nordmannianae, Dreyfusia piceae, Drosicha spp., Dysaphis spp. e.g. D. plan¬taginea, D. pyri, D. radicola; Dysaulacorthum pseudosolani, Dysdercus spp. e.g. D. cingulatus, D. intermedius; Dysmicoccus spp., Edessa spp., Geocoris spp., Empo¬asca spp. e.g. E. fabae, E. solana; Epidiaspis leperii, Eriosoma spp. e.g. E. lanigerum, E. pyricola; Erythroneura spp., Eurygaster spp. e.g. E. integriceps; Euscelis bilobatus, Euschistus spp. e.g. E. heros, E. impictiventris, E. servus; Fiorinia theae, Geococcus coffeae, Glycaspis brimblecombei, Halyomorpha spp. e.g. H. halys; Heliopeltis spp., Homalodisca vitripennis (=H. coagulata), Horcias nobilellus, Hyalopterus pruni, Hyperomyzus lactucae, Icerya spp. e.g. I. purchase; Idiocerus spp., Idioscopus spp., Laodelphax striatellus, Lecanium spp., Leca¬noi¬deus floccissimus, Lepidosaphes spp. e.g. L. ulmi; Leptocorisa spp., Leptoglossus phyllo¬pus, Lipaphis erysimi, Lygus spp. e.g. L. hesperus, L. lineolaris, L. praten-sis; Maconellicoccus hirsutus, Marchalina hellenica, Macropes excavatus, Macrosiphum spp. e.g. M. rosae, M. avenae, M. euphorbiae; Macrosteles quadrilineatus, Mahanarva fimbriolata, Megacopta cri¬braria, Megoura viciae, Melanaphis pyrarius, Melanaphis sacchari, Melanocallis (=Tinocallis) caryaefoliae, Metcafiella spp., Metopolophium dirhodum, Monellia costalis, Mo-nelliopsis peca¬nis, Myzocallis coryli, Murgantia spp., Myzus spp. e.g. M. ascalonicus, M. cerasi, M. nicotianae, M. persicae, M. varians; Nasonovia ribisnigri, Neotoxoptera formosana, Neomegalotomus spp, Nephotettix spp. e.g. N. malayanus, N. nigropictus, N. parvus, N. vires-cens; Ne¬zara spp. e.g. N. viridula; Nilaparvata lugens, Nysius huttoni, Oebalus spp. e.g. O. pug¬nax; Oncometopia spp., Orthezia praelonga, Oxycaraenus hyalinipennis, Parabemisia myricae, Parlatoria spp., Parthe¬nolecanium spp. e.g. P. corni, P. persicae; Pemphigus spp. e.g. P. bursarius, P. populivenae; Peregrinus maidis, Perkinsiella saccharicida, Phenacoc¬cus spp. e.g. P. aceris, P. gossypii; Phloeomyzus passerinii, Phorodon humuli, Phylloxera spp. e.g. P. devastatrix, Piesma quadrata, Piezodorus spp. e.g. P. guildinii; Pinnaspis aspidistrae, Planococcus spp. e.g. P. citri, P. ficus; Prosapia bicincta, Protopulvinaria pyriformis, Psallus seriatus, Pseudacysta persea, Pseu¬daulacaspis pentagona, Pseudococcus spp. e.g. P. com¬stocki; Psylla spp. e.g. P. mali; Pteromalus spp., Pulvinaria amygdali, Pyrilla spp., Qua¬draspidiotus spp., e.g. Q. perniciosus; Que¬sada gigas, Rastrococcus spp., Reduvius senilis, Rhizoecus americanus, Rhodnius spp., Rho¬palomyzus ascalonicus, Rhopalo¬si¬phum spp. e.g. R. pseudobrassicas, R. insertum, R. maidis, R. padi; Sagatodes spp., Sahlbergella singula¬ris, Saissetia spp., Sappaphis mala, Sappaphis mali, Scaptocoris spp., Scaphoides titanus, Schi¬zaphis graminum, Schizoneura lanuginosa, Scotinophora spp., Selenaspidus articulatus, Sitobion avenae, Sogata spp., Sogatella furcifera, Solubea insularis, Spissistilus festinus (=Stictocephala festina), Stephanitis nashi, Stephanitis pyrioides, Stephanitis takeyai, Tenala¬phara malayensis, Tetraleurodes perseae, Therioaphis maculate, Thyanta spp. e.g. T. accerra, T. perditor; Tibraca spp., Tomaspis spp., Toxoptera spp. e.g. T. aurantii; Trialeurodes spp. e.g. T. abutilonea, T. ricini, T. vaporariorum; Triatoma spp., Trioza spp., Typhlocyba spp., Unaspis spp. e.g. U. citri, U. yanonensis; and Viteus vitifolii,
Insects from the order Hymenoptera e.g. Acanthomyops interjectus, Athalia rosae, Atta spp. e.g. A. capiguara, A. cephalotes, A. cephalotes, A. laevigata, A. robusta, A. sex¬dens, A. texana, Bombus spp., Brachymyrmex spp., Camponotus spp. e.g. C. floridanus, C. pennsylvanicus, C. modoc; Cardiocondyla nuda, Chalibion sp, Crematogaster spp., Dasymutilla occidentalis, Diprio¬n spp., Dolichovespula maculata, Dorymyrmex spp., Dryocosmus kuriphilus, Formica spp., Hoplocampa spp. e.g. H. minuta, H. testudinea; Iridomyrmex humilis, Lasius spp. e.g. L. niger, Linepithema humile, Liometopum spp., Leptocybe invasa, Monomorium spp. e.g. M. pharaonis, Monomorium, Nylandria fulva, Pachycondyla chinensis, Paratre¬chi¬na longicornis, Paravespula spp., e.g. P. germanica, P. pennsylvanica, P. vulgaris; Pheidole spp. e.g. P. megacephala; Po¬go¬nomyrmex spp. e.g. P. barbatus, P. californicus, Polistes rubiginosa, Prenolepis impairs, Pseu¬domyrmex gracilis, Schelipron spp., Sirex cyaneus, Solenopsis spp. e.g. S. geminata, S.invicta, S. molesta, S. richteri, S. xyloni, Sphecius speciosus, Sphex spp., Tapinoma spp. e.g. T. melanocephalum, T. sessile; Tetramorium spp., e.g. T. caespitum, T. bicarinatum, Vespa spp., e.g. V. crabro; Vespula spp., e.g. V. squamosal; Wasmannia auropunctata, Xylocopa sp;
Insects from the order Orthoptera e.g. Acheta domesticus, Calliptamus italicus, Chortoicetes terminifera, Ceuthophilus spp., Diastrammena asynamora, Dociostaurus maroccanus, Gryllotalpa spp. e.g. G. africana, G. gryllotalpa; Gryllus spp., Hieroglyphus daganensis, Kraussa¬ria angulifera, Locusta spp. e.g. L. migratoria, L. pardalina; Melanoplus spp. e.g. M. bivittatus, M. femurrubrum, M. mexicanus, M. sanguinipes, M. spretus; Nomadacris septemfasciata, Oedaleus senegalensis, Scapteriscus spp., Schistocerca spp. e.g. S. americana, S. gregaria, Stemopelmatus spp., Tachycines asynamorus, and Zonozerus variegatus;
Pests from the Class Arachnida e.g. Acari,e.g. of the families Argasidae, Ixodidae and Sarcop¬tidae, e.g. Amblyomma spp. (e.g. A. americanum, A. variegatum, A. maculatum), Argas spp. e.g. A. persicu), Boophilus spp. e.g. B. annulatus, B. decoloratus, B. micro¬plus, Dermacentor spp. e.g. D.silvarum, D. andersoni, D. variabilis, Hyalomma spp. e.g. H. truncatum, Ixodes spp. e.g. I. ricinus, I. rubicundus, I. scapularis, I. holocyclus, I. pacificus, Rhipicephalus sanguineus, Ornithodorus spp. e.g. O. moubata, O. hermsi, O. turicata, Ornithonyssus bacoti, Otobius meg¬nini, Dermanyssus gallinae, Psoroptes spp. e.g. P. ovis, Rhi¬picephalus spp. e.g. R. sanguineus, R. appendiculatus, Rhipicephalus evertsi, Rhizogly¬phus spp., Sarcoptes spp. e.g. S. Scabiei; and Family Eriophyidae including Aceria spp. e.g. A. sheldoni, A. anthocoptes, Acallitus spp., Aculops spp. e.g. A. lycopersici, A. pelekassi; Aculus spp. e.g. A. schlechtendali; Colomerus vitis, Epitrimerus pyri, Phyllocoptruta oleivora; Eriophytes ribis and Eriophyes spp. e.g. Eriophyes sheldoni; Family Tarsonemidae including Hemitarsonemus spp., Phytonemus pallidus and Polyphagotarsonemus latus, Steno¬tar¬so¬nemus spp. Steneotarsonemus spinki; Family Tenuipalpidae including Brevipalpus spp. e.g. B. phoenicis; Family Tetranychidae including Eotetranychus spp., Eutetranychus spp., Oligonychus spp., Petrobia latens, Tetranychus spp. e.g. T. cinnabarinus, T. evansi, T. kan¬zawai, T, pacificus, T. phaseulus, T. telarius and T. urticae; Bryobia praetiosa; Panonychus spp. e.g. P. ulmi, P. citri; Metatetranychus spp. and Oligonychus spp. e.g. O. pratensis, O. perseae, Vasates lycopersici; Raoiella indica, Family Carpoglyphidae including Car¬poglyphus spp.; Penthaleidae spp. e.g. Halotydeus destructor; Family Demodicidae with species e.g. Demodex spp.; Family Trombicidea including Trombicula spp.; Family Macronyssidae including Ornothonyssus spp.; Family Pyemotidae including Pyemotes tritici; Tyrophagus putrescen¬tiae; Family Acaridae includ-ing Acarus siro; Family Araneida including Latrodectus mactans, Tegenaria agrestis, Chi-racanthium sp, Lycosa sp Achaearanea tepidariorum and Loxosceles reclusa;
Pests from the Phylum Nematoda, e.g. plant parasitic nematodes e.g. root-knot nematodes, Me¬loidogyne spp. e.g. M. hapla, M. incognita, M. javanica; cyst-forming nematodes, Globodera spp. e.g. G. rostochiensis; Heterodera spp. e.g. H. avenae, H. glyci¬nes, H. schachtii, H. trifolii; Seed gall nematodes, Anguina spp.; Stem and foliar nematodes, Aphelenchoides spp. e.g. A. besseyi; Sting nematodes, Belonolaimus spp. e.g. B. longicaudatus; Pine nematodes, Bursaphelenchus spp. e.g. B. lignicolus, B. xylophilus; Ring nematodes, Criconema spp., Criconemella spp. e.g. C. xenoplax and C. ornata; and, Criconemoides spp. e.g. Criconemoides informis; Mesocriconema spp.; Stem and bulb nematodes, Ditylenchus spp. e.g. D. destructor, D. dipsaci; Awl nematodes, Dolichodorus spp.; Spiral nematodes, Heliocotylenchus multicinctus; Sheath and sheathoid nematodes, Hemicycliophora spp. and Hemicriconemoides spp.; Hirshmanniella spp.; Lance nematodes, Hoploaimus spp.; False rootknot nematodes, Nacobbus spp.; Needle nematodes, Longidorus spp. e.g. L. elongatus; Lesion nematodes, Pratylenchus spp. e.g. P. brachyurus, P. neglectus, P. penetrans, P. curvitatus, P. goodeyi; Burrowing nema-todes, Radopholus spp. e.g. R. similis; Rhadopholus spp.; Rhodopholus spp.; Reniform nematodes, Rotylenchus spp. e.g. R. robustus, R. reniformis; Scutellonema spp.; Stubby-root nematode, Trichodorus spp. e.g. T. obtusus, T. primitivus; Paratrichodorus spp. e.g. P. minor; Stunt nematodes, Tylenchorhynchus spp. e.g. T. claytoni, T. dubius; Citrus nematodes, Tylenchulus spp. e.g. T. semipenetrans; Dagger nematodes, Xiphinema spp.; and other plant parasitic nematode species;
Insects from the order Blattodea e.g. Macrotermes spp. e.g. M. natalensis; Cornitermes cumulans, Procornitermes spp., Globitermes sulfureus, Neocapritermes spp. e.g. N. opa¬cus, N. parvus; Odontotermes spp., Nasutitermes spp. e.g. N. corniger; Coptotermes spp. e.g. C. formo¬sanus, C. gestroi, C. acinaciformis; Reticulitermes spp. e.g. R. hesperus, R. tibialis, R. speratus, R. flavipes, R. grassei, R. lucifugus, R. virginicus; Heterotermes spp. e.g. H. aureus, H. longiceps, H. tenuis; Cryptotermes spp. e.g. C. brevis, C. cavifrons; Incisitermes spp. e.g. I. minor, I. snyderi; Marginitermes hubbardi, Kalotermes flavicollis, Neotermes spp. e.g. N. castaneus, Zootermopsis spp. e.g. Z. angusticollis, Z. nevadensis, Mastotermes spp. e.g. M. darwiniensis; Blatta spp. e.g. B. orientalis, B. lateralis; Blattella spp. e.g. B. asahinae, B. germanica; Rhyparobia maderae, Panchlora nivea, Periplaneta spp. e.g. P. americana, P. australasiae, P. brunnea, P. fuliginosa, P. japonica; Supella longipalpa, Parcoblatta pennsylvanica, Eurycotis floridana, Pycnoscelus surinamensis,
Insects from the order Siphonoptera e.g. Cediopsylla simples, Ceratophyllus spp., Ctenocephalides spp. e.g. C. felis, C. canis, Xenopsylla cheopis, Pulex irritans, Trichodectes canis, Tunga penetrans, and Nosopsyllus fasciatus,
Insects from the order Thysanura e.g. Lepisma saccharina, Ctenolepisma urbana, and Thermobia domestica,
Pests from the class Chilopoda e.g. Geophilus spp., Scutigera spp. e.g. Scutigera coleoptrata;
Pests from the class Diplopoda e.g. Blaniulus guttulatus, Julus spp., Narceus spp.,
Pests from the class Symphyla e.g. Scutigerella immaculata,
Insects from the order Dermaptera, e.g. Forficula auricularia,
Insects from the order Collembola, e.g. Onychiurus spp., e.g. Onychiurus armatus,
Pests from the order Isopoda, e.g. Armadillidium vulgare, Oniscus asellus, Porcellio scaber, Insects from the order Phthiraptera, e.g. Damalinia spp., Pediculus spp. e.g. Pediculus humanus capitis, Pediculus humanus corporis, Pediculus humanus humanus; Pthirus pubis, Haematopinus spp. e.g. Haematopinus eurysternus, Haematopinus suis; Linognathus spp. e.g. Linognathus vituli; Bovicola bovis, Menopon gallinae, Menacanthus stramineus and Solenopotes capillatus, Trichodectes spp.,

Further pest species which may be controlled by compounds I include: from the Phylum Mollusca, class Bivalvia, e.g., Dreissena spp.; class Gastropoda, e.g., Arion spp., Biomphalaria spp., Bulinus spp., Deroceras spp., Galba spp., Lymnaea spp., Oncomelania spp., Pomacea canaliclata, Succinea spp.; from the class of the helminths, e.g., Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp. e.g. Haemonchus contortus; Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp., Strongyloides fuel-leborni, Strongyloides stercora lis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

The compounds of the invention are particularly suitable for efficiently combating
insects from the sub-order of Auchenorrhyncha, e.g. Amrasca biguttula, Empoasca spp., Nephotettix virescens, Sogatella furcifera, Mahanarva spp., Laodelphax striatellus, Nilapar-vata lugens, Diaphorina citri;
Lepidoptera, e.g. Helicoverpa spp., Heliothis virescens, Lobesia botrana, Ostrinia nubilalis, Plutella xylostella, Pseudoplusia includens, Scirpophaga incertulas, Spodoptera spp., Trichoplusia ni, Tuta absoluta, Cnaphalocrocis medialis, Cydia pomonella, Chilo suppressalis, Anticarsia gemmatalis, Agrotis ipsilon, Chrysodeixis includens;
True bugs, e.g. Lygus spp., Stink bugs such as Euschistus spp., Halyomorpha halys, Nezara viridula, Piezodorus guildinii, Dichelops furcatus;
Thrips, e.g. Frankliniella spp., Thrips spp., Dichromothrips corbettii;
Aphids, e.g. Acyrthosiphon pisum, Aphis spp., Myzus persicae, Rhopalosiphum spp., Schizaphis graminum, Megoura viciae;
Whiteflies, e.g. Trialeurodes vaporariorum, Bemisia spp.;
Coleoptera, e.g. Phyllotreta spp., Melanotus spp., Meligethes aeneus, Leptinotarsa decimlineata, Ceutorhynchus spp., Diabrotica spp., Anthonomus grandis, Atomaria linearia, Agriotes spp., Epilachna spp.;
Flies, e.g. Delia spp., Ceratitis capitate, Bactrocera spp., Liriomyza spp.;
Coccoidea, e.g. Aonidiella aurantia, Ferrisia virgate;
Anthropods of class Arachnida (Mites), e.g. Penthaleus major, Tetranychus spp.;
Nematodes, e.g. Heterodera glycines, Meloidogyne sp., Pratylenchus spp., Caenorhabditis elegans.

The compounds of the invention are suitable for use in treating or protecting animals against infestation or infection by parasites. Therefore, the invention also relates to the use of a compound of the invention for the manufacture of a medicament for the treatment or protection of animals against infestation or infection by parasites. Furthermore, the invention relates to a method of treating or protecting animals against infestation and infection by parasites, which comprises orally, topically or parenterally administering or applying to the animals a parasiticidally effective amount of a compound of the invention.

The invention also relates to the non-therapeutic use of compounds of the invention for treating or protecting animals against infestation and infection by parasites. Moreover, the invention relates to a non-therapeutic method of treating or protecting animals against infestation and infection by parasites, which comprises applying to a locus a parasiticidally effective amount of a compound of the invention.

The compounds of the invention are further suitable for use in combating or controlling parasites in and on animals. Furthermore, the invention relates to a method of combating or controlling parasites in and on animals, which comprises contacting the parasites with a parasitically effective amount of a compound of the invention.

The invention also relates to the non-therapeutic use of compounds of the invention for controlling or combating parasites. Moreover, the invention relates to a non-therapeutic method of combating or controlling parasites, which comprises applying to a locus a parasiticidally effective amount of a compound of the invention.

The compounds of the invention can be effective through both contact (via soil, glass, wall, bed net, carpet, blankets or animal parts) and ingestion (e.g. baits). Furthermore, the compounds of the invention can be applied to any and all developmental stages.

The compounds of the invention can be applied as such or in form of compositions comprising the compounds of the invention.

The compounds of the invention can also be applied together with a mixing partner, which acts against pathogenic parasites, e.g. with synthetic coccidiosis compounds, polyetherantibiotics e.g. Amprolium, Robenidin, Toltrazuril, Monensin, Salinomycin, Maduramicin, Lasalocid, Narasin or Semduramicin, or with other mixing partners as defined above, or in form of compositions comprising said mixtures.

The compounds of the invention and compositions comprising them can be applied orally, parenterally or topically, e.g. dermally. The compounds of the invention can be systemically or non-systemically effective.

The application can be carried out prophylactically, therapeutically or non-therapeutically. Furthermore, the application can be carried out preventively to places at which occurrence of the parasites is expected.

As used herein, the term "contacting" includes both direct contact (applying the compounds/compositions directly on the parasite, including the application directly on the animal or excluding the application directly on the animal, e.g. at its locus for the latter) and indirect contact (applying the compounds/compositions to the locus of the parasite). The contact of the parasite through application to its locus is an example of a non-therapeutic use of the compounds of the invention.

The term "locus" means the habitat, food supply, breeding ground, area, material or environment in which a parasite is growing or may grow outside of the animal.

As used herein, the term "parasites" includes endo- and ectoparasites. In some embodiments of the invention, endoparasites can be preferred. In other embodiments, ectoparasites can be preferred. Infestations in warm-blooded animals and fish include lice, biting lice, ticks, nasal bots, keds, biting flies, muscoid flies, flies, myiasitic fly larvae, chiggers, gnats, mosquitoes and fleas.

The compounds of the invention are especially useful for combating parasites of the following orders and species, respectively:
fleas (Siphonaptera), e.g. Ctenocephalides felis, C. canis, Xenopsylla cheopis, Pulex irri-tans, Tunga penetrans, and Nosopsyllus fasciatus; cockroaches (Blattaria - Blattodea), e.g. Blattella germanica, B. asahinae, Periplaneta americana, P. japonica, P. brunnea, P. fuligginosa, P. australasiae, and Blatta orientalis; flies, mosquitoes (Diptera), e.g. Aedes aegypti, A. albopictus, A. vexans, Anastrepha ludens, Anopheles maculipennis, A. crucians, A. albimanus, A. gambiae, A. freeborni, A. leucosphyrus, A. minimus, A. quadrimaculatus, Calliphora vicina, Chrysomya bezziana, C. hominivorax, C. macellaria, Chrysops discalis, C. silacea, C. atlanticus, Cochliomyia hominivorax, Cordylobia anthropophaga, Culicoides furens, Culex pipiens, C. nigripalpus, C. quinquefasciatus, C. tarsalis, Culiseta inornata, C. melanura, Dermatobia hominis, Fannia canicularis, Gasterophilus intestinalis, Glossina morsitans, G. palpalis, G. fuscipes, G. tachinoides, Haematobia irritans, Haplodiplosis equestris, Hippelates spp., Hypoderma line-ata, Leptoconops torrens, Lucilia caprina, L. cuprina, L. sericata, Lycoria pectoralis, Mansonia spp., Musca domestica, M. stabulans, Oestrus ovis, Phlebotomus argentipes, Psorophora columbiae, P. discolor, Prosimulium mixtum, Sarcophaga spp., S. haemorrhoidalis, Simulium vittatum, Stomoxys calcitrans, Tabanus bovinus, T. atratus, T. lineola, and T. similis; lice (Phthiraptera), e.g. Pediculus humanus capitis, P. humanus humanus, Pthirus pubis, Haematopinus eurysternus, H. suis, Linognathus vituli, Bovicola bovis, Menopon gallinae, Menacanthus stramineus, and Solenopotes capillatus; ticks and parasitic mites (Parasitiformes): ticks (Ixodida), e.g. Ixodes scapularis, I. holocyclus, I. pacificus, Rhiphicephalus sanguineus, Dermacentor andersoni, D. variabilis, Amblyomma americanum, A. maculatum, Ornithodorus hermsi, O. turicata and parasitic mites (Mesostigmata), e.g. Ornithonyssus bacoti, Dermanyssus gallinae; Actinedida (Prostigmata) and Acaridida (Astigmata), e.g. Acarapis spp., Cheyletiella spp., Ornithocheyletia spp., Myobia spp., Psorergates spp., Demo-dex spp., Trombicula spp., Listro¬phorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., and Laminosioptes spp; Bugs (Heteropterida): Cimex lectularius, C. hemipterus, Reduvius senilis, Triatoma spp., Rhodnius ssp., Panstrongylus ssp., and Arilus critatus; Anoplurida, e.g. Haematopinus spp., Linognathus spp., Pediculus spp., Phtirus spp., and Solenopotes spp.; Mallophagida (suborders Arnblycerina and Ischnocerina), e.g. Tri¬menopon spp., Menopon spp., Trinoton spp., Bovicola spp., Werneckiella spp., Lepikentron spp., Trichodectes spp., and Felicola spp.; Roundworms Nematoda: Wipeworms and Trichi¬nosis (Trichosyringida), e.g. Tri¬chinellidae (Trichinella spp.), (Trichuridae) Trichuris spp., Capillaria spp.; Rhabditida, e.g. Rhabditis spp., Strongyloides spp., Helicephalobus spp.; Strongylida, e.g. Strongylus spp., Ancylo¬sto¬ma spp., Necator americanus, Bunostomum spp. (Hookworm), Trichostrongylus spp., Haemonchus contortus, Ostertagia spp., Cooperia spp., Nematodirus spp., Dictyocaulus spp., Cyatho¬sto¬ma spp., Oesophagostomum spp., Stephanurus dentatus, Ollula¬nus spp., Chabertia spp., Stephanurus dentatus, Syngamus trachea, Ancylostoma spp., Uncinaria spp., Globocephalus spp., Necator spp., Metastrongylus spp., Muellerius capillaris, Protostrongylus spp., Angiostrongylus spp., Parelaphostrongylus spp., Aleurostrongylus abstrusus, and Dioctophyma renale; Intestinal roundworms (Ascaridida), e.g. Ascaris lumbricoides, Ascaris suum, Ascaridia galli, Parascaris equorum, Enterobius vermicularis (Threadworm), Toxocara canis, Toxascaris leonine, Skrjabinema spp., and Oxyuris equi; Camallanida, e.g. Dracunculus medinensis (gui¬nea worm); Spirurida, e.g. Thelazia spp., Wuchereria spp., Brugia spp., Onchocerca spp., Dirofilari spp.a, Dipetalonema spp., Setaria spp., Elaeophora spp., Spirocerca lupi, and Habronema spp.; Thorny headed worms (Acanthocephala), e.g. Acanthocephalus spp., Macracanthorhynchus hirudinaceus and Oncicola spp.; Planarians (Plathelminthes): Flukes (Trematoda), e.g. Faciola spp., Fascioloides magna, Paragonimus spp., Dicrocoelium spp., Fasciolopsis buski, Clonorchis sinensis, Schistosoma spp., Trichobilharzia spp., Alaria alata, Paragonimus spp., and Nanocyetes spp.; Cercomeromorpha, in particular Cestoda (Tapeworms), e.g. Diphyllobothrium spp., Tenia spp., Echinococcus spp., Dipylidium caninum, Multiceps spp., Hymenolepis spp., Mesocestoides spp., Vampirolepis spp., Moniezia spp., Anoplocephala spp., Sirometra spp., Anoplocephala spp., and Hymenolepis spp..

The term "animal" includes warm-blooded animals (including humans) and fish. Preferred are mammals, e.g. cattle, sheep, swine, camels, deer, horses, pigs, poultry, rabbits, goats, dogs and cats, water buffalo, donkeys, fallow deer and reindeer, and also in fur-bearing animals e.g. mink, chinchilla and raccoon, birds e.g. hens, geese, turkeys and ducks and fish e.g. fresh- and salt-water fish e.g. trout, carp and eels. Particularly preferred are domestic animals, e.g. dogs or cats.

Generally, "parasiticidally effective amount" means the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions e.g. desired parasiticidal effect and duration, target species, mode of application.

Generally, it is favorable to apply the compounds of the invention in total amounts of 0.5 mg/kg to 100 mg/kg per day, preferably 1 mg/kg to 50 mg/kg per day.

For oral administration to warm-blooded animals, the compounds I may be formulated as animal feeds, animal feed premixes, animal feed concentrates, pills, solutions, pastes, suspensions, drenches, gels, tablets, boluses and capsules. In addition, the compounds I may be ad-ministered to the animals in their drinking water. For oral administration, the dosage form chosen should provide the animal with 0.01 mg/kg to 100 mg/kg of animal body weight per day of the compounds I, preferably with 0.5 mg/kg to 100 mg/kg of animal body weight per day.

Alternatively, the compounds I may be administered to animals parenterally, e.g., by intraruminal, intramuscular, intravenous or subcutaneous injection. The compounds I may be dispersed or dissolved in a physiologically acceptable carrier for subcutaneous injection. Alternatively, the compounds I may be formulated into an implant for subcutaneous administration. In addition the compounds I may be transdermally administered to animals. For parenteral administration, the dosage form chosen should provide the animal with 0.01 mg/kg to 100 mg/kg of animal body weight per day of the compounds I.

The compounds I may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the compounds I. In addition, the compounds I may be formulated as ear tags for animals, particularly quadrupeds e.g. cattle and sheep.

Suitable preparations are:
- Solutions e.g. oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations e.g. powders, premixes or concentrates, granules, pellets, tablets, boluses, capsules; aerosols and inhalants, and active compound-containing shaped articles.

Compositions suitable for injection are prepared by dissolving the active ingredient in a suitable solvent and optionally adding further auxiliaries e.g. acids, bases, buffer salts, preservatives, and solubilizers. Suitable auxiliaries for injection solutions are known in the art. The solutions are filtered and filled sterile.

Oral solutions are administered directly. Concentrates are administered orally after prior dilution to the use concentration. Oral solutions and concentrates are prepared according to the state of the art and as described above for injection solutions, sterile procedures not being necessary.

Solutions for use on the skin are trickled on, spread on, rubbed in, sprinkled on or sprayed on. Solutions for use on the skin are prepared according to the state of the art and according to what is described above for injection solutions, sterile procedures not being necessary. Gels are applied to or spread on the skin or introduced into body cavities. Gels are prepared by treating solutions which have been prepared as described in the case of the injection solutions with sufficient thickener that a clear material having an ointment-like consistency results. Suitable thickeners are known in the art.

Pour-on formulations are poured or sprayed onto limited areas of the skin, the active compound penetrating the skin and acting systemically. Pour-on formulations are prepared by dissolving, suspending or emulsifying the active compound in suitable skin-compatible solvents or solvent mixtures. If appropriate, other auxiliaries e.g. colorants, bioabsorption-promoting substances, antioxidants, light stabilizers, adhesives are added. Suitable such auxiliaries are known in the art.

Emulsions can be administered orally, dermally or as injections. Emulsions are either of the water-in-oil type or of the oil-in-water type. They are prepared by dissolving the active compound either in the hydrophobic or in the hydrophilic phase and homogenizing this with the solvent of the other phase with the aid of suitable emulsifiers and, if appropriate, other auxiliaries e.g. colorants, absorption-promoting substances, preservatives, antioxidants, light stabilizers, viscosity-enhancing substances. Suitable hydrophobic phases (oils), suitable hydrophilic phases, suitable emulsifiers, and suitable further auxiliaries for emulsions are known in the art.

Suspensions can be administered orally or topically/dermally. They are prepared by suspending the active compound in a suspending agent, if appropriate with addition of other auxiliaries e.g. wetting agents, colorants, bioabsorption-promoting substances, preservatives, antioxidants, light stabilizers. Suitable suspending agents, and suitable other auxiliaries for suspensions including wetting agents are known in the art.

Semi-solid preparations can be administered orally or topically/dermally. They differ from the suspensions and emulsions described above only by their higher viscosity.

For the production of solid preparations, the active compound is mixed with suitable excipients, if appropriate with addition of auxiliaries, and brought into the desired form. Suitable auxiliaries for this purpose are known in the art.

The compositions which can be used in the invention can comprise generally from about 0.001 to 95% of the compound of the invention.

Ready-to-use preparations contain the compounds acting against parasites, preferably ectoparasites, in concentrations of 10 ppm to 80% by weight, preferably from 0.1 to 65% by weight, more preferably from 1 to 50% by weight, most preferably from 5 to 40% by weight.

Preparations which are diluted before use contain the compounds acting against ectoparasites in concentrations of 0.5 to 90% by weight, preferably of 1 to 50% by weight.

Furthermore, the preparations comprise the compounds of formula I against endoparasites in concentrations of 10 ppm to 2% by weight, preferably of 0.05 to 0.9% by weight, very particularly preferably of 0.005 to 0.25% by weight.

Topical application may be conducted with compound-containing shaped articles e.g. collars, medallions, ear tags, bands for fixing at body parts, and adhesive strips and foils.

Generally it is favorable to apply solid formulations which release compounds of the invention in total amounts of 10 mg/kg to 300 mg/kg, preferably 20 mg/kg to 200 mg/kg, most preferably 25 mg/kg to 160 mg/kg body weight of the treated animal in the course of three weeks.

### Examples:

With appropriate modification of the starting materials, the procedures as described in the preparation examples below were used to obtain further compounds of formula I. The compounds obtained in this manner are listed in the table C that follows, together with physical data.

Compounds can be characterized e.g., by coupled High Performance Liquid Chromatography / mass spectrometry (HPLC/MS), by 1H-NMR and/or by their melting points. Analytical HPLC - Method 1: Agilent Eclipse Plus C18, 50 × 4,6 mm, ID 5µm; Elution: A = 10 mM Amm. Formate (0.1 % Formic Acid), B = Acetonitrile (0.1 % Formic Acid), Flow = 1.2 ml/min. at 30 °C; Gradient: 10% B to 100 % B - 3 min, hold for 1 min, 1 min - 10% B. Run Time = 5.01 min.

Analytical HPLC - Method 2: Kinetex XB C18 1,7µ 50 × 2,1mm; A = Water + 0.1 % TFA, B = Acetonitrile, Flow = 0.8 ml/min - 1.0 ml/min in 1.5 min. at 60 °C; Gradient: 5 % B to 100 % B - 1.5 min.

1H-NMR: The signals are characterized by chemical shift (ppm, δ [delta]) vs. tetramethylsilane respectively, CDCl₃ for ¹³C-NMR, by their multiplicity and by their integral (relative number of hydrogen atoms given). The following abbreviations are used to characterize the multiplicity of the signals: m = multiplet, q = quartet, t = triplet, d = doublet and s = singlet. Abbreviations used are: d for day(s), h for hour(s), min for minute(s), r.t./room temperature for 20 - 25 °C, Rt for retention time; DMSO for dimethyl sulfoxide, OAc for acetate, EtOAc for ethyl acetate, THF for tetrahydrofuran, DMF for N,N-Dimethylformamide, t-BuOH for tert-butanol and RH for relative humidity.

### Example C-1:

Synthesis of 1-[(E)-[4-[1,4-dimethyl-5-[1-oxo-5-(trifluoromethoxy)isoindolin-2-yl]pyrazol-3-yl]phenyl]methyleneamino]-3-(2-isopropyl-5-methyl-phenyl)thiourea:
Step 1: Synthesis of 3-(4-bromophenyl)-2-methyl-3-oxo-propanenitrile:
   Under an argon atmosphere, methyl 4-bromobenzoate (50 g, 232 mmol) and propanenitrile (21.5 g, 390 mmol) were dissolved in tetrahydrofuran (500 ml) and a solution of lithium bis(trimethylsilyl)amide (370 ml, 1.1 M, 407 mmol) was added dropwise at ambient temperature. The reaction mixture was stirred overnight. The reaction was quenched by addition of water and extracted with dichloromethane. The organic phase was discarded. The aqueous phase was acidified with aqueous hydrochloric acid to pH 2 and extracted with dichloromethane (3x). The combined organic phase extracts were dried over sodium sulfate and concentrated. The desired product thus obtained (32 g, 57% yield) was used in the next step without further purification.
Step 2: Synthesis of 5-(4-bromophenyl)-2,4-dimethyl-pyrazol-3-amine:
   Methylhydrazine sulfate (20 g, 175 mmol) was added to a stirred solution of 3-(4-bromophenyl)-2-methyl-3-oxo-propanenitrile (32 g, 134 mmol) in EtOH (3000 mL). Resulting mixture was stirred overnight at 80 °C and evaporated. Crude residue was treated with 2M NaOH to pH = 9 filtered and concentrated under vacuum to give 5-(4-bromophenyl)-2,4-dimethyl-pyrazol-3-amine (22 g, 82 mmol, 47% yield) that was used in the next step without further purification.
Step 3: Synthesis of methyl 2-methyl-4-(trifluoromethoxy)benzoate:
   Mixture of 2-methyl-4-(trifluoromethoxy)benzoic acid (30 g, 136 mmol), Mel (25.2 g, 177 mmol), and potassium carbonate (28.2 g, 204 mmol) in DMF (150 mL) was stirred under argon atmosphere at RT for 18 h. Obtained mixture was diluted with water (150 mL) and extracted with EtOAc (150 mL × 3), Combined organic layers were washed with water (150 mL × 2) and brine (200 mL), dried over sodium sulfate, filtered, and concentrated under vacuum affording methyl 2-methyl-4-(trifluoromethoxy)benzoate (31 g, 87% yield).
Step 4: Synthesis of methyl 2-(bromomethyl)-4-(trifluoromethoxy)benzoate: 2-methyl-4-(trifluoromethoxy)benzoate (30 g, 128 mmol) and benzoyl peroxide (BPO, 0.56 g, 2.3 mmol) was dissolved in CCl₄ (300 mL) and heated to reflux, NBS (25.2 g, 141 mmol) was added in portions and continue to heat reflux 2h, after cooling to room temperature, the reaction was filtered and concentrated under vacuum. The crude residue was purified by flash column chromatography to give methyl 2-(bromomethyl)-4-(trifluoromethoxy)benzoate (18 g, 44% yield).
Step 5: Synthesis of 2-[5-(4-bromophenyl)-2,4-dimethyl-pyrazol-3-yl]-5-(trifluoromethoxy) isoindolin-1-one:
   Mixture of methyl 2-(bromomethyl)-4-(trifluoromethoxy)benzoate (10 g, 31 mmol), 5-(4-bromophenyl)-2,4-dimethyl-pyrazol-3-amine (8.5 g, 32 mmol) and *N,N*-diisopropylethylamine (33 ml, 186 mmol) in DMF (100 mL) was stirred under argon atmosphere at 80 °C for 18 h. Obtained mixture was diluted with water (100 mL) and extracted with EtOAc (100 mL × 3), Combined organic layers were washed with water (50 mL × 2) and brine (20 mL), dried over sodium sulfate, filtered, and concentrated under vacuum. The crude residue was purified by flash column chromatography to give 2-[5-(4-bromophenyl)-2,4-dimethyl-pyrazol-3-yl]-5-(trifluoromethoxy)isoindolin-1-one (6 g, 41% yield).
Step 6: Synthesis of 2-[2,4-dimethyl-5-(4-vinylphenyl)pyrazol-3-yl]-5-(trifluoromethoxy)isoindolin-1-one:
   Pd2(dba)3 (0.23 g, 0.25 mmol) was added to a solution of 2-[5-(4-bromophenyl)-2,4-dimethyl-pyrazol-3-yl]-5-(trifluoromethoxy) isoindolin-1-one (11.25 g, 24 mmol), P(Cy)3 (0.18 g, 0.62 mmol), tripotassium phosphate (7.2 g, 33 mmol) and potassium ethenyltrifluoroboranide (6.7 g, 48 mmol) in dioxane (150 mL)/water (50 mL) mixture under argon atmosphere and the reaction mixture was stirred at 100 ° C for 18 h. After it was cooled to room temperature, partitioned between EtOAc (150 mL) and water (100 mL) and the aqueous phase was extracted with EtOAc (100 mL × 2). The combined organic layers were washed with brine (100 mL), dried over sodium sulfate, filtered and concentrated under vacuum. The residue was subjected to flash column chromatography purification that afforded 2-[2,4-dimethyl-5-(4-vinylphenyl)pyrazol-3-yl]-5-(trifluoromethoxy) isoindolin-1-one (6 g, 58% yield).
Step 7: Synthesis of 4-[1,4-dimethyl-5-[1-oxo-5-(trifluoromethoxy)isoindolin-2-yl]pyrazol-3-yl]benzaldehyde:
   A stirred solution of 8 (6 g, 14.27 mmol) in 1,4-dioxane (86 mL) was treated dropwise with water (57 mL), then sodium periodate (6 g, 30.0 mmol) and osmium tetroxide (0.2g, 0.7 mmol). The reaction mixture was stirred at room temperature for 4 h and then concentrated. The residue was partitioned between water and EtOAc. The organic layer was washed with one portion of saturated aqueous sodium thiosulfate solution, one portion of brine, dried over sodium sulfate, filtered, and concentrated. The crude residue was purified by flash column chromatography to give 4-[1,4-dimethyl-5-[1-oxo-5-(trifluoromethoxy)isoindolin-2-yl]pyrazol-3-yl]benzaldehyde (1.8 g, 28% yield). ¹H NMR (400 MHz, Chloroform-d) δ 10.01 (s, 1H), 8.01 (s, 1H), 7.84 (d, 2H), 7.82 (d, 2H), 7.42 - 7.38 (m, 2H), 4.78 (s, 2H), 3.89 (s, 3H), 2.08 (s, 3H).
Step 8: Synthesis of 1-[(E)-[4-[1,4-dimethyl-5-[1-oxo-5-(trifluoromethoxy)isoindolin-2-yl]pyrazol-3-yl]phenyl]methyleneamino]-3-(2-isopropyl-5-methyl-phenyl)thiourea (C-1):
   To a stirred solution of 400 mg (0.96 mmol) 4-[1,4-dimethyl-5-[1-oxo-5-(trifluoromethoxy)isoindolin-2-yl]pyrazol-3-yl]benzaldehyde in 32 mL ethanol was added 258 mg (1.16 mmol, 2 equivalents) of 1-amino-3-(2-isopropyl-5-methyl-phenyl)thiourea. The solution was heated at 60 °C for a period of three hours. Upon disappearance of the starting material as judged by TLC, the reaction mixture was cooled to room temperature, solvents evaporated and the crude reaction mixture subjected to flash chromatography to yield 510 mg (85% yield) of 1-[(E)-[4-[1,4-dimethyl-5-[1-oxo-5-(trifluoromethoxy)isoindolin-2-yl]pyrazol-3-yl]phenyl]methyleneamino]-3-(2-isopropyl-5-methyl-phenyl)thiourea. ¹H NMR (400 MHz, Chloroform-d) δ 9.59 (s, 1H), 8.98 (s, 1H), 8.04 (dd, J = 7.9, 1.0 Hz, 1H), 7.90 (s, 1H), 7.79 - 7.68 (m, 4H), 7.47 - 7.40 (m, 2H), 7.27 (d, J = 8.0 Hz, 1H), 7.18 - 7.11 (m, 1H), 4.77 (s, 2H), 3.81 (s, 3H), 3.14 (p, J = 6.9 Hz, 1H), 2.37 (s, 3H), 2.15 (s, 3H), 1.32 - 1.20 (m, 6H).

### Example C-2:

Synthesis of (2Z)-2-[(E)-[4-[1,4-dimethyl-5-[1-oxo-5-(trifluoromethoxy)isoindolin-2-yl]pyrazol-3-yl]phenyl]methylenehydrazono]-3-(2-isopropyl-5-methyl-phenyl)thiazolidin-4-one:
Step 1: Synthesis of (2Z)-2-[(E)-[4-[1,4-dimethyl-5-[1-oxo-5-(trifluoromethoxy)isoindolin-2-yl]pyrazol-3-yl]phenyl]methylenehydrazono]-3-(2-isopropyl-5-methyl-phenyl)thiazolidin-4-one (C-2):
To a stirred solution of 300 mg (0.48 mmol) of 1-[(E)-[4-[1 ,4-dimethyl-5-[1-oxo-5-(trifluoromethoxy)isoindolin-2-yl]pyrazol-3-yl]phenyl]methyleneamino]-3-(2-isopropyl-5-methyl-phenyl)thiourea in 20 mL ethanol was first added 79 mg (1 mmol, 2 equivalents) sodium acetate, followed by 147 mg (1 mmol, 2 equivalents) methyl bromoacetate. The reaction mixture was heated at 60 °C for 2 hours. Upon disappearance of starting material as judged by TLC, the solvents were evaporated and the crude mixture purified by flash chromatography to yield 262 mg (82%) of (2Z)-2-[(E)-[4-[1,4-dimethyl-5-[1-oxo-5-(trifluoromethoxy)isoindolin-2-yl]pyrazol-3-yl]phenyl] methylenehydrazono]-3-(2-isopropyl-5-methyl-phenyl)thiazolidin-4-one. ¹H NMR (400 MHz, Chloroform-d) δ 8.30 (s, 1H), 8.06 - 7.99 (m, 1H), 7.78 (d, J = 8.4 Hz, 2H), 7.73 (d, J = 8.4 Hz, 2H), 7.46 - 7.40 (m, 2H), 7.36 (d, J = 8.0 Hz, 1H), 7.29 (dd, J = 8.1, 1.9 Hz, 3H), 7.05 - 6.96 (m, 1H), 4.77 (s, 2H), 3.99 (d, J = 1.7 Hz, 2H), 3.80 (s, 3H), 2.78 (hept, J = 6.8 Hz, 1H), 2.37 (s, 3H), 2.14 (s, 3H), 1.22-1.20 (m, 6H).

### Example C-3:

Synthesis of [(Z)-N'-[(E)-[4-[1,4-dimethyl-5-[1-oxo-5-(trifluoromethoxy)isoindolin-2-yl]pyrazol-3-yl]phenyl]methyleneamino]-N-(2-isopropyl-5-methyl-phenyl)carbamimidoyl]sulfanylmethyl 2-methylpropanoate:
Step 1: Synthesis of [(Z)-N'-[(E)-[4-[1,4-dimethyl-5-[1-oxo-5-(trifluoromethoxy)isoindolin-2-yl]pyrazol-3-yl]phenyl]methyleneamino]-N-(2-isopropyl-5-methyl-phenyl)carbamimidoyl] sulfanylmethyl 2-methylpropanoate (C-3):
To a stirred solution of 250 mg (0.40 mmol) of 1-[(E)-[4-[1,4-dimethyl-5-[1-oxo-5-(trifluoromethoxy)isoindolin-2-yl]pyrazol-3-yl]phenyl]methyleneamino]-3-(2-isopropyl-5-methyl-phenyl)thiourea in 5 ml acetone was added 165 mg (1.20 mmol, 3 equivalents) of chloromethyl 2-methylpropanoate, 0.28 mL (1.61 mmol, 4 equivalents) of *N,N*-diisopropylamine and 18 mg (0.12 mmol, 0.3 equivalents) of sodium iodide. After stirring at 70 °C for 12 hours, the reaction mixture was cooled down to room temperature, solvents evaporated and the crude mixture purified by flash chromatography to yield 100 mg (35% yield) of [(Z)-N'-[(E)-[4-[1,4-dimethyl-5-[1-oxo-5-(trifluoromethoxy)isoindolin-2-yl]pyrazol-3-yl]phenyl]methyleneamino]-N-(2-isopropyl-5-methyl-phenyl)carbamimidoyl]sulfanylmethyl 2-methylpropanoate. ¹H NMR (500 MHz, Chloroform-d) δ 8.50 (s, 1H), 8.18 (s, 1H), 8.05 - 8.00 (m, 1H), 7.86 - 7.80 (m, 2H), 7.80 - 7.68 (m, 2H), 7.46 - 7.40 (m, 2H), 7.28 - 7.06 (m, 3H), 5.74 (s, 2H), 4.76 (s, 2H), 3.80 (s, 3H), 3.21 (hept, J = 6.9 Hz, 1H), 2.58 (hept, J = 7.0 Hz, 1H), 2.33 (s, 3H), 2.15 (s, 3H), 1.23 (d, J = 6.9 Hz, 6H), 1.16 (d, J = 7.0 Hz, 6H).

### Example C-4:

Synthesis of [(Z)-N'-[(E)-[4-[1,4-dimethyl-5-[1-oxo-5-(trifluoromethoxy)isoindolin-2-yl]pyrazol-3-yl]phenyl]methyleneamino]-N-(2-isopropyl-5-methyl-phenyl)carbamimidoyl]sulfanylmethyl ethyl carbonate :
Step 1: Synthesis of [(Z)-N'-[(E)-[4-[1,4-dimethyl-5-[1-oxo-5-(trifluoromethoxy)isoindolin-2-yl]pyrazol-3-yl]phenyl]methyleneamino]-N-(2-isopropyl-5-methyl-phenyl)carbamimidoyl] sulfanylmethyl ethyl carbonate (C-4)
To a stirred solution of 700 mg (1.12 mmol) of 1-[(E)-[4-[1,4-dimethyl-5-[1-oxo-5-(trifluoromethoxy)isoindolin-2-yl]pyrazol-3-yl]phenyl]methyleneamino]-3-(2-isopropyl-5-methyl-phenyl)thiourea in 5 ml acetone was added 467 mg (3.37 mmol, 3 equivalents) of chloromethylethyl carbonate, 581 mg (4.50 mmol, 4 equivalents) of *N,N*-diisopropylamine and 50 mg (0.34 mmol, 0.3 equivalents) of sodium iodide. After stirring at 70 °C for 12 hours, the reaction mixture was cooled down to room temperature, solvents evaporated and the crude mixture purified by flash chromatography to yield 160 mg (18% yield) of [(Z)-N'-[(E)-[4-[1,4-dimethyl-5-[1-oxo-5-(trifluoromethoxy)isoindolin-2-yl]pyrazol-3-yl]phenyl]methyleneamino]-N-(2-isopropyl-5-methyl-phenyl)carbamimidoyl]sulfanylmethyl ethyl carbonate. ¹H NMR (400 MHz, Chloroform-d) δ 8.51 (s, 1H), 8.16 (s, 1H), 8.06 - 8.00 (m, 1H), 7.82 (d, J = 8.2 Hz, 2H), 7.74 (d, J = 8.2 Hz, 2H), 7.26-7.23 (m, 2H), 7.18-7.07 (m, 3H), 5.80 (s, 2H), 4.77 (s, 2H), 4.21 (q, J = 7.1 Hz, 2H), 3.80 (s, 3H), 3.20 (h, J = 6.8 Hz, 1H), 2.34 (3, 3H), 2.33 (s, 3H), 2.15 (s, 3H), 1.39 - 1.24 (m, 9H).

### Example C-7:

Synthesis of 1-[(E)-[4-[1,4-dimethyl-5-[1-oxo-6-(trifluoromethoxy)-3,4-dihydroisoquinolin-2-yl]pyrazol-3-yl]phenyl]methyleneamino]-3-(2-isopropyl-5-methyl-phenyl)thiourea:
Step 1: Synthesis of methyl 2-bromo-4-(trifluoromethoxy)benzoate:
   To a stirred solution of 2-bromo-1-iodo-4-(trifluoromethoxy) benzene (100 g, 272 mmol) in methanol (800 mL) mixture were added sodium methoxide 30% solution (73.62 g, 408 mmol) and 1,1'-Bis(Diphenyl phosphine) ferrocene-palladium(II)dichloride dichloromethane complex (2.26 g, 2.72 mmol ) at ambient temperature. The whole reaction mixture was stirred at 27 °C for 16 hours under CO gas pressure (5 bar). The progress of the reaction was monitored by LCMS analysis. The reaction mixture was filtered through a celite pad and the filtrate was concentrated under reduced pressure to get the crude product. The crude product was purified by column chromatography using EtOAc and heptane as eluent to offer the desired product as colorless liquid (61.2 g, 75.2 %). HPLC/MS: Rt: 2.16 min; m / z = 301 (M+2)⁺.
Step 2: Synthesis of methyl 4-(trifluoromethoxy)-2-vinyl-benzoate :
   Solution of methyl 2-bromo-4-(trifluoromethoxy) benzoate (39.0 g, 130.30 mmol) in THF (300 mL) and water (70 mL) was degassed with Nitrogen gas for 30 min. To the stirred degassed solution were added potassium vinyl trifluoroborate (26.20 g, 195.63 mmol), cesium carbonate (131.72 g, 404.29 mmol), triphenylphosphine (3.42 g,13.04 mmol) and palladium (II) chloride (1.15 g, 6.52 mmol) at ambient temperature. Reaction mass was heated for 12 hours at 55 °C. The progress of the reaction was monitored by TLC and LCMS. After the reaction was completed, the reaction mixture was filtered through a celite pad and washed with EtOAc (100 mL). The reaction Filtrate was diluted with water (200 mL) then extracted with EtOAc (100 mL × 2). The combined organic layers were dried over sodium sulphate and concentrated under reduced pressure to get the crude product. The crude product was purified by column chromatography using EtOAc and heptane as eluent to offer the desired product as color less liquid (30.5 g, 94.9%). GC/MS: Rt: 4.57 min; m / z = 246 (M).
Step 3: Synthesis of 4-(trifluoromethoxy)-2-vinyl-benzoic acid :
   To a stirred solution of methyl 4-(trifluoromethoxy)-2-vinyl-benzoate (31.0 g, 125.92 mmol) in THF (260 mL) and water (50 mL) was added lithium hydroxide (15.07 g, 629.62 mmol) at ambient temperature. The reaction mixture was stirred at 27 °C for 16 hours. The progress of the reaction was monitored by TLC. THF was evaporated through reduced pressure. Reaction mass was diluted with water (100 mL), extracted with EtOAc (2 × 100 mL). Aqueous layer was acidified with 2.0 N HCl solution (100 mL) to pH 3, followed by extraction with EtOAc (3 × 150 mL). Combined organic layers were dried over sodium sulphate and evaporated under reduced pressure to obtain the crude product as solid. The crude product was purified by column chromatography using EtOAc and heptane as eluent to afford the desired product as off-white solid (26.0 g, 89.6 %). HPLC/MS (Method 1): Rt: 2.01 min; m / z = 231.1(M-1) +
Step 4: Synthesis of 3-(4-bromophenyl)-2-methyl-3-oxo-propanenitrile:
   Under an argon atmosphere, ethyl 4-bromobenzoate (30 g, 130.9 mmol) and propanenitrile (8.65 g, 157.16 mmol) were dissolved in THF (600 ml) and potassium tert-butoxide (29.33 g, 261.93 mmol) was added portion wise at ambient temperature. The reaction mixture was stirred at 27 °C for 4 hours. The progress of the reaction was monitored by TLC. The reaction was quenched by addition of saturated ammonium chloride solution and followed by extraction with EtOAc (3 × 200 mL). Combined organic layers were dried over sodium sulphate and evaporated under reduced pressure to obtain the crude product as yellow solid (23.0 g) was used in the next step without further purification. ¹H NMR (500 MHz, CDCl3) δ 7.91 - 7.84 (m, 2H), 7.73 - 7.67 (m, 2H), 4.33 (q, J = 7.2 Hz, 1H), 1.66 (d, J = 7.2 Hz, 3H).
Step 5: Synthesis of 5-(4-bromophenyl)-2,4-dimethyl-pyrazol-3-amine:
   Methylhydrazine (6.67 g, 115.93 mmol, 80% in water) was added to a stirred solution of 3-(4-bromophenyl)-2-methyl-3-oxo-propanenitrile (23 g, 96.06 mmol) in ethanol (230 mL). Resulting mixture was stirred overnight at 80 °C and evaporated. Crude residue was treated with methyl tert-butyl ether, filtered, and concentrated under vacuum to give 5-(4-bromophenyl)-2,4-dimethyl-pyrazol-3-amine (20 g, 75% yield) that was used in the next step without further purification. HPLC/MS: Rt:1.79 min; m / z = 268 (M+2)⁺.
Step 6: Synthesis of N-[5-(4-bromophenyl)-2,4-dimethyl-pyrazol-3-yl]-4-(trifluoromethoxy)-2-vinyl-benzamide:
   To a stirred solution of 5-(4-bromophenyl)-2,4-dimethyl-pyrazol-3-amine (10 g, 37.57 mmol) in dichloromethane (100 mL) was added 4-hydroxy-2-vinyl-benzoic acid (9.59 g, 41.33 mmol) followed by addition of *N,N*-diisopropylethylamine (19.4 mL, 112.72 mmol) at 10 °C. The reaction mixture was cooled to 0 °C and to the reaction mixture was added 1-pro-panephosphonic anhydride solution, (67.07 mL, 50% solution in EtOAc, 112.72 mmol) dropwise over a period of 30 min. The reaction mixture was stirred at 27 °C for 16 hours. The progress of the reaction was monitored by TLC. After completion of reaction, the reaction mixture was added to water dropwise (750 mL) and stirred for 60 min. White precipitate was formed which was filtered and dried under reduced pressure to obtain the desired product as white solid compound (15.2 g, 84%). HPLC/MS (Method 1): Rt: 2.29 min; m / z = 483.2(M+2)⁺.
Step 7: Synthesis of 2-[5-(4-bromophenyl)-2,4-dimethyl-pyrazol-3-yl]-6-(trifluoromethoxy)-3,4-dihydroisoquinolin-1-one:

   To a stirred solution of N-[5-(4-bromophenyl)-2,4-dimethyl-pyrazol-3-yl]-4-(trifluoromethoxy)-2-vinyl-benzamide (1.0 g, 31.23 mmol) in dry DMF (8 mL) was added potassium tert-butoxide (0.07 g, 9.36 mmol) at ambient temperature. The reaction mixture was heated at 120 °C for 1 hour under microwave irradiation. The progress of the reaction was monitored by TLC. The reaction mixture was diluted with water (100 mL) and extracted with EtOAc (50 mL × 2). The combined organic layers were dried over sodium sulfate and concentrated under reduced pressure to get the crude product which was purified by column chromatography using EtOAc and heptane as eluent to afford the desired product as beige solid (0.48 g, 48%). HPLC/MS (Method 1): Rt: 2.35 min; m / z = 482.1 (M+2)⁺.
Step 8: Synthesis of 4-[1,4-dimethyl-5-[1-oxo-6-(trifluoromethoxy)-3,4-dihydroisoquinolin-2-yl]pyrazol-3-yl]benzaldehyde:
   To a stirred solution of 2-[5-(4-bromophenyl)-2,4-dimethyl-pyrazol-3-yl]-6-(trifluoromethoxy)-3,4-dihydroisoquinolin-1-one (1.1 g, 2.29 mmol) in dry DMF (25 mL) were added triethylsilane (0.79 g, 6.87 mmol), triethylamine (0.69 g, 6.87 mmol) and (1,3-Bis(diphenylphosphino)propane) palladium(II) chloride (0.20 g, 0.34 mmol) at ambient temperature. The reaction mixture was stirred at 80 °C for 16 hours under CO atmosphere (9 bar) in autoclave. The progress of the reaction was monitored by TLC. The reaction mixture was diluted with water (100 mL) and extracted with EtOAc (75 mL × 2). The combined organic layers were dried over sodium sulfate and concentrated under reduced pressure to get the crude product which was purified by column chromatography using EtOAc and heptane as eluent to afford the desired product as beige solid (0.9 g, 96% yield). ¹H NMR (300 MHz, DMSO-d6) δ 10.04 (s, 1H), 8.10 (d, J = 8.6 Hz, 1H), 7.96 (q, J = 8.3 Hz, 4H), 7.51 (s, 1H), 7.42 (d, J = 8.5 Hz, 1H), 3.93 (t, J = 6.5 Hz, 2H), 3.32 (t, J = 6.5 Hz, 2H) 3.75 (s, 3H), 2.16 (s, 3H). HPLC/MS (Method 1): Rt: 2.07 min; m / z = 430.3 (M+1)⁺.
Step 9: Synthesis of 1-[(E)-[4-[1,4-dimethyl-5-[1-oxo-6-(trifluoromethoxy)-3,4-dihydroisoquinolin-2-yl]pyrazol-3-yl]phenyl]methyleneamino]-3-(2-isopropyl-5-methyl-phenyl)thiourea (C-7):
   To a stirred solution of 4-[1,4-dimethyl-5-[1-oxo-6-(trifluoromethoxy)-3,4-dihydroisoquinolin-2-yl]pyrazol-3-yl]benzaldehyde (0.350 g, 0.815 mmol) in acetic acid (4 mL) was added 1-amino-3-(2-isopropylphenyl)thiourea (0.182 g, 0.815 mmol) at 0 °C. The reaction mass was stirred for 3 hours and the progress of the reaction was monitored by TLC analysis. After completion of reaction, reaction mixture was quenched in water (15 mL). The precipitated product was filtered through a filter paper and dried under reduced pressure to get the title compound as a solid 0.470 g (86.03 % yield). LC/MS (Method 1): Rt: 2.34 min; m / z = 635 (M+1)+; ¹H NMR (300 MHz, DMSO-d6) δ 11.69 (s, 1H), 9.88 (s, 1H), 8.10 (s, 1H), 8.02 (d, J = 8.6 Hz, 1H), 7.89 (d, J = 8.1 Hz, 2H), 7.65 (d, J = 8.1 Hz, 2H), 7.43 (s, 1H), 7.35 (d, J = 8.7 Hz, 1H), 7.16 (d, J = 7.9 Hz, 1H), 7.04 (d, J = 8.0 Hz, 1H), 6.96 (s, 1H), 3.84 (t, J = 6.4 Hz, 2H), 3.65 (s, 3H), 3.21 (t, J = 7.2 Hz, 2H), 3.02 (p, J = 6.9 Hz, 1H), 2.22 (s, 3H), 2.05 (s, 3H), 1.10 (d, J = 6.8 Hz, 6H).

### Example C-8:

Synthesis of (2Z)-2-[(E)-[4-[1,4-dimethyl-5-[1-oxo-6-(trifluoromethoxy)-3,4-dihydroisoquinolin-2-yl]pyrazol-3-yl]phenyl]methylenehydrazono]-3-(2-isopropyl-5-methyl-phenyl)thiazolidin-4-one:
Step 1: Synthesis of (2Z)-2-[(E)-[4-[1,4-dimethyl-5-[1-oxo-6-(trifluoromethoxy)-3,4-dihydroisoquinolin-2-yl]pyrazol-3-yl]phenyl]methylenehydrazono]-3-(2-isopropyl-5-methyl-phenyl)thiazolidin-4-one (C-8):
To a stirred solution of 1-[(E)-[4-[1,4-dimethyl-5-[1-oxo-6-(trifluoromethoxy)-3,4-dihydroisoquinolin-2-yl]pyrazol-3-yl]phenyl]methyleneamino]-3-(2-isopropyl-5-methyl-phenyl)thiourea (0.250 g, 0.393 mmol) in ethanol (5.0 mL) were added sodium acetate (0.065 g, 0.787 mmol) and methyl bromo acetate (0.090 g, 0.590 mmol) at ambient temperature. Then reaction mass was stirred at ambient temperature for 12 hours and monitored by TLC analysis. After completion of reaction, reaction mass was diluted with water (20 mL) and followed by extracted in EtOAc (20 mLx 2). The combined organic extracts were dried over sodium sulphate, filtered, and concentrated under reduced pressure. The crude product was purified by preparative HPLC to afford title compound as a solid 0.160 g (60.01 % yield). LC/MS (Method 1): Rt: 2.38 min; m / z = 675 (M+1)+; ¹H NMR (300 MHz, DMSO-d6) δ 8.36 (s, 1H), 8.10 (d, J = 8.6 Hz, 1H), 7.86 - 7.72 (m, 4H), 7.50 (s, 1H), 7.46 - 7.35 (m, 2H), 7.29 (dd, J = 8.4, 1.8 Hz, 1H), 7.11 - 7.04 (m, 1H), 4.25 (d, J = 17.4 Hz, 1H), 4.13 (d, J = 17.3 Hz, 1H), 3.92 (t, J = 6.5 Hz, 2H), 3.73 (s, 3H), 3.22 (s, 2H), 2.75 (p, J = 6.8 Hz, 1H), 2.32 (s, 3H), 2.12 (s, 3H), 1.13 (dd, J = 9.3, 6.8 Hz, 6H).

### Example C-9:

Synthesis of (2Z)-3-(2-isopropyl-5-methyl-phenyl)-2-[(E)-[4-[1-methyl-5-[1-oxo-6-(trifluoromethoxy)-3,4-dihydroisoquinolin-2-yl]-1,2,4-triazol-3-yl]phenyl]methylenehydrazono] thiazolidin-4-one:
Step 1: Synthesis of 3,5-dibromo-1-methyl-1,2,4-triazole:
   To a stirred solution of 3,5-dibromo-1H-1,2,4-triazole (300 g, 1322 mmol) in 1,2 dichloroethane (1500 mL) mixture were added 2.0 M sodium hydroxide aqueous solution (750 mL,1500 mmol), tetrabutylammonium hydrogen sulfate (44.8 g, 132.2 mmol) and dimethyl sulphate (175.13 g, 1388.5 mmol), at ambient temperature. The whole reaction mixture was stirred at 27 °C for 5 hours. The progress of the reaction was monitored by HPLC analysis. The reaction mixture was diluted with water (500 mL) and extracted with DCM (500 mL × 2). The combined organic layers were washed with saturated NaHCO3 solution (1000 mL) dried over sodium sulfate and concentrated under reduced pressure to get the crude product which was purified by column chromatography using EtOAc and heptane as eluent to afford the desired product as beige solid (298 g, 93%). GCMS (Method 1): Rt: 4.68 min; m / z = 241 (M).
Step 2: 5-bromo-N-[(4-methoxyphenyl)methyl]-2-methyl-1,2,4-triazol-3-amine:

   To a stirred solution of 3,5-dibromo-1-methyl-1,2,4-triazole (300 g, 1245.4 mmol) in N-Methylpyrrolidone (3000 mL), were added 4-methoxy benzylamine (179.3 g, 1307.7mmol) and potassium carbonate (258.1 g, 1868.2 mmol) at ambient temperature. The whole reaction mixture was stirred at 120 °C for 16 hours. The progress of the reaction was monitored by HPLC and LCMS. After the reaction was completed, the reaction mixture was added drop wise into water (10000 mL) under stirring over 45 min. White precipitate was formed which was filtered and dried under reduced pressure to obtain the desired product as white solid (285 g, 77%). LC/MS: Rt: 1.82 min; m / z = 301 (M+2) +.
Step 3: Synthesis of 4-[5-[(4-methoxyphenyl) methylamino]-1-methyl-1,2,4-triazol-3-yl] benzaldehyde:
   Solution of 5-bromo-N-[(4-methoxyphenyl)methyl]-2-methyl-1,2,4-triazol-3-amine (300.0 g, 1009.6 mmol) in 1,4-Dioxane (2500 mL) and water (500 mL) was degassed with Nitrogen gas for 30 min. To the stirred and degassed solution were followed added 4-formylphenyl boronic acid (158.9 g, 1060.1 mmol), cesium carbonate (657.89 g, 2019.2 mmol), [1,1'-Bis(diphenylphosphino)ferrocene]dichloropalladium(II) (14.7 g, 20.192 mmol) at ambient temperature. Reaction mass was heated for 6 hours at 90 °C. The progress of the reaction was monitored by HPLC and LCMS. After the completion of reaction, the reaction mixture was filtered through a celite pad and washed with EtOAc (1000 mL). The combined organic layers were washed with saturated sodium bicarbonate solution (1000 mL), dried over sodium sulfate, and concentrated under reduced pressure to get the crude product (brown solid). Crude solid was treated with methanol (300 mL × 2) and filtered to afford the desired product as pale-yellow solid (295 g,77 %). HPLC/MS (Method 1): Rt: 1.91 min; m / z = 323.2 (M+1) +.
Step 4: Synthesis of 4-(5-amino-1-methyl-1,2,4-triazol-3-yl) benzaldehyde:
   To a stirred solution of 4-[5-[(4-methoxyphenyl) methylamino]-1-methyl-1,2,4-triazol-3-yl] benzaldehyde (50 g, 155.1 mmol) in 1,2 dichloromethane (500 mL) was added triflic acid (23.27 g, 155.1 mmol) at 0 °C. The reaction mixture was stirred at 27 °C for 3 hours. The progress of the reaction was monitored by LCMS and HPLC. After completion of reaction, the reaction mixture was dosed into an aqueous potassium carbonate solution (1.5 eq, 500 mL) over 30 min. White precipitate was formed which was filtered and dried under reduced pressure to obtain the desired product as white solid (28.2 g, 90%). ¹H NMR (500 MHz, DMSO) δ 10.01 (s, 1H), 8.09 - 8.04 (m, 2H), 7.96 - 7.91 (m, 2H), 6.41 (s, 2H), 3.62 (s, 3H).: Rt: 1.62 min; m / z = 203.1 (M+1)⁺.
Step 5: Synthesis of (2Z)-2-[(E)-[4-(5-amino-1-methyl-1,2,4-triazol-3-yl) phenyl] methylene-hydrazono]-3-(2-isopropyl-5-methyl-phenyl) thiazolidin-4-one:
   To a stirred solution of 4-(5-amino-1-methyl-1,2,4-triazol-3-yl) benzaldehyde (4.0 g, 19.78 mmol) in DMF (40 mL) was added 1-amino-3-(2-isopropyl-5-methyl-phenyl) thiourea (4.6 g, 20.77 mmol). The solution was stirred at 27 °C for a period of 3 hours. Upon disappearance of the starting material as judged by HPLC and LCMS, sodium acetate (2.43 g, 29.62 mmol) followed by methyl bromoacetate (3.32 g, 21.76 mmol) were added. The reaction mixture was heated at 60 °C for 16 hours. Upon disappearance of starting material as judged by HPLC and LCMS, the reaction mixture was added dropwise to water (750 mL) and stirred for 15 min. White precipitate was formed which was filtered and dried under reduced pressure to obtain the desired product as white solid compound (7.1 g, 80% yield). ¹H NMR (500 MHz, DMSO) δ 8.33 (s, 1H), 7.95 - 7.89 (m, 2H), 7.78 - 7.73 (m, 2H), 7.38 (d, J = 8.0 Hz, 1H), 7.28 (dd, J = 8.2, 1.9 Hz, 1H), 7.09 - 7.05 (m, 1H), 6.35 (s, 2H), 4.24 (d, J = 17.3 Hz, 1H), 4.13 (d, J = 17.3 Hz, 1H), 3.59 (s, 3H), 2.73 (q, J = 6.5 Hz, 1H), 2.32 (s, 3H), 1.12 (dd, J = 13.1, 6.8 Hz, 6H).. HPLC/MS (Method 1): Rt: 1.99 min; m / z = 448.3 (M+1)⁺.
Step 6: Synthesis of N-[5-[4-[(E)-[(Z)-[3-(2-isopropyl-5-methyl-phenyl)-4-oxo-thiazolidin-2-ylidene]hydrazono]methyl]phenyl]-2-methyl-1,2,4-triazol-3-yl]-4-(trifluoromethoxy)-2-vinylbenzamide:
   To a stirred solution of (2Z)-2-[(E)-[4-(5-amino-1-methyl-1,2,4-triazol-3-yl)phenyl] methylene-hydrazono]-3-(2-isopropyl-5-methyl-phenyl)thiazolidin-4-one (0.3 g, 0.67 mmol) and 4-(trifluo-romethoxy)-2-vinyl-benzoic acid (0.187 g, 0.80 mmol) in pyridine (3 mL) at 0 °C was added phosphorus oxychloride (0.125 ml, 1.34 mmol). The reaction mixture was stirred at 27°C for 15 min. The progress of the reaction was monitored by TLC. After completion of reaction, the reaction mixture was quenched with methanol (0.5 mL) and concentrated under reduced pressure to get the crude product which was purified by column chromatography using EtOAc and heptane as eluent. Product obtained was dissolved in methanol (10 ml) and potassium carbonate (0.278 g, 2.01 mmol) was added and stirred for5 min. The reaction mixture was diluted with water (50 mL) and was extracted with EtOAc (50 mL × 2). The combined organic layers were dried over sodium sulfate and concentrated under reduced pressure to get the crude product which was purified by column chromatography using EtOAc and heptane as eluent to afford the desired product as beige solid (0.25 g, 53%). HPLC/MS (Method 1): Rt: 2.29 min; m / z = 660 (M+1)⁺.
Step 7: Synthesis of (2Z)-3-(2-isopropyl-5-methyl-phenyl)-2-[(E)-[4-[1-methyl-5-[1-oxo-6-(trifluoromethoxy)-3,4-dihydroisoquinolin-2-yl]-1,2,4-triazol-3-yl]phenyl]methylenehydrazono] thiazolidin-4-one (C-9):
   To a stirred solution of N-[5-[4-[(E)-[(Z)-[3-(2-isopropyl-5-methyl-phenyl)-4-oxo-thiazolidin-2-ylidene]hydrazono]methyl]phenyl]-2-methyl-1,2,4-triazol-3-yl]-4-(trifluoromethoxy)-2-vinylbenzamide (0.2 g, 0.3 mmol) in dry DMF (5 mL) was added potassium tert-butoxide (0.027 g, 0.24 mmol) at ambient temperature. The reaction mixture was heated at 100 °C for 48 hours. The progress of the reaction was monitored by TLC. The reaction mixture was diluted with water (50 mL) and extracted with EtOAc (50 mL × 2). The combined organic layers were dried over sodium sulfate and concentrated under reduced pressure to get the crude product which was purified by column chromatography using EtOAc and heptane as eluent to afford the desired product as beige solid (0.065 g, 29%). HPLC/MS (Method 1): Rt: 2.37 min; m / z = 662.3 (M+1)⁺. ¹H NMR (300 MHz, DMSO-d6) δ 8.37 (s, 1H), 8.12 (d, J = 8.6 Hz, 1H), 8.04 (d, J = 8.1 Hz, 2H), 7.84 (d, J = 8.3 Hz, 2H), 7.52 (d, J = 2.5 Hz, 1H), 7.47 - 7.35 (m, 2H), 7.28 (d, J = 8.1 Hz, 1H), 7.08 (d, J = 1.7 Hz, 1H), 4.30 - 4.04 (m, 4H), 3.77 (s, 3H), 3.27 (m, 2H), 2.74 (t, J = 6.8 Hz, 1H), 2.32 (s, 3H), 1.19 - 1.05 (m, 6H).

Example C-11:

### Synthesis of (3Z)-1-[4-[1,4-dimethyl-5-[1-oxo-6-(trifluoromethoxy)-3,4-dihydroisoquinolin-2-yl]pyrazol-3-yl] phenyl]-3-[3-(2-isopropyl-5-methyl-phenyl)-4-oxo-thiazolidin-2-ylidene]urea

Step 1: Synthesis of 2-[5-(4-aminophenyl)-2,4-dimethyl-pyrazol-3-yl]-6-(trifluoromethoxy)-3,4-dihydroisoquinolin-1-one:
   A solution of 2-[5-(4-bromophenyl)-2,4-dimethyl-pyrazol-3-yl]-6-(trifluoromethoxy)-3,4-dihydroisoquinolin-1-one (7.1 g, 14.78 mmol) and boc-amide (2.59 g, 22.17 mmol) in 1,4-dioxane (80 mL) was degassed with Nitrogen gas for 30 min. To the strirred and degassed solution were added potassium tert-butoxide (3.31 g, 29.56 mmol), Tris(dibenzylideneacetone)dipalladium (0.677 g, 0.73 mmol), 5-(Di-tert-butylphosphino)-1', 3', 5'- triphenyl-1'H-[1,4']bipyrazole (0.749 g, 1.47 mmol) at ambient temperature. Reaction mass was heated for 12 hours at 90 °C. The progress of the reaction was monitored by TLC and LCMS. After the reaction was complete, the reaction mixture was filtered through a celite pad and washed with EtOAc (100 mL). Filtrate was concentrated to its one-third volume and extracted with EtOAc (2 × 100 mL). Organic layers were dried over sodium sulphate and evaporated under reduced pressure to obtain the crude product as off-white solid (7.1 g). To this crude compound was added dichloromethane (70 mL) followed by dropwise addition of 4.0 M HCl in 1,4 dioxane (10.23 mL, 40.94 mmol) at 10°C. Reaction mass was stirred for 12 hours at ambient temperature. The progress of the reaction was monitored by TLC and LCMS. After the completion of the reaction, it was added to water (100 mL) dropwise under stirring, extracted with EtOAc (2 × 50 mL). Aqueous layer was basified with saturated sodium carbonate solution (100 mL) followed by extraction with EtOAc (3 × 100 mL). Combined organic layers were dried over sodium sulphate and evaporated under reduced pressure to obtain the crude product as off-white solid (5.2 g). The crude product was purified by column chromatography using EtOAc and heptane as eluent to afford the desired product as off-white solid (4.6 g, 71.2 %). HPLC/MS (Method 1): Rt: 1.93 min; m / z = 417(M+1)⁺ .
Step 2: Synthesis of (3Z)-1-[4-[1,4-dimethyl-5-[1-oxo-6-(trifluoromethoxy)-3,4-dihydroisoquinolin-2-yl]pyrazol-3-yl] phenyl]-3-[3-(2-isopropyl-5-methyl-phenyl)-4-oxo-thiazolidin-2-ylidene]urea (C-11):
   To a solution of 2-[5-(4-aminophenyl)-2,4-dimethyl-pyrazol-3-yl]-6-(trifluoromethoxy)-3,4-dihydroisoquinolin-1-one (0.15 g, 0.360 mmol) in dry acetonitrile (5 mL) was added pyridine (0.116 mL, 1.44 mmol) at ambient temperature. The reaction mixture was cooled to 0 °C and N, N'-disuccinimidyl carbonate (0.097 g, 0.378 mmol) was added at 0 °C. The reaction mixture was allowed to stir at ambient temperature for 3 hours. The progress of the reaction was monitored by TLC. Pyridine (0.116 mL, 1.44 mmol) followed by 2-imino-3-(2-isopropyl-5-methylphenyl) thiazolidin-4-one (0.089 g, 0.360 mmol) was added to the reaction mixture and stirred at 60 °C for 3 hours. The progress of the reaction was monitored by TLC. The reaction mixture was diluted with water (30 mL) and extracted with EtOAc (50 mL × 2). The combined organic layers were dried over sodium sulfate and concentrated under reduced pressure to get the crude product which was purified by column chromatography using EtOAc and heptane as eluent to afford the desired product as beige solid (0.175 g). HPLC/MS (Method 1): Rt: 2.24 min; m / z = 691 (M+1)⁺. ¹H NMR (500 MHz, DMSO-d6) δ 9.86 (s, 1H), 8.09 (d, J = 8.6 Hz, 1H), 7.76 - 7.67 (m, 2H), 7.56 (d, J = 8.8 Hz, 2H), 7.50 (s, 1H), 7.41 (t, J = 7.5 Hz, 2H), 7.28 (d, J = 8.0 Hz, 1H), 7.07 (s, 1H), 4.20 (d, J = 17.7 Hz, 1H), 4.08 (d, J = 18.1 Hz, 1H), 3.89 (t, J = 6.5 Hz, 2H), 3.68 (s, 3H), 3.33 - 3.23 (m, 2H), 2.72 - 2.63 (m, 1H), 2.32 (s, 3H), 2.05 (s, 3H), 1.18 (d, J = 6.9 Hz, 3H),, 1.10 (d, J = 6.8 Hz, 3H).

### Example C-12:

Synthesis of (3Z)-1-[2-chloro-4-[1,4-dimethyl-5-[1-oxo-6-(trifluoromethoxy)-3,4-dihydroisoquinolin-2-yl] pyrazol-3-yl] phenyl]-3-[3-(2-isopropyl-5-methyl-phenyl)-4-oxo-thiazolidin-2-ylidene] urea:
Step 1: Synthesis of 2-[5-(4-amino-3-chloro-phenyl)-2,4-dimethyl-pyrazol-3-yl]-6-(trifluoromethoxy)-3,4-dihydroisoquinolin-1-one:
   To a stirred solution of 2-[5-(4-aminophenyl)-2,4-dimethyl-pyrazol-3-yl]-6-(trifluoromethoxy)-3,4-dihydroisoquinolin-1-one (0.25 g, 0.600 mmol) in acetonitrile (10 mL) was added N-chlorosuccinimide (0.080 g, 0.600 mmol) at ambient temperature. The reaction mixture was heated at 70 °C for 7 hours. The progress of the reaction was monitored by TLC. The reaction mixture was diluted with saturated sodium bicarbonate solution (20 mL) and extracted with EtOAc (50 mL × 2). The combined organic layers were dried over sodium sulfate and concentrated under reduced pressure to get the crude product which was purified by column chromatography using EtOAc and heptane as eluent to afford the desired product as beige solid (0.22 g). HPLC/MS (Method 1): Rt: 2.10 min; m / z = 451 (M+1)+. ¹H NMR (500 MHz, DMSO-d6) δ 8.01 (d, J = 8.6 Hz, 1H), 7.43 (s, 1H), 7.39 (d, J = 2.0 Hz, 1H), 7.34 (dd, J = 8.6, 1.5 Hz, 1H), 7.27 (dd, J = 8.4, 2.0 Hz, 1H), 6.78 (d, J = 8.4 Hz, 1H), 5.39 (s, 2H), 3.81 (t, J = 6.5 Hz, 2H), 3.59 (s, 3H), 3.25 - 3.13 (m, 2H), 1.96 (s, 3H).
Step 2: Synthesis of (3Z)-1-[2-chloro-4-[1,4-dimethyl-5-[1-oxo-6-(trifluoromethoxy)-3,4-dihydroisoquinolin-2-yl] pyrazol-3-yl] phenyl]-3-[3-(2-isopropyl-5-methyl-phenyl)-4-oxo-thiazolidin-2-ylidene] urea (C-12):
   To a solution of 2-[5-(4-amino-3-chloro-phenyl)-2,4-dimethyl-pyrazol-3-yl]-6-(trifluoromethoxy)-3,4-dihydroisoquinolin-1-one (0.220 g, 0.488 mmol) in dry acetonitrile (10 mL) was added pyridine (0.158 mL, 1.951 mmol) at ambient temperature. The reaction mixture was cooled to 0 °C and N, N'-disuccinimidyl carbonate (0.131 g, 0.512 mmol) was added. The reaction mixture was allowed to stir at ambient temperature for 3 hours. The progress of the reaction was monitored by TLC. Pyridine (0.158 mL, 1.951 mmol) followed by 2-imino-3-(2-isopropyl-5-methyl-phenyl) thiazolidin-4-one (0.122 g, 0.488 mmol) was added to the reaction mixture and stirred at 60 °C for 3 hours. The progress of the reaction was monitored by TLC. The reaction mixture was diluted with water (30 mL) and was extracted with EtOAc (30 mL × 2). The combined organic layers were dried over sodium sulfate and concentrated under reduced pressure to get the crude product which was purified by column chromatography using EtOAc and heptane as eluent to afford the desired product as beige solid (0.175 g). HPLC/MS (Method 1): Rt: 2.36 min; m / z = 725 (M+1)⁺. ¹H NMR (500 MHz, DMSO-d6) δ 9.00 (s, 1H), 8.02 (d, J = 8.6 Hz, 1H), 7.68 - 7.48 (m, 3H), 7.43 (s, 1H), 7.38 - 7.30 (m, 2H), 7.21 (d, J = 8.0 Hz, 1H), 7.00 (s, 1H), 4.14 (d, J = 18.0 Hz, 1H), 4.02 (d, J = 18.0 Hz, 1H), 3.84 (t, J = 6.5 Hz, 2H), 3.64 (s, 3H), 3.24-3.17 (m, 2H), 2.66 - 2.56 (m, 1H), 2.25 (s, 3H), 2.02 (s, 3H), 1.12 (d, J = 6.3 Hz, 3H), 1.03 (d, J = 6.8 Hz, 3H).

All other examples enlisted in the table C are synthesized analogous to the methods mentioned in either general procedure or experimental procedure mentioned above.

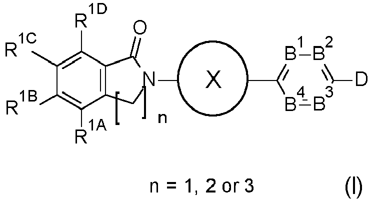

**Table C:**

| **No** | 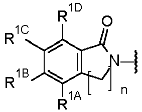 | 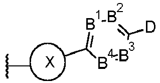 | **D** | **HPLC/MS** | **Rt min** |
|---|---|---|---|---|---|
| C1 | 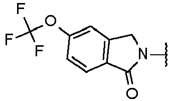 | 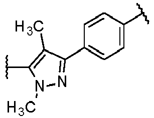 | 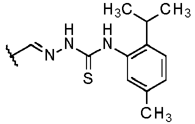 | 621 (Method 2) | 1.41 |
| C2 | 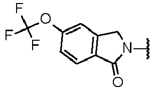 | 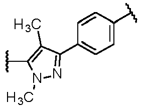 | 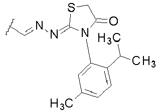 | 661 (Method 2) | 1.44 |
| C3 | 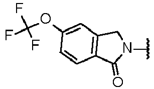 | 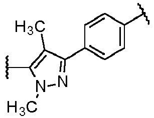 | 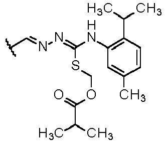 | - | - |
| C4 | 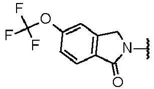 | 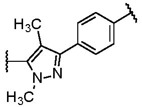 | 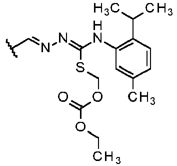 | - | - |
| C5 | 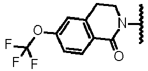 | 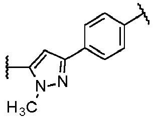 | 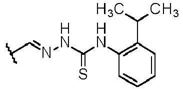 | 607 (Method 1) | 2.12 |
| **No** | 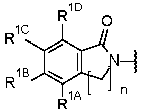 | 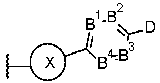 | **D** | **HPLC/MS** | **Rt min** |
| C6 | 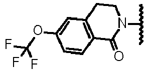 | 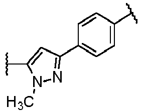 | 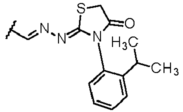 | 647 (Method 1) | 2.16 |
| C7 | 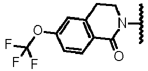 | 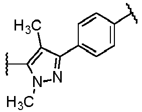 | 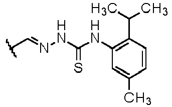 | 635 (Method 1) | 2.34 |
| C8 | 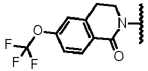 | 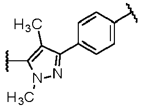 | 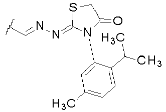 | 675 (Method 1) | 2.38 |
| C9 | 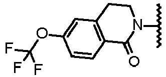 | 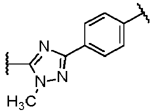 | 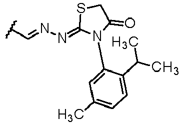 | 662 (Method 1) | 2.37 |
| C10 | 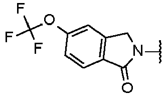 | 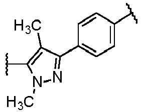 | 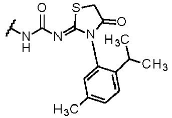 | 677 (Method 2) | 1.36 |
| C11 | 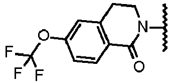 | 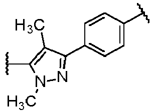 | 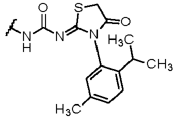 | 691 (Method 1) | 2.24 |
| C12 | 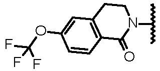 | 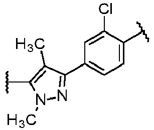 | 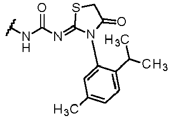 | 725 (Method 1) | 2.36 |
| C13 | 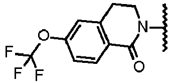 | 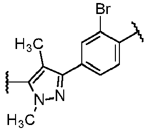 | 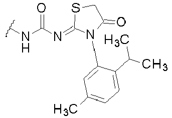 | 771 (Method 1) | 2.37 |
| C14 | 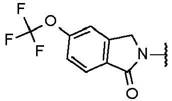 | 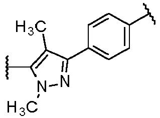 | 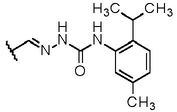 | 605 (Method 2) | 1.37 |

### Biological examples:

### Example B1: Action on Yellow fever mosquito (Aedes aegypti)

For evaluating control of yellow fever mosquito (*Aedes aegypti*) the test unit consisted of 96-well-microtiter plates containing 200µL of tap water per well and 5-15 freshly hatched A. *aegypti* larvae.

The active compounds or mixtures were formulated using a solution containing 75% (v/v) water and 25% (v/v) DMSO. Different concentrations of formulated compounds or mixtures were sprayed onto the insect diet at 2.5µL, using a custom-built micro atomizer, at two replications.

For experimental mixtures in these tests identical volumes of both mixing partners at the desired concentrations respectively, were mixed together.

After application, microtiter plates were incubated at 28 ± 1°C, 80 ± 5 % relative humidity for 2 days. Larval mortality was then visually assessed.

In this test, compounds C-1, C-2, C-3, C-4, C-5, C-7, C-8, C-9, C-10, C-11, C-12, C-13 and C-14 at 800 ppm showed at least 50% mortality in comparison with untreated controls.

### Example B2: Action on Orchid thrips (Dichromothrips corbetti)

Dichromothrips corbetti adults used for bioassay were obtained from a colony maintained continuously under laboratory conditions. For testing purposes, the test compound is diluted in a 1:1 mixture of acetone:water (vol:vol), plus Kinetic^{®} HV at a rate of 0.01% v/v.

Thrips potency of each compound was evaluated by using a floral-immersion technique. All petals of individual, intact orchid flowers were dipped into treatment solution and allowed to dryin Petri dishes. Treated petals were placed into individual re-sealable plastic along with about 20 adult thrips. All test arenas were held under continuous light and a temperature of about 28°C for duration of the assay. After 3 days, the numbers of live thrips were counted on each petal. The percent mortality was recorded 72 hours after treatment.

In this test, compounds C-1, C-2, C-3, C-4, C-5, C-6, C-7, C-8, C-9, C-11, C-12, C-13 and C-14 at 300 ppm showed at least 75% mortality in comparison with untreated controls.

### Example B3: Action on Boll weevil (Anthonomus grandis )

For evaluating control of boll weevil (Anthonomus grandis) the test unit consisted of 96-well-microtiter plates containing an insect diet and 5-10 A. grandis eggs.

The compounds were formulated using a solution containing 75% (v/v) water and 25% (v/v) DMSO. Different concentrations of formulated compounds were sprayed onto the insect diet at 5 µL, using a custom-built micro atomizer, at two replications.

After application, microtiter plates were incubated at about 25 + 1°C and about 75 + 5 % relative humidity for 5 days. Egg and larval mortality were then visually assessed.

In this test, compounds C-1, C-2, C-3, C-4, C-5, C-7, C-8, C-9, C-10, C-11, C-12, C-13 and C-14 at 800 ppm showed at least 75 % mortality in comparison with untreated controls.

### Example B4: Action on Silverleaf whitefly (Bemisia argentifolii) (adults)

The active compounds were formulated by a Tecan liquid handler in 100% cyclohexanone as a 10,000 ppm solution supplied in tubes. The 10,000 ppm solution was serially diluted in 100% cyclohexanone to make interim solutions. These served as stock solutions for which final dilutions were made by the Tecan in 50% acetone:50% water (v/v) into 5 or 10ml glass vials. A nonionic surfactant (Kinetic^{®}) was included in the solution at a volume of 0.01% (v/v). The vials were then inserted into an automated electrostatic sprayer equipped with an atomizing nozzle for application to plants/insects.

Cotton plants at the cotyledon stage (one plant per pot) were sprayed by an automated electrostatic plant sprayer equipped with an atomizing spray nozzle. The plants were dried in the sprayer fume hood and then removed from the sprayer. Each pot was placed into a plastic cup and about 10 to 12 whitefly adults (approximately 3-5 days old) were introduced. The insects were collected using an aspirator and a nontoxic Tygon^{®} tubing connected to a barrier pipette tip. The tip, containing the collected insects, was then gently inserted into the soil containing the treated plant, allowing insects to crawl out of the tip to reach the foliage for feeding. Cups were covered with a reusable screened lid. Test plants were maintained in a growth room at about 25°C and about 20-40% relative humidity for 3 days, avoiding direct exposure to fluorescent light (24 hour photoperiod) to prevent trapping of heat inside the cup. Mortality was assessed 3 days after treatment, compared to untreated control plants.

In this test, compound C-2, C-5 at 300 ppm showed at least 75 % mortality in comparison with untreated controls.

### Example B5: Action on Tobacco budworm (Heliothis virescens)

For evaluating control of tobacco budworm (*Heliothis virescens*) the test unit consisted of 96-well-microtiter plates containing an insect diet and 15-25 H. virescens eggs.

The compounds were formulated using a solution containing 75% v/v water and 25% v/v DMSO. Different concentrations of formulated compounds were sprayed onto the insect diet at 10 µl, using a custom-built micro atomizer, at two replications.

After application, microtiter plates were incubated at about 28 + 1°C and about 80 + 5 % relative humidity for 5 days. Egg and larval mortality were then visually assessed.

In this test, compounds C-1, C-2, C-3, C-4, C-5, C-7, C-8, C-9, C-10, C-11, C-12, C-13 and C-14, at 800 ppm showed at least 75 % mortality in comparison with untreated controls.

### Example B6: Action on Diamond back moth (Plutella xylostella)

The active compound is dissolved at the desired concentration in a mixture of 1:1 (v/v) distilled water: acetone. Surfactant (Kinetic^{®} HV) is added at a rate of 0.01% (v/v). The test solution is prepared at the day of use.

Leaves of cabbage were dipped in test solution and air-dried. Treated leaves were placed in petri dishes lined with moist filter paper and inoculated with ten third instar larvae. Mortality was recorded 72 hours after treatment. Feeding damages were also recorded using a scale of 0-100%.

In this test, compounds C-1, C-2, C-5, C-6, C-8, C-11, C-12, C-13 and C-14 at 300 ppm showed at least 75 % mortality in comparison with untreated controls.

### Example B7: Action on Southern armyworm (Spodoptera eridania), second instar larvae

The active compounds were formulated by a Tecan liquid handler in 100% cyclohexanone as a 10,000 ppm solution supplied in tubes. The 10,000 ppm solution was serially diluted in 100% cyclohexanone to make interim solutions. These served as stock solutions for which final dilutions were made by the Tecan in 50% acetone:50% water (v/v) into 10 or 20 mL glass vials. A nonionic surfactant (Kinetic^{®}) was included in the solution at a volume of 0.01% (v/v). The vials were then inserted into an automated electrostatic sprayer equipped with an atomizing nozzle for application to plants/insects.

Lima bean plants (variety Sieva) were grown 2 plants to a pot and selected for treatment at the 1st true leaf stage. Test solutions were sprayed onto the foliage by an automated electrostatic plant sprayer equipped with an atomizing spray nozzle. The plants were dried in the sprayer fume hood and then removed from the sprayer. Each pot was placed into perforated plastic bags with a zip closure. About 10 to 11 armyworm larvae were placed into the bag and the bags
zipped closed. Test plants were maintained in a growth room at about 25°C and about 20-40% relative humidity for 4 days, avoiding direct exposure to fluorescent light (24 hour photoperiod) to prevent trapping of heat inside the bags. Mortality and reduced feeding were assessed 4 days after treatment, compared to untreated control plants.

In this test, compounds C-1, C-2, C-3, C-4, C-5, C-6, C-7, C-8, C-9, C-10, C-11, C-12, C-13 and C-14 at 300 ppm showed at least 75 % mortality in comparison with untreated controls.

### Example B8: Action on Diamond back moth (Plutella xylostella)

For evaluating control of diamond back moth (*Plutella xylostella*) the test unit consisted of 96-well-microtiter plates containing an insect diet and 15-25 *P*. *xylostella* eggs. The compounds or mixtures were formulated using a solution containing 75% water and 25% DMSO. Different concentrations of formulated compounds or mixtures were sprayed onto the insect diet at 5µl, using a custom-built micro atomizer, at two replications. For experimental mixtures in these tests identical volumes of both mixing partners at the desired concentrations respectively, were mixed together. After application, microtiter plates were incubated at 28 ± 1°C, 80 ± 5 % relative humidity for 5 days. Egg and larval mortality was then visually assessed.

In this test, compounds C-3, C-4, C-7, C-9 and C-10 at 800 ppm showed at least 75 % mortality in comparison with untreated controls.

## Claims

1. Compounds of the formula I 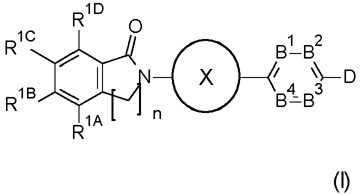 wherein
R^{1A}, R^{1B}, R^{1C} and R^{1D} are, identical or different, H, halogen, SF₅, OH, CN, C₁-C₆-alkyl, C₁-C₆-alkoxy, C₃-C₆-cycloalkyl, C₃-C₆-cycloalkoxy, wherein the alkyl, alkoxy, cycloalkyl, and cycloalkoxy moieties are unsubstituted or substituted with halogen or CN;
NR⁸R⁹, C=O(NR⁸R⁹), S(=O)ₘ(NR⁸R⁹), or NHS(=O)ₘR⁸;
m is 1 or 2;
n is 1, 2 or 3;
X is defined by either one of the following two rings X1 or X2 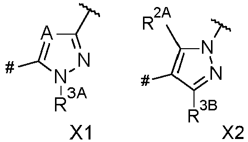
wherein
# denotes connection to the lactam moiety;
A is N or CR^{2A};
R^{2A} is H, C₁-C₆-alkyl, C₃-C₆-cycloalkyl, C₁-C₆-alkoxy, wherein the alkyl, alkoxy and cycloalkyl moieties are unsubstituted or substituted with halogen or CN; halogen, CN, OR⁶, or NR⁶R⁷;
R^{3A} is H, C₁-C₆-alkyl, C₃-C₆-cycloalkyl, C₁-C₆-alkoxy, halogen, or CN wherein the alkyl, alkoxy, and cycloalkyl moieties are unsubstituted or substituted with halogen, CN, or C₁-C₆-alkoxy;
R^{3B} is H, C₁-C₆-alkyl, C₃-C₆-cycloalkyl, C₁-C₆-alkoxy, wherein the alkyl, alkoxy, and cycloalkyl moieties are unsubstituted or substituted with halogen or CN; halogen, CN, OR⁶, or NR⁶R⁷;
R⁶ and R⁷ are, identical or different, H, C₁-C₆-alkyl, C₃-C₆-cycloalkyl, phenyl,-CH₂-phenyl, 5- or 6- membered heteroaryl, -CH₂-5- or 6- membered heteroaryl, 1,3-dioxolan-2-ylmethyl, or 2-(methylamino)-2-oxo-ethyl, wherein the alkyl, cycloalkyl, phenyl and heteroaryl moieties are unsubstituted or substituted with halogen, CN, C₁-C₆-alkyl or C₁-C₆-alkoxy;
B¹ is N or CR^{B1};
B² is N or CR^{B2};
B³ is N or CR^{B3};
B⁴ is CR^{B4};
R^{B1}, R^{B2}, R^{B3}, and R^{B4} are, identical or different, H, halogen, OH, CN, C₁-C₆-alkyl, C₃-C₆-cycloalkyl, or C₁-C₆-alkoxy, wherein the alkyl, alkoxy, and cycloalkyl moieties are unsubstituted or substituted with halogen;
D is the moiety DA, DB, DC, DD, DE, or DF 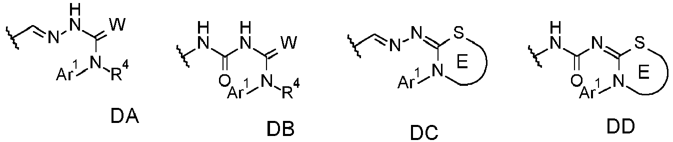 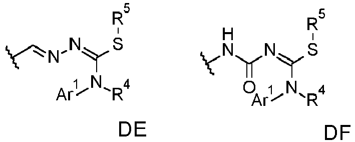
wherein
W isSorO;
R⁴ is H, C₁-C₆-alkyl, or C₃-C₆-cycloalkyl, wherein the alkyl and cycloalkyl moieties are unsubstituted or substituted with halogen or CN;
R⁵ is H, C₁-C₆-alkyl, or C₃-C₆-cycloalkyl, wherein the alkyl and cycloalkyl moieties are unsubstituted or substituted with -O-(C=O)-C₁-C₆-alkoxy, -O-(C=O)-C₁-C₆-alkyl or CN;
E is a 5- or 6-membered carbocyclic group, wherein 1 or 2 CH₂ moieties of the carbocyclic group may be replaced by a carbonyl group, O, or S, wherein the carbocyclic group is unsubstituted or substituted with R¹⁰;
Ar¹ is phenyl or 5- or 6-membered heteroaryl, which are unsubstituted or substituted with R^{Ar1}, wherein
R^{Ar1} is halogen, SF₅, OH, CN, C₁-C₆-alkyl, C₁-C₆-alkoxy, C₃-C₆-cycloalkyl, C₃-C₆-cycloalkoxy, wherein the alkyl, alkoxy, cycloalkyl, and cycloalkoxy moieties are unsubstituted or substituted with halogen or CN; NR⁸R⁹, C=O(NR⁸R⁹), S(=O)ₘ(NR⁸R⁹), NHS(=O)ₘR⁸;
R⁸ and R⁹ are, identical or different, H, C₁-C₆-alkyl, C₃-C₆-cycloalkyl wherein the alkyl, and cycloalkyl moieties are unsubstituted or substituted with halogen;
R¹⁰ is halogen, C₁-C₆-alkyl, or C₁-C₆-alkoxy;
and the N-oxides, stereoisomers, tautomers and agriculturally or veterinarily acceptable salts thereof.

2. Compounds of formula I according to claim 1, wherein B¹ is CR^{B1}, B² is CR^{B2}, and B³ is CR^{B3}.

3. Compounds of formula I according to claim 1 or claim 2, wherein D is DA, DB, DC, DD, or DE.

4. Compounds of formula I according to any one of the preceding claims, wherein R^{1A}, R^{1B}, R^{1C} and R^{1D} independently of each other are selected from H, halogen, CN, C₁-C₆-alkyl, and C₁-C₆-alkoxy, wherein the alkyl and alkoxy moieties are unsubstituted or substituted with halogen, or CN.

5. Compounds of formula I according to any one of the preceding claims, wherein R^{2A} is H, C₁-C₆-alkyl, C₃-C₆-cycloalkyl, or halogen, wherein the alkyl and cycloalkyl moieties are unsubstituted or substituted with halogen or CN.

6. Compounds of formula I according to any one of the preceding claims, wherein n is 1 or 2.

7. Compounds of formula I according to any one of the preceding claims, wherein Ar¹ is phenyl which is unsubstituted or substituted with R^{Ar1}, and wherein
R^{Ar1} is halogen, CN, C₁-C₆-alkyl, C₁-C₆-alkoxy, or C₃-C₆-cycloalkyl, wherein the alkyl, alkoxy and cycloalkyl moieties are unsubstituted or substituted with halogen.

8. Compounds of formula I according to claim 1, wherein
n is 1 or 2;
R^{1A}, R^{1B}, R^{1C} and R^{1D} independently of each other are selected from H, halogen, CN, C₁-C₆-haloalkyl, and C₁-C₆-haloalkoxy;
X is X1 or X2;
A is N or CR^{2A};
R^{2A} is H, halogen, or C₁-C₆-alkyl;
R^{3A} is H or C₁-C₆-alkyl, wherein the alkyl is unsubstituted or substituted with halogen, CN or C₁-C₆-alkoxy;
R^{3B} is H or C₁-C₆-alkyl, preferably CH₃;
B¹ is CR^{B1}, B² is CR^{B2}, and B³ is CR^{B3};
R^{B1}, R^{B2}, R^{B3}, and R^{B4} independently of each other are H, halogen, C₁-C₆-alkyl, or CN;
D is DA, DB, DC, DD, or DE;
W is O or S;
R⁴ is H;
R⁵ is C₁-C₆-alkyl, which is unsubstituted or substituted with -O-(C=O)-C₁-C₆-alkoxy or-O-(C=O)-C₁-C₆-alkyl;
Ar¹ is phenyl which is unsubstituted or substituted with R^{Ar1};
R^{Ar1} is CN, C₁-C₆-alkyl, C₃-C₆-cycloalkyl, NR⁸R⁹ wherein alkyl and cycloalkyl moieties are unsubstituted or substituted with halogen;
R⁸ and R⁹ are, identical or different, H or C₁-C₆-alkyl.

9. A composition, comprising one compound of formula I according to any of claims 1 to 8, an N-oxide or an agriculturally acceptable salt thereof, and a further active substance.

10. A method for combating or controlling invertebrate pests, which method comprises contacting said pest or its food supply, habitat or breeding grounds with a pesticidally effective amount of at least one compound according to any of claims 1 to 8 or the composition according to claim 9.

11. A method for protecting growing plants from attack or infestation by invertebrate pests, which method comprises contacting a plant, or soil or water wherein the plant is growing, with a pesticidally effective amount of at least one compound according to any of claims 1 to 8 or the composition according to claim 9.

12. Seed comprising a compound according to any of claims 1 to 8, or the enantiomers, diastereomers or salts thereof or comprising a composition according to claim 9, in an amount of from 0.1 g to 10 kg per 100 kg of seed.

13. A use of a compound of the formula I according to any of claims 1 to 8, and of an agriculturally acceptable salt thereof or of the compositions according to claim 9, for protecting growing plants from attack or infestation by invertebrate pests.

14. A method for treating or protecting an animal from infestation or infection by invertebrate pests which comprises bringing the animal in contact with a pesticidally effective amount of at least one compound of the formula I according to any of claims 1 to 8, a stereoisomer thereof and/or at least one veterinarily acceptable salt thereof.
